# EUROPEAN PATENT APPLICATION

(11) **EP 4 369 901 A2**
(43) Date of publication of application: **15.05.2024**
(21) Application number: 22837969.9
(22) Date of filing: 06.07.2022
(51) Int. Cl.: H10K 99/00, H10K 50/00

(54) **COMPOSITION FOR ORGANIC OPTOELECTRONIC DIODE, ORGANIC OPTOELECTRONIC DIODE, AND DISPLAY DEVICE**

(30) Priority: 06.07.2021 KR 20210088620
(71) Applicant: Samsung SDI Co., Ltd., Yongin-si, Gyeonggi-do 17084 (KR)
(72) Inventor: CHO, Pyeongseok, Suwon-si, Gyeonggi-do 16678 (KR); KIM, Hyung Sun, Suwon-si, Gyeonggi-do 16678 (KR); LEE, Mijin, Suwon-si, Gyeonggi-do 16678 (KR); LIM, Youngmook, Suwon-si, Gyeonggi-do 16678 (KR); LYU, Seungchul, Suwon-si, Gyeonggi-do 16678 (KR); LUI, Jinhyun, Suwon-si, Gyeonggi-do 16678 (KR); JUNG, Kyunghag, Suwon-si, Gyeonggi-do 16678 (KR); JUNG, Sung-Hyun, Suwon-si, Gyeonggi-do 16678 (KR); JUNG, Ho Kuk, Suwon-si, Gyeonggi-do 16678 (KR); HUH, Dalho, Suwon-si, Gyeonggi-do 16678 (KR)
(74) Representative: Michalski Hüttermann & Partner Patentanwälte mbB
(86) International application number: PCT/KR2022/009751
(87) International publication number: WO 2023/282617

(57) **Abstract**

Provided are a composition for an organic optoelectronic device, and an organic optoelectronic device including the same, and a display device, the composition for an organic optoelectronic device including a first compound represented by Chemical Formula 1, and a second compound represented by a combination of Chemical Formula 2 and Chemical Formula 3.

Details of Chemical Formula 1 to Chemical Formula 3 are as described in the specification.

## Description

### [Technical Field]

A composition for an organic optoelectronic device, an organic photoelectronic device, and a display device are disclosed.

### [Background Art]

An organic optoelectronic device (organic optoelectronic diode) is a device capable of converting electrical energy and optical energy to each other.

Organic optoelectronic devices may be largely divided into two types according to a principle of operation. One is a photoelectric device that generates electrical energy by separating excitons formed by light energy into electrons and holes, and transferring the electrons and holes to different electrodes, respectively and the other is light emitting device that generates light energy from electrical energy by supplying voltage or current to the electrodes.

Examples of the organic optoelectronic device include an organic photoelectric element, an organic light emitting diode, an organic solar cell, and an organic photo conductor drum.

Of these, an organic light emitting diode (OLED) has recently drawn attention due to an increase in demand for flat panel displays. The organic light emitting diode is a device that converts electrical energy into light, and the performance of the organic light emitting diode is greatly influenced by an organic material between electrodes.

### [Disclosure]

An embodiment provides a composition for an organic optoelectronic device capable of realizing an organic optoelectronic device having a low-driving, high-efficiency, and long life-span.

Another embodiment provides an organic optoelectronic device including the composition for an organic optoelectronic device.

Another embodiment provides a display device including the organic optoelectronic device.

According to an embodiment, a composition for an organic optoelectronic device includes a first compound represented by Chemical Formula 1, and a second compound represented by a combination of Chemical Formula 2 and Chemical Formula 3.

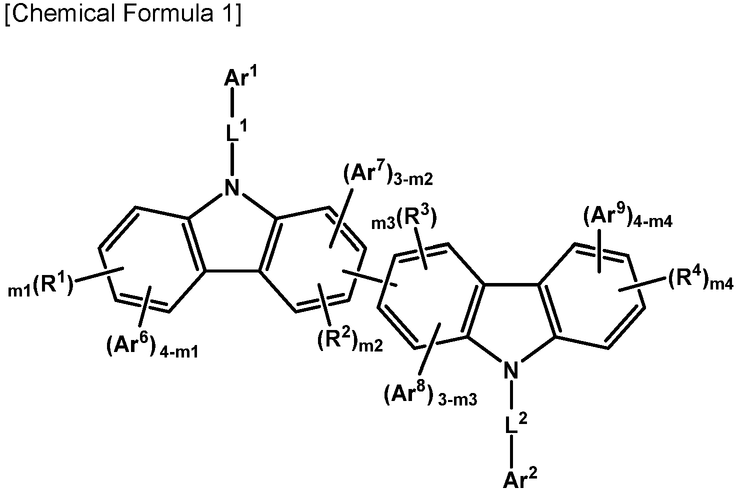

In Chemical Formula 1,
L¹ and L² are each independently a single bond or a substituted or unsubstituted C6 to C30 arylene group,
Ar¹ and Ar² are each independently a substituted or unsubstituted C6 to C30 aryl group or a substituted or unsubstituted C2 to C30 heterocyclic group,
R¹ to R⁴ are each independently hydrogen, deuterium or a substituted or unsubstituted C6 to C30 aryl group,
Ar⁶ to Ar⁹ are each independently hydrogen or a substituted or unsubstituted C6 to C30 aryl group,
m1 and m4 are each independently an integer from 1 to 4,
m2 and m3 are each independently an integer from 1 to 3, and
Chemical Formula 1 simultaneously satisfies (i) and (ii),
   (i) at least one of Ar¹ and Ar² is a C6 to C30 aryl group substituted with at least one deuterium or a C2 to C30 heterocyclic group substituted with at least one deuterium, and
   (ii) at least one of R¹ to R⁴ is deuterium; 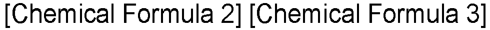 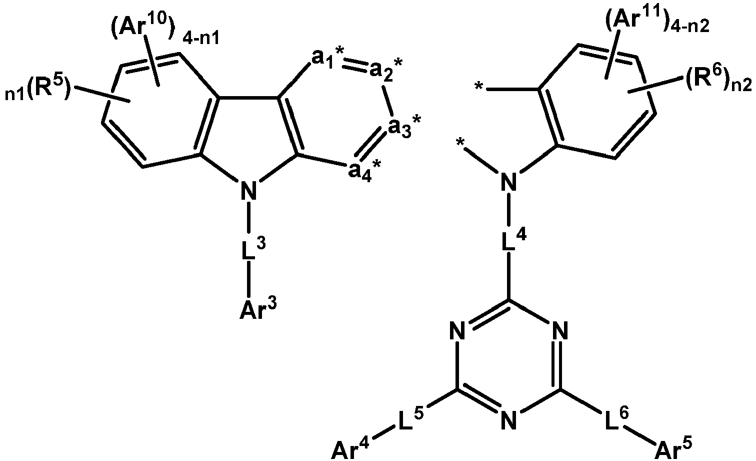
      wherein, in Chemical Formula 2 and Chemical Formula 3,
      Ar³ to Ar⁵ are each independently a substituted or unsubstituted C6 to C20 aryl group or a substituted or unsubstituted C2 to C30 heterocyclic group,
      the two adjacent ones among a₁* to a₄* in Chemical Formula 2 are linking carbons each linked to * in Chemical Formula 3,
      the remaining two of a₁* to a₄* in Chemical Formula 2 that are not linked to * in Chemical Formula 3 are CR^{a},
      L³ to L⁶ are each independently a single bond, a substituted or unsubstituted C6 to C20 arylene group or a substituted or unsubstituted C2 to C20 heteroarylene group,
      R^{a}, R⁵, and R⁶ are each independently hydrogen, deuterium, a cyano group, a halogen, a substituted or unsubstituted amino group, a substituted or unsubstituted C1 to C30 alkyl group, a substituted or unsubstituted C6 to C30 aryl group or a substituted or unsubstituted C2 to C30 heterocyclic group,
      Ar¹⁰ and Ar¹¹ are each independently hydrogen or a substituted or unsubstituted C6 to C30 aryl group,
      n1 and n2 are each independently an integer from 1 to 4, and
      Chemical Formula 2 and Chemical Formula 3 satisfy at least one of the following conditions (iii) and (iv):
         (iii) at least one of Ar3 to Ar5 is a C6 to C20 aryl group substituted with at least one deuterium or a C2 to C30 heterocyclic group substituted with at least one deuterium, and
         (iv) at least one of R^{a}, R⁵, and R⁶ is deuterium.

According to another embodiment, an organic optoelectronic device includes an anode and a cathode facing each other, and at least one organic layer between the anode and the cathode, wherein the organic layer includes the composition for an organic optoelectronic device.

According to another embodiment, a display device including the organic optoelectronic device is provided.

Organic optoelectronic devices with low driving, high efficiency, and long life-span can be implemented.

### [Description of the Drawings]

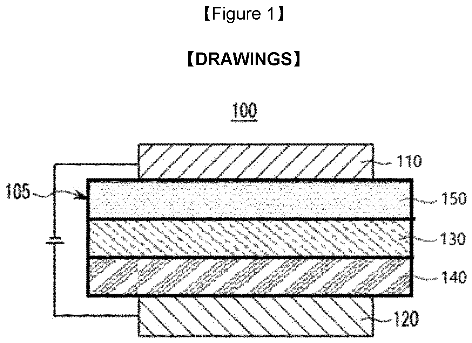

FIG. 1 is cross-sectional view illustrating an organic light emitting diode according to an embodiment.

### [Mode for Invention]

Hereinafter, embodiments of the present invention are described in detail. However, these embodiments are exemplary, the present invention is not limited thereto and the present invention is defined by the scope of claims.

In the present specification, when a definition is not otherwise provided, "substituted" refers to replacement of at least one hydrogen of a substituent or a compound by deuterium, a halogen, a hydroxyl group, an amino group, a substituted or unsubstituted C1 to C30 amine group, a nitro group, a substituted or unsubstituted C1 to C40 silyl group, a C1 to C30 alkyl group, a C1 to C10 alkylsilyl group, a C6 to C30 arylsilyl group, a C3 to C30 cycloalkyl group, a C3 to C30 heterocycloalkyl group, a C6 to C30 aryl group, a C2 to C30 heteroaryl group, a C1 to C20 alkoxy group, a C1 to C10 trifluoroalkyl group, a cyano group, or a combination thereof.

In one example of the present invention, "substituted" refers to replacement of at least one hydrogen of a substituent or a compound by deuterium, a C1 to C30 alkyl group, a C1 to C10 alkylsilyl group, a C6 to C30 arylsilyl group, a C3 to C30 cycloalkyl group, a C3 to C30 heterocycloalkyl group, a C6 to C30 aryl group, a C2 to C30 heteroaryl group, or a cyano group. In addition, in specific examples of the present invention, "substituted" refers to replacement of at least one hydrogen of a substituent or a compound by deuterium, a C1 to C20 alkyl group, a C6 to C30 aryl group, or a cyano group. In addition, in specific examples of the present invention, "substituted" refers to replacement of at least one hydrogen of a substituent or a compound by deuterium, a C1 to C5 alkyl group, a C6 to C18 aryl group, or a cyano group. In addition, in specific examples of the present invention, "substituted" refers to replacement of at least one hydrogen of a substituent or a compound by deuterium, a cyano group, a methyl group, an ethyl group, a propyl group, a butyl group, a phenyl group, a biphenyl group, a terphenyl group, or a naphthyl group.

"Unsubstituted" refers to non-replacement of a hydrogen atom by another substituent and remaining of the hydrogen atom.

In the present specification, "hydrogen (-H)" may include "deuterium substitution (-D)" or "tritium substitution (-T)."

In the present specification, when a definition is not otherwise provided, "hetero" refers to one including one to three heteroatoms selected from N, O, S, P, and Si, and remaining carbons in one functional group.

In the present specification, "aryl group" refers to a group including at least one hydrocarbon aromatic moiety, and may include a group in which all elements of the hydrocarbon aromatic moiety have p-orbitals which form conjugation, for example a phenyl group, a naphthyl group, and the like, a group in which two or more hydrocarbon aromatic moieties may be linked by a sigma bond, for example a biphenyl group, a terphenyl group, a quarterphenyl group, and the like, and a group in which two or more hydrocarbon aromatic moieties are fused directly or indirectly to provide a non-aromatic fused ring, for example, a fluorenyl group, and the like.

The aryl group may include a monocyclic, polycyclic or fused ring polycyclic (i.e., rings sharing adjacent pairs of carbon atoms) functional group.

In the present specification, "heterocyclic group" is a generic concept of a heteroaryl group, and may include at least one heteroatom selected from N, O, S, P, and Si instead of carbon (C) in a cyclic compound such as an aryl group, a cycloalkyl group, a fused ring thereof, or a combination thereof. When the heterocyclic group is a fused ring, the entire ring or each ring of the heterocyclic group may include one or more heteroatoms.

For example, "heteroaryl group" refers to an aryl group including at least one heteroatom selected from N, O, S, P, and Si. Two or more heteroaryl groups are linked by a sigma bond directly, or when the heteroaryl group includes two or more rings, the two or more rings may be fused. When the heteroaryl group is a fused ring, each ring may include one to three heteroatoms.

More specifically, the substituted or unsubstituted C6 to C30 aryl group refers to a substituted or unsubstituted phenyl group, a substituted or unsubstituted naphthyl group, a substituted or unsubstituted anthracenyl group, a substituted or unsubstituted phenanthrenyl group, a substituted or unsubstituted naphthacenyl group, a substituted or unsubstituted pyrenyl group, a substituted or unsubstituted biphenyl group, a substituted or unsubstituted p-terphenyl group, a substituted or unsubstituted m-terphenyl group, a substituted or unsubstituted o-terphenyl group, a substituted or unsubstituted chrysenyl group, a substituted or unsubstituted triphenylene group, a substituted or unsubstituted perylenyl group, a substituted or unsubstituted fluorenyl group, a substituted or unsubstituted indenyl group, a substituted or unsubstituted furanyl group, or a combination thereof, but is not limited thereto.

More specifically, the substituted or unsubstituted C2 to C30 heterocyclic group refers to a substituted or unsubstituted thiophenyl group, a substituted or unsubstituted pyrrolyl group, a substituted or unsubstituted pyrazolyl group, a substituted or unsubstituted imidazolyl group, a substituted or unsubstituted triazolyl group, a substituted or unsubstituted oxazolyl group, a substituted or unsubstituted thiazolyl group, a substituted or unsubstituted oxadiazolyl group, a substituted or unsubstituted thiadiazolyl group, a substituted or unsubstituted pyridyl group, a substituted or unsubstituted pyrimidinyl group, a substituted or unsubstituted pyrazinyl group, a substituted or unsubstituted triazinyl group, a substituted or unsubstituted benzofuranyl group, a substituted or unsubstituted benzothiophenyl group, a substituted or unsubstituted benzimidazolyl group, a substituted or unsubstituted indolyl group, a substituted or unsubstituted quinolinyl group, a substituted or unsubstituted isoquinolinyl group, a substituted or unsubstituted quinazolinyl group, a substituted or unsubstituted quinoxalinyl group, a substituted or unsubstituted naphthyridinyl group, a substituted or unsubstituted benzoxazinyl group, a substituted or unsubstituted benzthiazinyl group, a substituted or unsubstituted acridinyl group, a substituted or unsubstituted phenazinyl group, a substituted or unsubstituted phenothiazinyl group, a substituted or unsubstituted phenoxazinyl group, a substituted or unsubstituted carbazolyl group, a substituted or unsubstituted dibenzofuranyl group, or a substituted or unsubstituted dibenzothiophenyl group, or a combination thereof, but is not limited thereto.

In the present specification, hole characteristics refer to an ability to donate an electron to form a hole when an electric field is applied and that a hole formed in the anode may be easily injected into the light emitting layer and transported in the light emitting layer due to conductive characteristics according to the highest occupied molecular orbital (HOMO) level.

In addition, electron characteristics refer to an ability to accept an electron when an electric field is applied and that electron formed in the cathode may be easily injected into the light emitting layer and transported in the light emitting layer due to conductive characteristics according to the lowest unoccupied molecular orbital (LUMO) level.

Hereinafter, a composition for an organic optoelectronic device according to an embodiment is described.

The composition for an organic optoelectronic device according to an embodiment includes a first compound represented by Chemical Formula 1, and a second compound represented by a combination of Chemical Formula 2 and Chemical Formula 3.

The first compound may be represented by Chemical Formula 1.

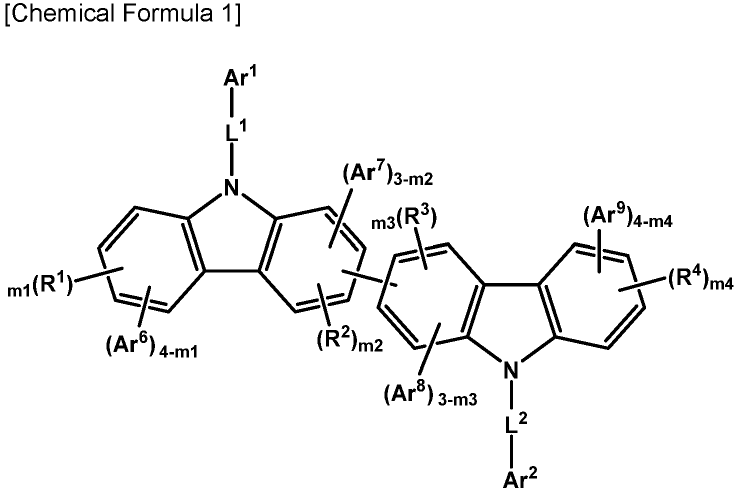

In Chemical Formula 1,
L¹ and L² are each independently a single bond or a substituted or unsubstituted C6 to C30 arylene group,
Ar¹ and Ar² are each independently a substituted or unsubstituted C6 to C30 aryl group or a substituted or unsubstituted C2 to C30 heterocyclic group,
R¹ to R⁴ are each independently hydrogen, deuterium or a substituted or unsubstituted C6 to C30 aryl group,
Ar⁶ to Ar⁹ are each independently hydrogen or a substituted or unsubstituted C6 to C30 aryl group,
m1 and m4 are each independently an integer from 1 to 4,
m2 and m3 are each independently an integer from 1 to 3, and
Chemical Formula 1 simultaneously satisfies the following conditions (i) and (ii):
   (i) at least one of Ar¹ and Ar² is a C6 to C30 aryl group substituted with at least one deuterium or a C2 to C30 heterocyclic group substituted with at least one deuterium, and
   (ii) at least one of R¹ to R⁴ is deuterium.

The first compound represented by Chemical Formula 1 has a structure in which biscarbazole is a basic skeleton, a benzene moiety of the carbazole is substituted with at least one deuterium, and at least one of Ar¹ and Ar² that are the 9th (N-direction) substituents of carbazole is substituted with deuterium.

As the benzene moiety of carbazole and the 9th (N-direction) substituent of carbazole are replaced with deuterium, a zero-point energy and vibration energy of the compound may be further lowered. As a result, an energy of the ground state is further lowered and the interaction between molecules is weakened, and thus it possible to make the thin film formed from this in an amorphous state, which improves heat resistance and effectively improves life-span. In other words, if this is applied, it is possible to implement an organic light emitting diode with low driving, high efficiency, and especially long life-span.

Chemical Formula 1 may be represented by one of, for example, any one of Chemical Formulas 1-1 to 1-10, depending on the linking position of carbazole.

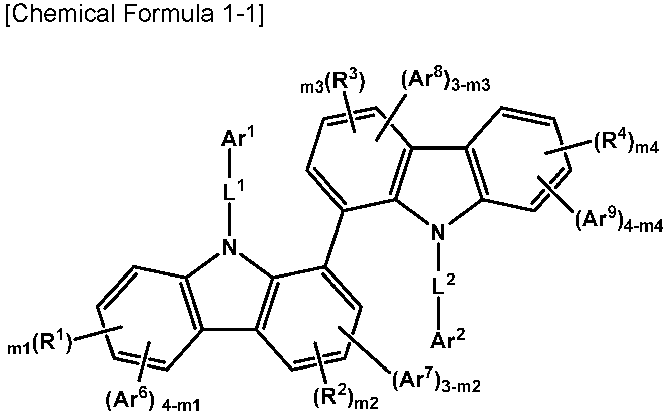

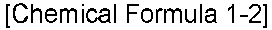

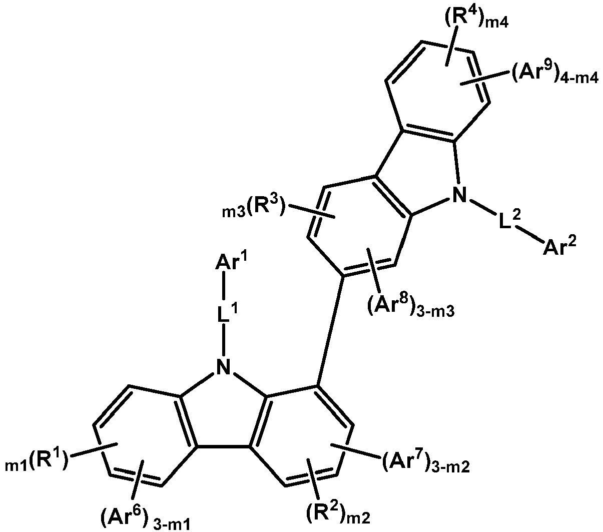

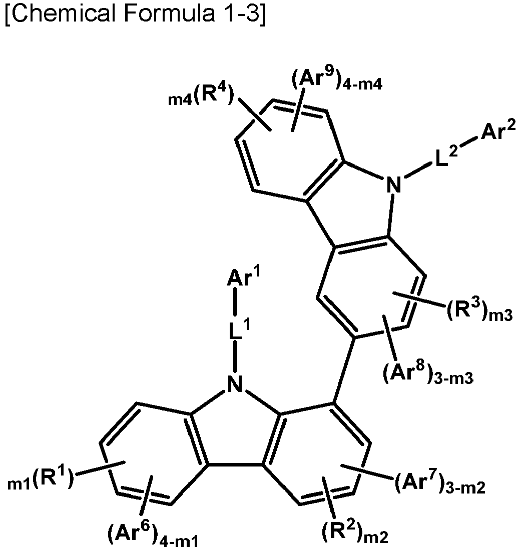

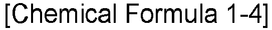

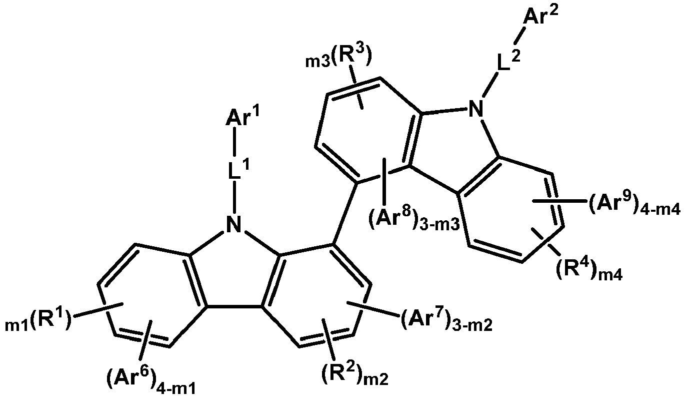

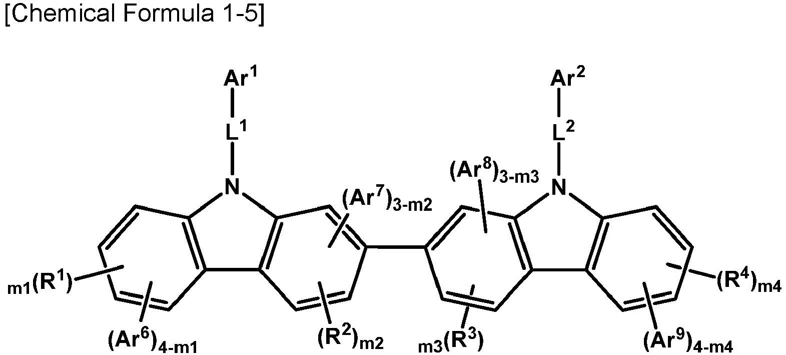

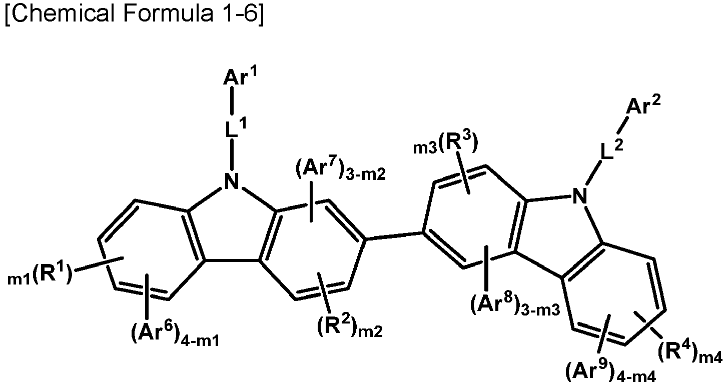

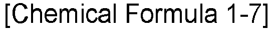

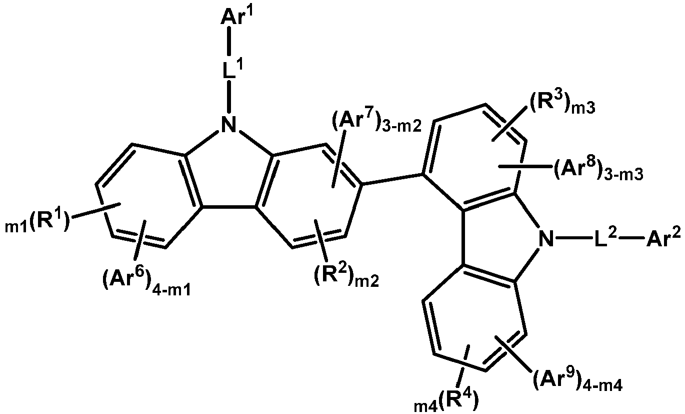

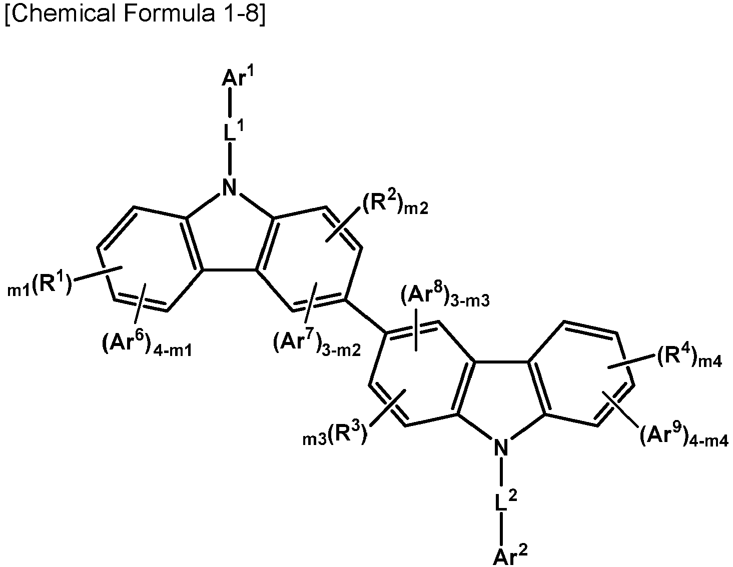

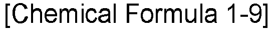

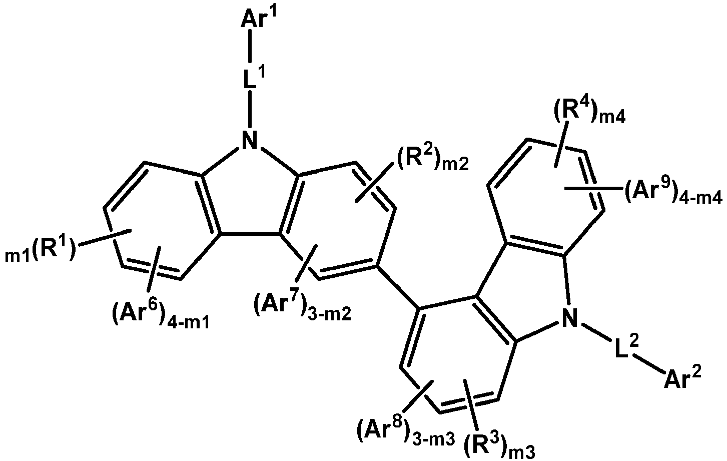

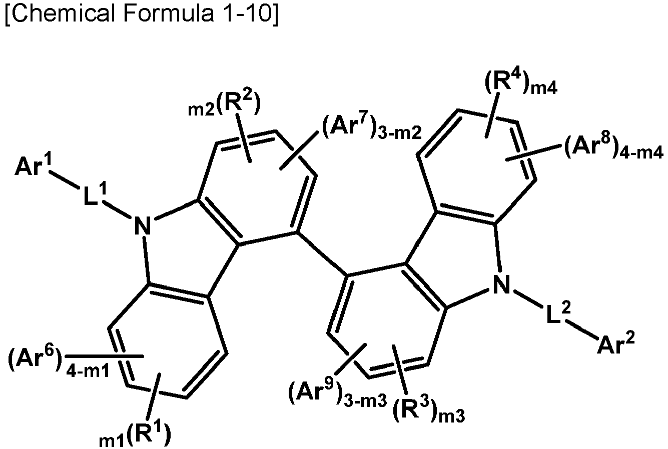

In Chemical Formula 1-1 to Chemical Formula 1-10,
L¹, L², Ar¹, Ar², Ar⁶ to Ar⁹, R¹ to R⁴, and m¹ to m⁴ are the same as described above.

When R¹ is greater than or equal to 2, each R¹ may be the same or different from each other.

When R² is greater than or equal to 2, each R² may be the same or different from each other.

When R³ is greater than or equal to 2, each R³ may be the same or different from each other.

When R⁴ is greater than or equal to 2, each R⁴ may be the same or different from each other.

When Ar⁶ is greater than or equal to 2, each Ar⁶ may be the same or different from each other.

When Ar⁷ is greater than or equal to 2, each Ar⁷ may be the same or different from each other.

When Ar⁸ is greater than or equal to 2, each Ar⁸ may be the same or different from each other.

When Ar⁹ is greater than or equal to 2, each Ar⁹ may be the same or different from each other.

For example, at least two of R¹ to R⁴ may be deuterium.

For example, R¹ to R⁴ may each be deuterium, m1 and m4 may each be an integer of 4, and m2 and m3 may each be an integer of 3.

For example, R¹ and R² may each be deuterium, m1 may be an integer from 1 to 4, m2 may be an integer from 1 to 3, and R³ and R⁴ may each be hydrogen.

For example, R³ and R⁴ may each be deuterium, m3 may be an integer from 1 to 3, m4 may be an integer from 1 to 4, and R¹ and R² may each be hydrogen.

For example, R¹ and R⁴ may each be deuterium, m1 and m4 may each be an integer from 1 to 4, and R² and R³ may each be hydrogen.

For example, R¹ to R³ may each be deuterium, m2 and m3 may each be an integer from 1 to 3, m 1 may be an integer from 1 to 4, and R⁴ may be deuterium or a C6 to C30 aryl group substituted or unsubstituted with deuterium.

As an example, depending on the substitution position of the deuterium substituted for R¹ to R⁴, Chemical Formula 1 may be represented by any of Chemical Formula 1a to Chemical Formula 1e. 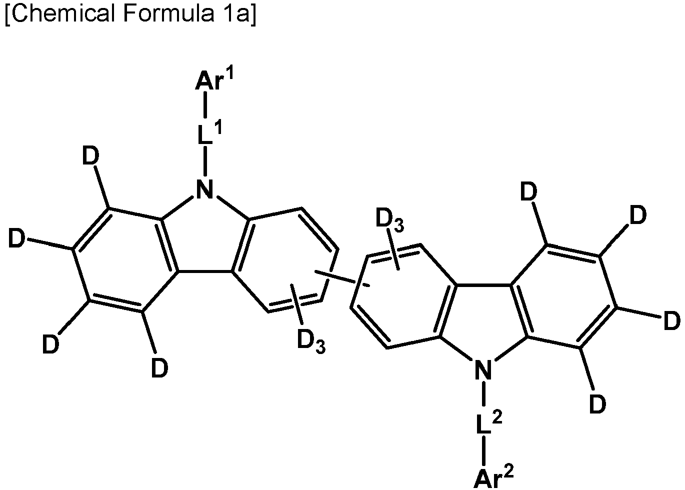 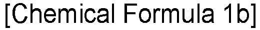 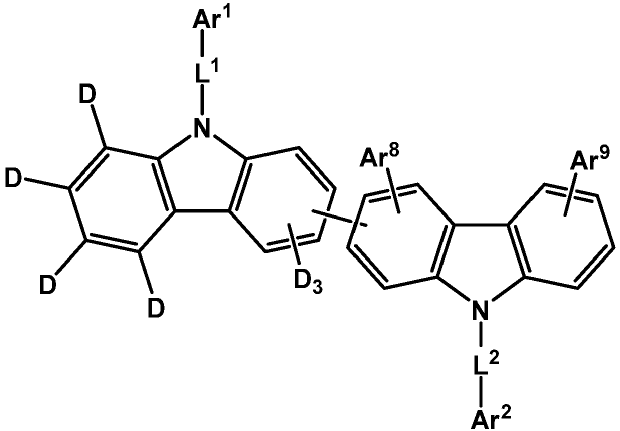 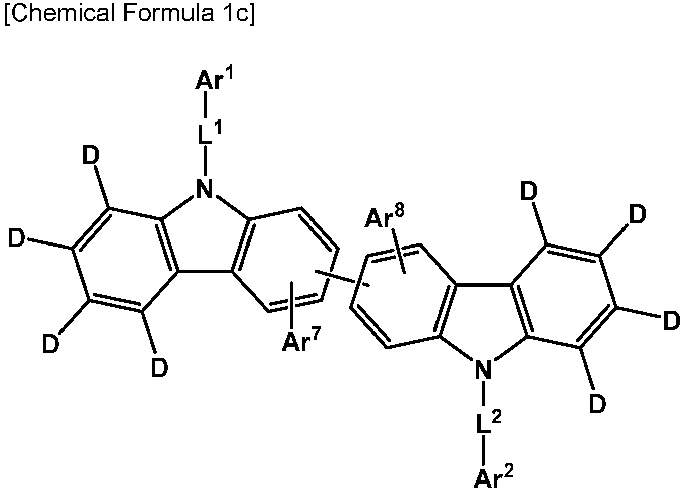 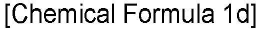 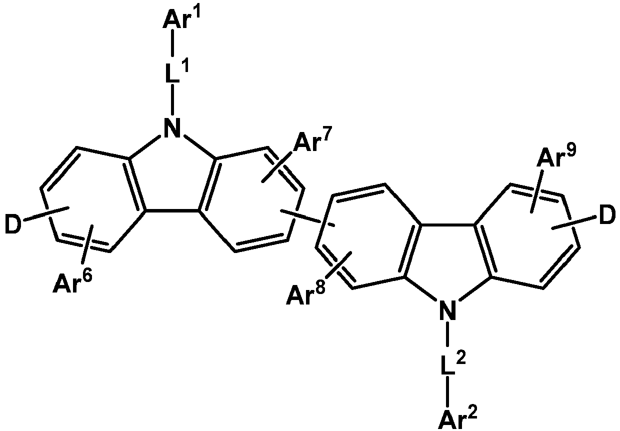 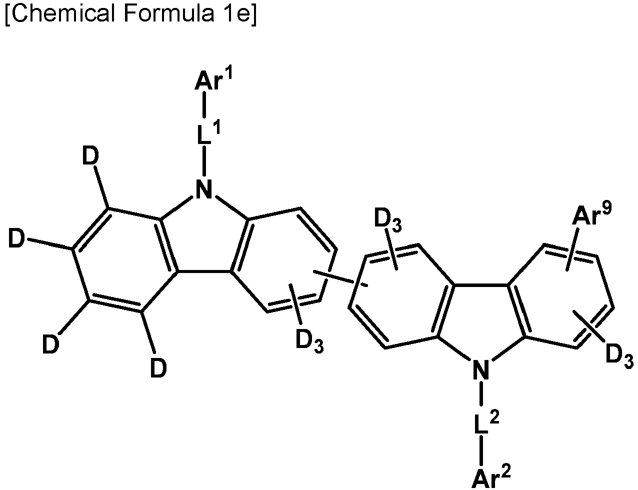
In Chemical Formula 1a to Chemical Formula 1e, L¹, L², Ar¹, Ar², and Ar⁶ to Ar⁹ are as described above,
Ar⁶ to Ar⁹ are each independently a C6 to C30 aryl group substituted or unsubstituted with hydrogen or deuterium, and
D₃ refers to replacement of three deuterium.

For example, at least one of Ar¹ and Ar² may be a phenyl group substituted with at least one deuterium, a biphenyl group substituted with at least one deuterium, a terphenyl group substituted with at least one deuterium, a naphthyl group substituted with at least one deuterium, an anthracenyl group substituted with at least one deuterium, a phenanthrenyl group substituted with at least one deuterium, a triphenylene group substituted with at least one deuterium, a fluorenyl group substituted with at least one deuterium, a dibenzofuranyl group substituted with at least one deuterium, or a dibenzothiophenyl group substituted with at least one deuterium.

As a specific example, at least one of Ar¹ and Ar² may be a phenyl group substituted with at least one deuterium, a biphenyl group substituted with at least one deuterium, a terphenyl group substituted with at least one deuterium, a triphenylene group substituted with at least one deuterium, a dibenzofuranyl group substituted with at least one deuterium, or a dibenzothiophenyl group substituted with at least one deuterium.

As a specific example, Ar⁶ to Ar⁹ may each independently be hydrogen or a C6 to C20 aryl group unsubstituted or substituted with at least one deuterium.

For example, Ar⁶ to Ar⁹ are each independently hydrogen or a phenyl group substituted or unsubstituted with deuterium, a biphenyl group substituted or unsubstituted with deuterium, a terphenyl group substituted or unsubstituted with deuterium, a naphthyl group substituted or unsubstituted with deuterium, a phenanthrenyl group substituted or unsubstituted with deuterium, an anthracenyl group substituted or unsubstituted with deuterium, a triphenylene group substituted or unsubstituted with deuterium, or a fluorenyl group substituted or unsubstituted with deuterium.

For example, L¹-Ar¹ and L²-Ar² of Chemical Formula 1 may each independently be selected from the substituents listed in Group I -1 and Group I -2, and at least one of L¹-Ar¹ and L²-Ar² may be selected from substituents listed in Group I -2.

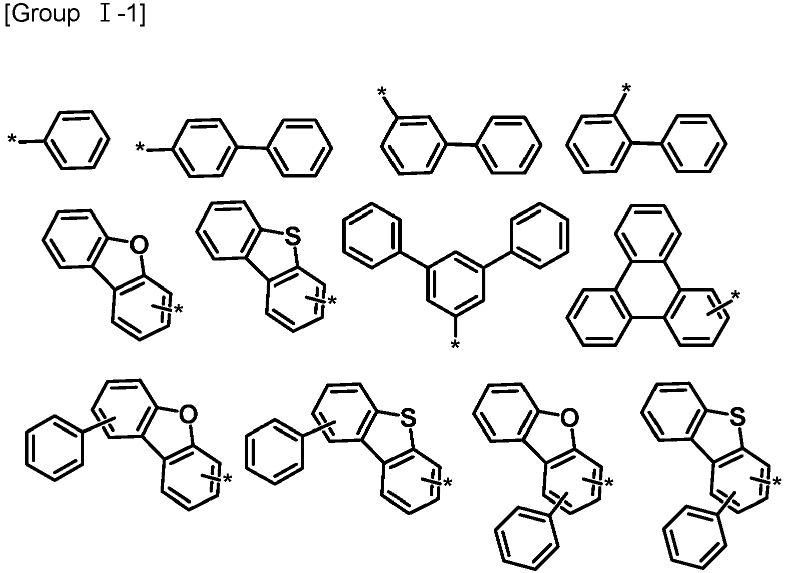

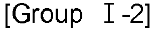

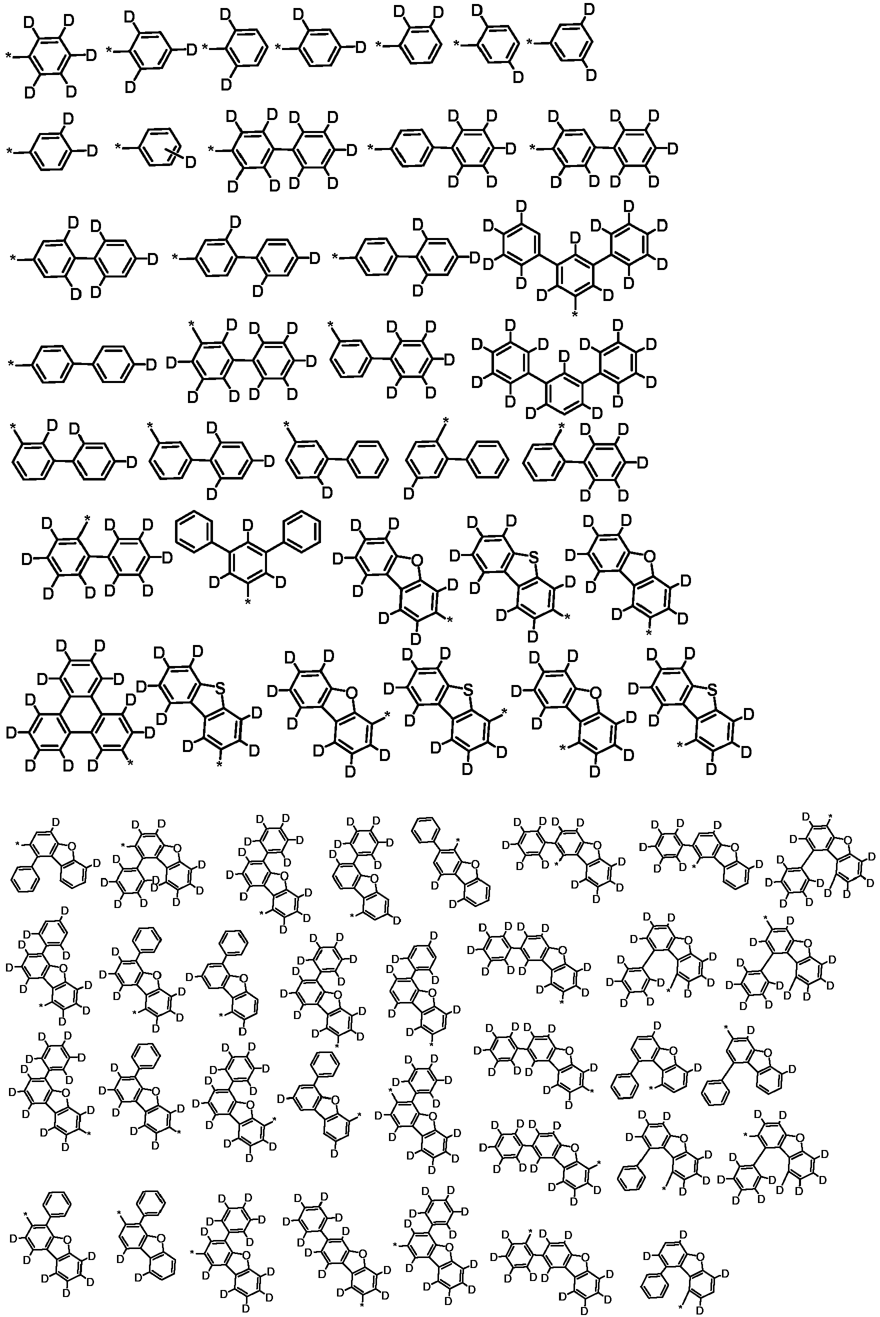

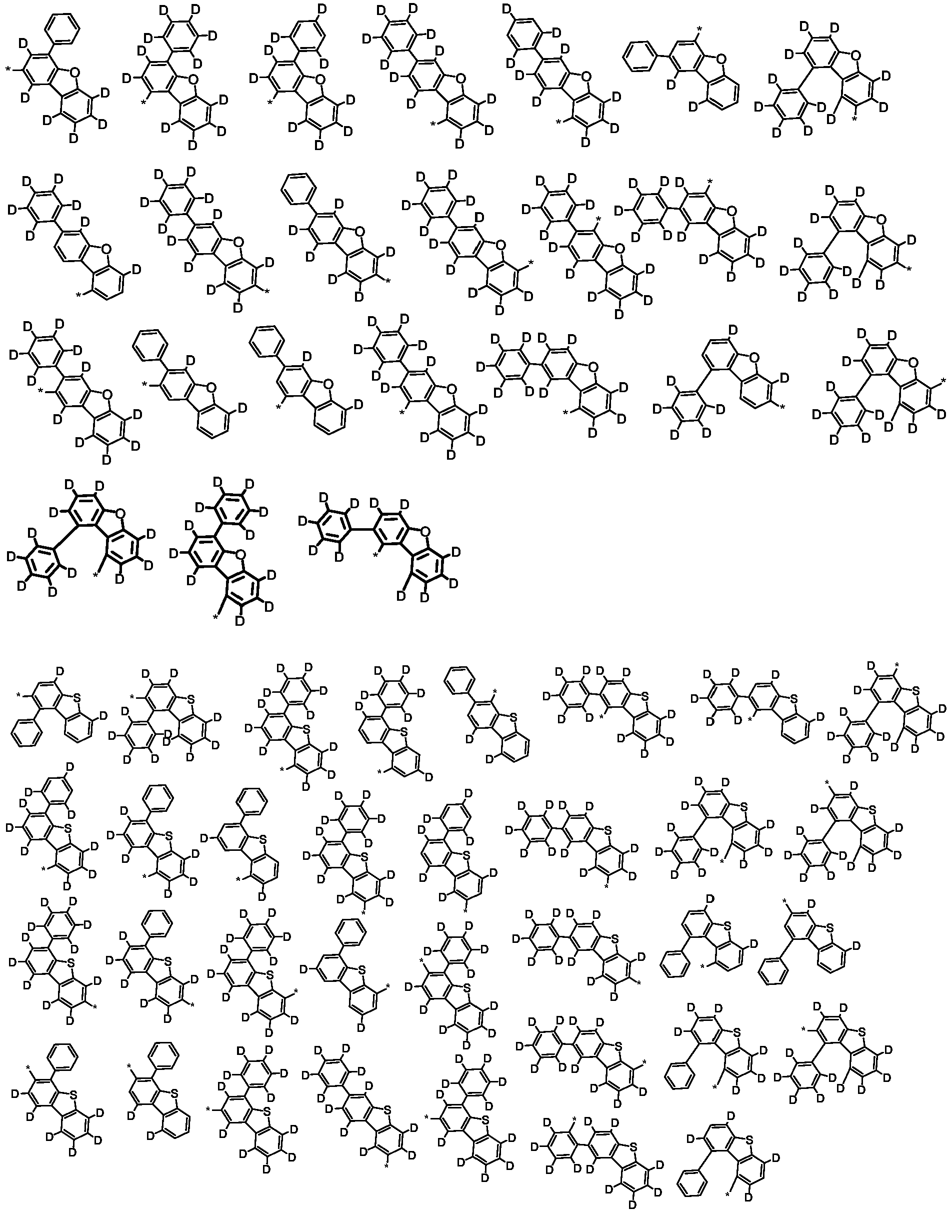

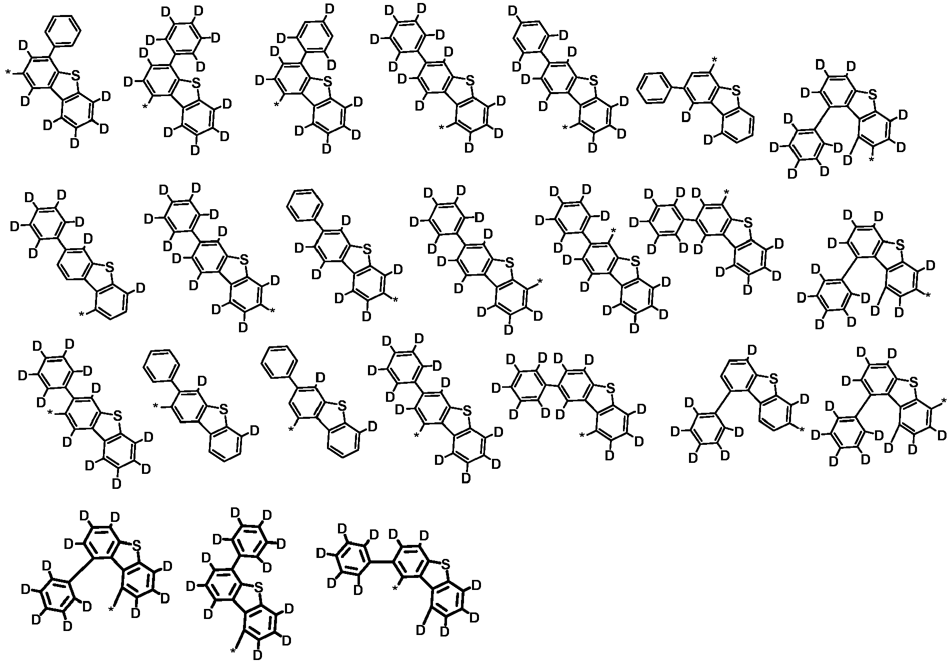

In Group I -1 and Group I -2, * is a linking point.

For example, Chemical Formula 1 may be represented by Chemical Formula 1-8a or Chemical Formula 1-8e.

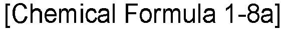

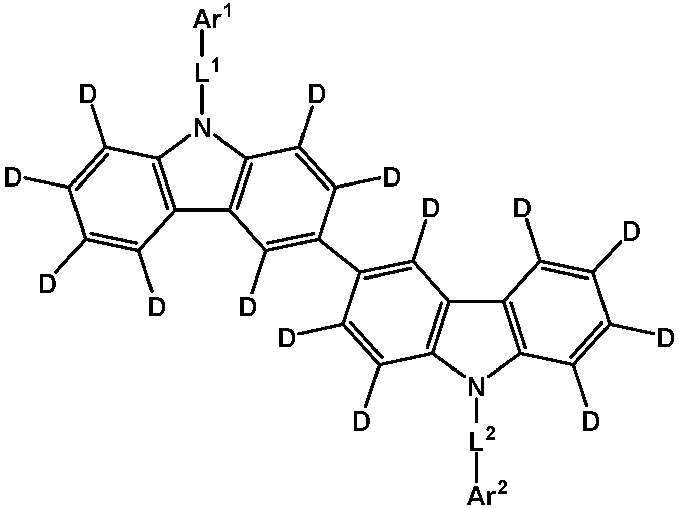

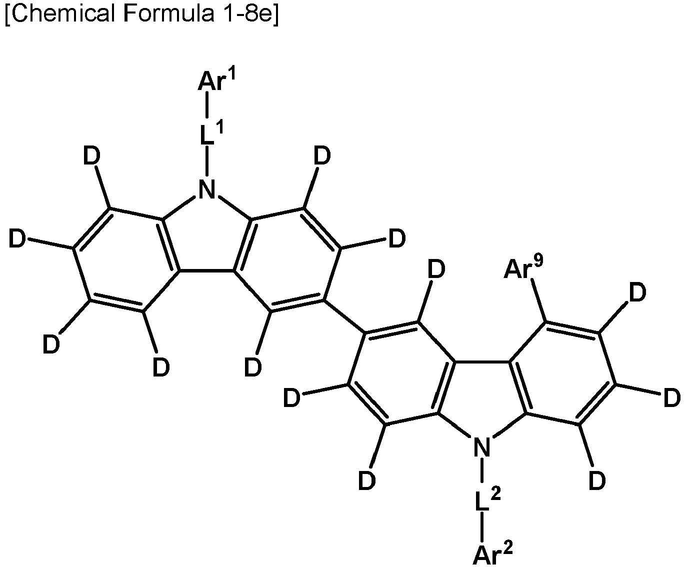

In Chemical Formula 1-8a and Chemical Formula 1-8e,
L¹, L², Ar¹, and Ar² are the same as described above, and Ar⁹ is a C6 to C30 aryl group substituted or unsubstituted with deuterium.

For example, Ar⁹ may be a phenyl group substituted or unsubstituted with deuterium, a biphenyl group substituted or unsubstituted with deuterium, a terphenyl group substituted or unsubstituted with deuterium, a naphthyl group substituted or unsubstituted with deuterium, a phenanthrenyl group substituted or unsubstituted with deuterium, an anthracenyl group substituted or unsubstituted with deuterium, a triphenylene group substituted or unsubstituted with deuterium, or a fluorenyl group substituted or unsubstituted with deuterium.

For example, the compound for an organic optoelectronic device represented by Chemical Formula 1 may be one selected from the compounds listed in Group 1, but is not limited thereto.

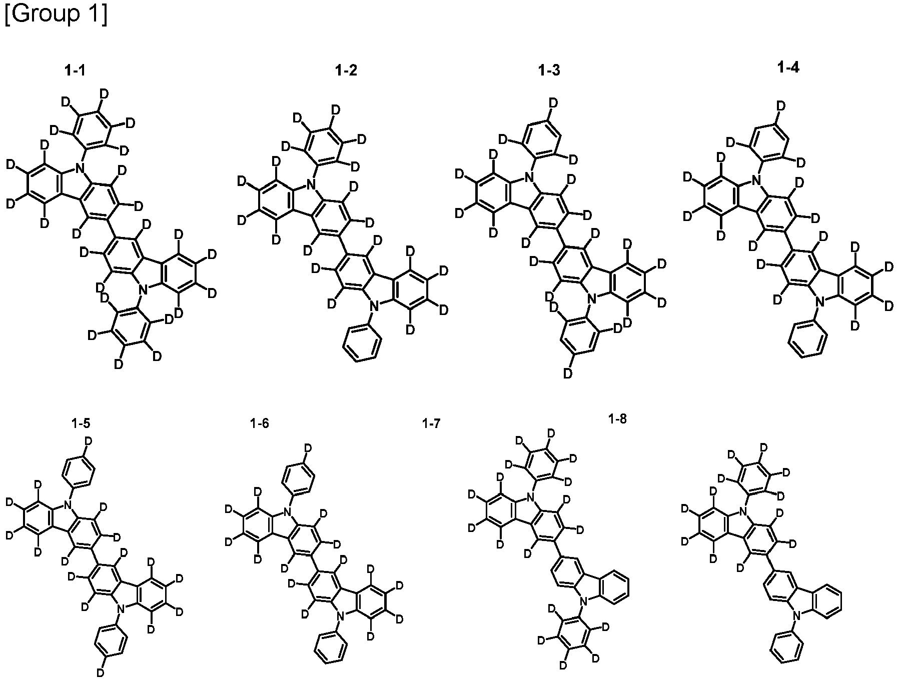

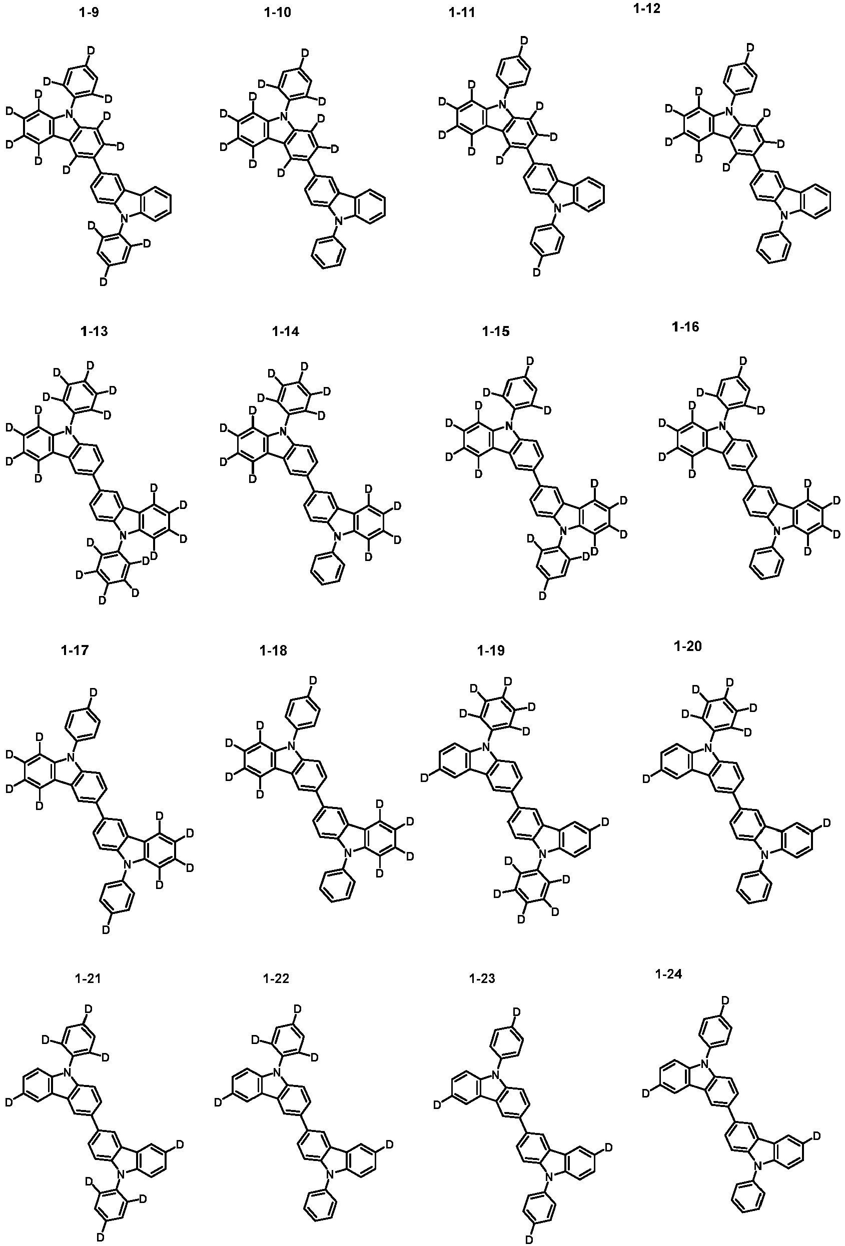

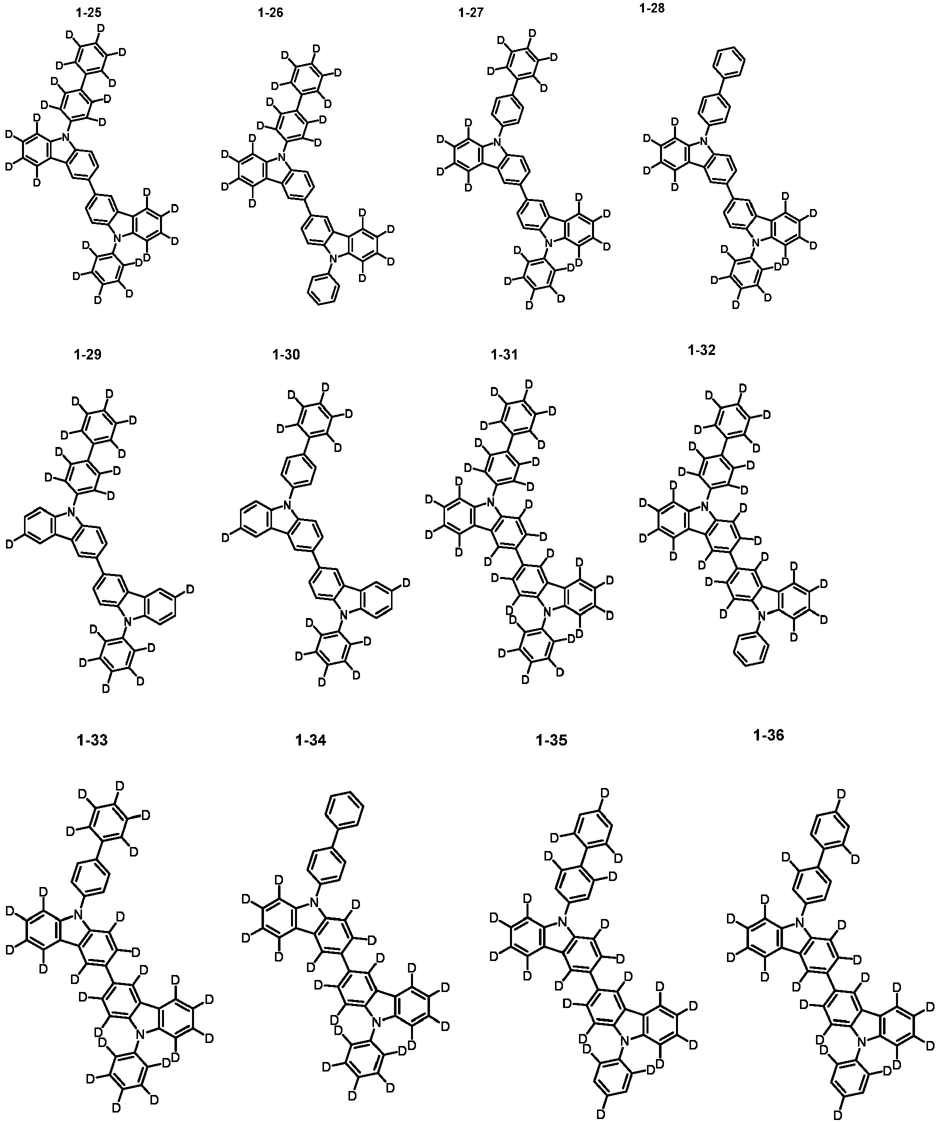

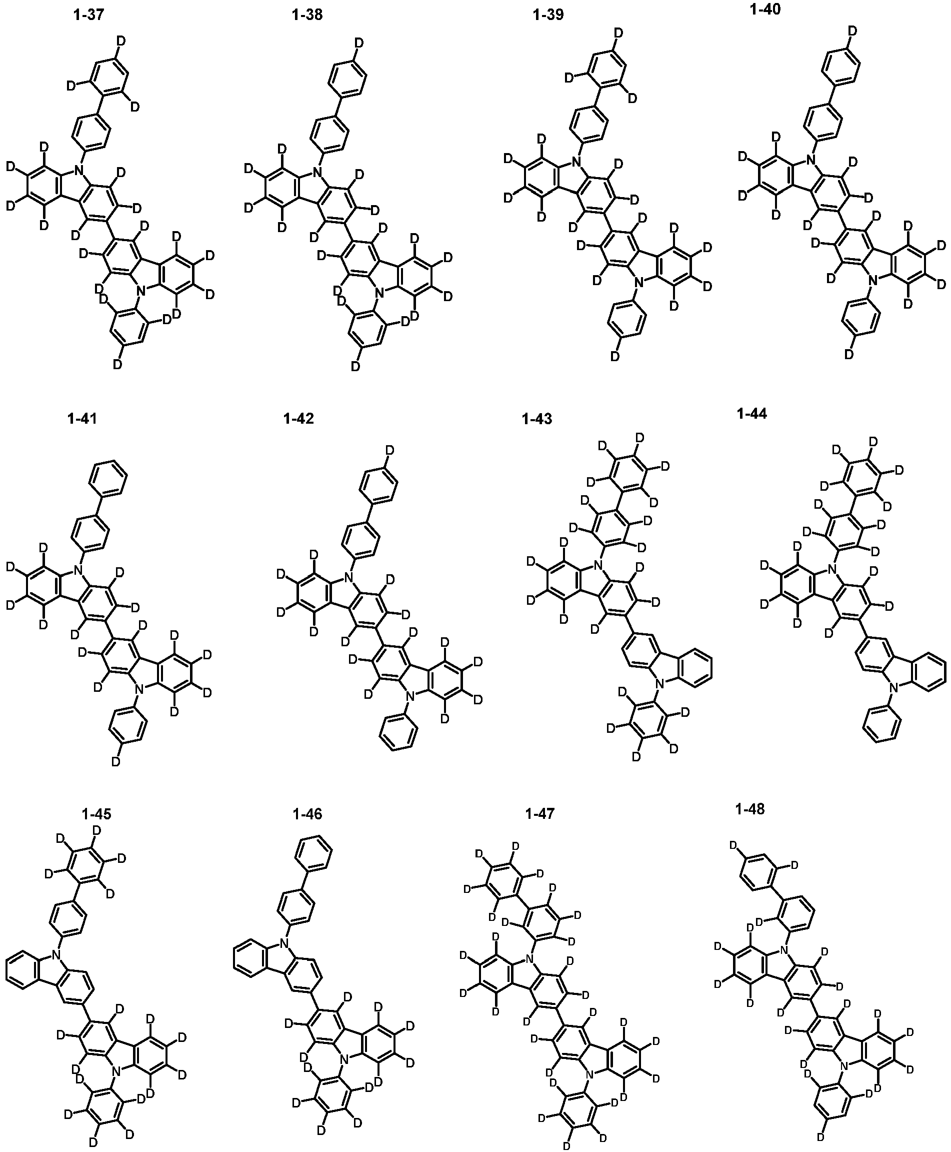

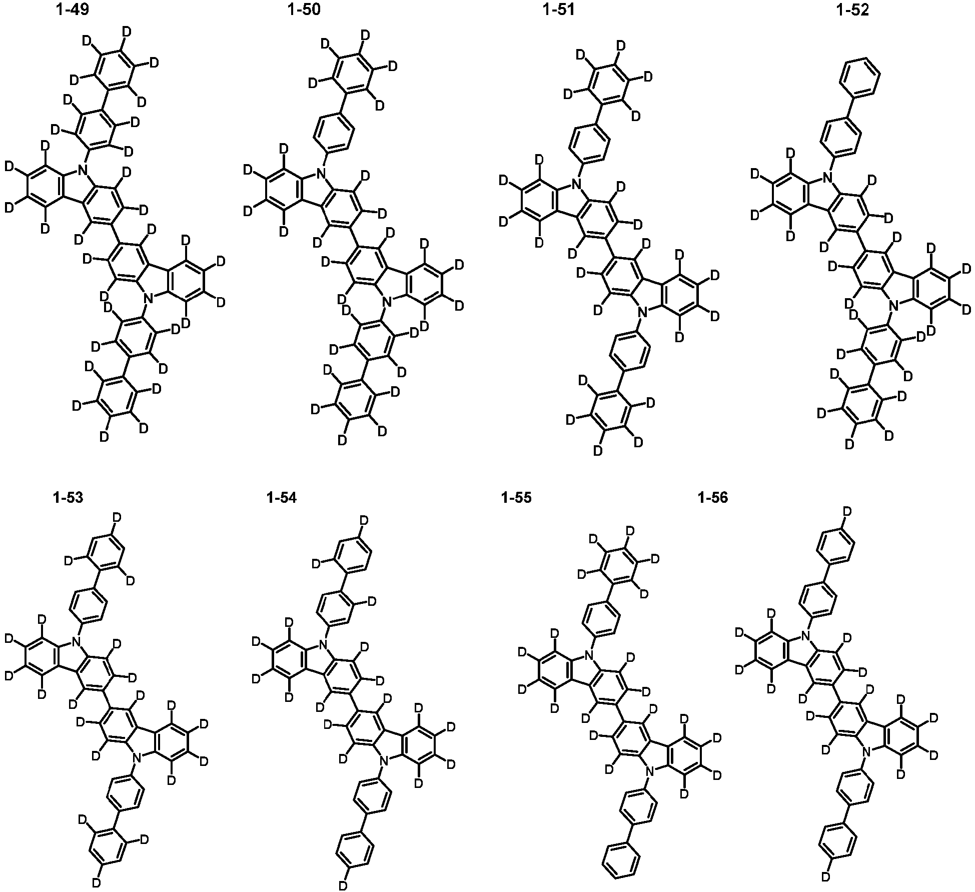

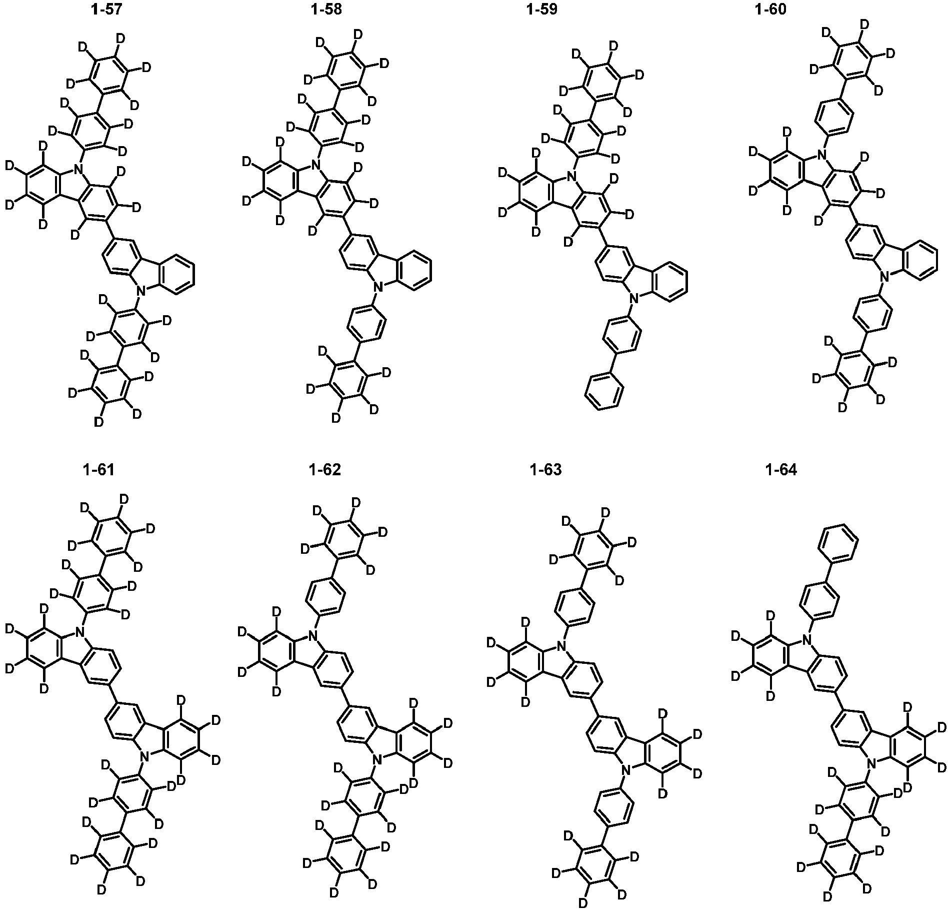

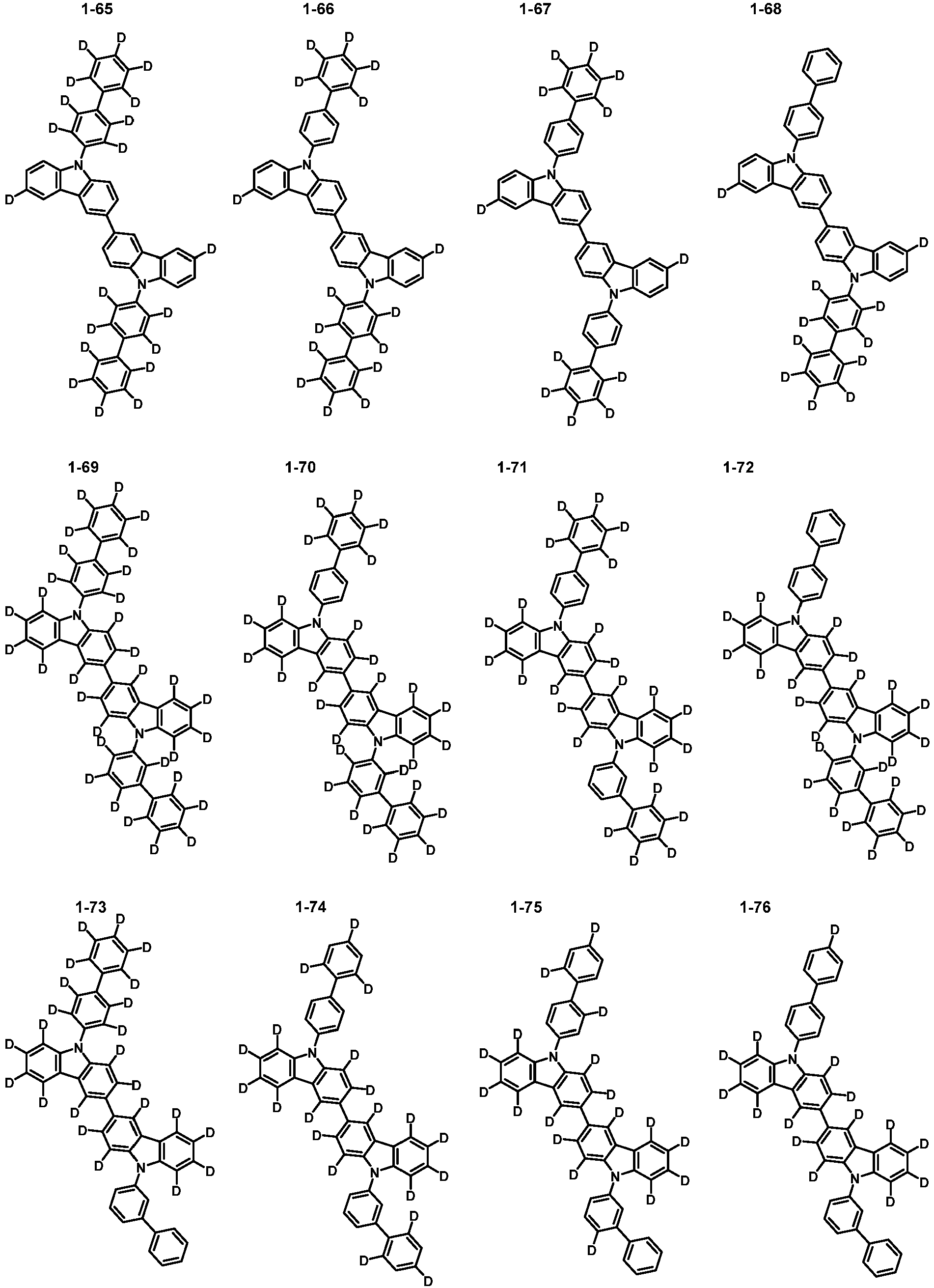

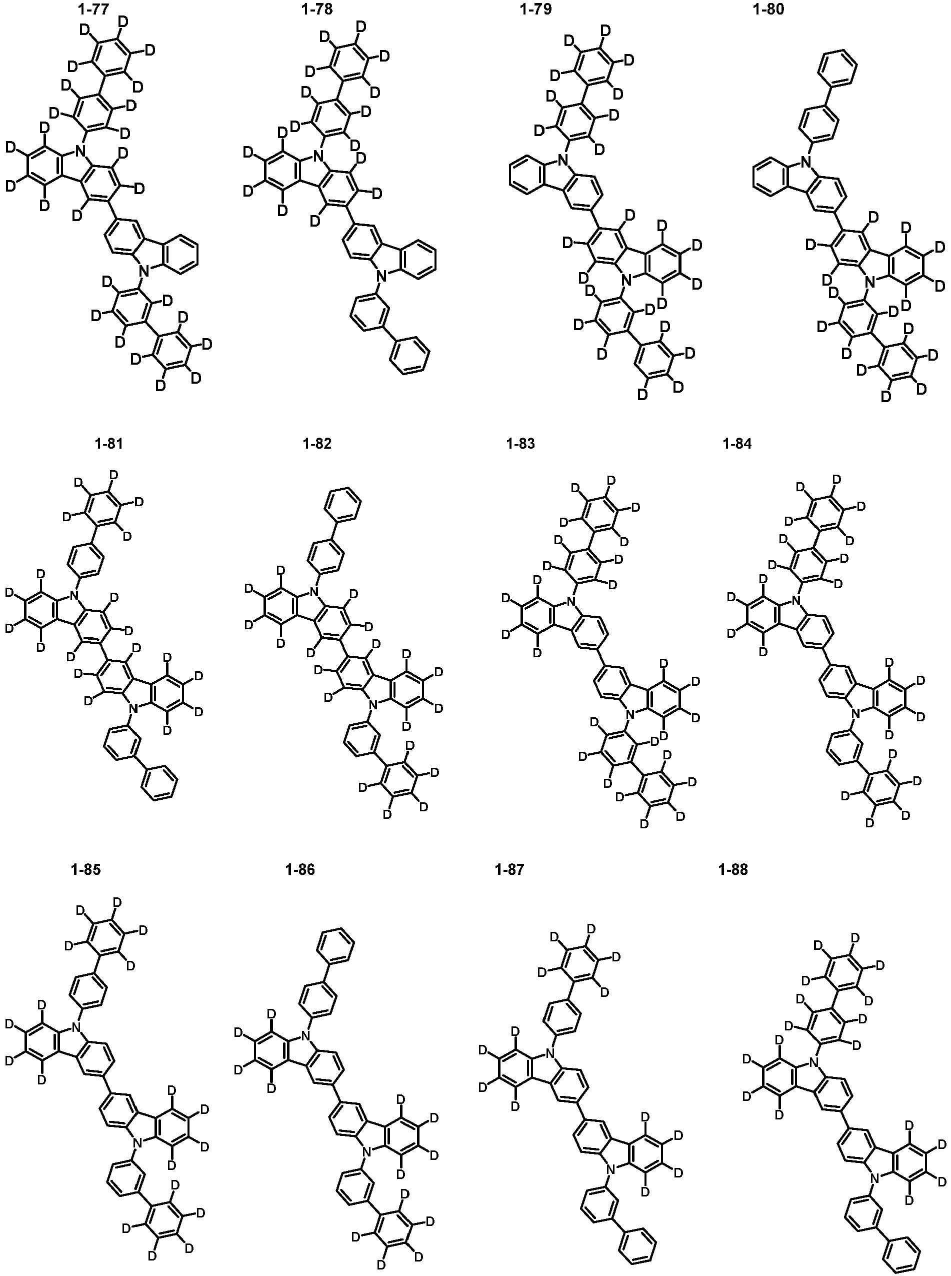

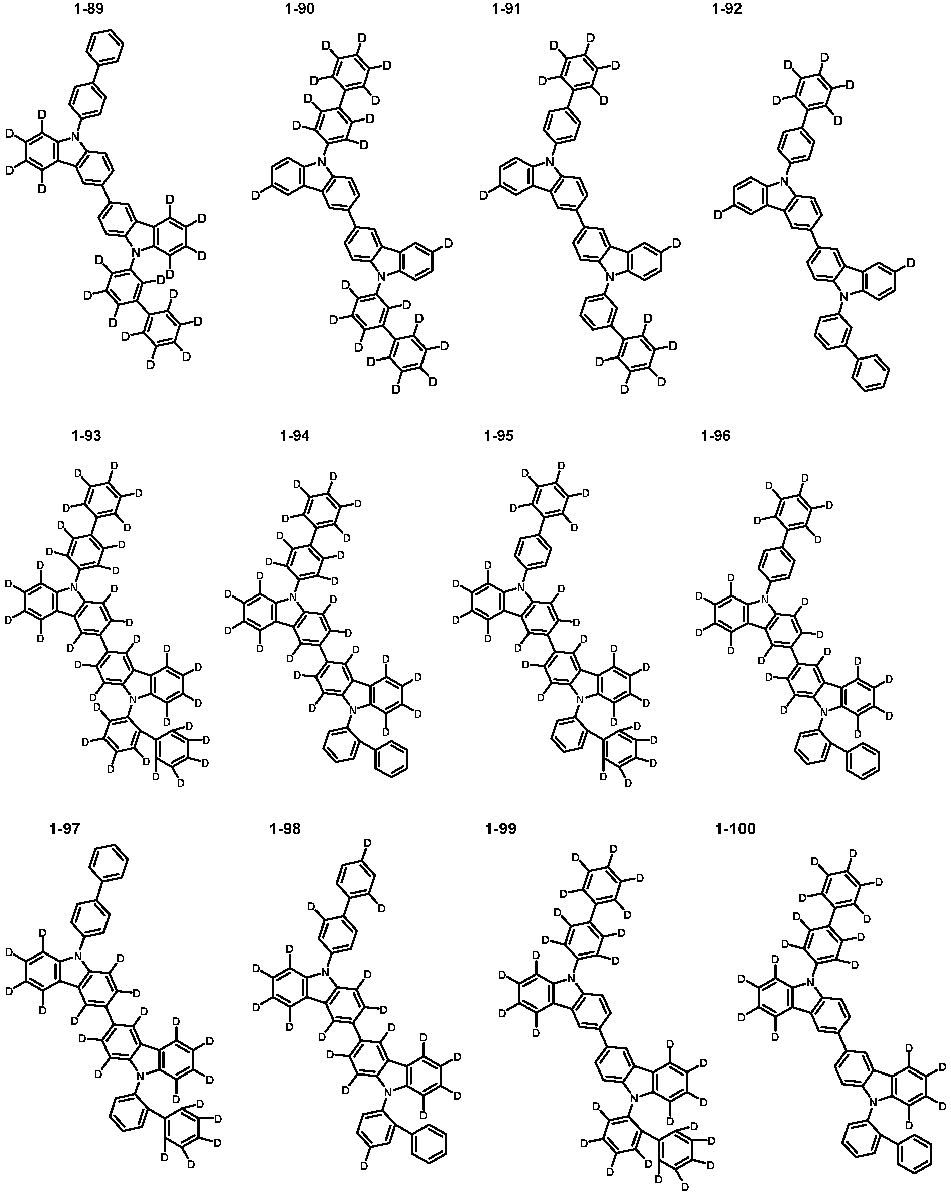

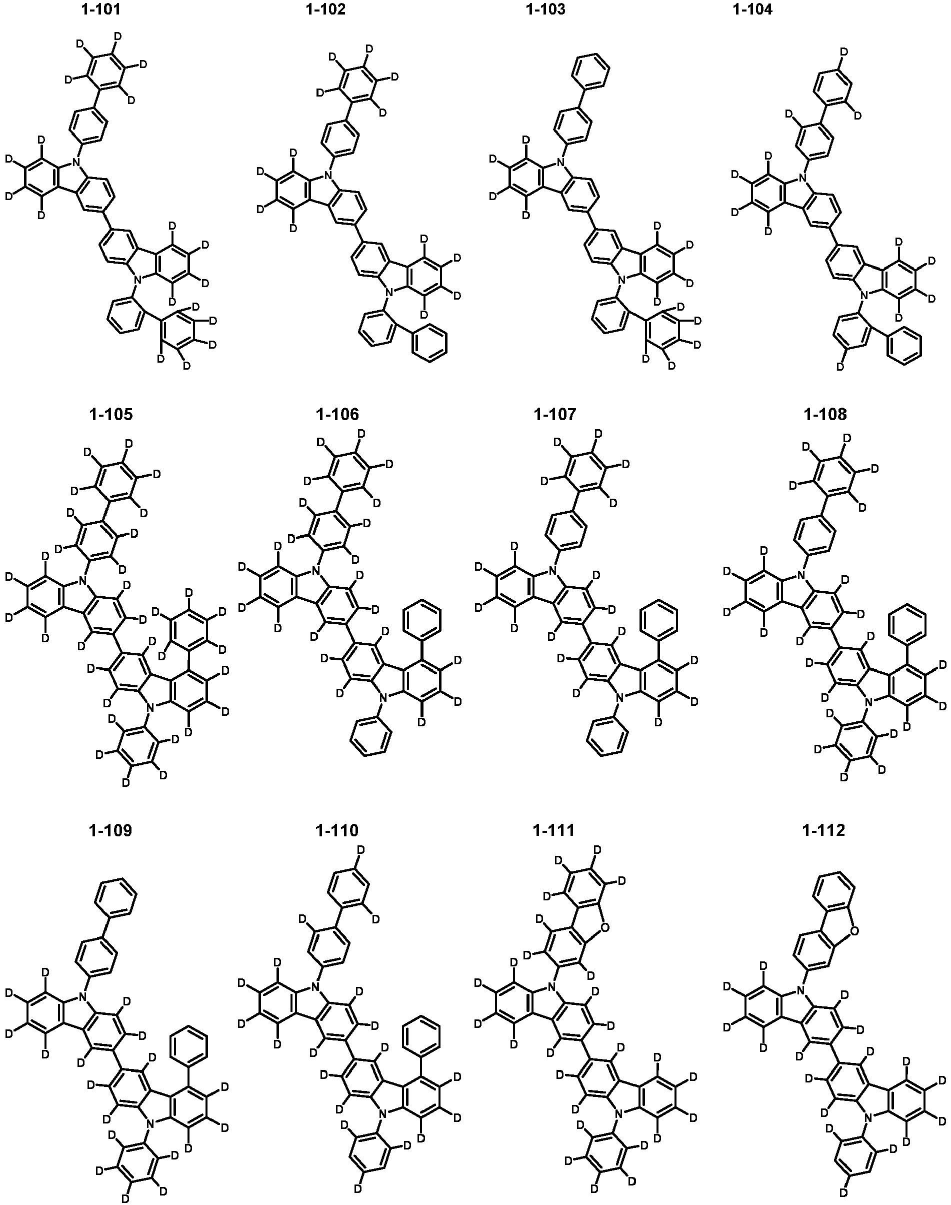

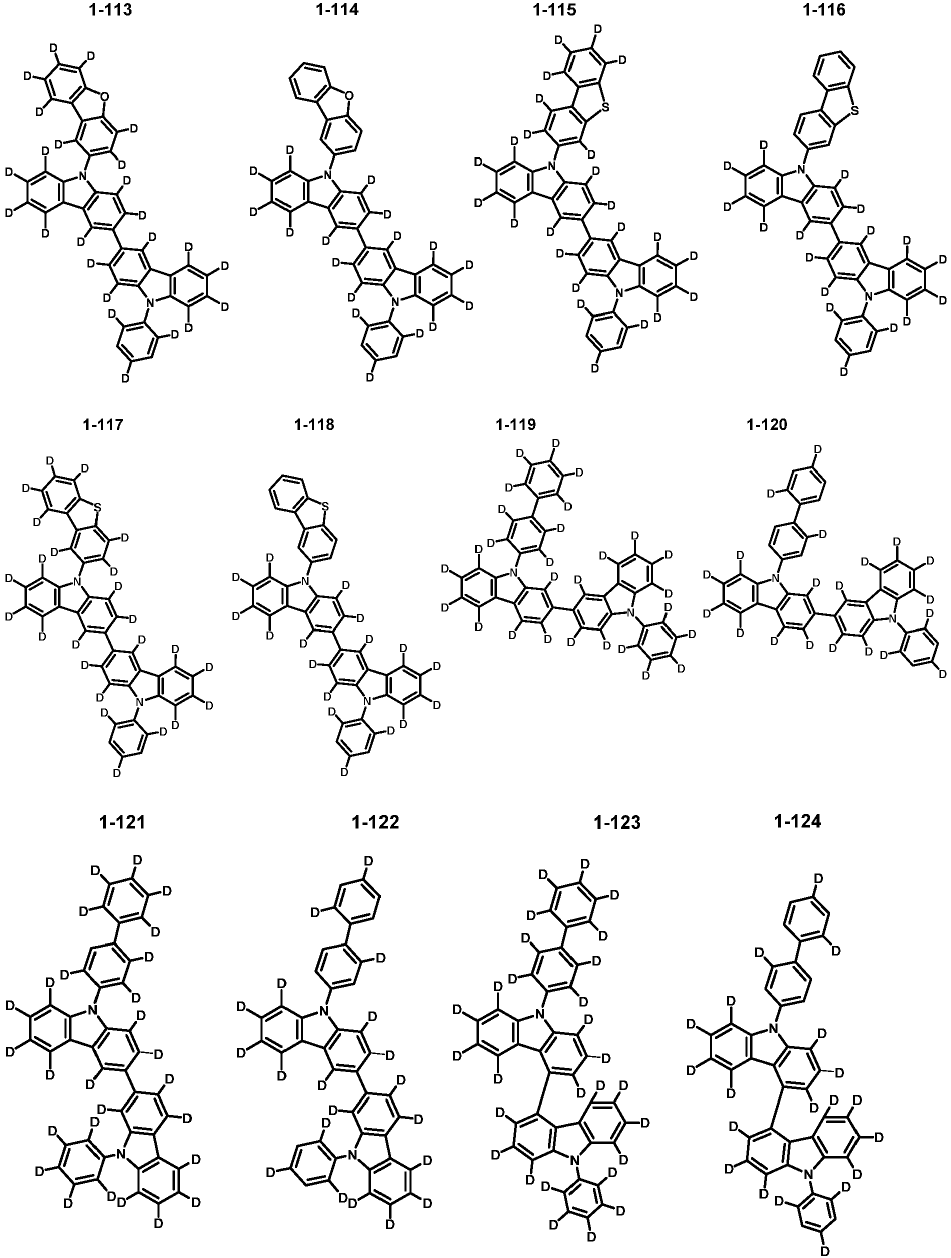

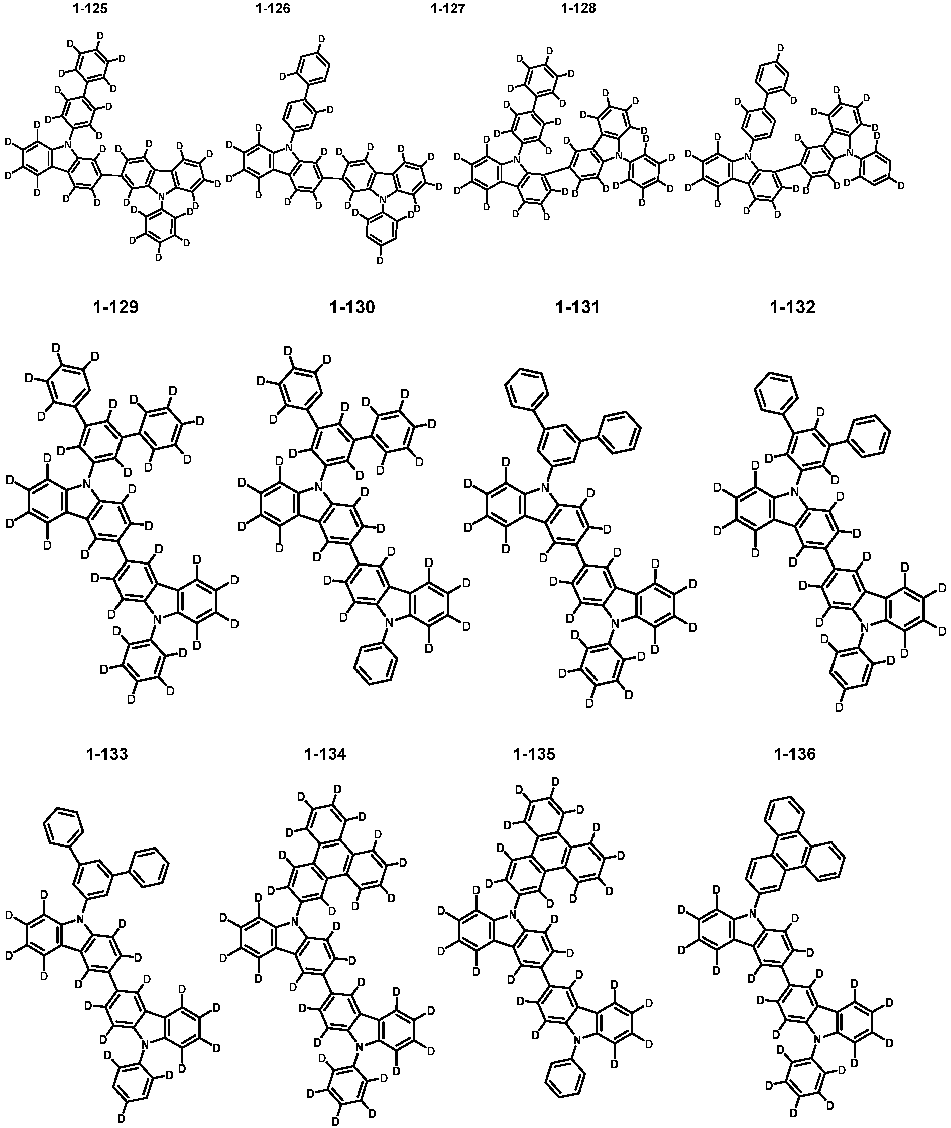

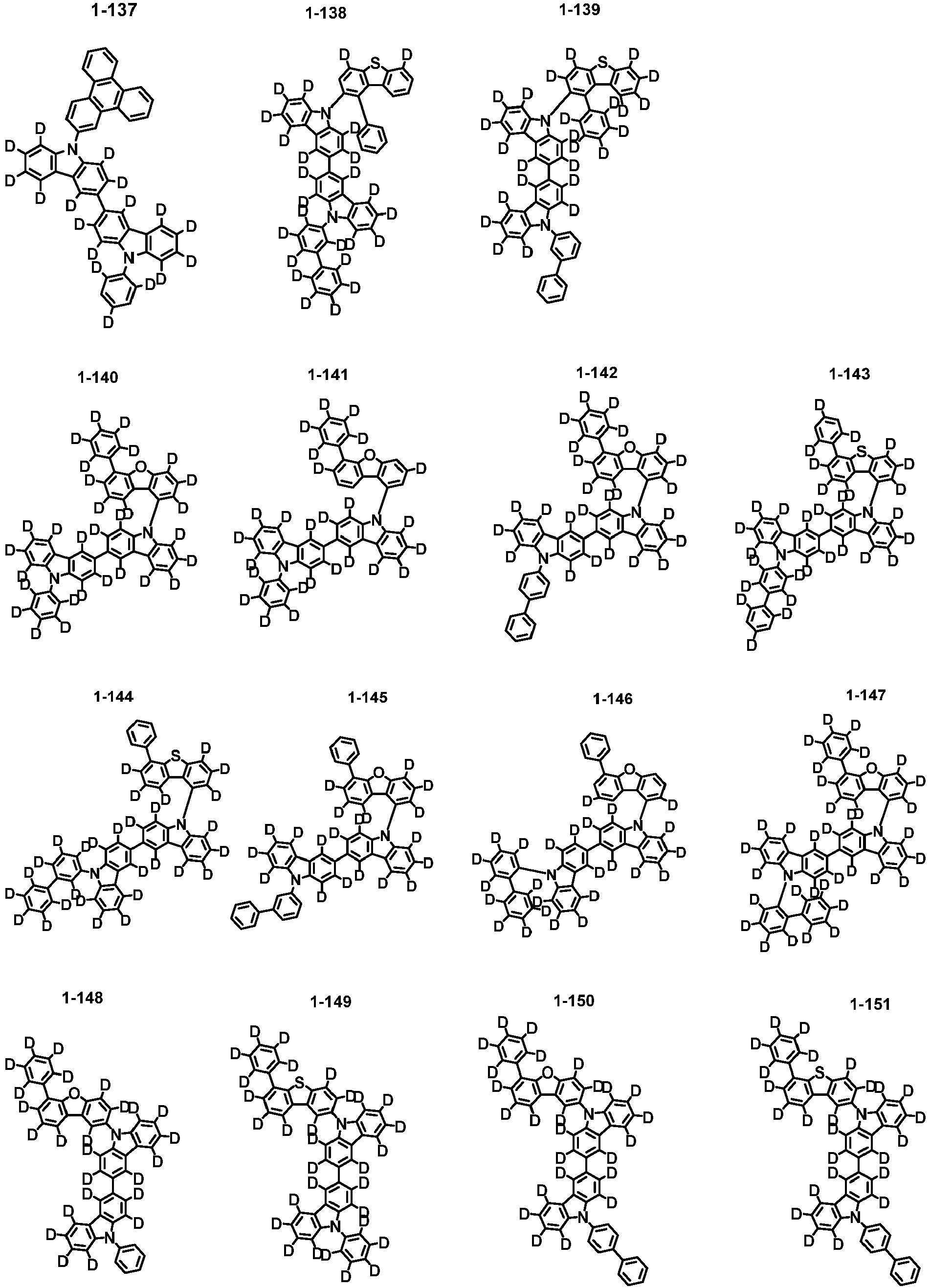

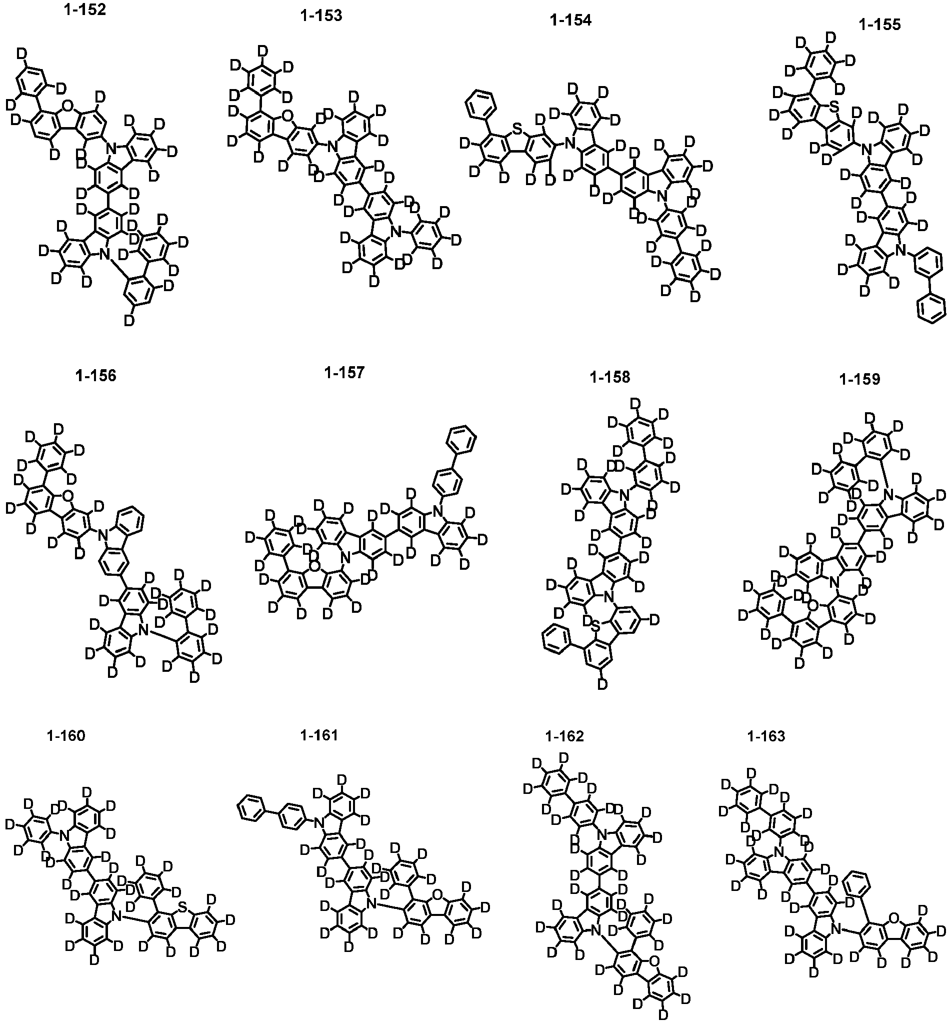

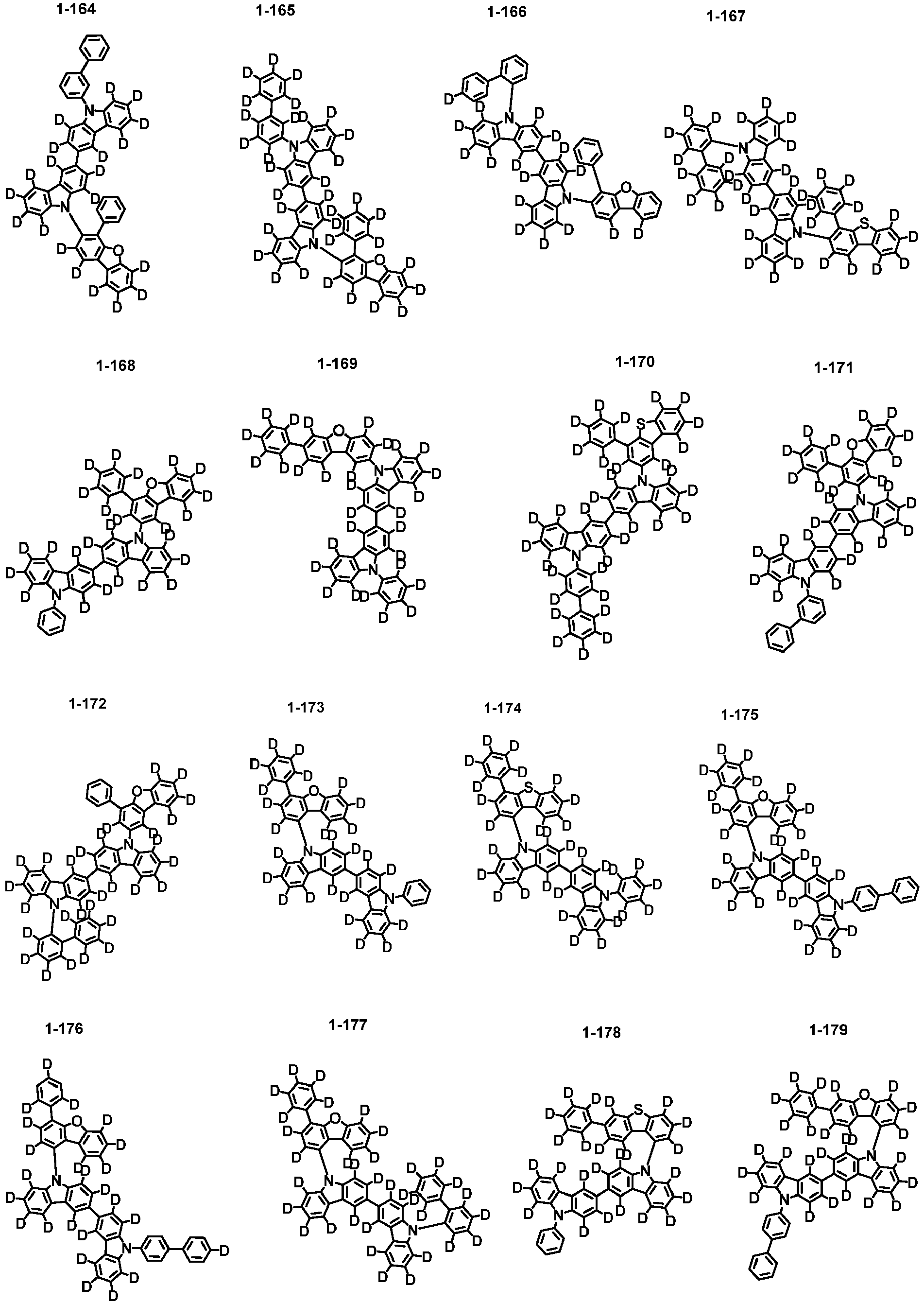

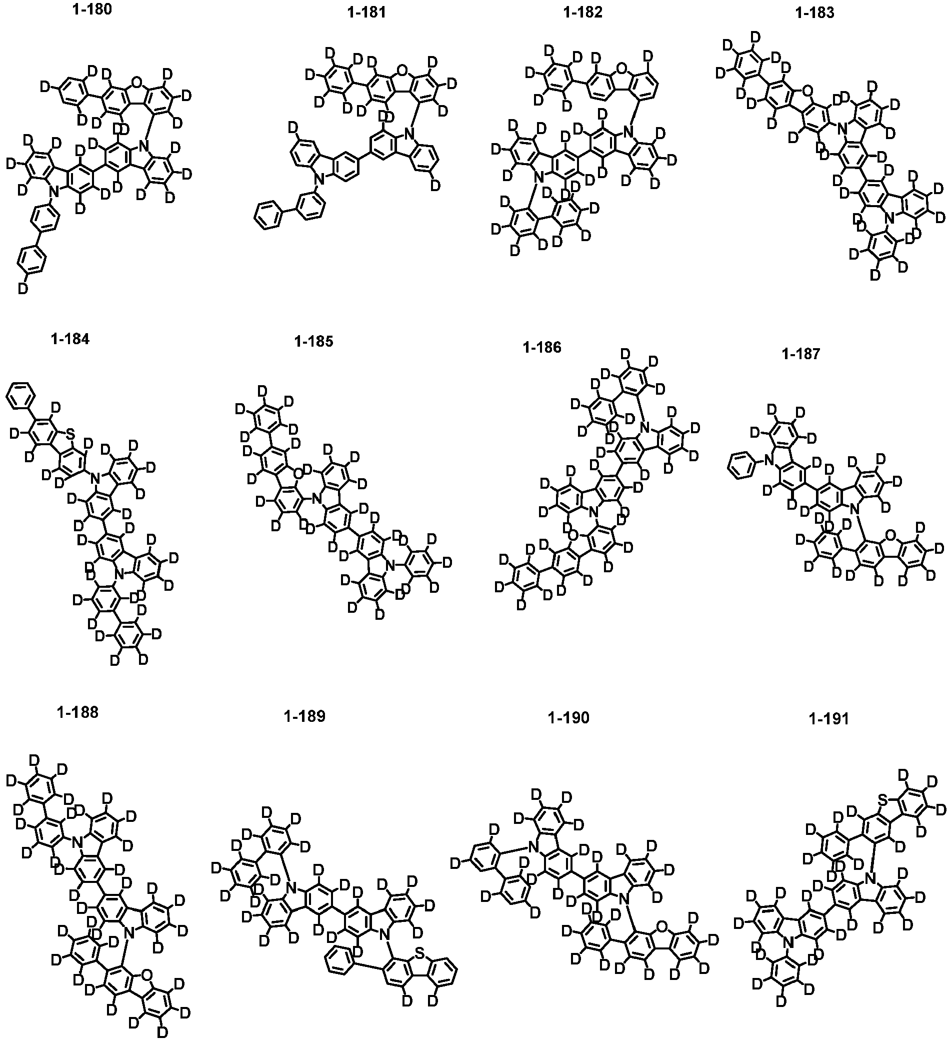

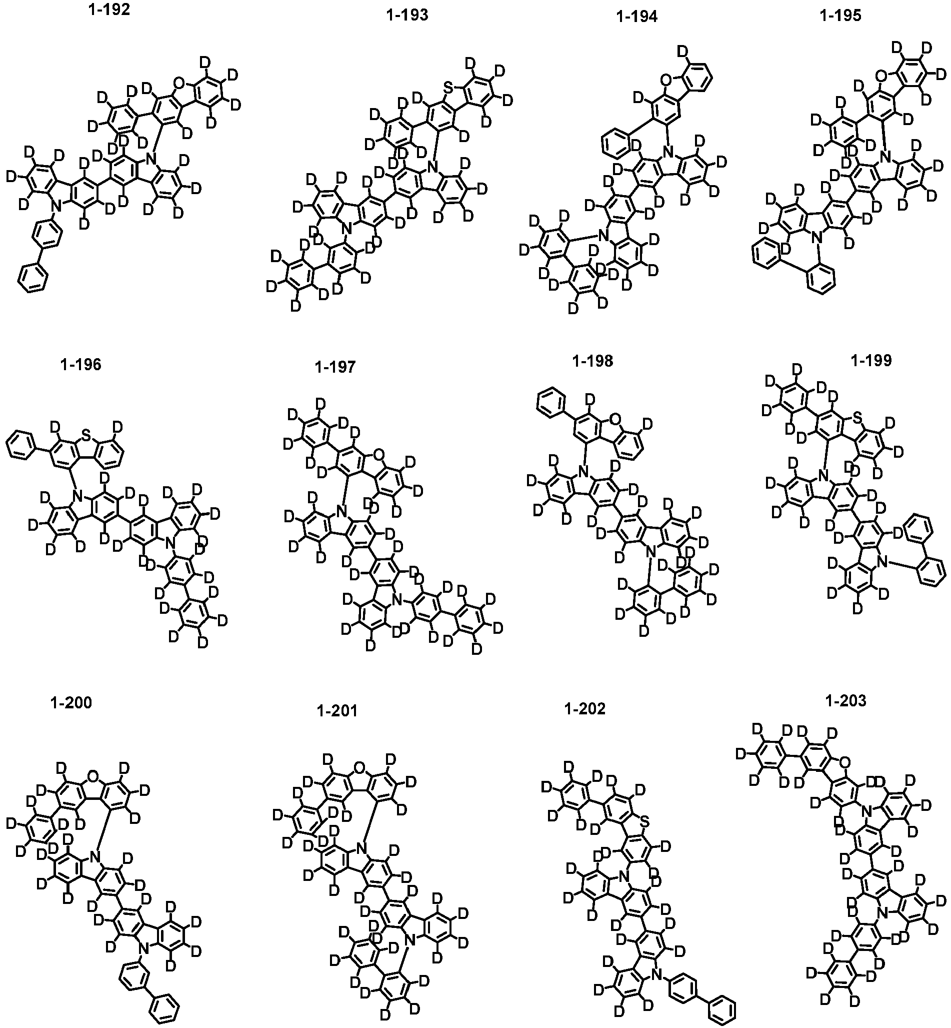

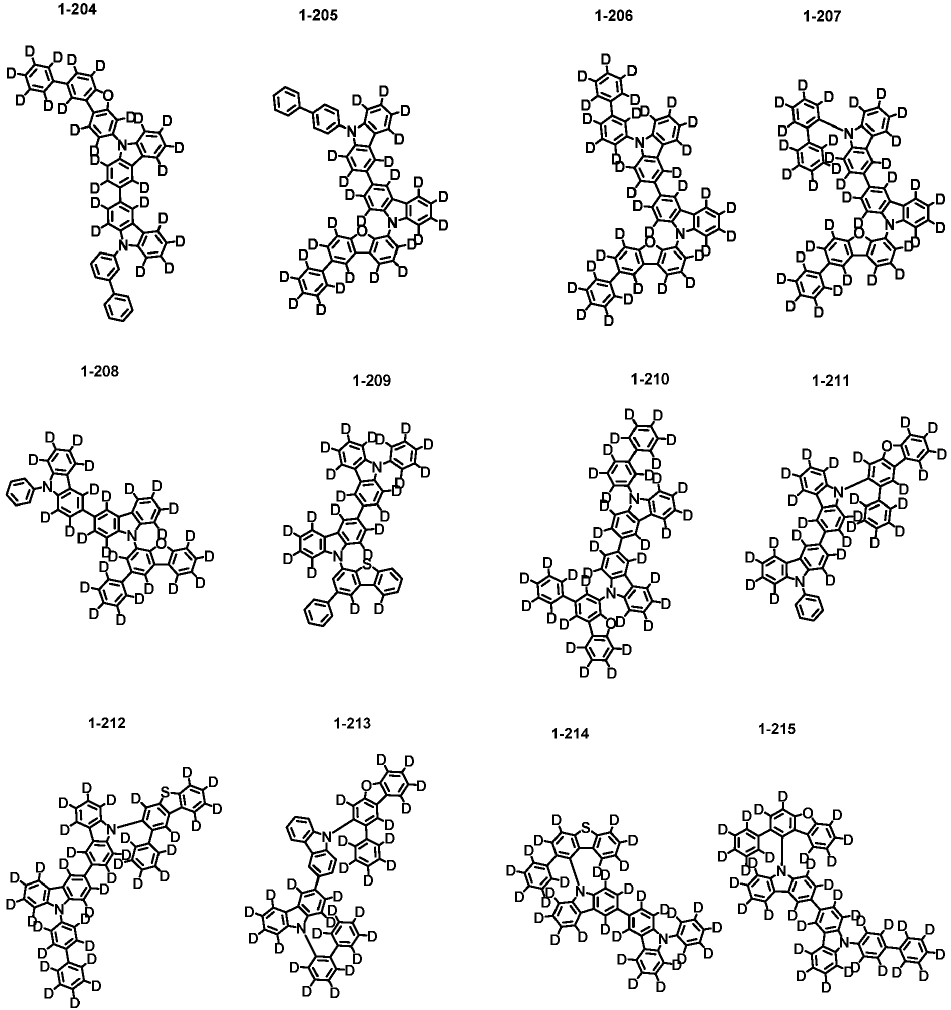

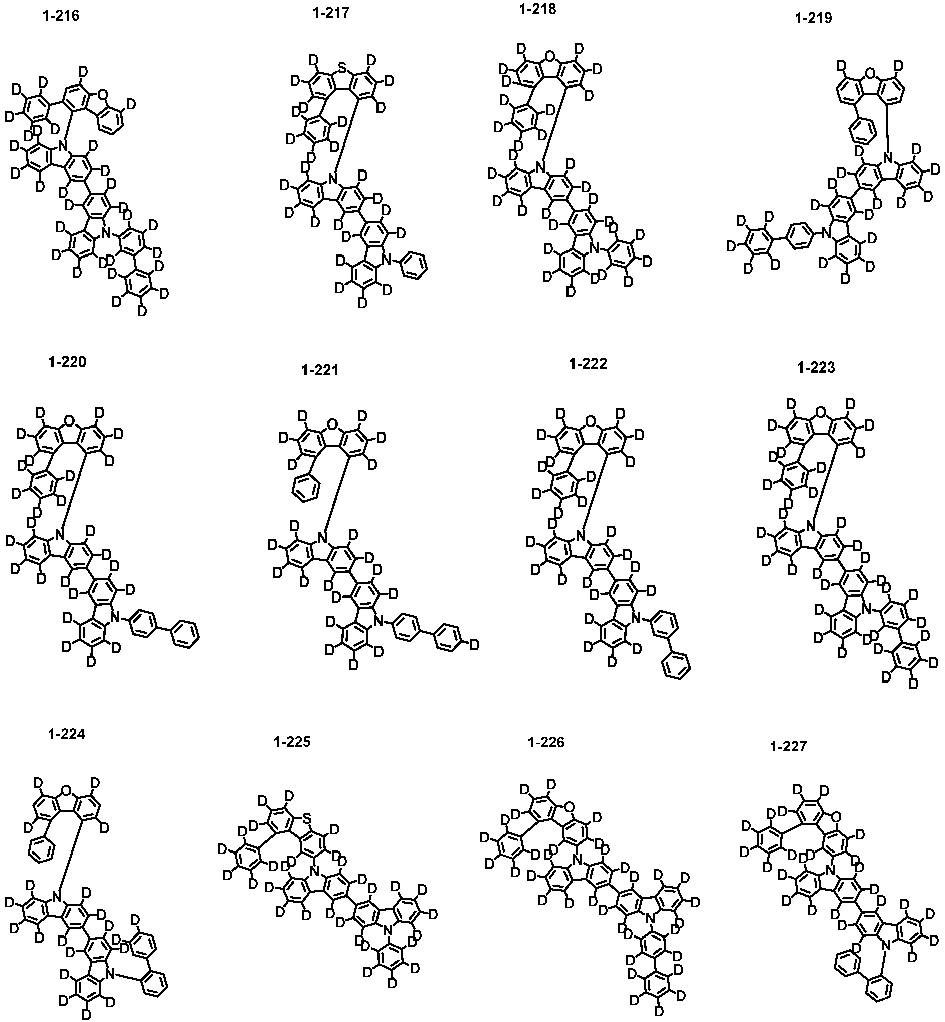

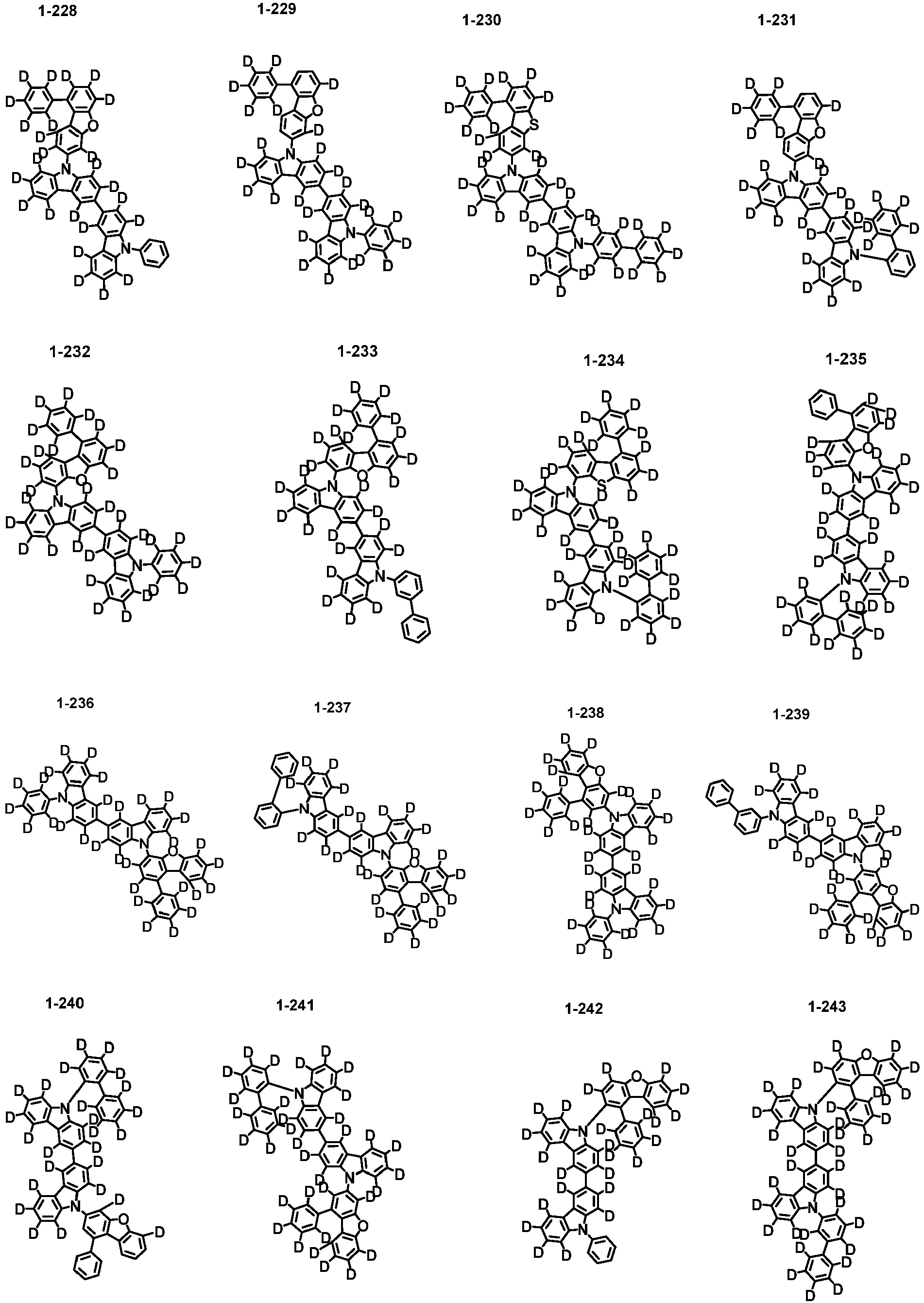

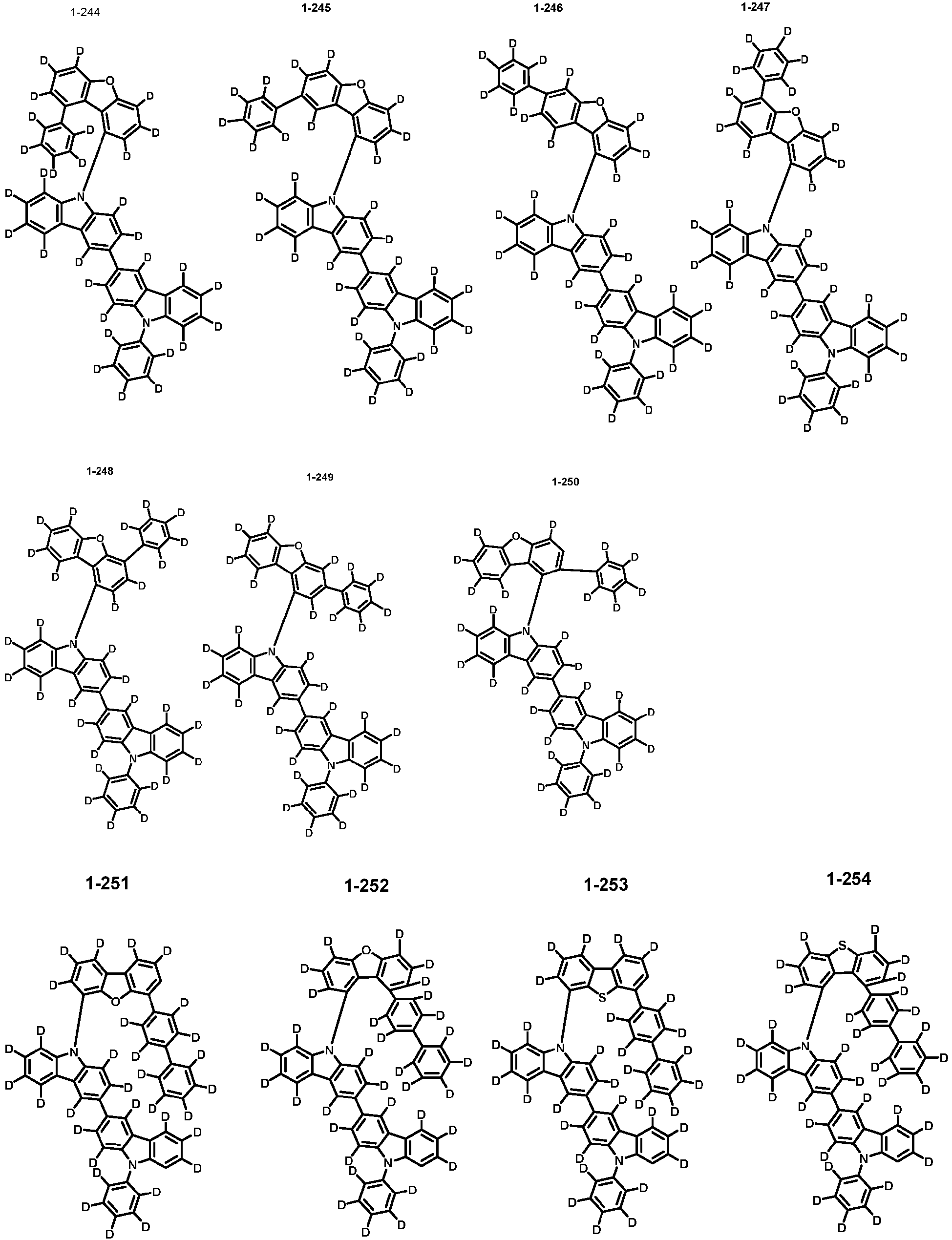

As a more specific example, the compound for an organic optoelectronic device according to the present invention may be represented by Chemical Formula 1-8a,

L¹ and L² may be a single bond or a substituted or unsubstituted phenylene group, and

Ar¹ and Ar² may each be a phenyl group substituted with deuterium, a biphenyl group substituted with deuterium, a terphenyl group substituted with deuterium, or a triphenylene group substituted with deuterium.

The second compound may be represented by a combination of Chemical Formula 2 and Chemical Formula 3.

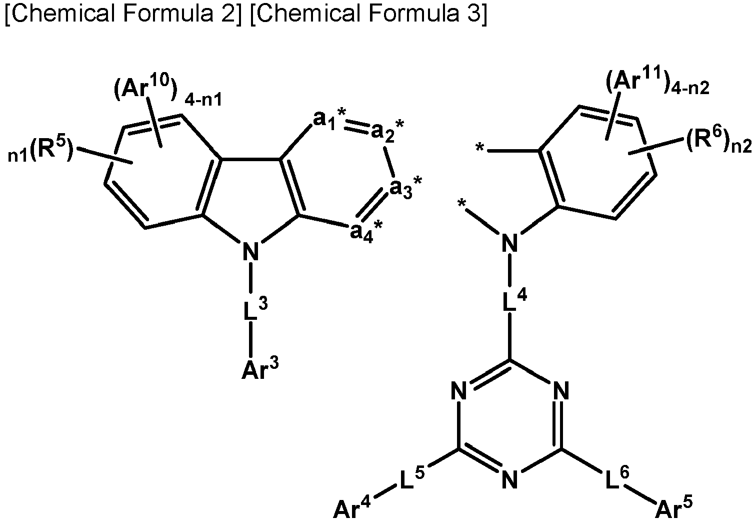

In Chemical Formula 2 and Chemical Formula 3,
Ar³ to Ar⁵ are each independently a substituted or unsubstituted C6 to C20 aryl group or a substituted or unsubstituted C2 to C30 heterocyclic group,
a₁* to a₄* in Chemical Formula 2 are each independently a linking carbon (C) or C-L^{a}-R^{a},
adjacent two of a₁* to a₄* in Chemical Formula 2 are each linked to * in Chemical Formula 3,
L^{a} and L³ to L⁶ are each independently a single bond, a substituted or unsubstituted C6 to C20 arylene group or a substituted or unsubstituted C2 to C20 heteroarylene group,
R^{a}, R⁵, and R⁶ are each independently hydrogen, deuterium, a cyano group, a halogen, a substituted or unsubstituted amino group, a substituted or unsubstituted C1 to C30 alkyl group, a substituted or unsubstituted C6 to C30 aryl group or a substituted or unsubstituted C2 to C30 heterocyclic group,
Ar¹⁰ and Ar¹¹ are each independently hydrogen or a substituted or unsubstituted C6 to C30 aryl group,
n1 and n2 are each independently an integer from 1 to 4, and
Chemical Formula 2 and Chemical Formula 3 satisfy at least one of the following conditions (iii) and (iv):
   (iii) at least one of Ar3 to Ar5 is a C6 to C20 aryl group substituted with at least one deuterium or a C2 to C30 heterocyclic group substituted with at least one deuterium, and
   (iv) at least one of R^{a}, R⁵, and R⁶ is deuterium.

The second compound is used with the first compound in a light emitting layer, and thereby charge mobility and stability are increased and luminous efficiency and life-span characteristics are improved.

In particular, the second compound represented by the combination of Chemical Formula 2 and Chemical Formula 3 has a structure in which indolocarbazole is a basic skeleton, and a benzene moiety of indolocarbazole is substituted with at least one deuterium, or at least one of the N-direction substituents Ar³ to Ar⁵ of indolocarbazole is substituted with deuterium.

As the benzene moiety of indolocarbazole or the N-direction substituent of indolocarbazole is substituted with deuterium, a zero-point energy and vibration energy of the compound may be further lowered. As a result, an energy of the ground state is further lowered and the interaction between molecules is weakened, and thus it possible to make the thin film formed from this in an amorphous state, which improves heat resistance and effectively improves life-span. In other words, if this is applied, it is possible to implement an organic light emitting diode with low driving, high efficiency, and especially long life-span.

As an example, the second compound may be represented by any one of Chemical Formula 2A, Chemical Formula 2B, Chemical Formula 2C, Chemical Formula 2D, Chemical Formula 2E and Chemical Formula 2F. 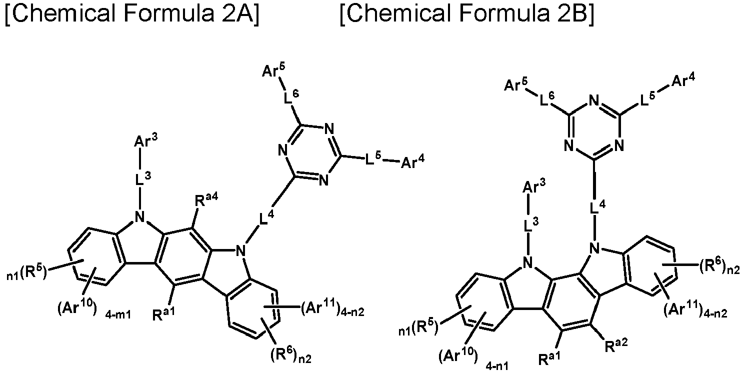 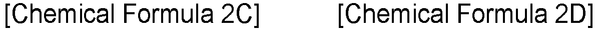 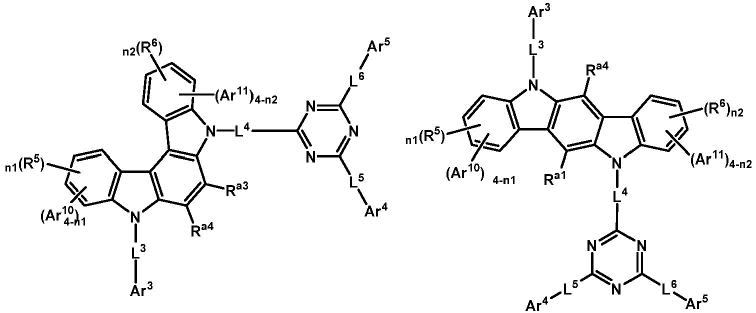 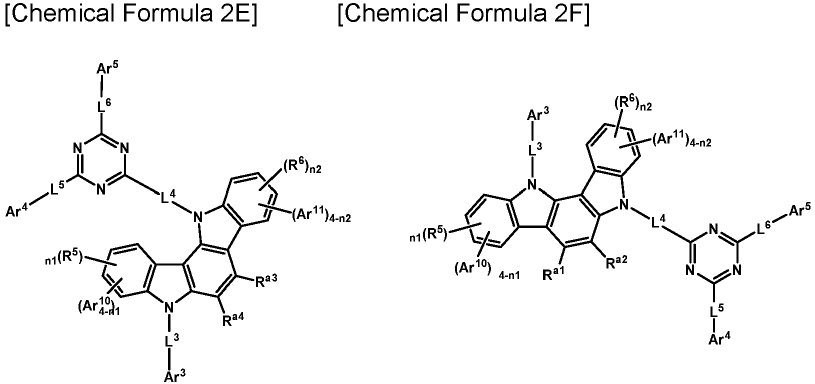
In Chemical Formula 2A to Chemical Formula 2F, Ar³ to Ar⁵, Ar¹⁰, Ar¹¹, L³ to L⁶, R' to R⁴, n1, and n2 are the same as described above,
L^{a1} to L^{a4} are the same as the definitions of L³ to L⁶ described above, and
R^{a1} to R^{a4} are each the same as the definition of R^{a} described above.

For example, at least one of R^{a1} to R^{a4}, R⁵ and R⁶ may be deuterium.

For example, Ar¹⁰ and Ar¹¹ may each independently be hydrogen or a C6 to C20 aryl group unsubstituted or substituted with at least one deuterium.

For example, Ar¹⁰ and Ar¹¹ may each independently be hydrogen or a phenyl group substituted or unsubstituted with deuterium, a biphenyl group substituted or unsubstituted with deuterium, a terphenyl group substituted or unsubstituted with deuterium, a naphthyl group substituted or unsubstituted with deuterium, a phenanthrenyl group substituted or unsubstituted with deuterium, an anthracenyl group substituted or unsubstituted with deuterium, a triphenylene group substituted or unsubstituted with deuterium, or a fluorenyl group substituted or unsubstituted with deuterium.

As an example, at least one of Ar³ to Ar⁵ may be a C6 to C20 aryl group substituted with at least one deuterium or a C2 to C30 heterocyclic group substituted with at least one deuterium.

As an example, at least one of R^{a1} to R^{a4}, R⁵ and R⁶ may be deuterium, and at least one of Ar³ to Ar⁵ may be a C6 to C20 aryl group substituted with at least one deuterium or a C2 to C30 heterocycle substituted with at least one deuterium.

For example, at least one of Ar³ to Ar⁵ may be a phenyl group substituted with at least one deuterium, a biphenyl group substituted with at least one deuterium, a terphenyl group substituted with at least one deuterium, a naphthyl group substituted with at least one deuterium, an anthracenyl group substituted with at least one deuterium, a phenanthrenyl group substituted with at least one deuterium, a triphenylene group substituted with at least one deuterium, a fluorenyl group substituted with at least one deuterium, a carbazolyl group substituted with at least one deuterium, a dibenzofuranyl group substituted with at least one deuterium, or a dibenzothiophenyl group substituted with at least one deuterium.

In a specific embodiment of the present invention, L³-Ar³, L⁵-Ar⁴, and L⁶-Ar⁵ of Chemical Formula 2 and Chemical Formula 3 may each independently be selected from the substituents listed in Group II-1 and Group II-2, and at least one of L³-Ar³, L⁵-Ar⁴, and L⁶-Ar⁵ may be selected from substituents listed in Group II-2.

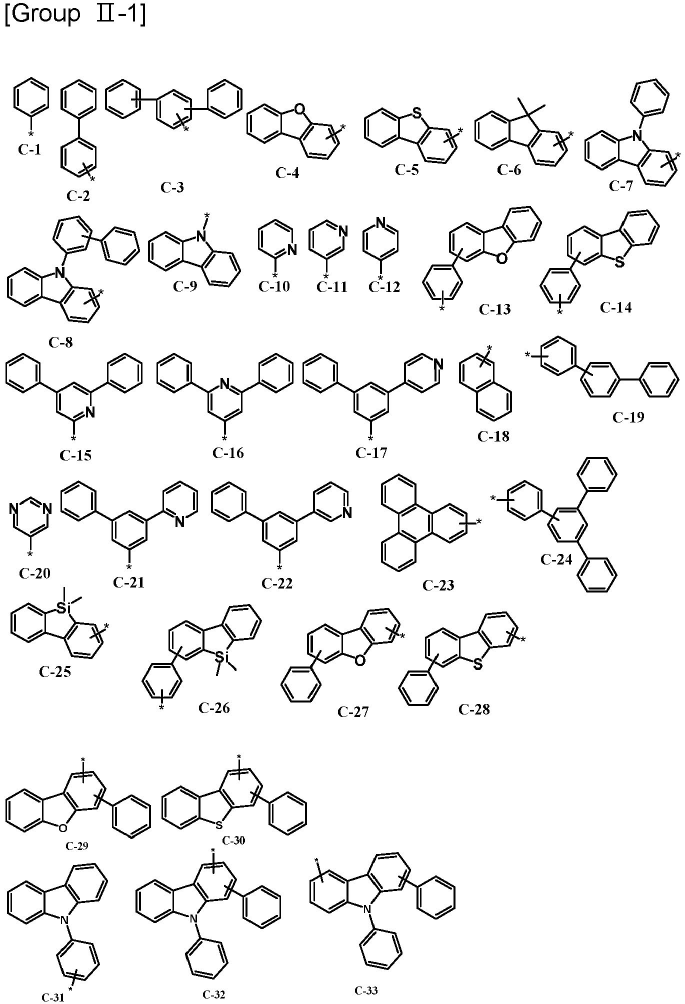

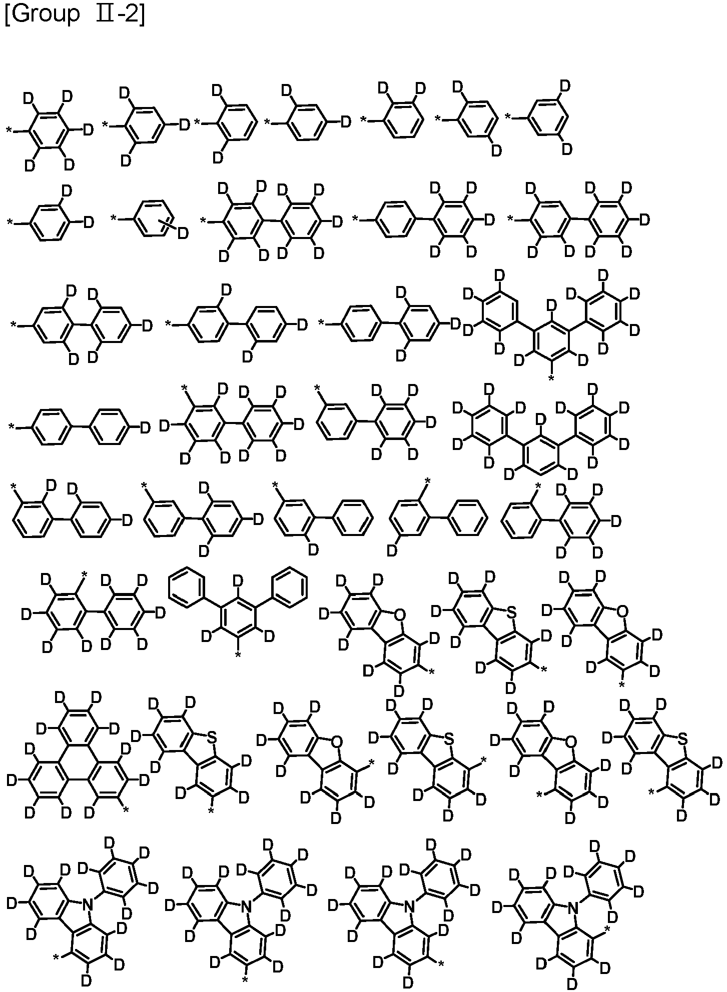

In Group II-1 and Group II-22, * is a linking point.

In a more specific embodiment of the present invention, the second compound may be represented by Chemical Formula 2B-a.

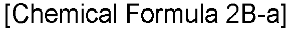

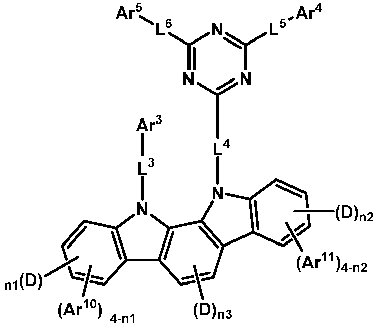

In Chemical Formula 2B-a,
Ar³ to Ar⁵, Ar¹⁰, Ar¹¹, and L³ to L⁶ are the same as described above,
n1 and n2 are each independently an integer from 1 to 4, and
n3 is an integer of 1 or 2.

For example, Ar³ to Ar⁵ may each independently be a substituted or unsubstituted phenyl group or a substituted or unsubstituted biphenyl group.

For example, L³ to L⁶ may each independently be a single bond or a substituted or unsubstituted phenylene group.

For example, the second compound may be one selected from the compounds of Group 2, but is not limited thereto.

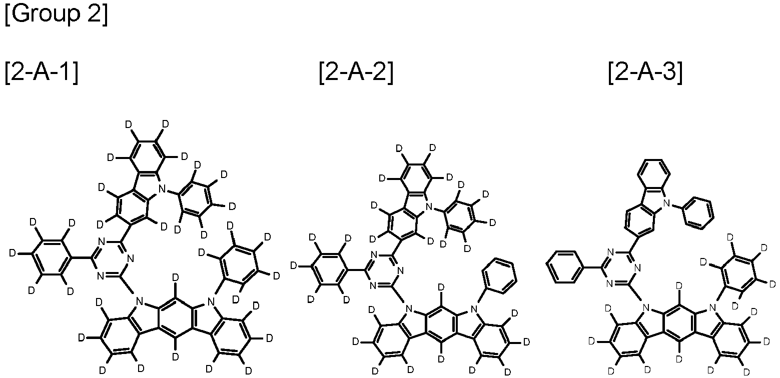

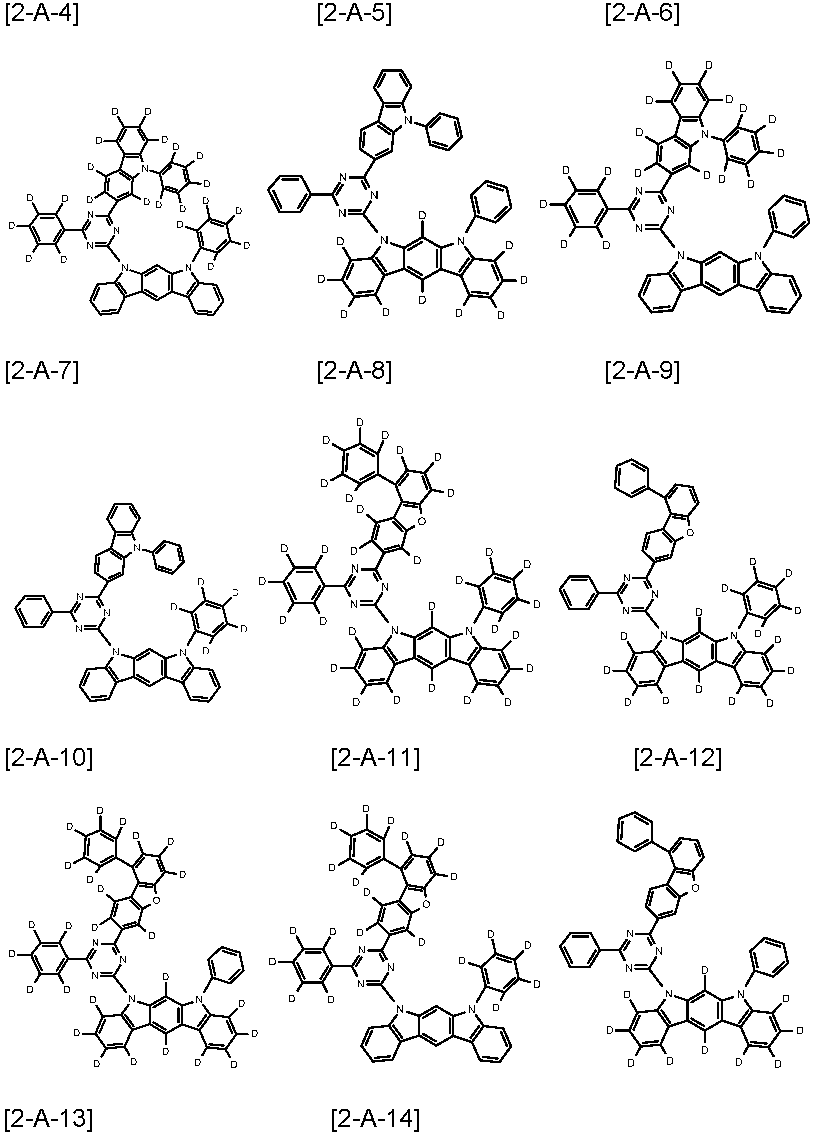

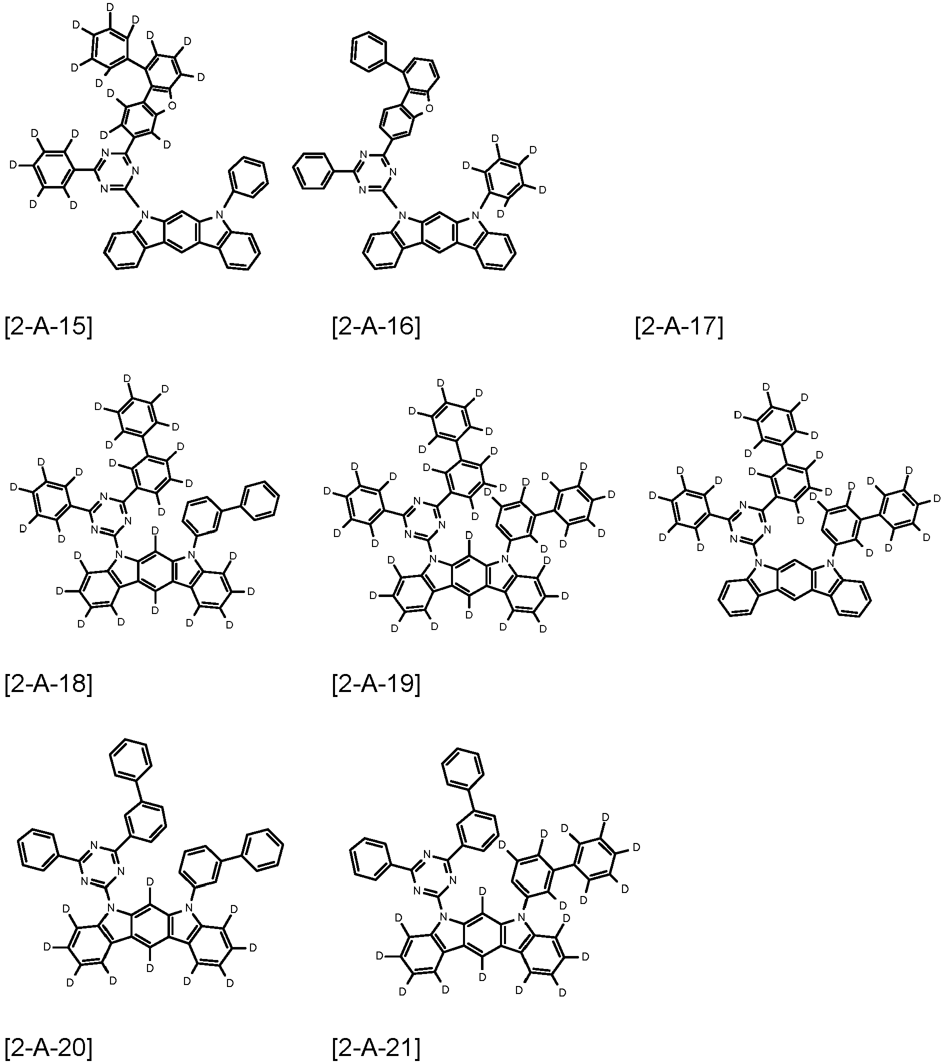

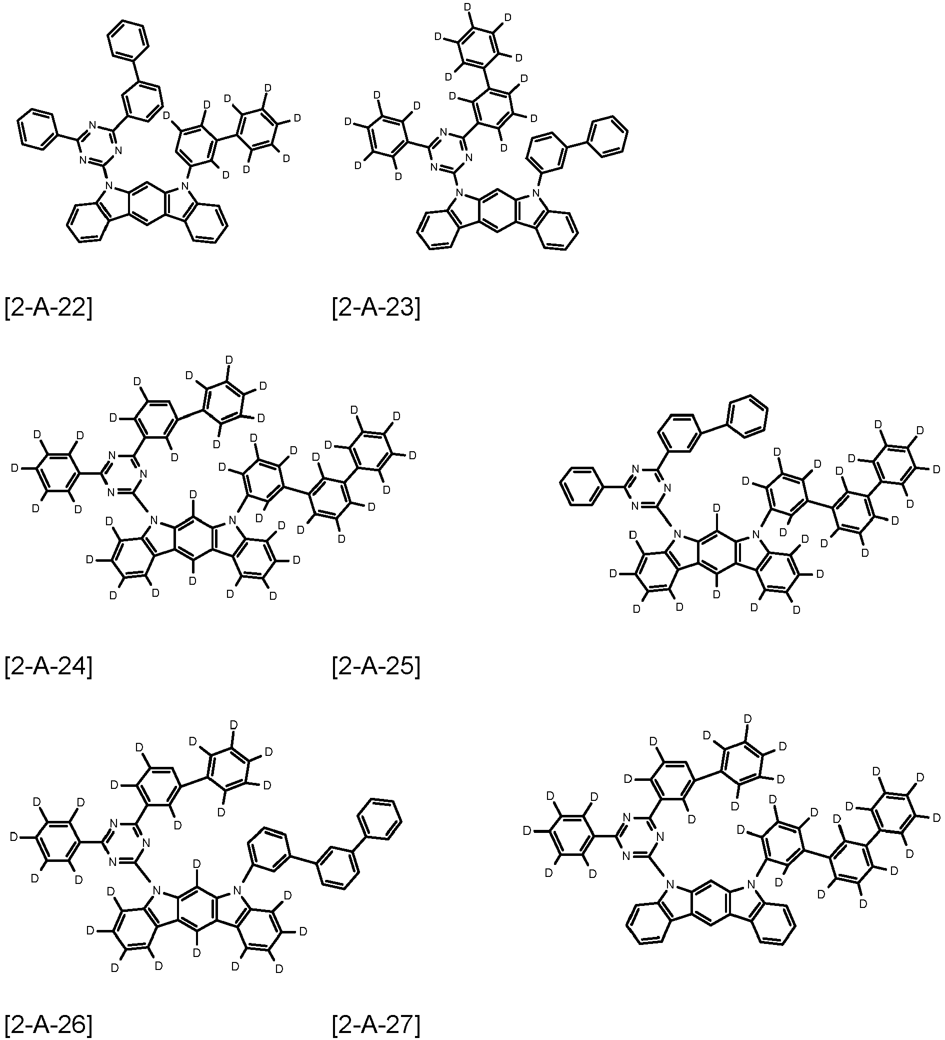

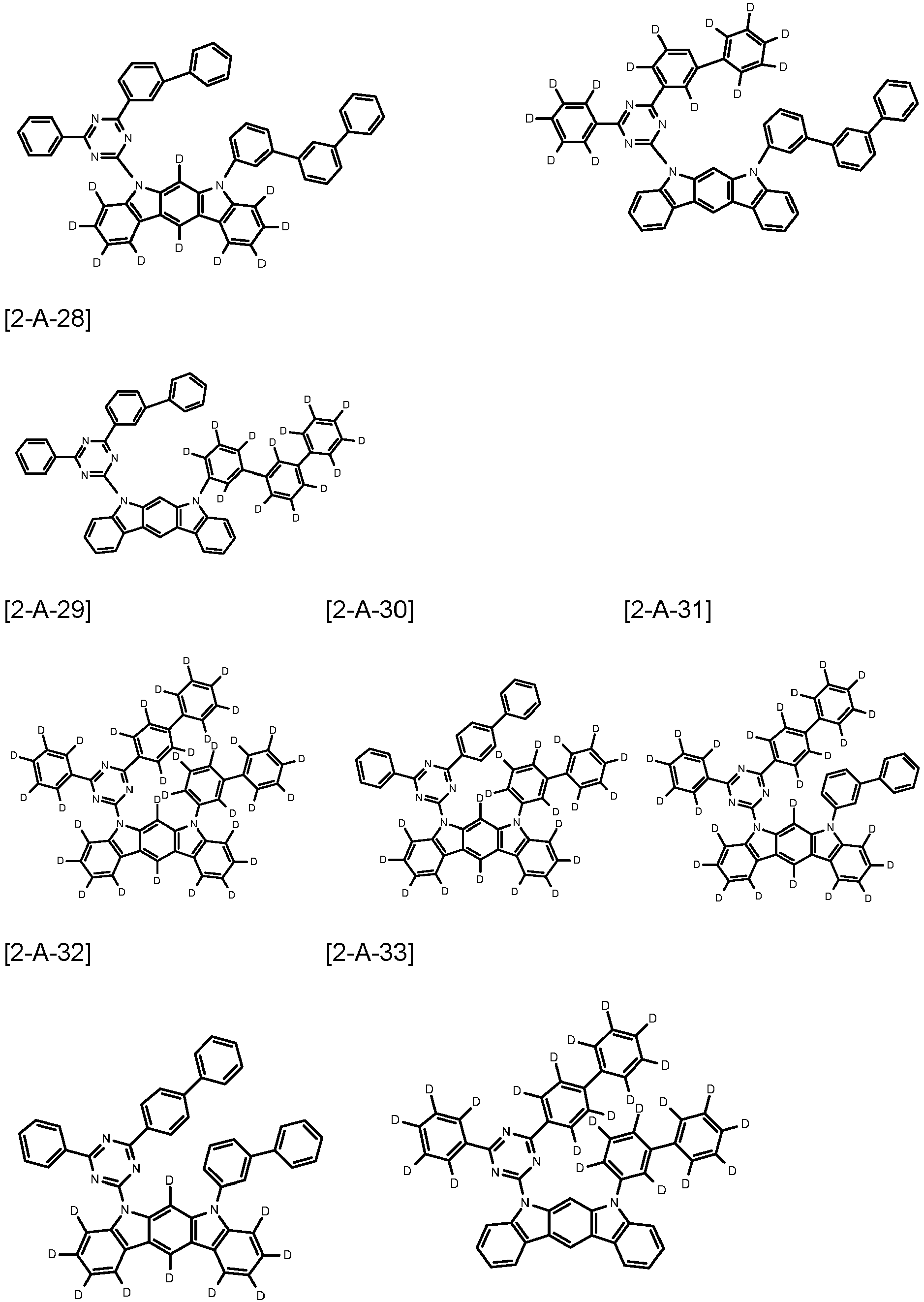

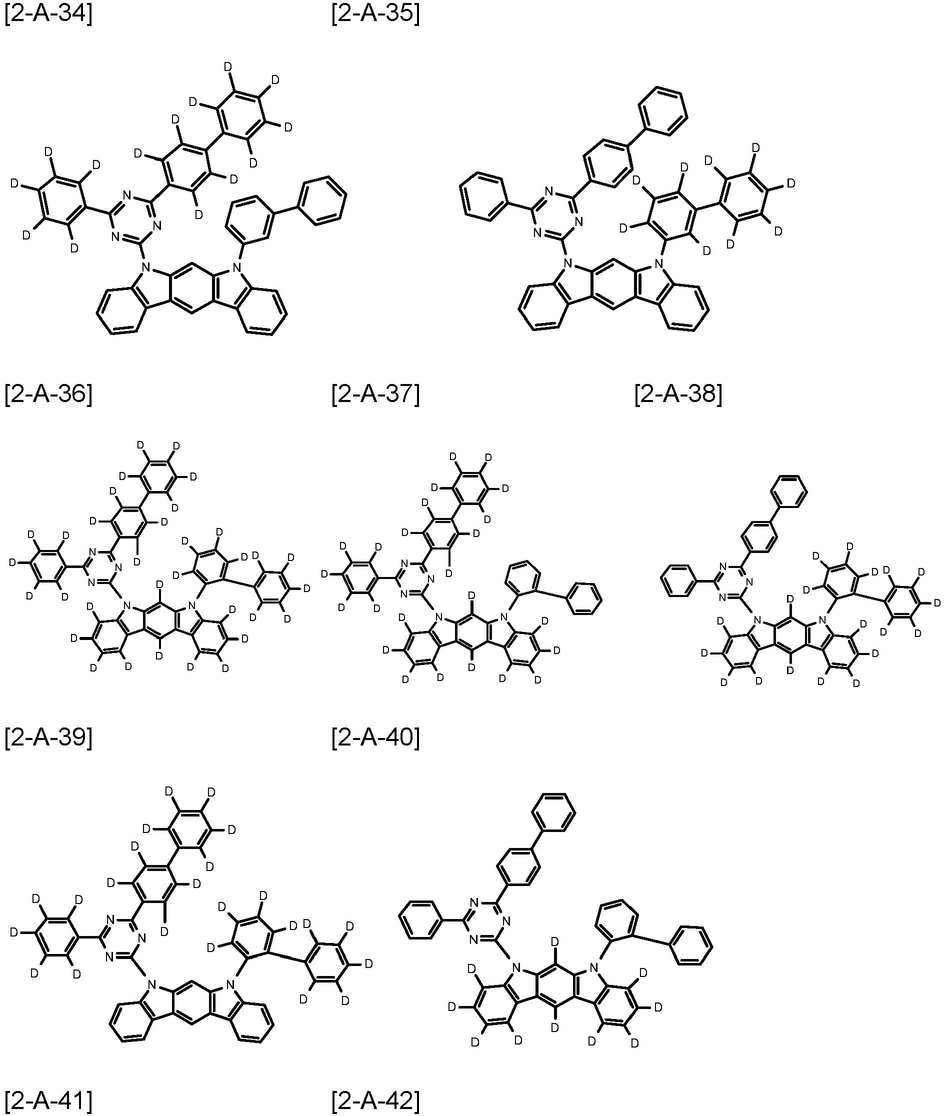

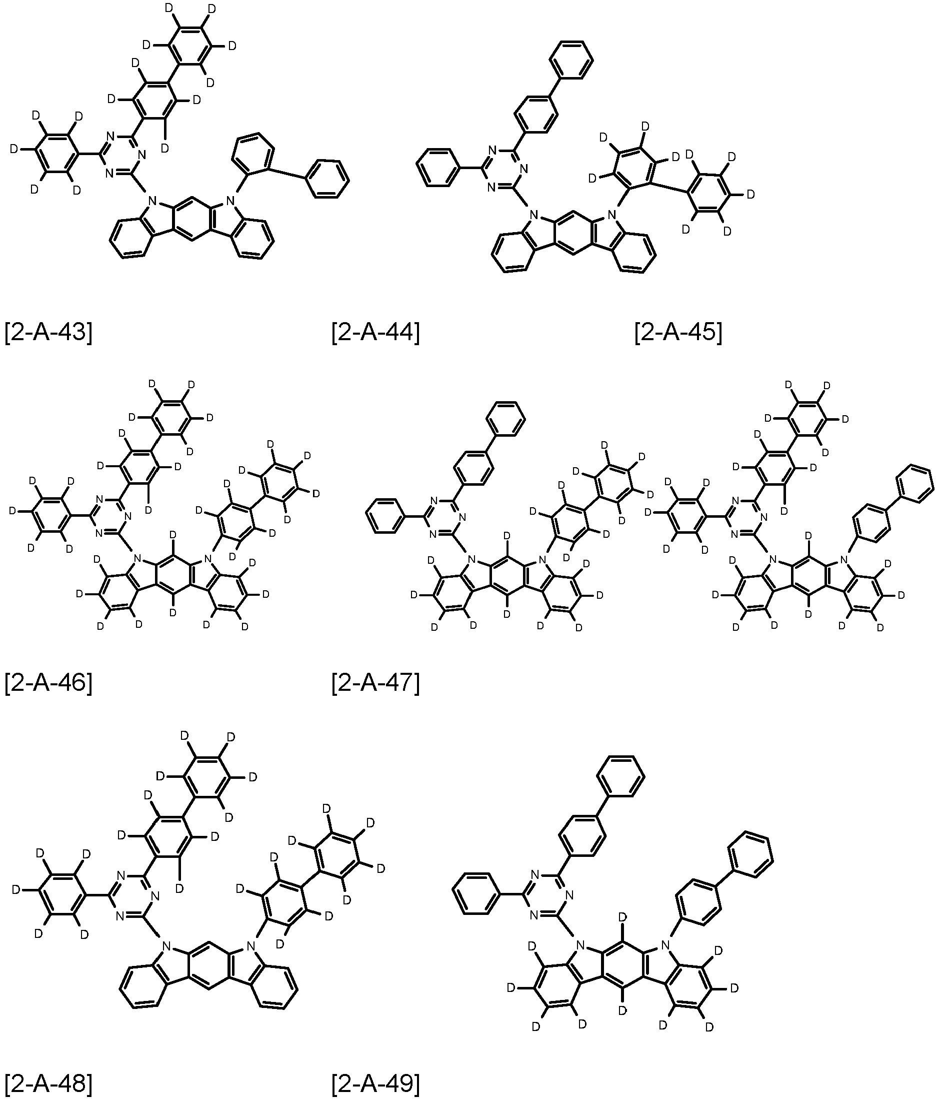

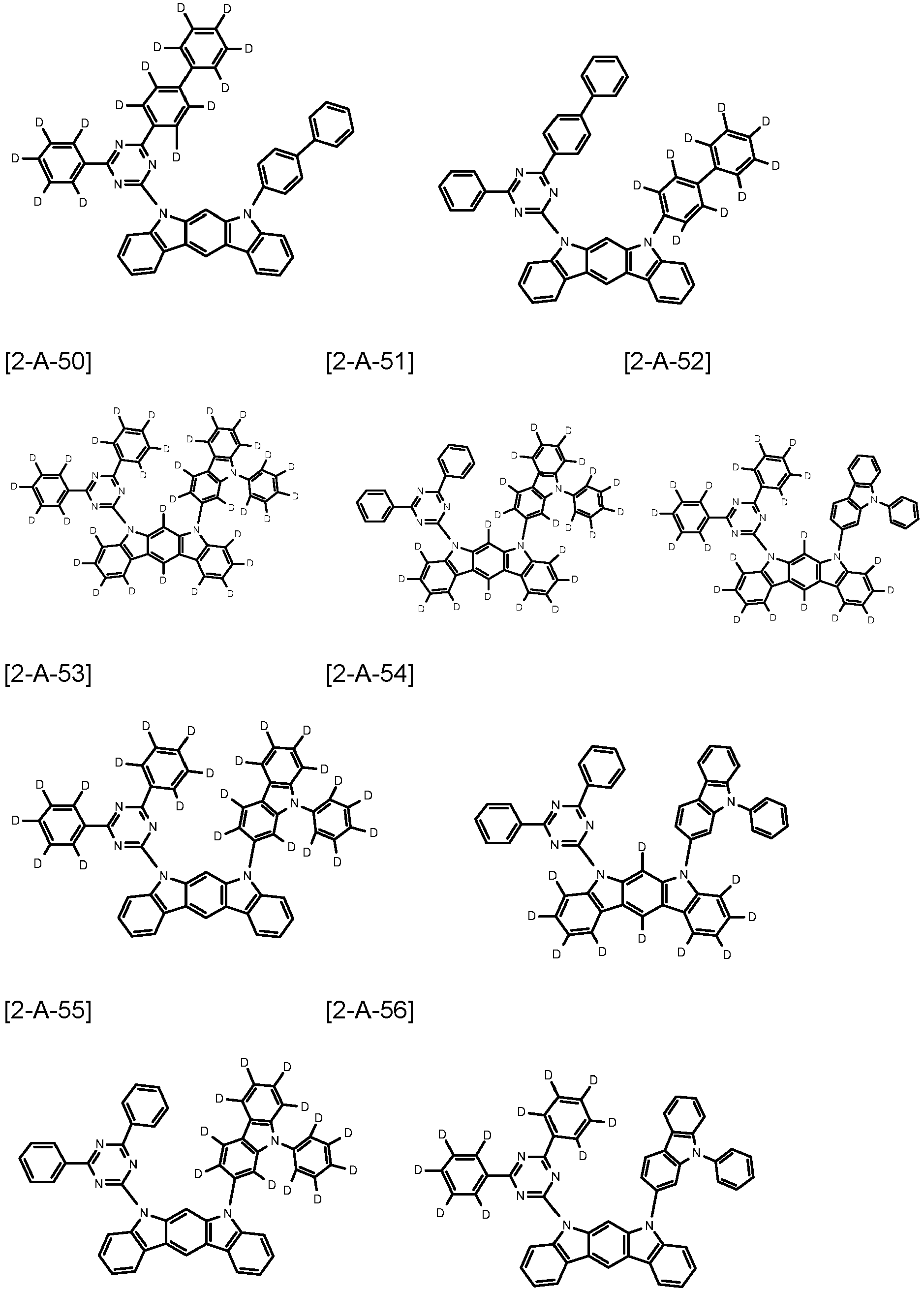

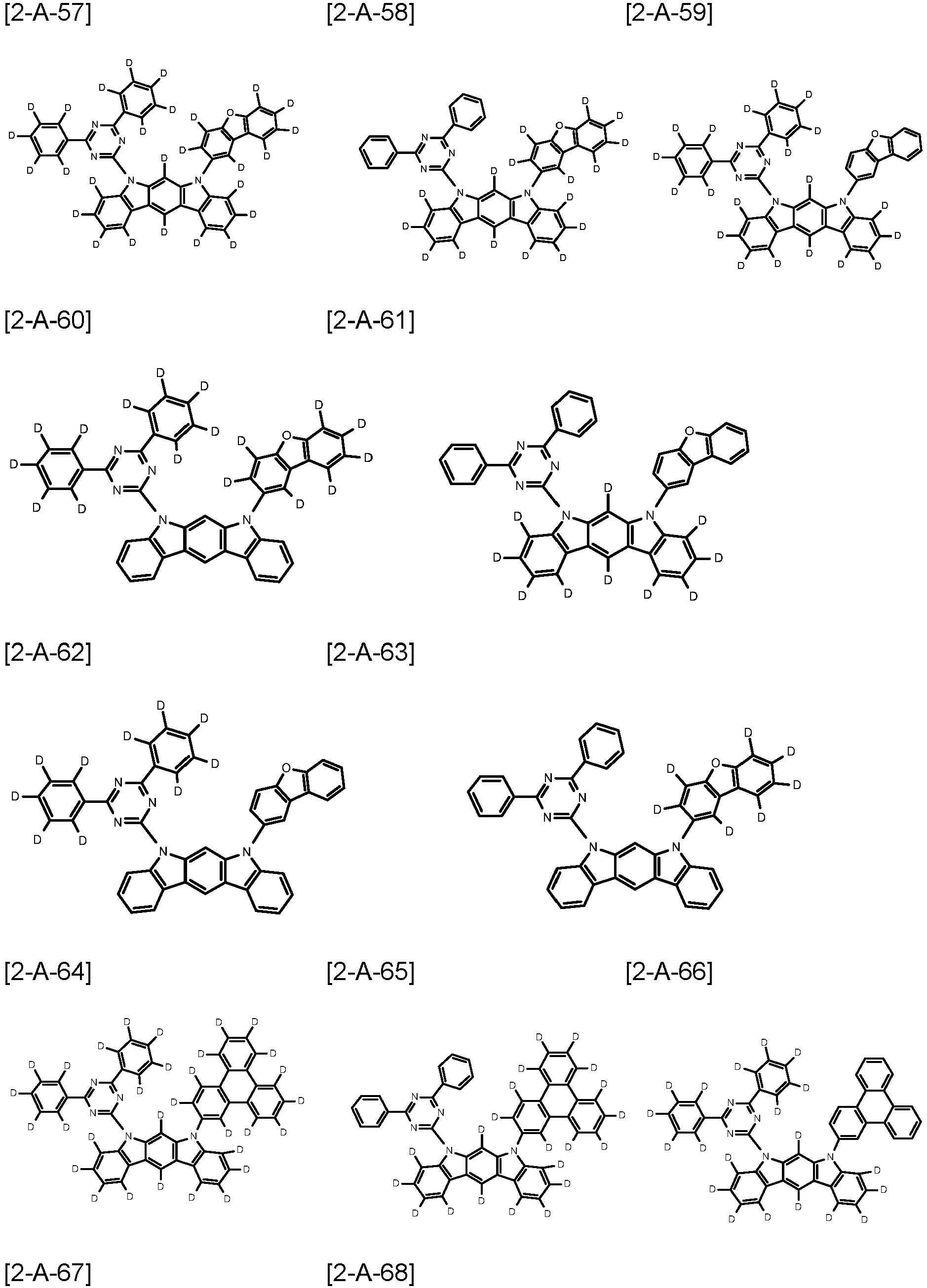

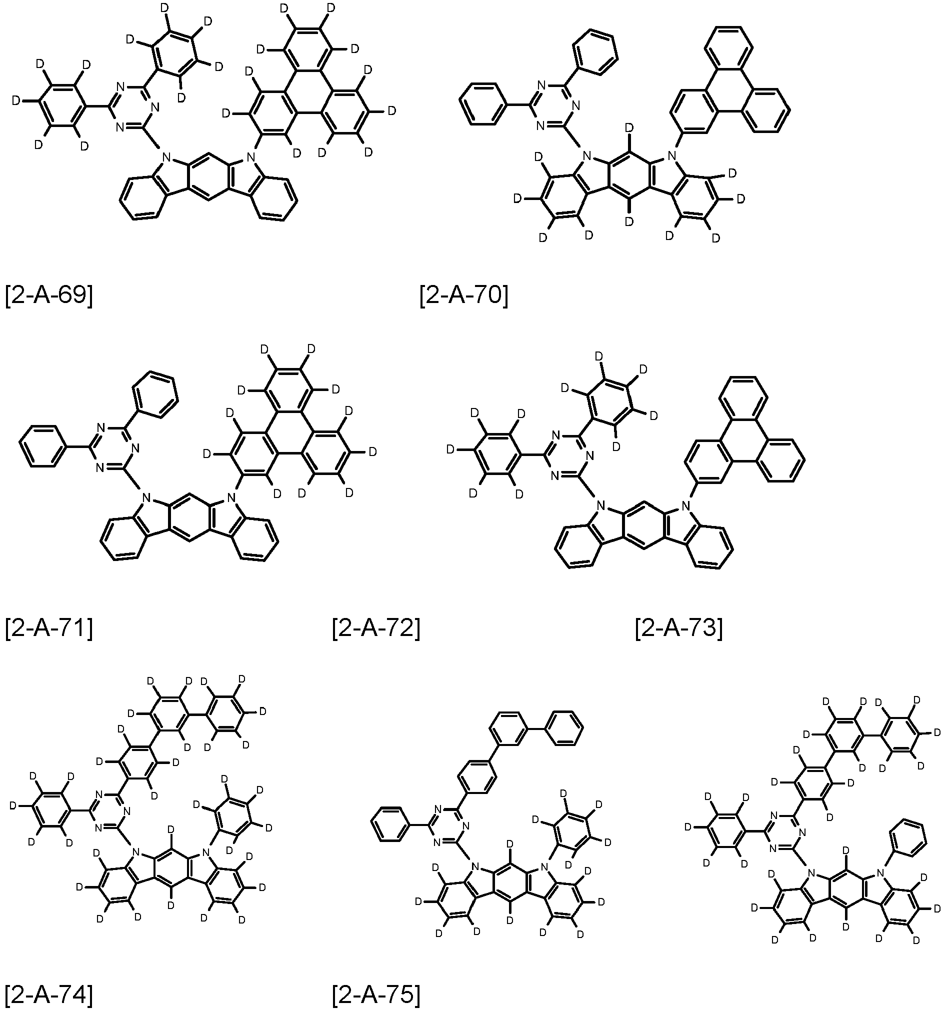

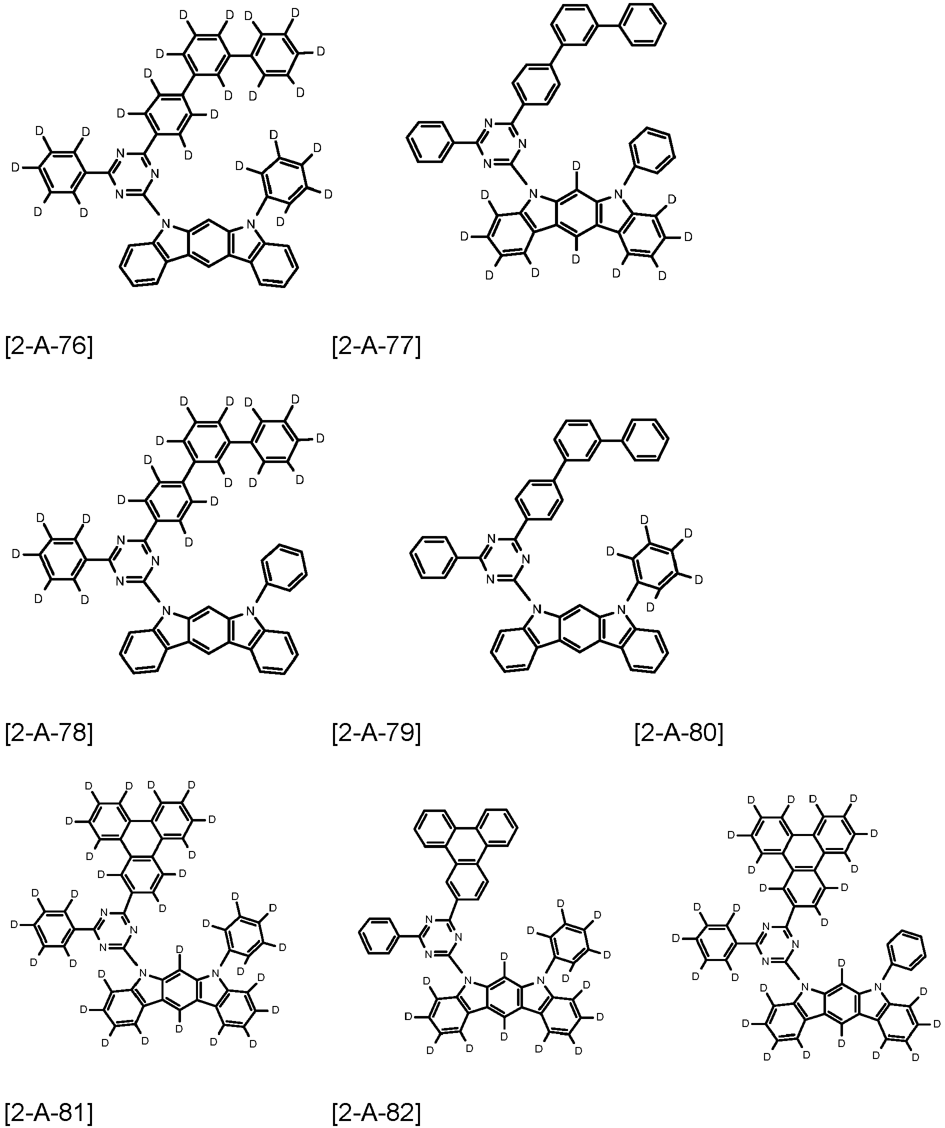

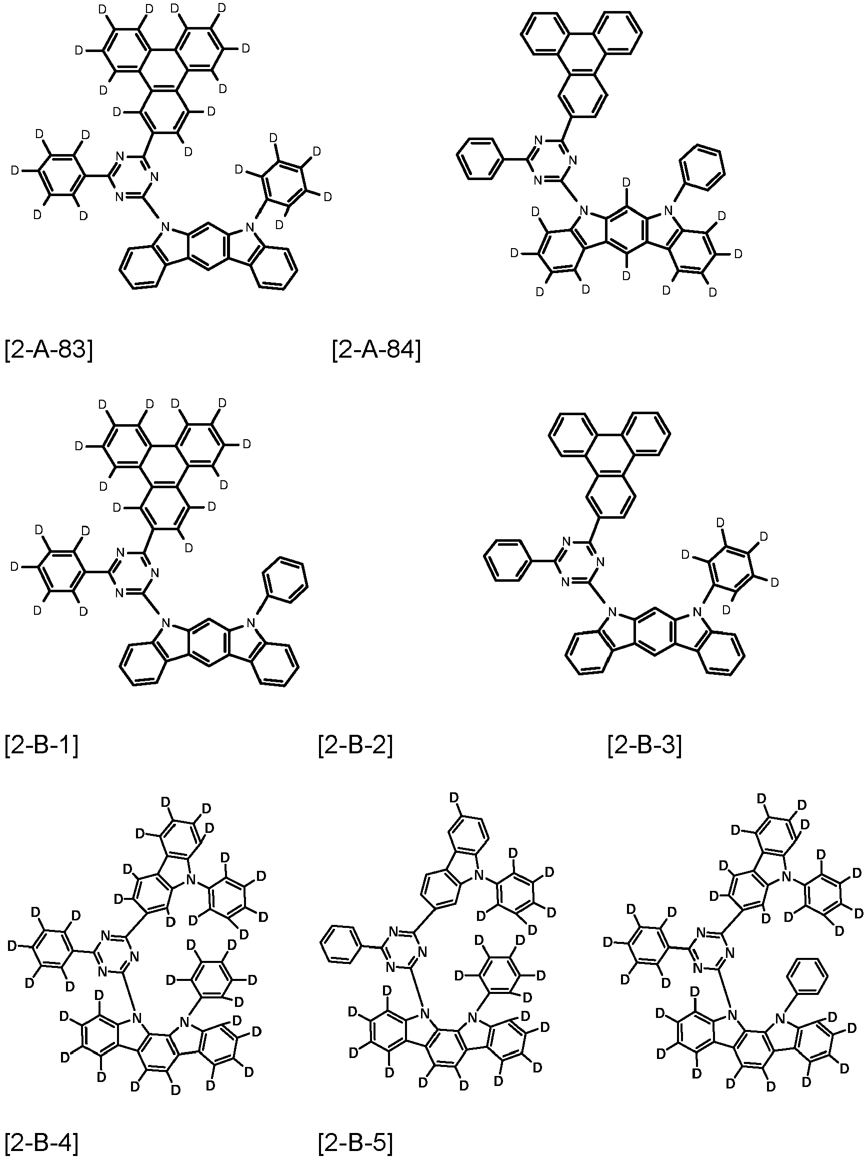

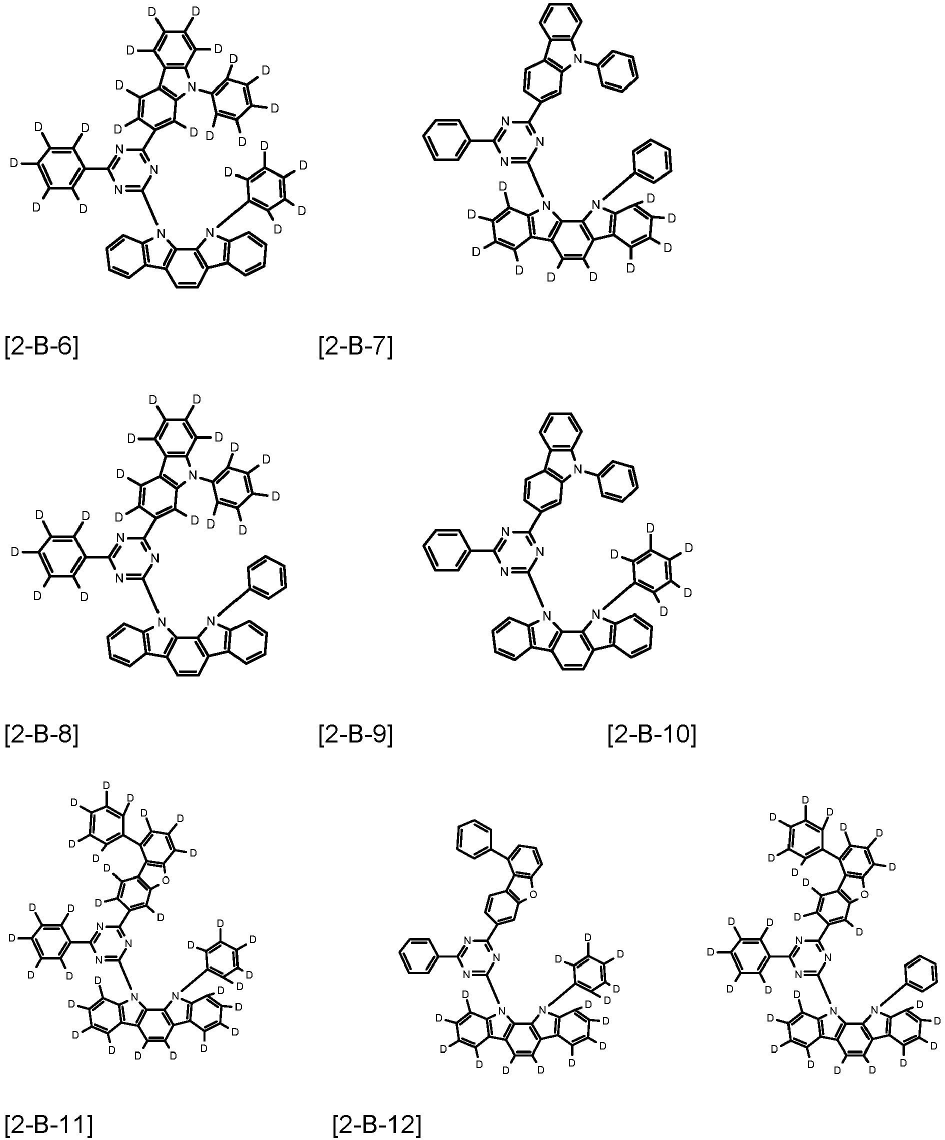

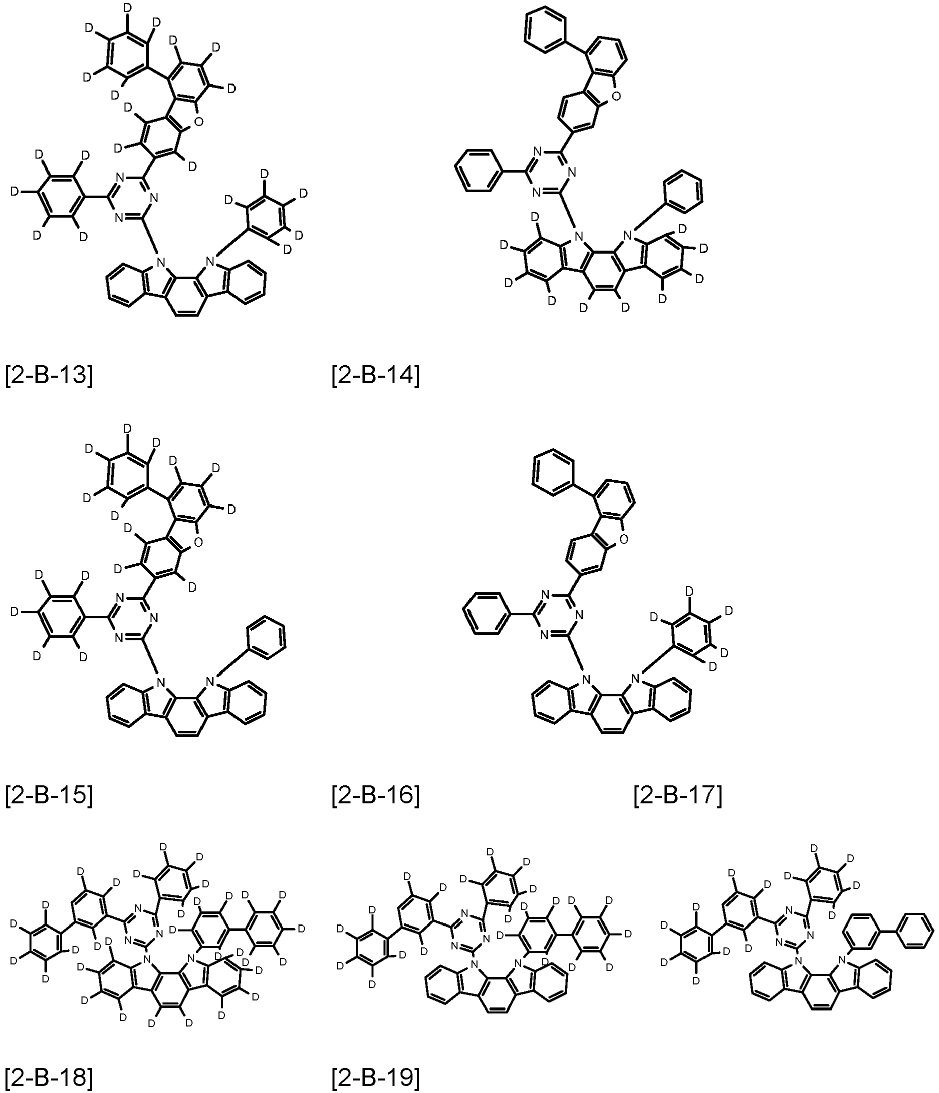

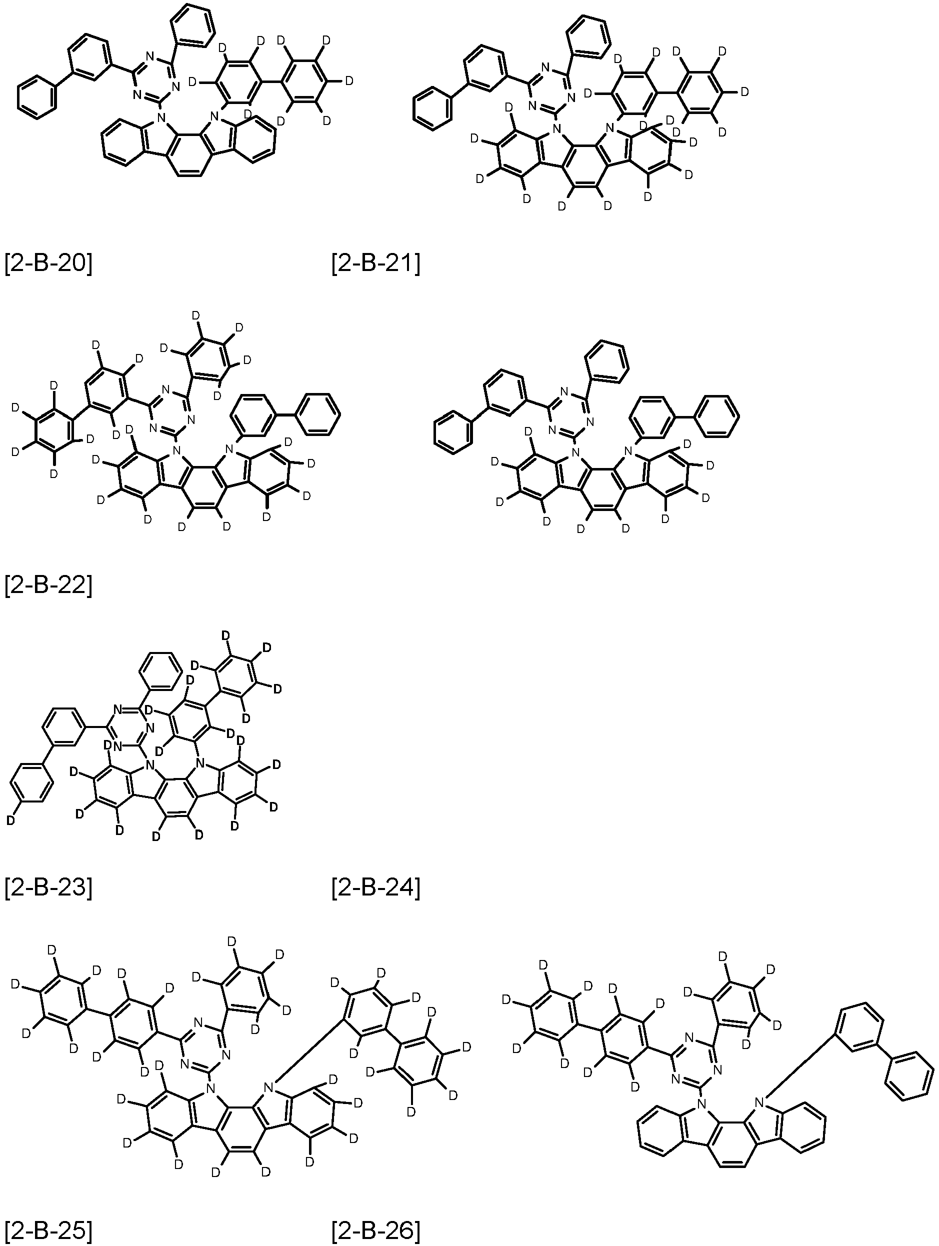

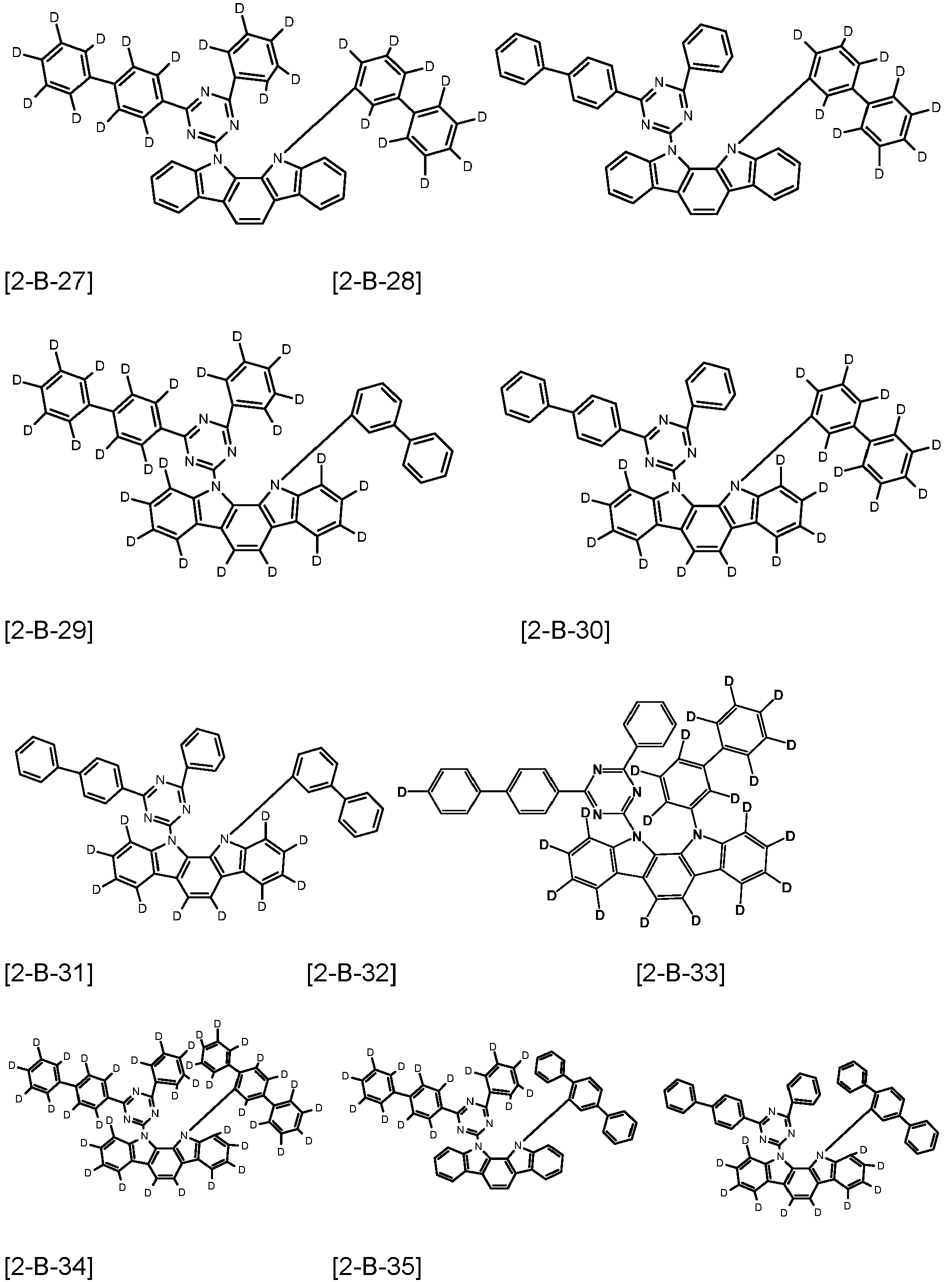

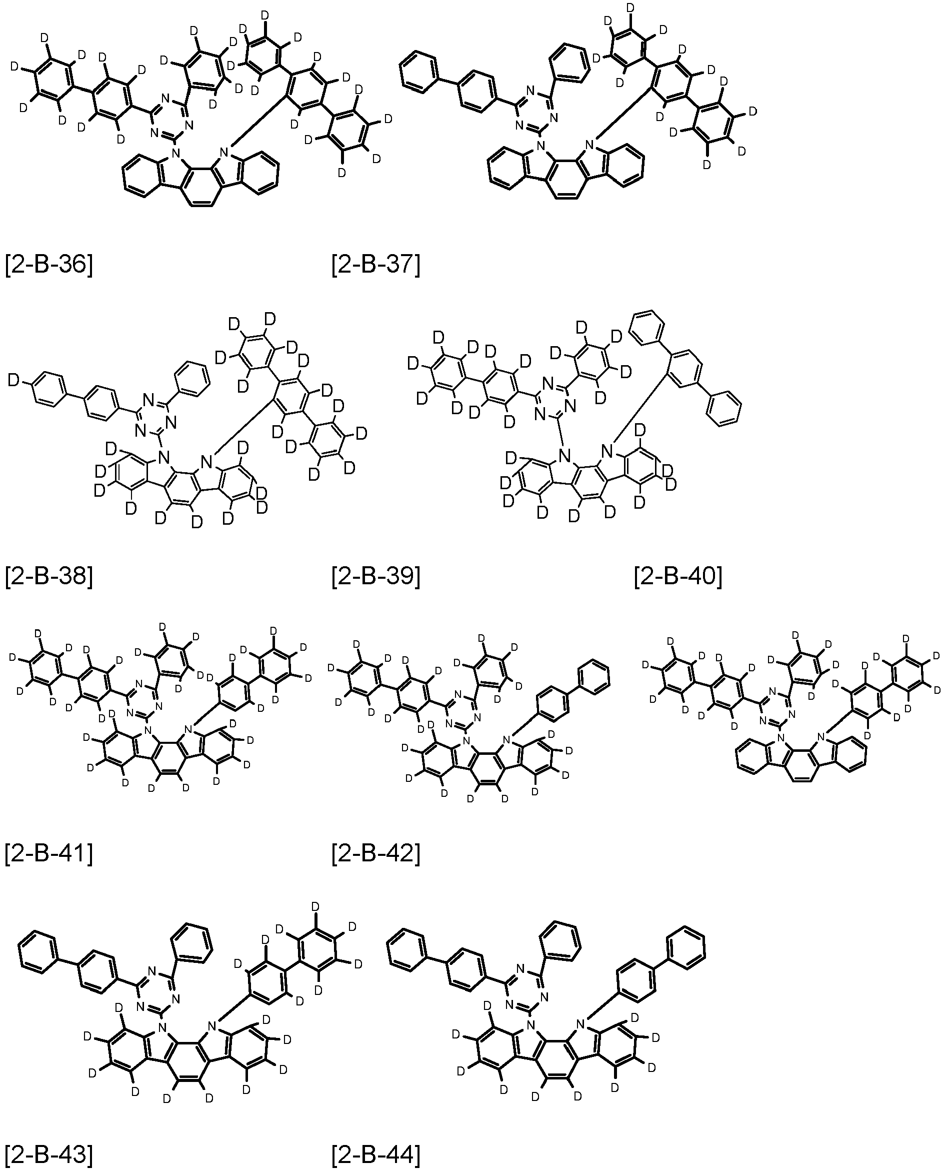

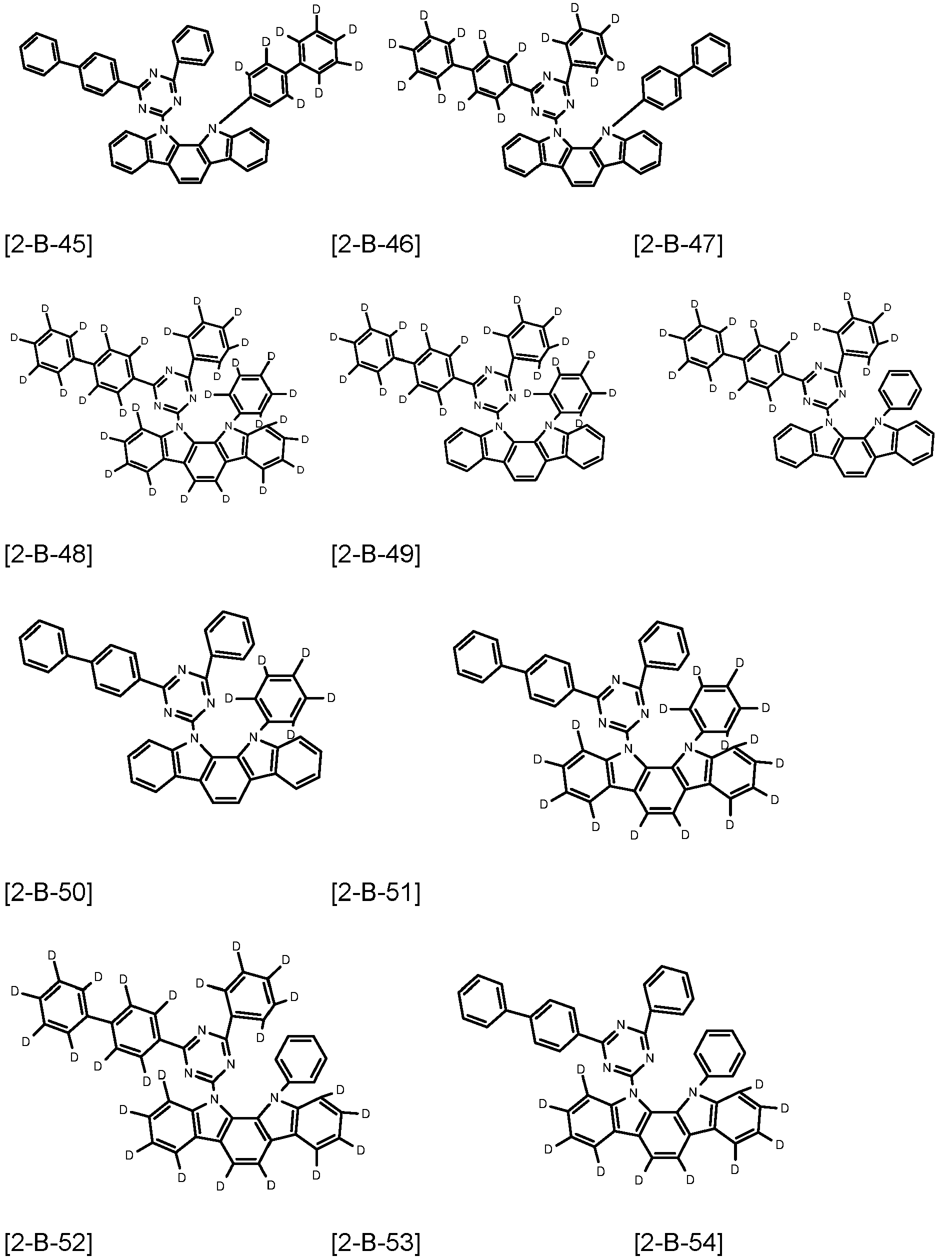

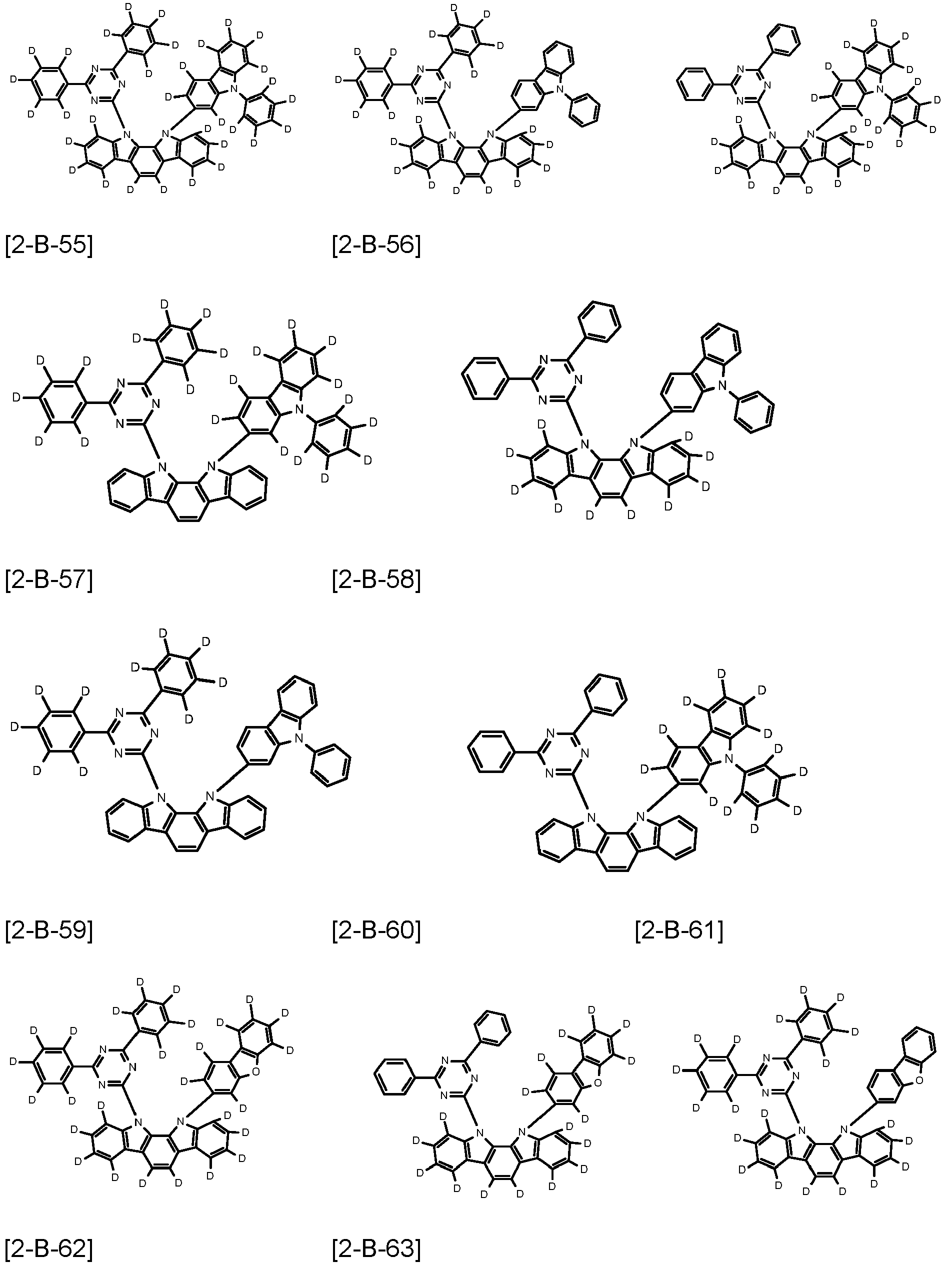

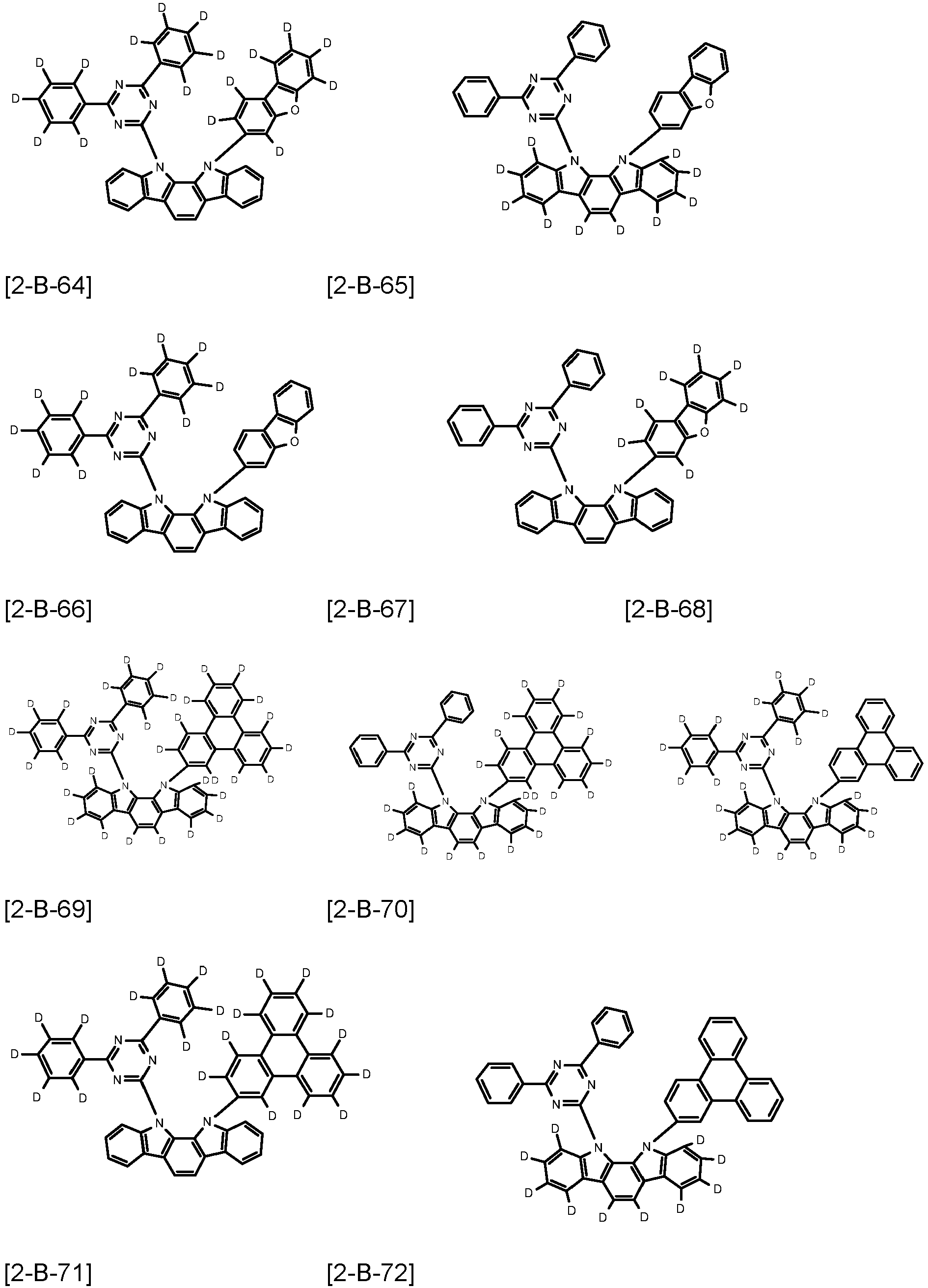

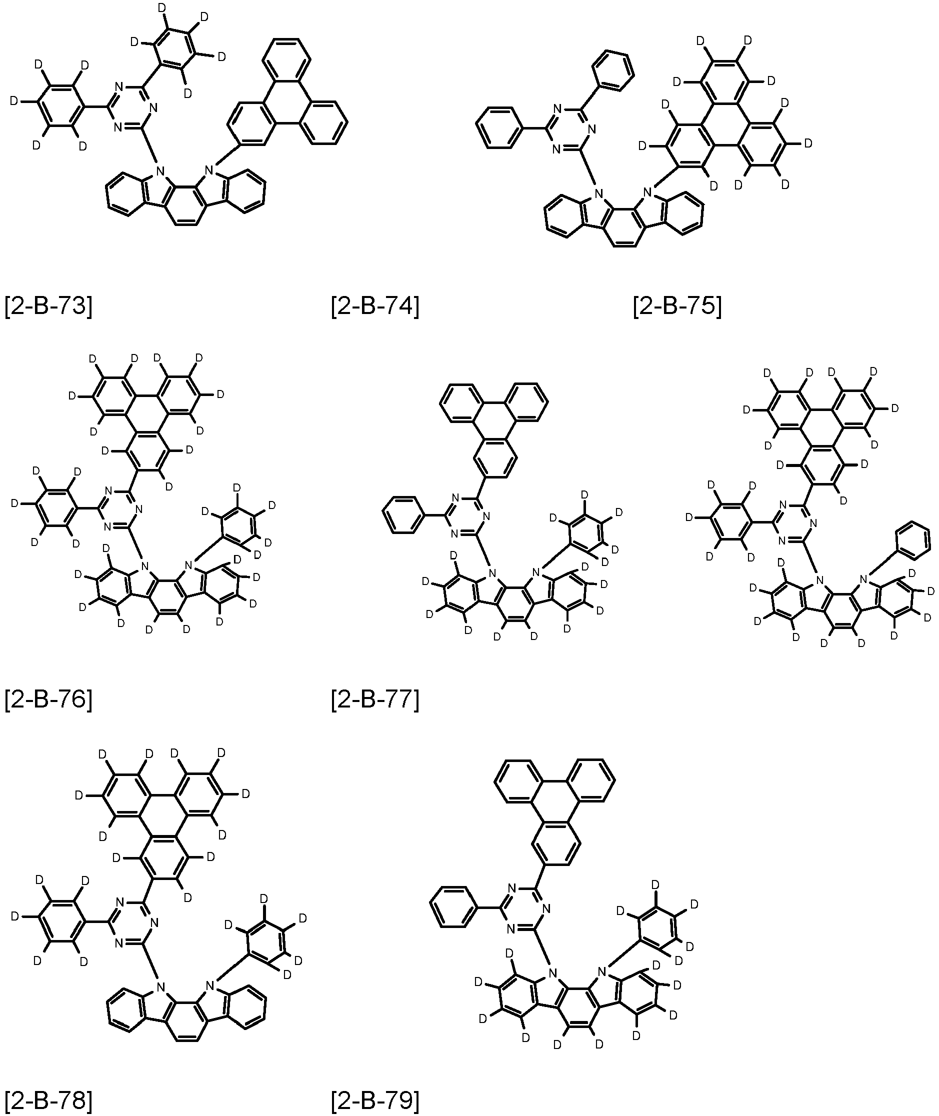

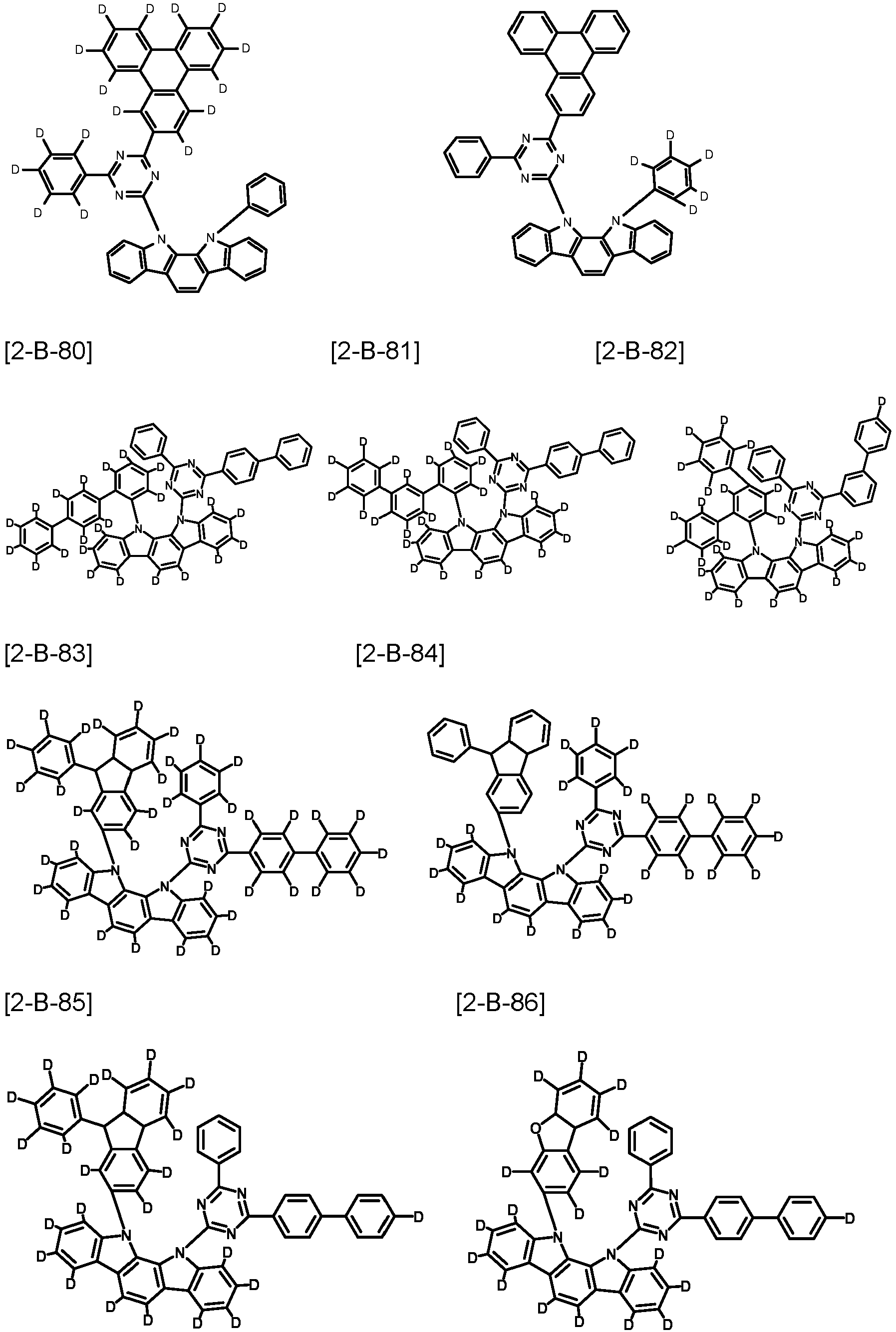

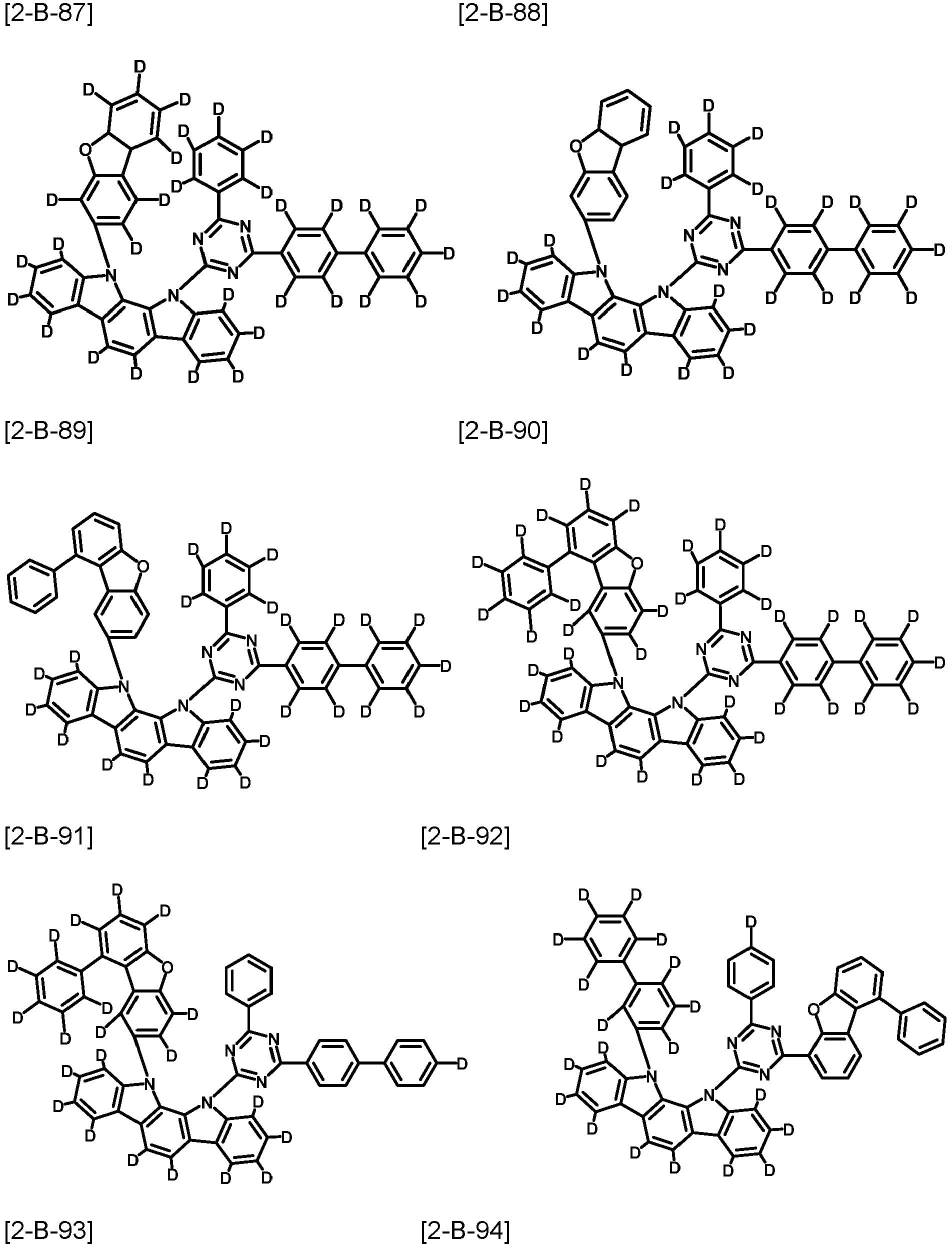

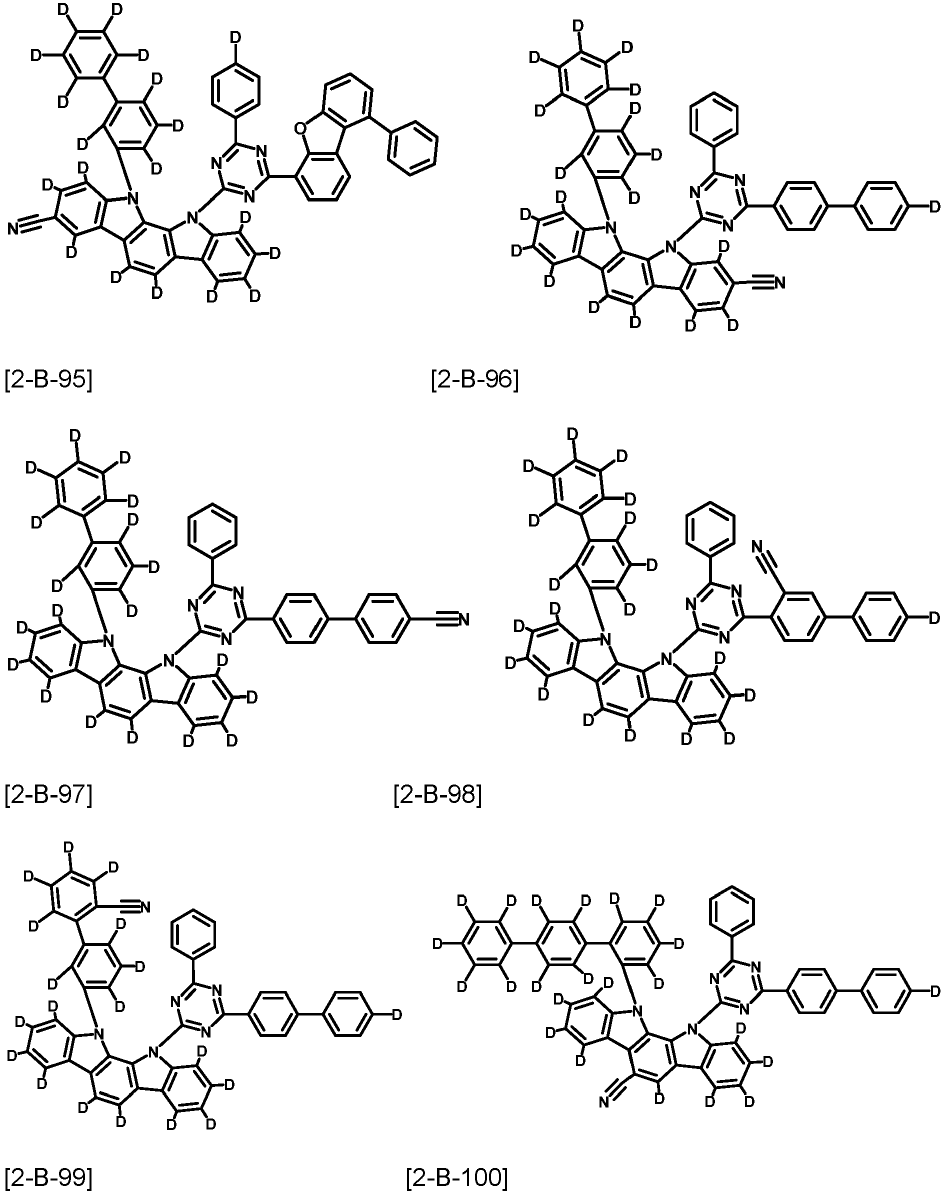

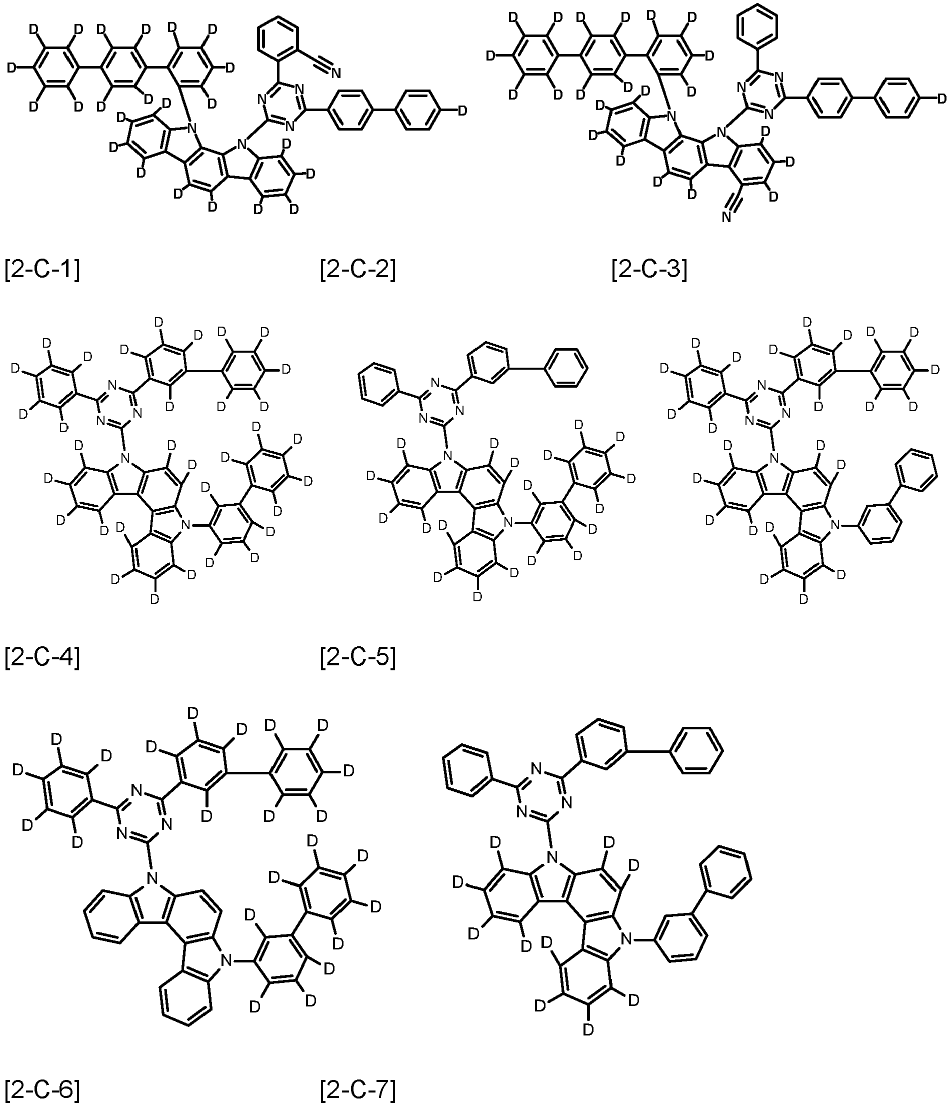

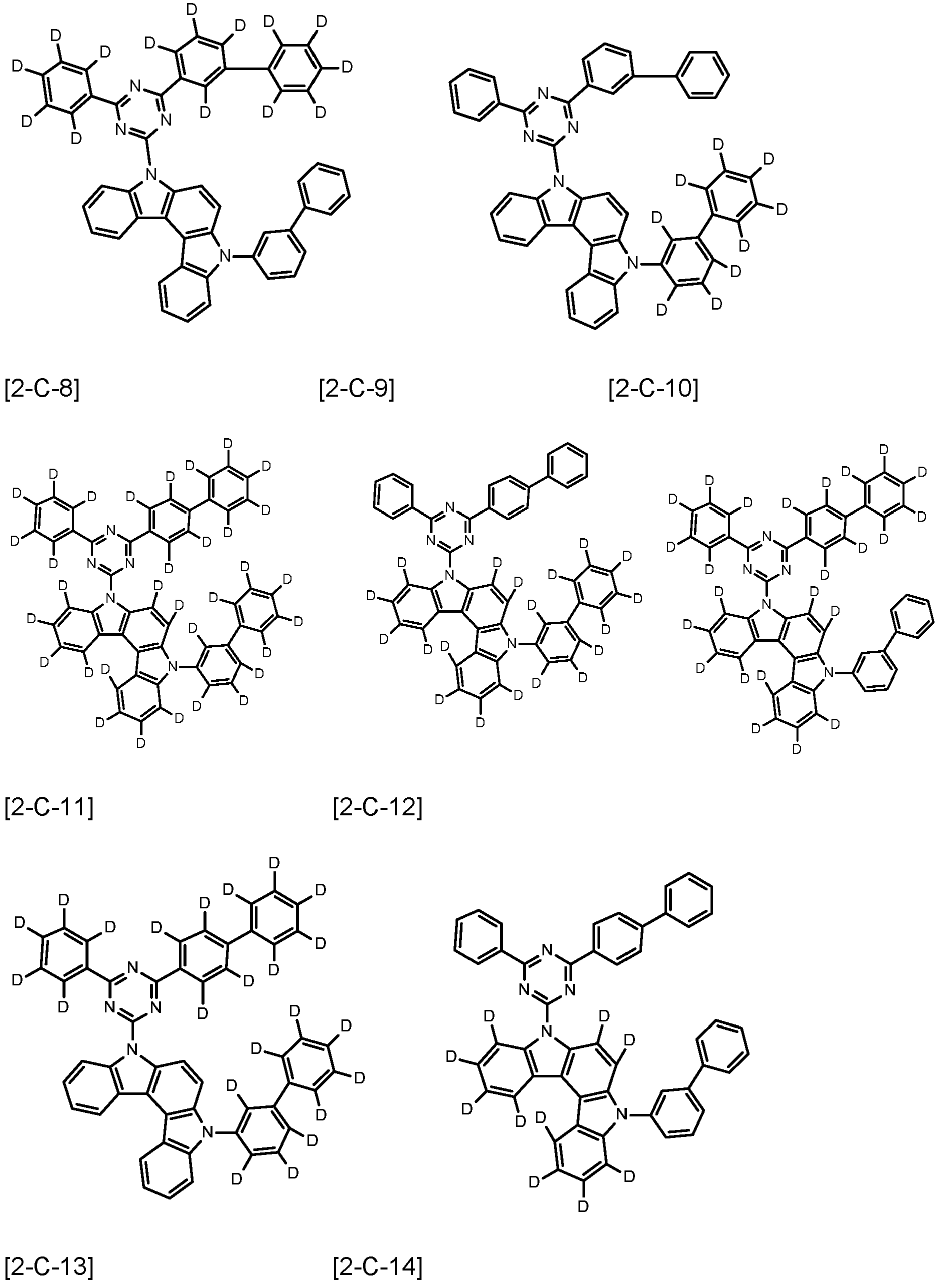

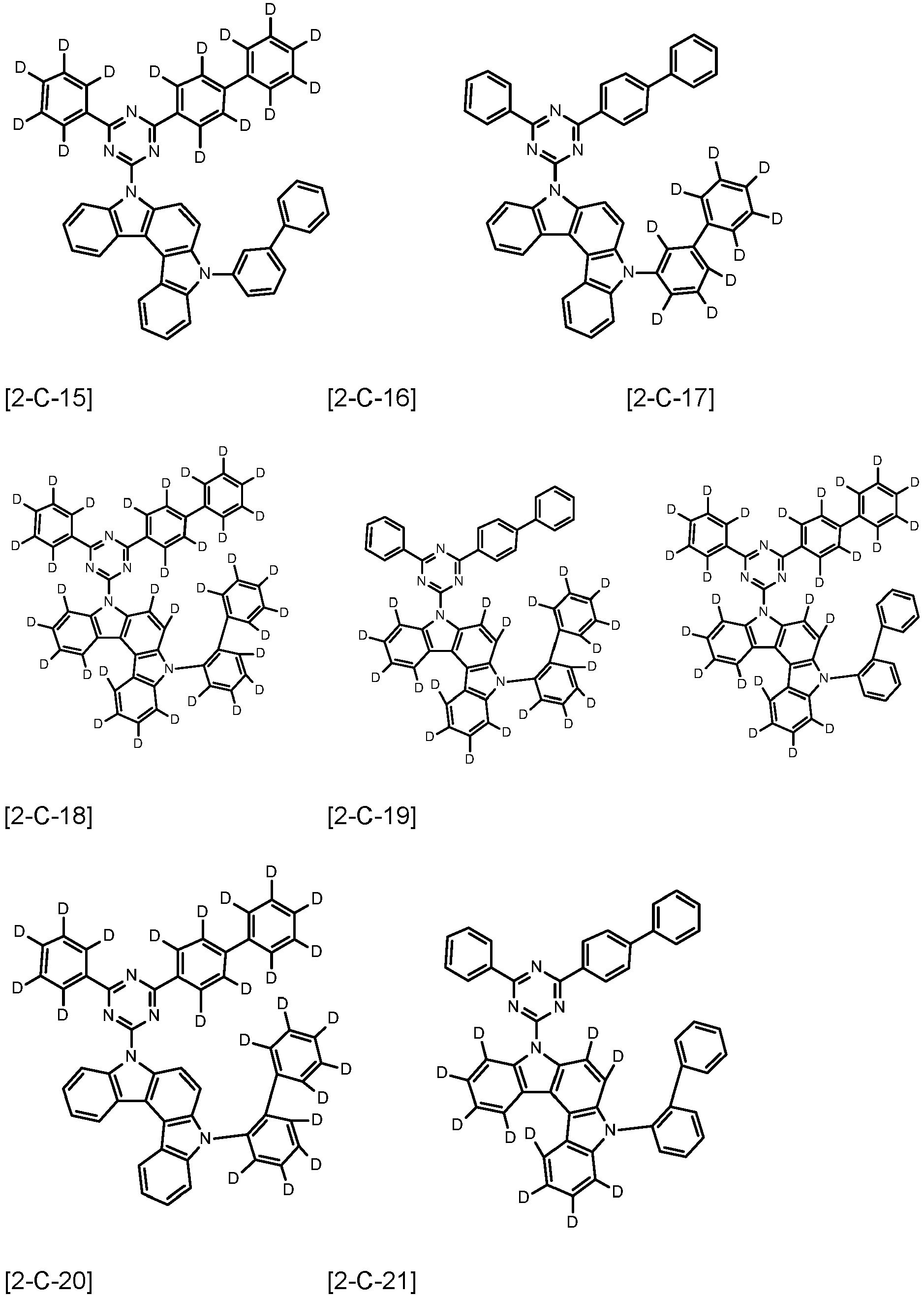

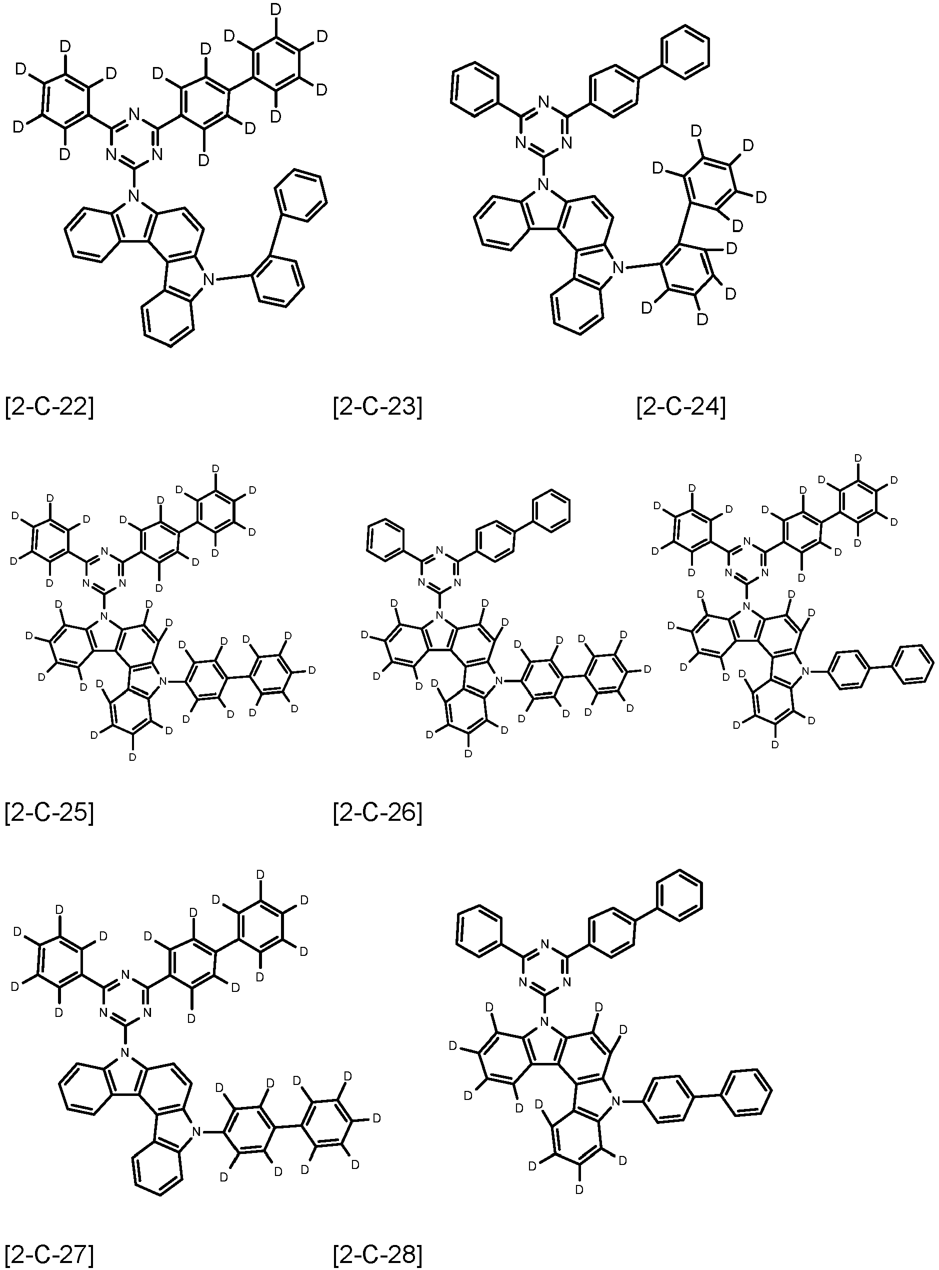

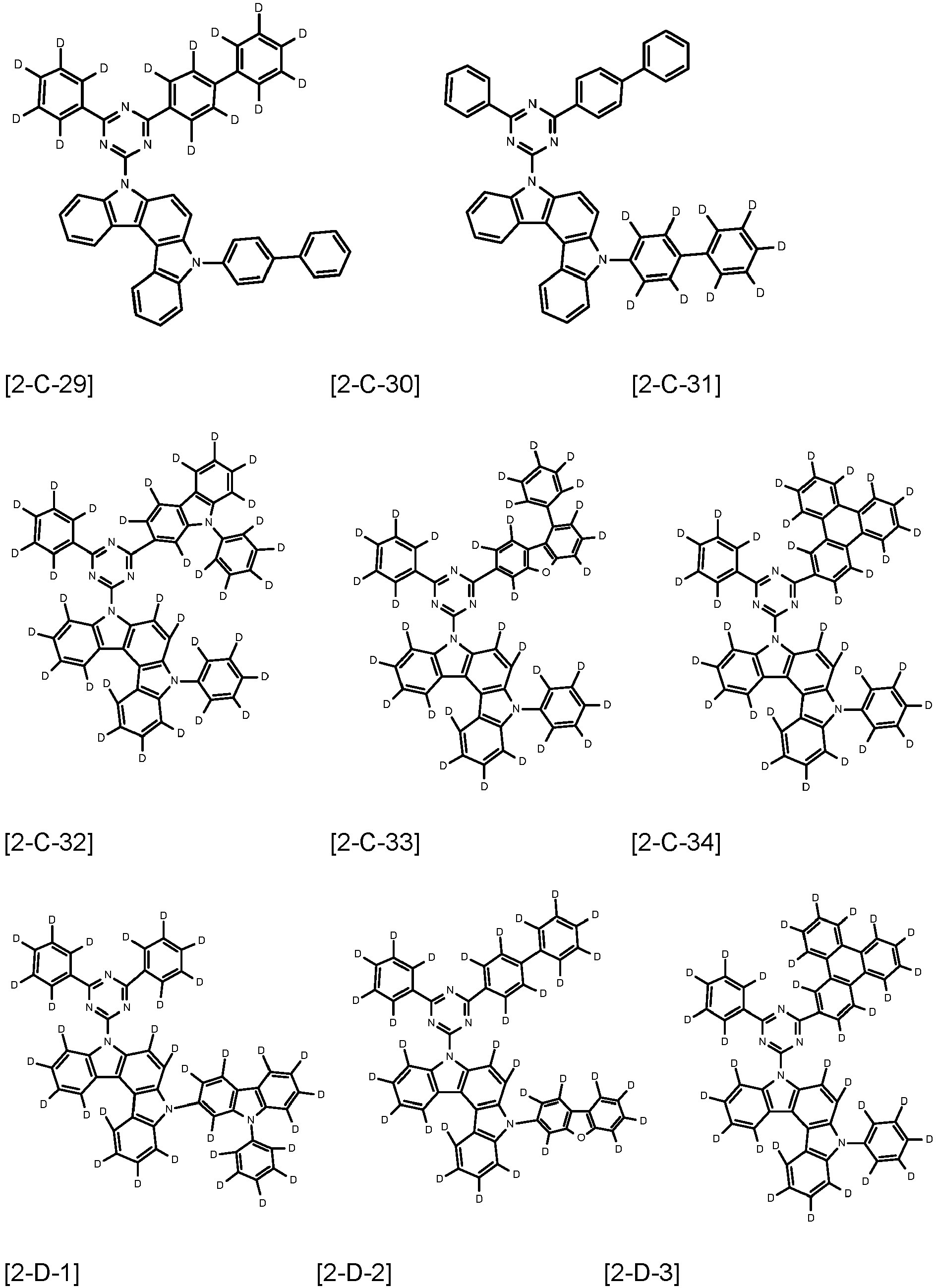

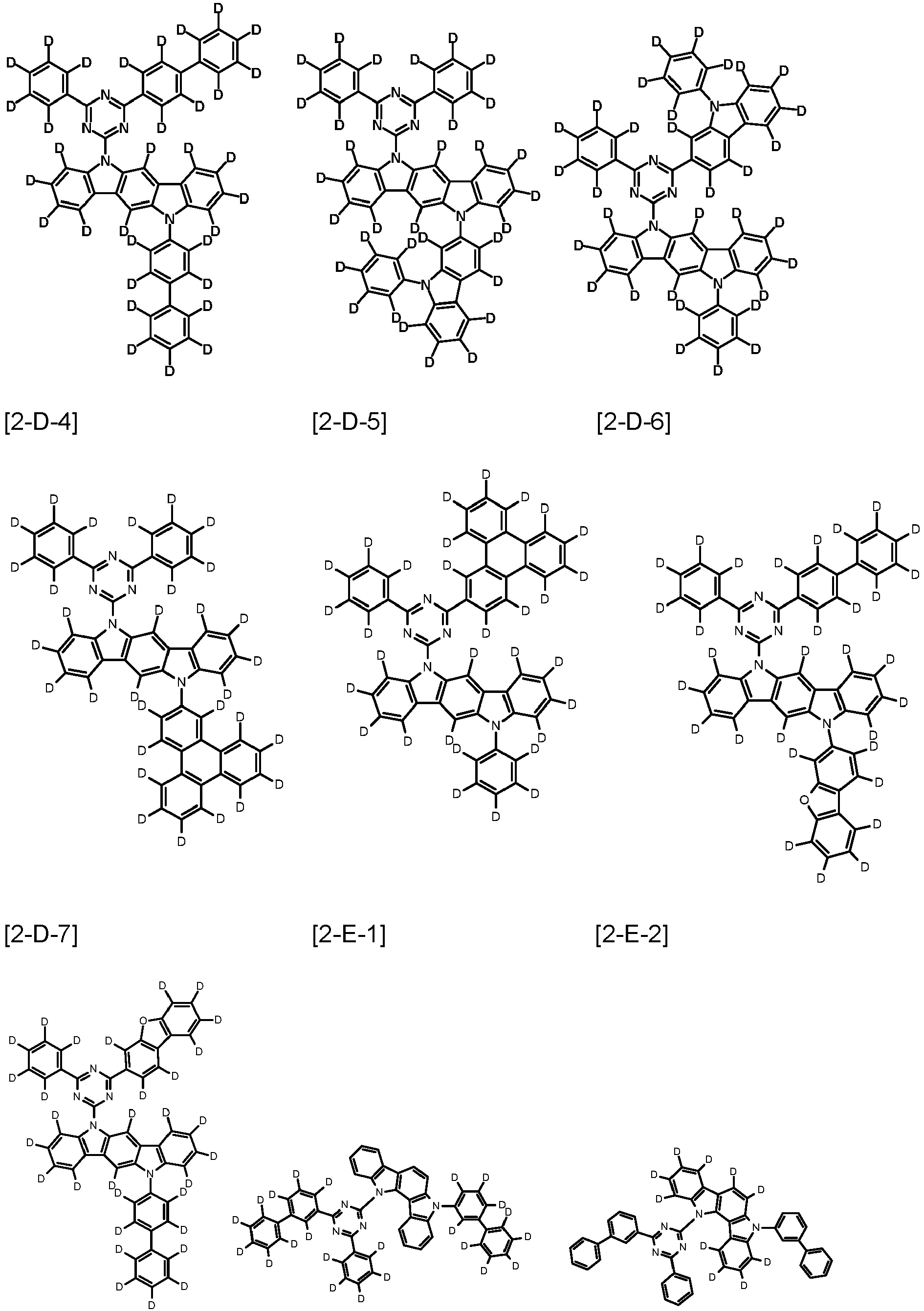

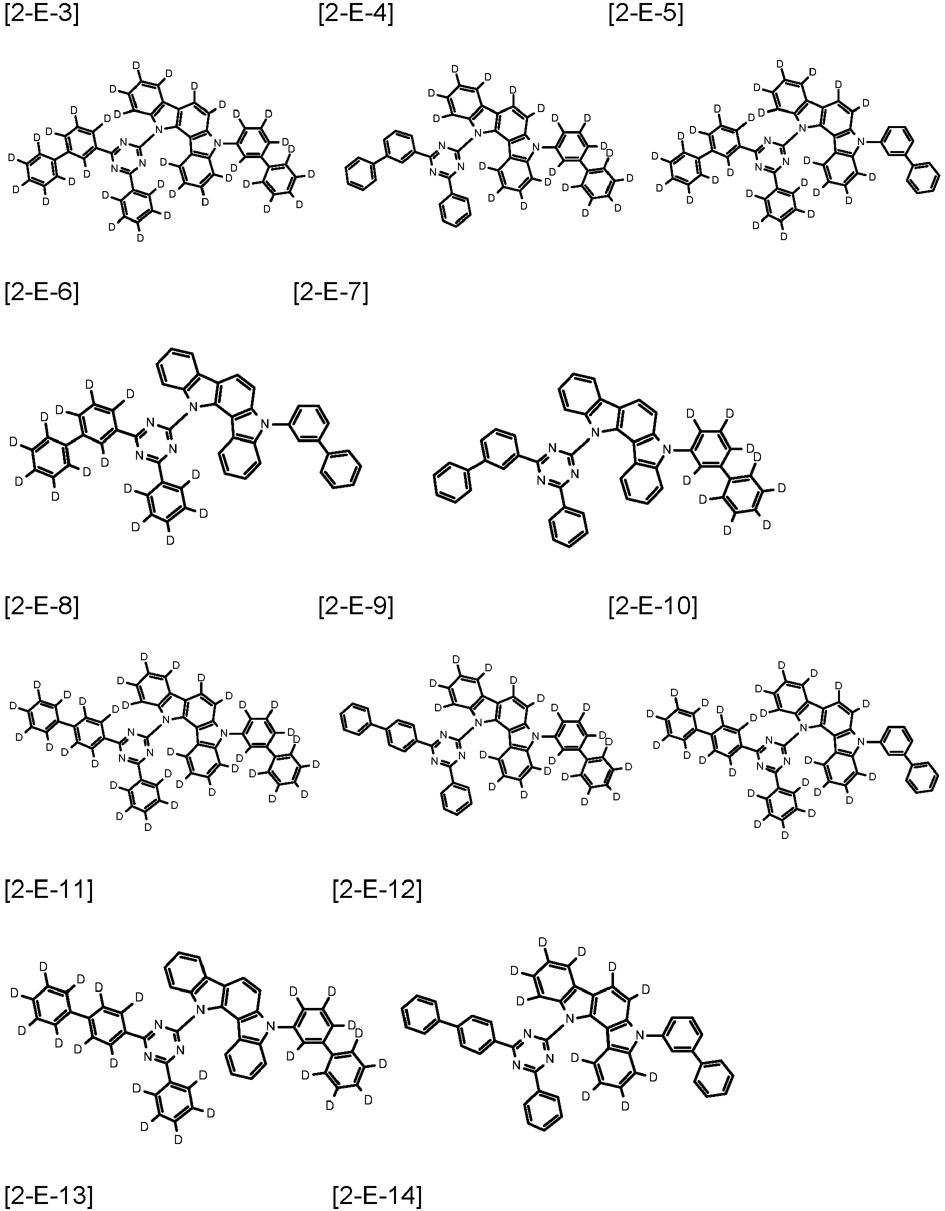

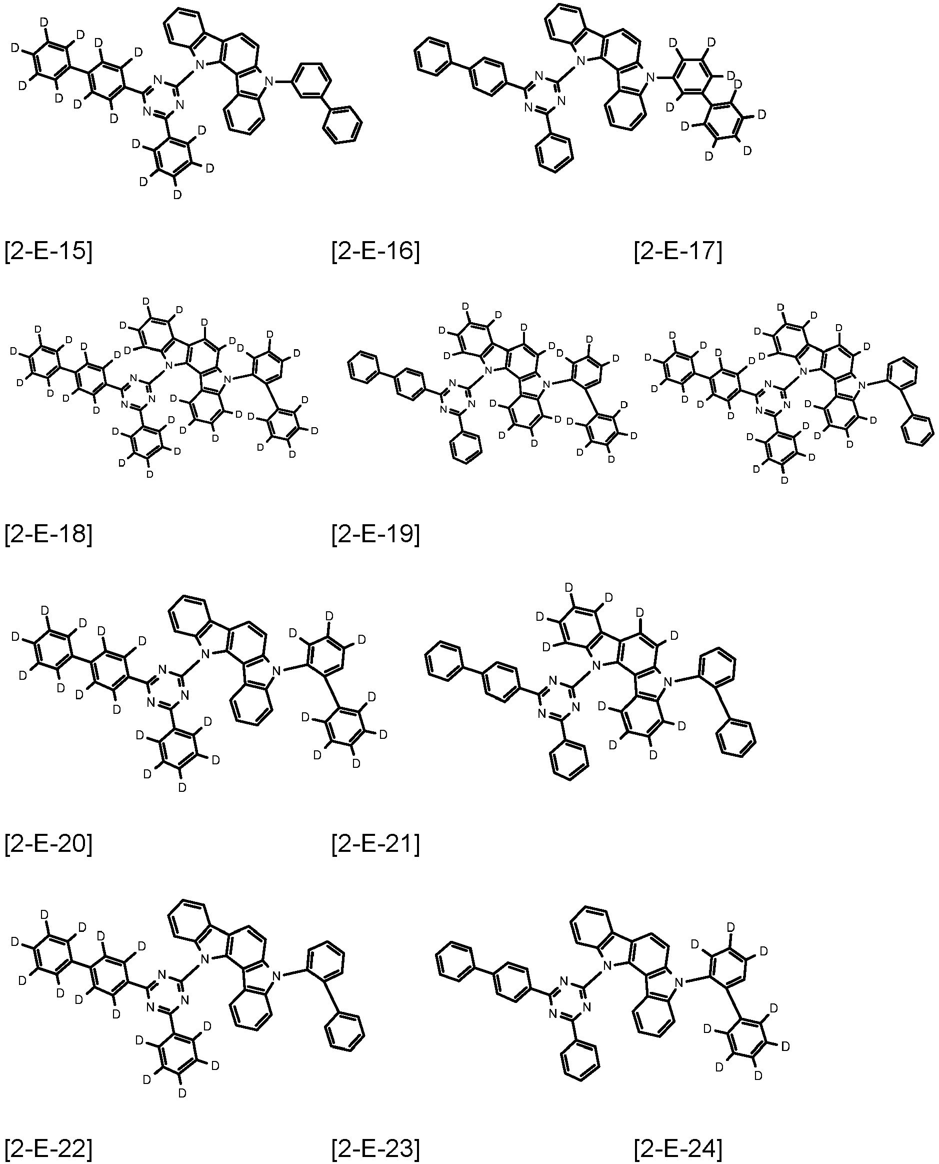

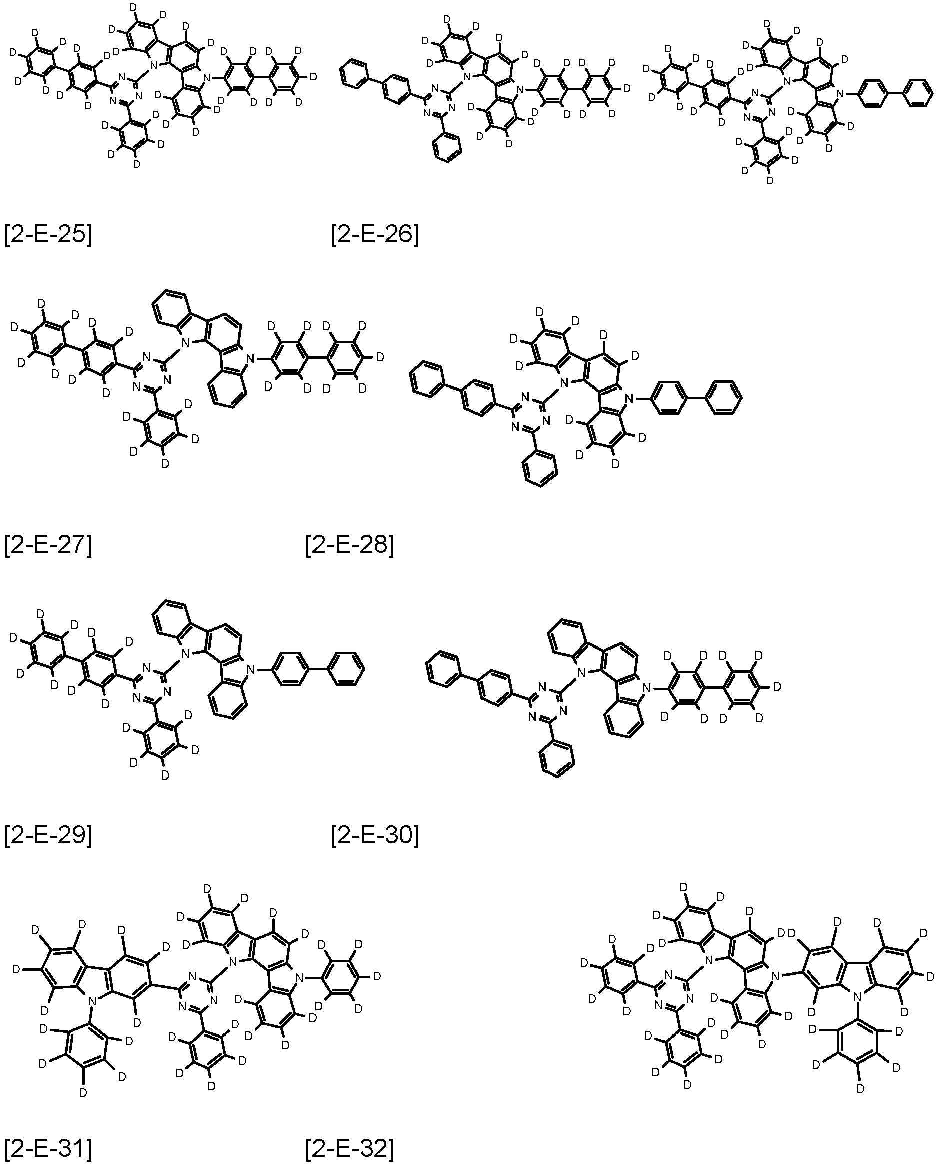

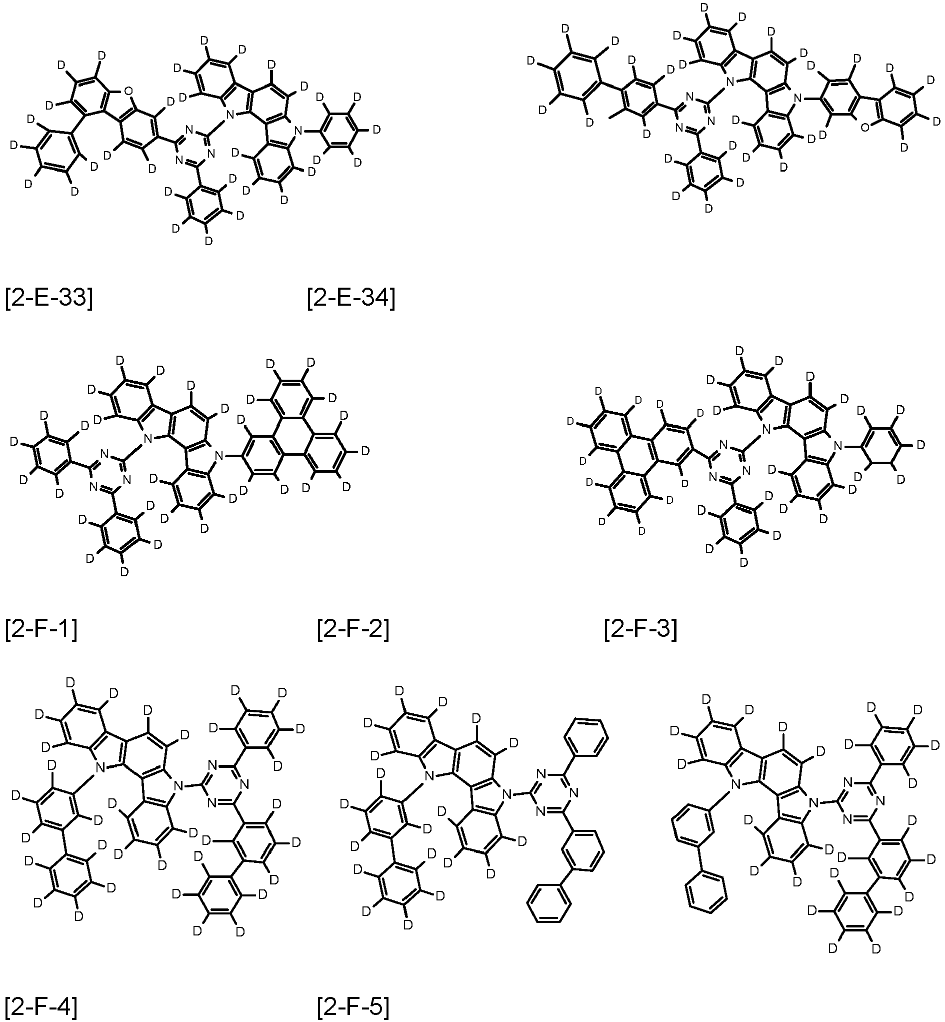

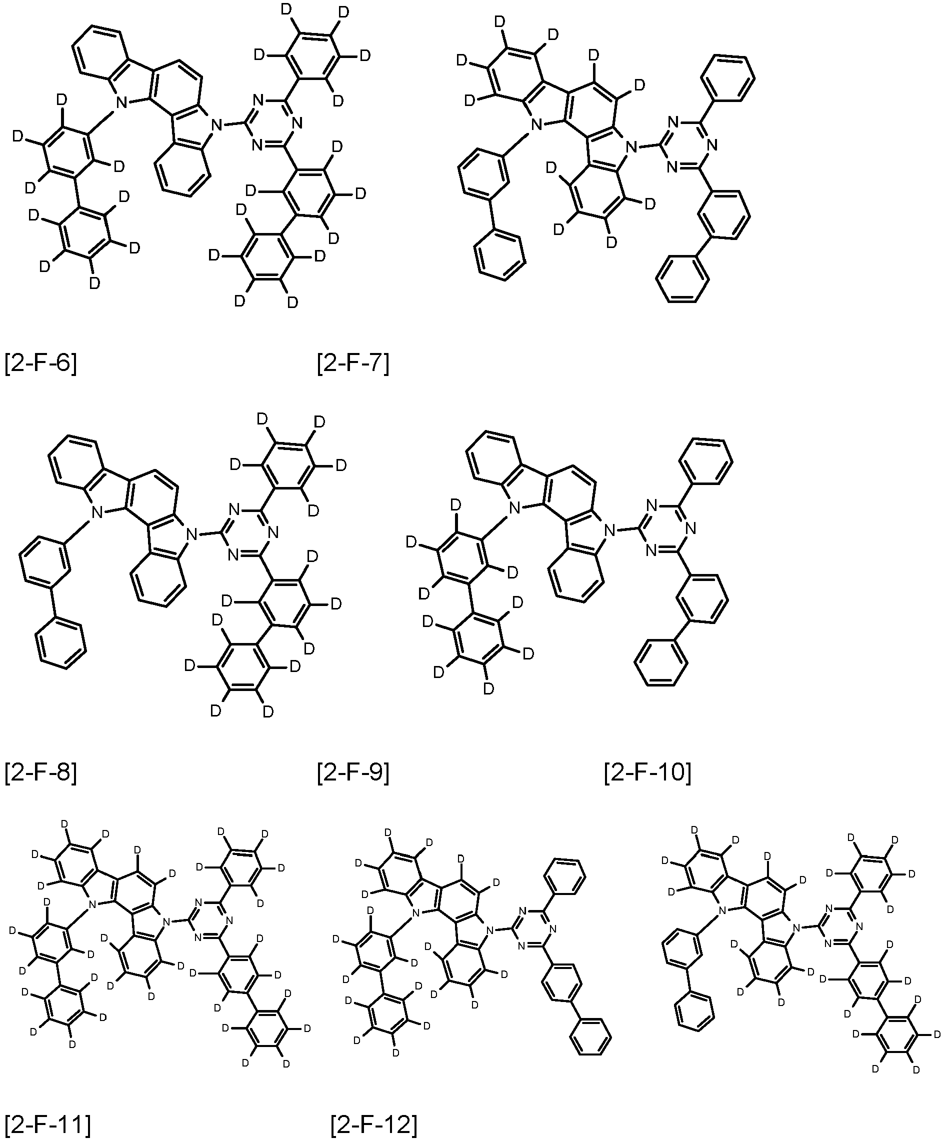

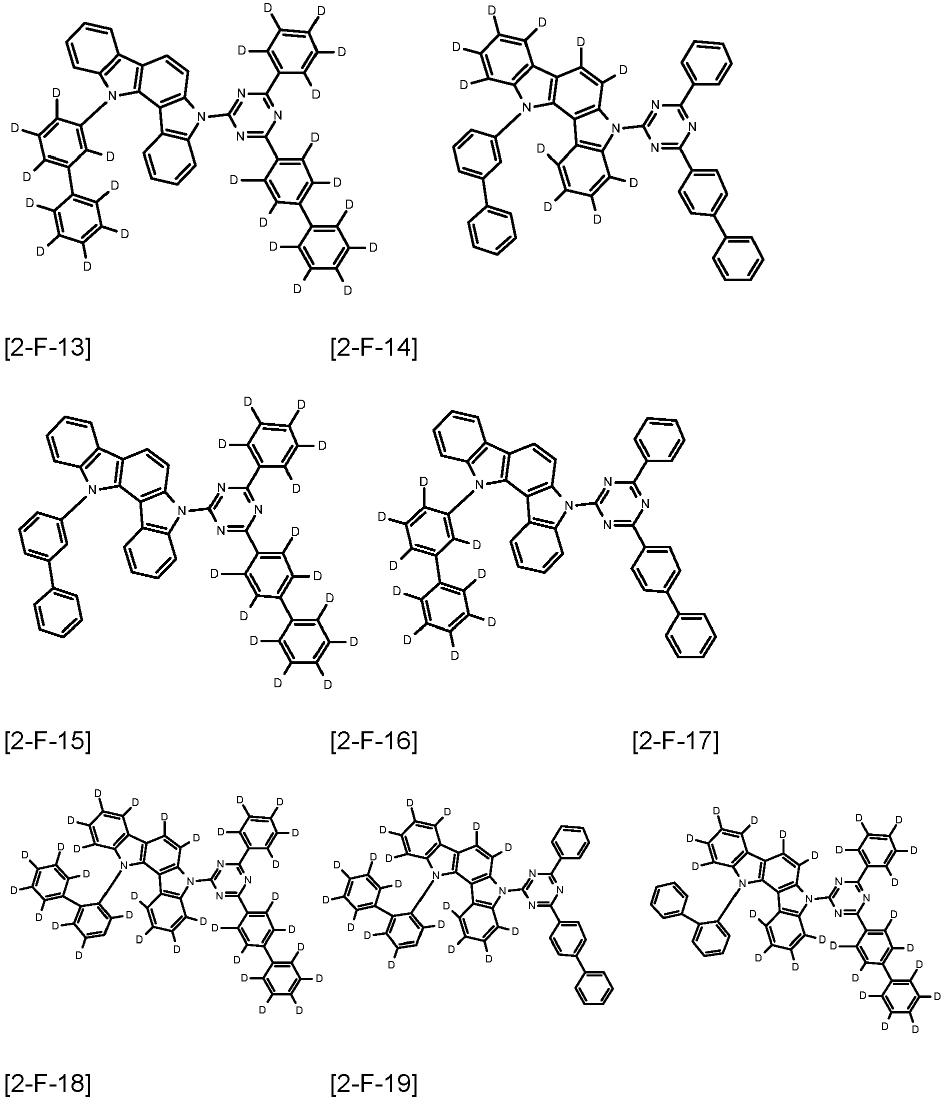

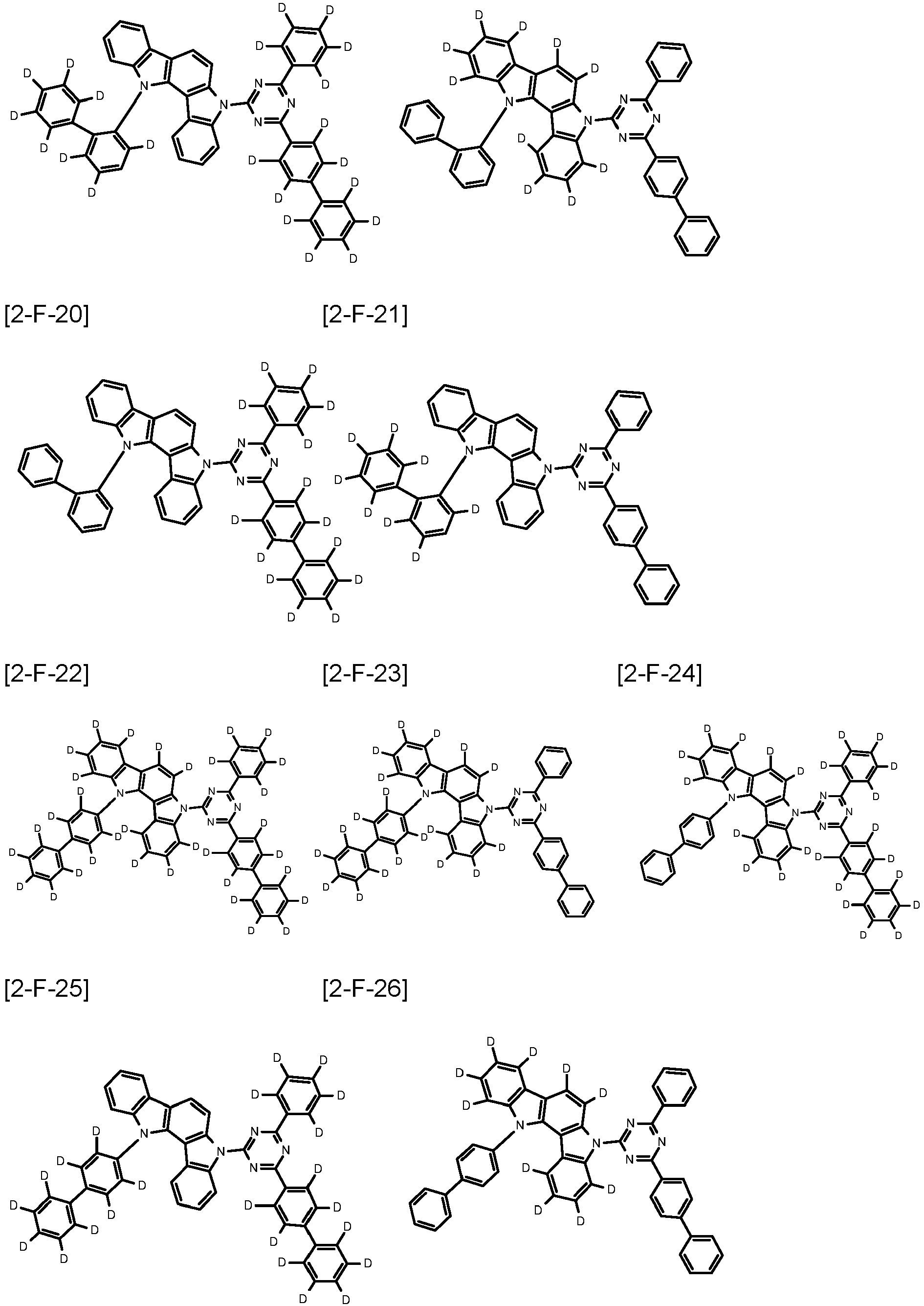

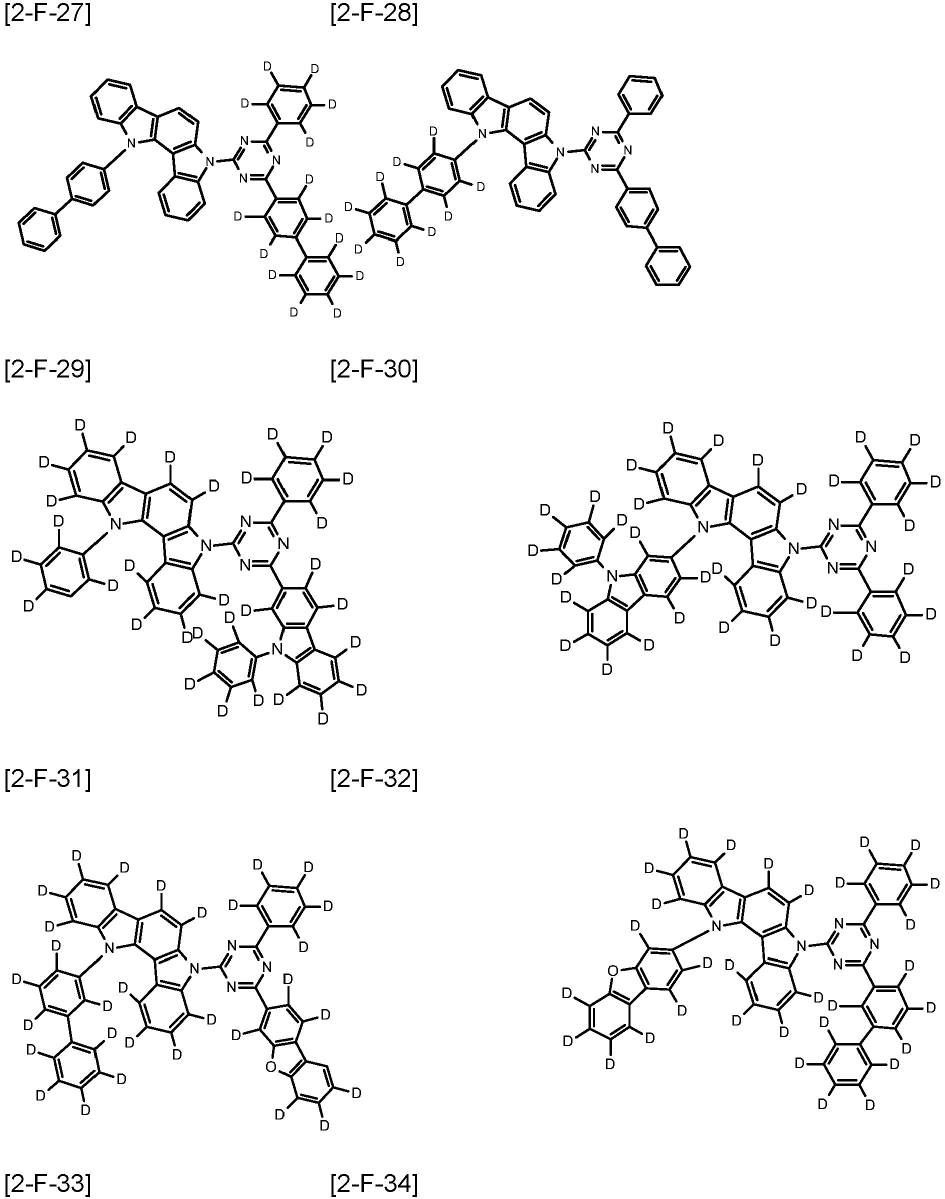

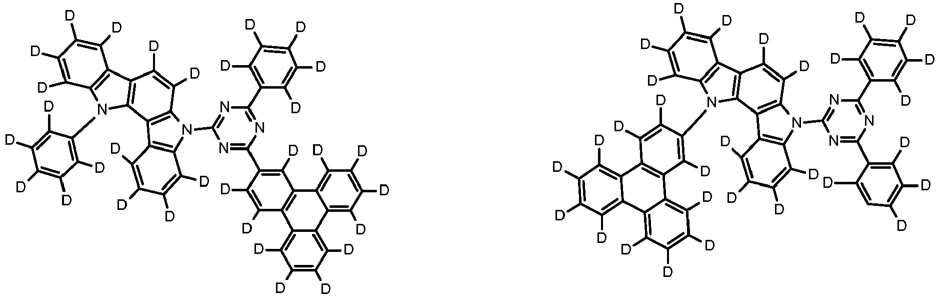

In a more specific embodiment of the present invention, the first compound may be represented by Chemical Formula 1-8a, and the second compound may be represented by Chemical Formula 2B-a-1.

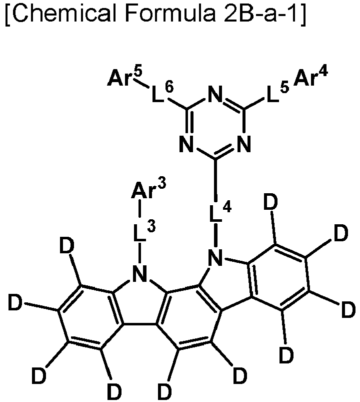

In Chemical Formula 2B-a-1,
Ar³ to Ar⁵ are each independently a substituted or unsubstituted phenyl group or a substituted or unsubstituted biphenyl group, and
L³ to L⁶ are each independently a single bond or a substituted or unsubstituted phenylene group.

The first compound and the second compound may be included in a weight ratio of, for example, 1:99 to 99:1. Within the range, a desirable weight ratio may be adjusted using an electron transport capability of the first compound and a hole transport capability of the second compound to realize bipolar characteristics and thus to improve efficiency and life-span. Within the range, they may be for example included in a weight ratio of 10:90 to 90:10, 20:80 to 80:20, for example, 20:80 to 70:30, 20:80 to 60:40, or 30:70 to 60:40. As a specific example, it may be included in a weight ratio of 40:60, 50:50, or 60:40.

In addition to the first and second compounds described above, one or more compounds may be further included.

For example, the composition for an organic optoelectronic device described above may further include a dopant.

The dopant may be, for example, a phosphorescent dopant, for example a phosphorescent dopant of red, green or blue, and may be, for example, a red phosphorescent dopant.

The dopant is a material mixed with the composition for an organic optoelectronic device in a small amount to cause light emission and may be generally a material such as a metal complex that emits light by multiple excitation into a triplet or more. The dopant may be, for example, an inorganic, organic, or organic-inorganic compound, and may include one or two or more types.

An example of the dopant may be a phosphorescent dopant, and examples of the phosphorescent dopant may include an organometallic compound including Ir, Pt, Os, Ti, Zr, Hf, Eu, Tb, Tm, Fe, Co, Ni, Ru, Rh, Pd, or a combination thereof. The phosphorescent dopant may be, for example, a compound represented by Chemical Formula Z, but is not limited thereto.

[Chemical Formula Z] L⁷MX

In Chemical Formula Z, M is a metal, and L⁷ and X are the same or different, and are a ligand to form a complex compound with M.

The M may be for example Ir, Pt, Os, Ti, Zr, Hf, Eu, Tb, Tm, Fe, Co, Ni, Ru, Rh, Pd, or a combination thereof, and L⁷ and X may be, for example a bidentate ligand.

Examples of the ligands represented by L⁷ and X may be selected from the chemical formulas listed in Group A, but are not limited thereto.

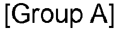

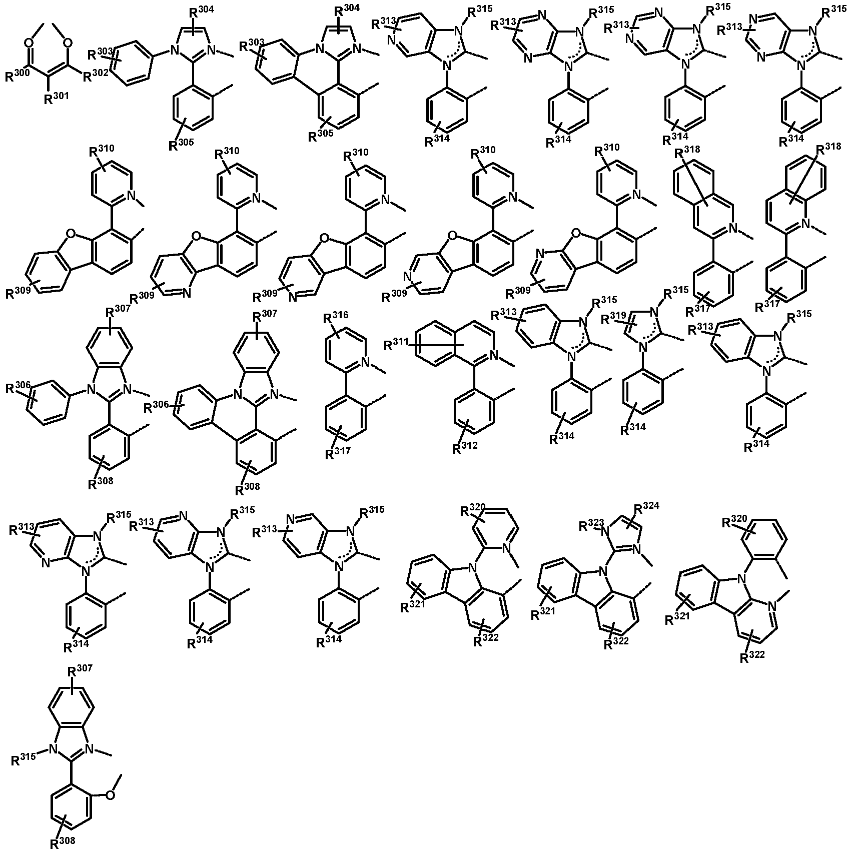

In Group A,
R³⁰⁰ to R³⁰² are each independently hydrogen, deuterium a C1 to C30 alkyl group that is substituted or unsubstituted with a halogen, a C6 to C30 aryl group that is substituted or unsubstituted with a C1 to C30 alkyl, or a halogen, and
R³⁰³ to R³²⁴ are each independently hydrogen, deuterium, a halogen, a substituted or unsubstituted C1 to C30 alkyl group, a substituted or unsubstituted C1 to C30 alkoxy group, a substituted or unsubstituted C3 to C30 cycloalkyl group, a substituted or unsubstituted C2 to C30 alkenyl group, a substituted or unsubstituted C6 to C30 aryl group, a substituted or unsubstituted C1 to C30 heteroaryl group, a substituted or unsubstituted C1 to C30 amino group, a substituted or unsubstituted C6 to C30 arylamino group, SF₅, a trialkylsilyl group having a substituted or unsubstituted C1 to C30 alkyl group, a dialkylarylsilyl group having a substituted or unsubstituted C1 to C30 alkyl group and C6 to C30 aryl group, or a triarylsilyl group having a substituted or unsubstituted C6 to C30 aryl group.

For example, a dopant represented by Chemical Formula V may be included.

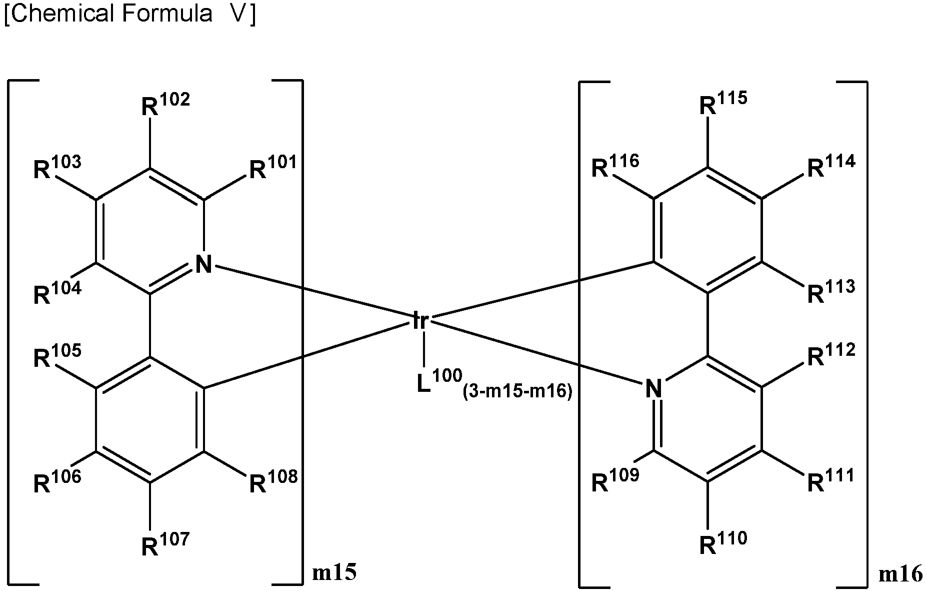

In Chemical Formula V,
R¹⁰¹ to R¹¹⁶ are each independently hydrogen, deuterium, a substituted or unsubstituted C1 to C10 alkyl group, a substituted or unsubstituted C6 to C20 aryl group, or -SiR¹³²R¹³³R¹³⁴,
R¹³² to R¹³⁴ are each independently a C1 to C6 alkyl group,
at least one of R¹⁰¹ to R¹¹⁶ is a functional group represented by Chemical Formula V-1,
L¹⁰⁰ may be a bidentate ligand of a monovalent anion, and is a ligand that coordinates to iridium through a lone pair of carbons or heteroatoms, and
m15 and m16 are each independently any one of integers of 0 to 3, and m15 + m16 is any one of integers of 1 to 3, 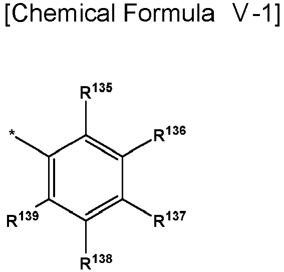
wherein, in Chemical Formula V-1,
R¹³⁵ to R¹³⁹ are each independently hydrogen, deuterium, a substituted or unsubstituted C1 to C10 alkyl group, a substituted or unsubstituted C6 to C20 aryl group, or -SiR¹³²R¹³³R¹³⁴,
R¹³² to R¹³⁴ are each independently a C1 to C6 alkyl group, and
* means a part connected to a carbon atom.

For example, the dopant represented by Chemical Formula Z-1 may be included. 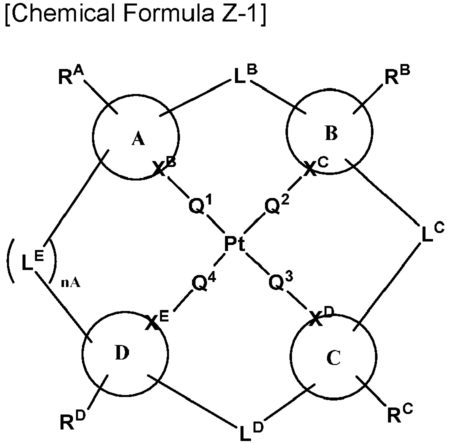
In Chemical Formula Z-1, rings A, B, C, and D are each independently a 5-membered or 6-membered carbocyclic or heterocyclic ring;
R^{A}, R^{B}, R^{C}, and R^{D} are each independently mono-, di-, tri-, or tetra-substitution, or unsubstitution;
L^{B}, L^{C}, and L^{D} are each independently a direct bond, BR, NR, PR, O, S, Se, C=O, S=O, SO₂, CRR', SiRR', GeRR', or a combination thereof. when nA is 1, L^{E} may be a direct bond, BR, NR, PR, O, S, Se, C=O, S=O, SO₂, CRR', SiRR', GeRR', or a combination thereof; and when nA is 0, L^{E} does not exist.

R^{A}, R^{B}, R^{C}, R^{D}, R, and R' are each independently hydrogen, deuterium, a halogen, an alkyl group, a cycloalkyl group, a heteroalkyl group, an arylalkyl group, an alkoxy group, an aryloxy group, an amino group, a silyl group, an alkenyl group, a cycloalkenyl group, a heteroalkenyl group, an alkynyl group, an aryl group, a heteroaryl group, an acyl group, a carbonyl group, a carboxylic acid group, an ester group, a nitrile group, an isonitrile group, a sulfanyl group, a sulfinyl group, a sulfonyl group, a phosphino group, or a combination thereof; any adjacent R^{A}, R^{B}, R^{C}, R^{D}, R, and R' are optionally linked to each other to provide a ring; X^{B}, X^{C}, X^{D}, and X^{E} are each independently selected from carbon and nitrogen; and Q¹, Q², Q³, and Q⁴ each represent oxygen or a direct bond.

The dopant according to an embodiment may be a platinum complex, and may be, e.g., represented by Chemical Formula VI.

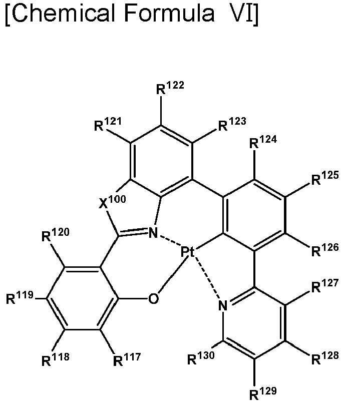

In Chemical Formula VI,
X¹⁰⁰ may be, e.g., O, S, or NR¹³¹,
R¹¹⁷ to R¹³¹ are each independently hydrogen, deuterium, a substituted or unsubstituted C1 to C10 alkyl group, a substituted or unsubstituted C6 to C20 aryl group, or - SiR¹³²R¹³³R¹³⁴,
R¹³² to R¹³⁴ are each independently a C1 to C6 alkyl group, and
at least one of R¹¹⁷ to R¹³¹ may be, e.g., -SiR¹³²R¹³³R¹³⁴ or a tert-butyl group.

Hereinafter, an organic optoelectronic device including the aforementioned compound for the organic optoelectronic device is described.

The organic optoelectronic device may be a suitable device to convert electrical energy into photoenergy and vice versa, e.g., an organic photoelectric device, an organic light emitting diode, an organic solar cell, or an organic photoconductor drum.

Herein, an organic light emitting diode as one example of an organic optoelectronic device is described referring to drawings.

FIG 1 is a cross-sectional view illustrating an organic light emitting diode according to an embodiment.

Referring to the FIG. 1, an organic light emitting diode 100 according to an embodiment may include, e.g., an anode 120 and a cathode 110 facing each other and an organic layer 105 between the anode 120 and cathode 110.

The anode 120 may be made of a conductor having a large work function to help hole injection, and may be, e.g., a metal, a metal oxide, or a conductive polymer. The anode 120 may be, e.g., a metal such as nickel, platinum, vanadium, chromium, copper, zinc, gold, and the like or an alloy thereof; a metal oxide such as zinc oxide, indium oxide, indium tin oxide (ITO), indium zinc oxide (IZO), and the like; a combination of a metal and an oxide such as ZnO and Al or SnO₂ and Sb; a conductive polymer such as poly(3-methylthiophene), poly(3,4-(ethylene-1,2-dioxy)thiophene) (PEDOT), polypyrrole, or polyaniline.

The cathode 110 may be made of a conductor having a small work function to help electron injection, and may be, e.g., a metal, a metal oxide, or a conductive polymer. The cathode 110 may be, e.g., a metal such as magnesium, calcium, sodium, potassium, titanium, indium, yttrium, lithium, gadolinium, aluminum silver, tin, lead, cesium, barium, or the like, or an alloy thereof; or a multi-layer structure material such as LiF/Al, LiO₂/Al, LiF/Ca, or BaF₂/Ca.

The organic layer 105 may include the aforementioned compound for an organic optoelectronic device.

The organic layer 105 may include, e.g., the light emitting layer 130, and the light emitting layer 130 may include, e.g., the aforementioned compound for an organic optoelectronic device.

The composition for an organic optoelectronic device further including a dopant may be, e.g., a red light-emitting composition.

The light emitting layer 130 may include, e.g., the aforementioned compound for an organic optoelectronic device as a phosphorescent host.

The organic layer may further include a charge transport region in addition to the light emitting layer.

The charge transport region may be, e.g., the hole transport region 140.

The hole transport region 140 may help further increase hole injection and/or hole mobility between the anode 120 and the light emitting layer 130 and block electrons.

In an implementation, the hole transport region 140 may include a hole transport layer between the anode 120 and the light emitting layer 130, and a hole transport auxiliary layer between the light emitting layer 130 and the hole transport layer, and at least one of the compounds of Group B may be included in at least one of the hole transport layer and the hole transport auxiliary layer.

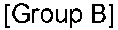

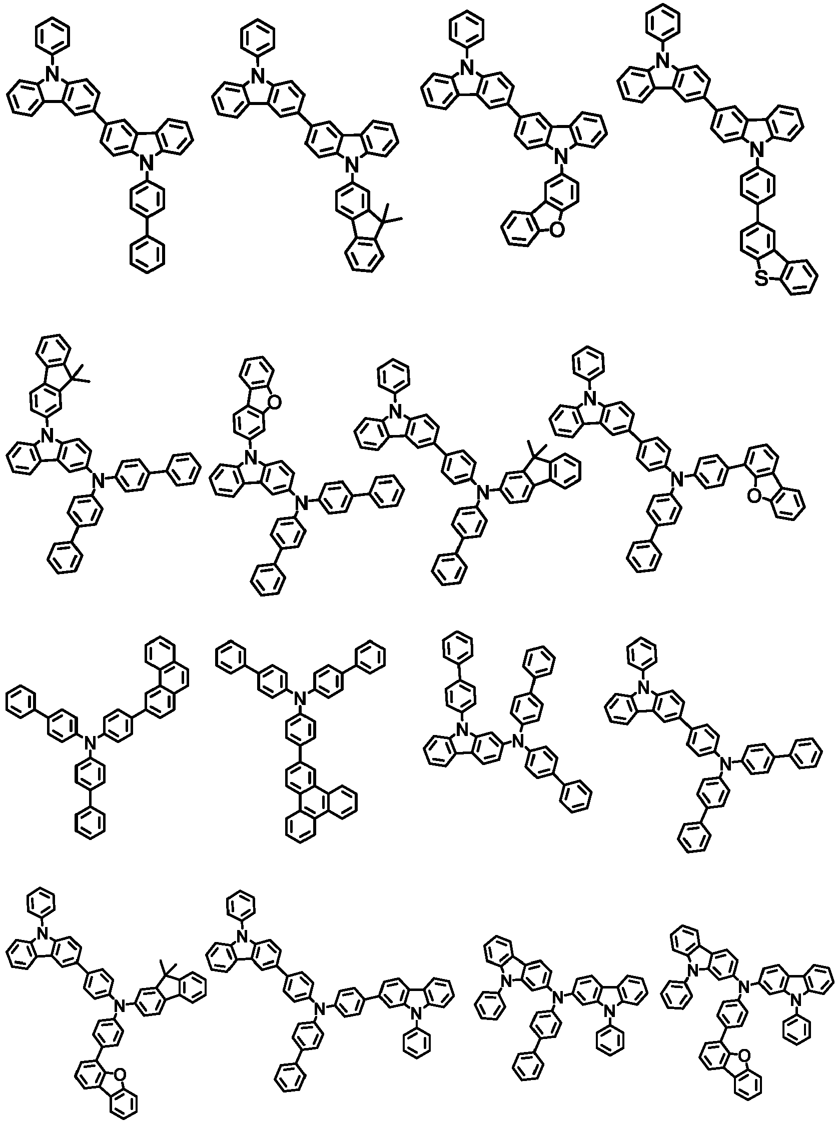

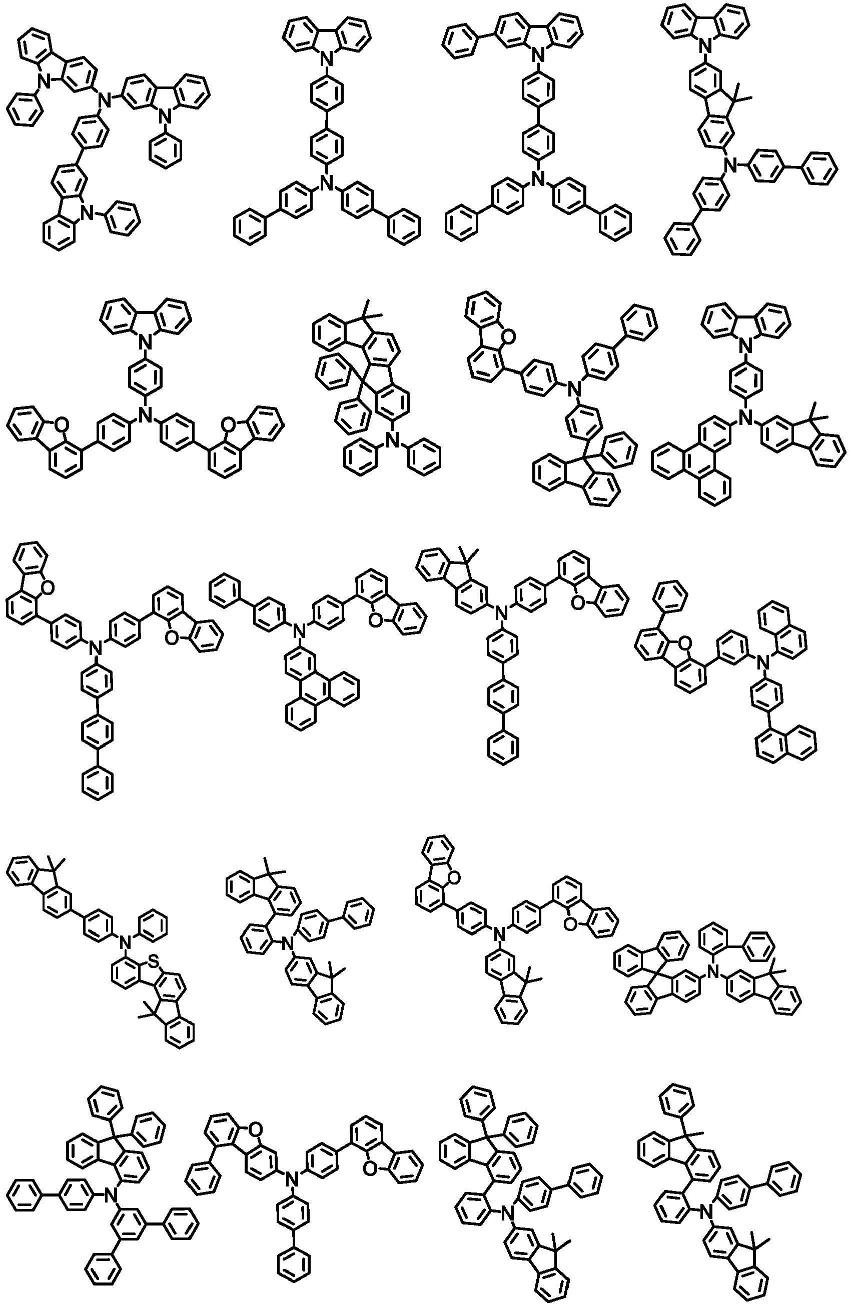

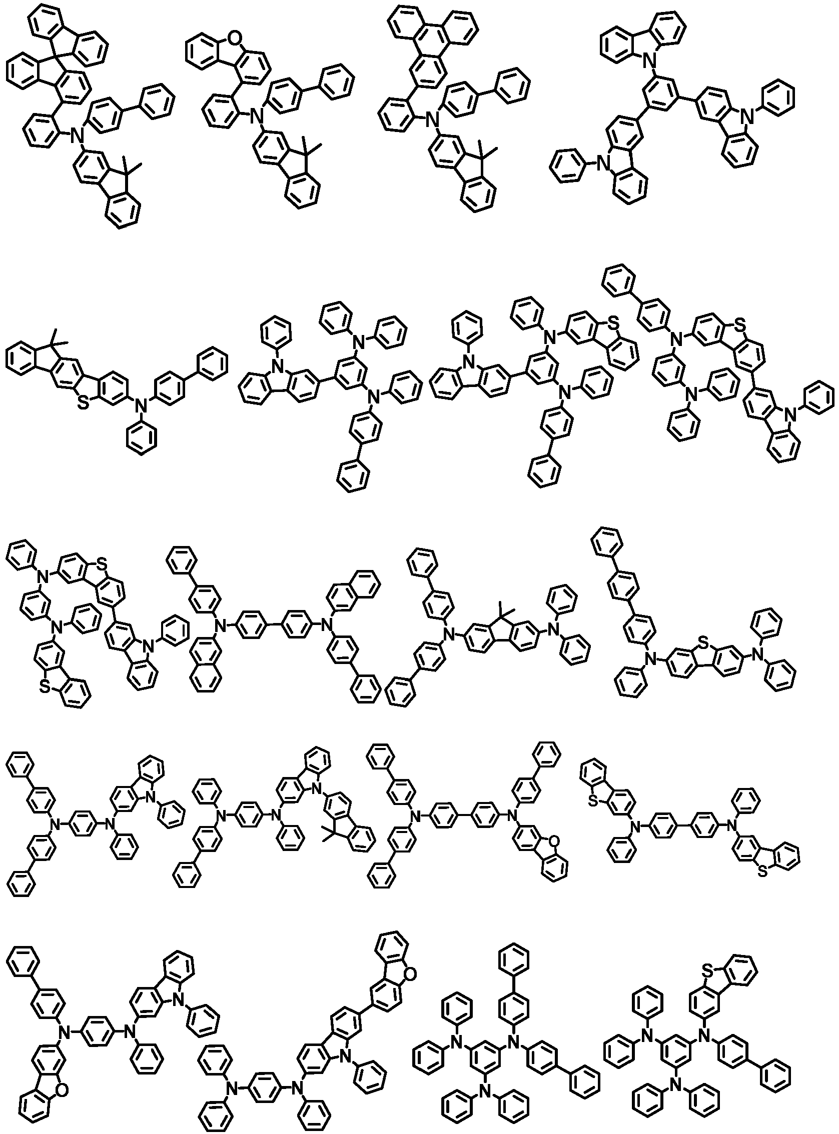

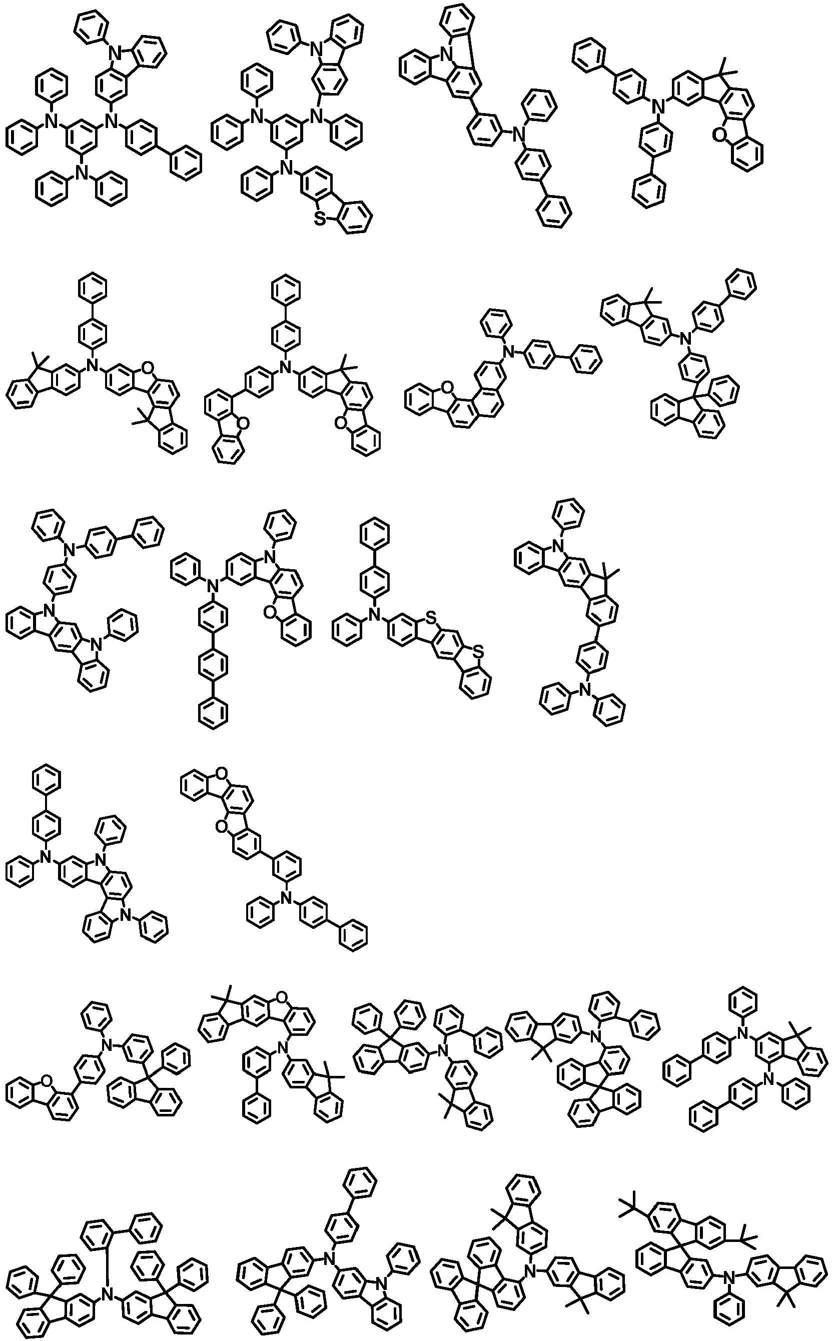

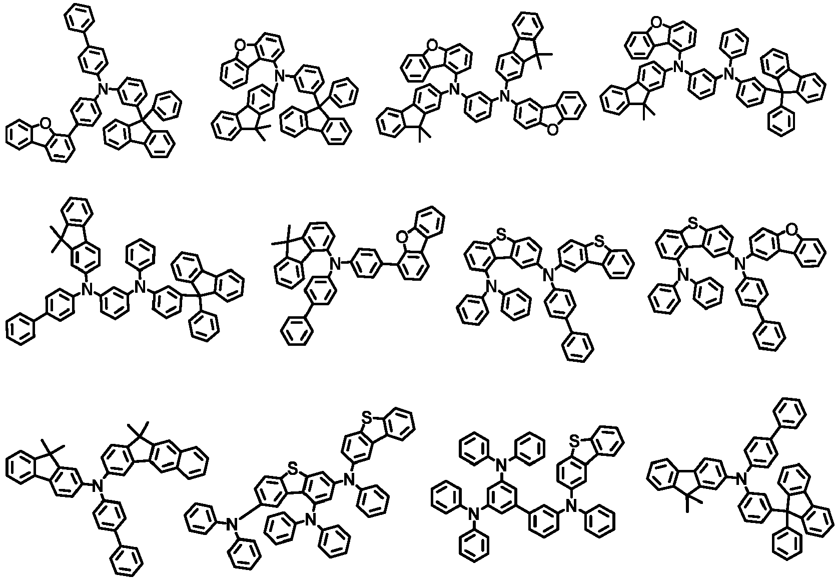

In the hole transport region, in addition to the compounds described above, known compounds disclosed in US5061569A, JP1993-009471A, WO1995-009147A1, JP1995-126615A, JP1998-095973A, etc. and compounds having a similar structure may also be used.

Also, the charge transport region may be, for example, the electron transport region 150.

The electron transport region 150 may further increase electron injection and/or electron mobility and block holes between the cathode 110 and the light emitting layer 130.

Specifically, the electron transport region 150 may include an electron transport layer between the cathode 110 and the light emitting layer 130, and an electron transport auxiliary layer between the light emitting layer 130 and the electron transport layer, and at least one of the compounds of Group C may be included in at least one of the electron transport layer and the electron transport auxiliary layer.

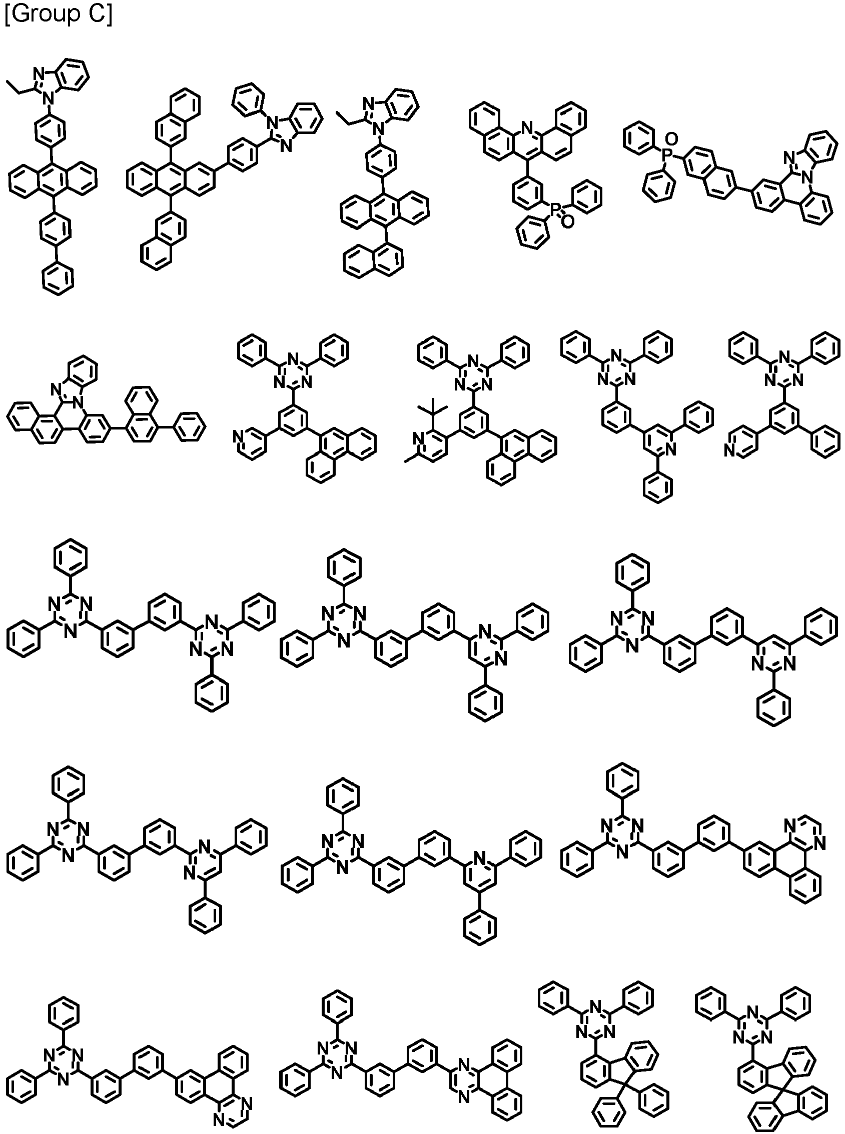

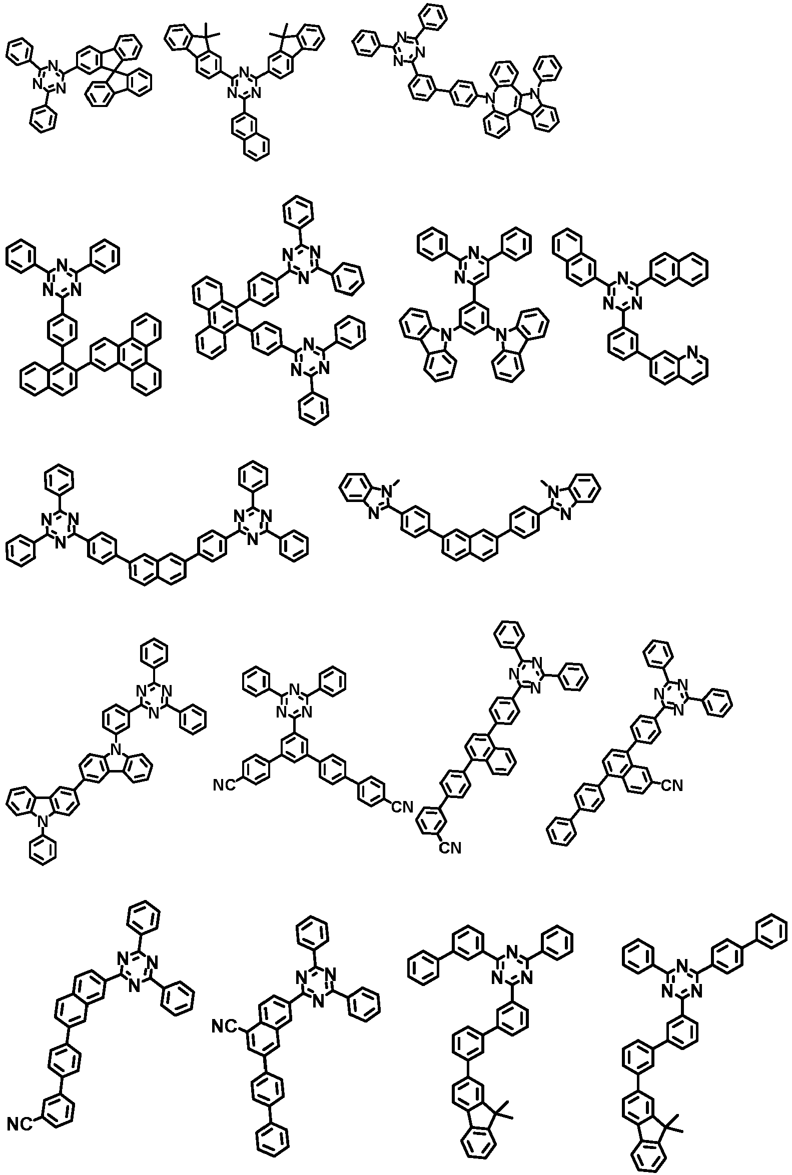

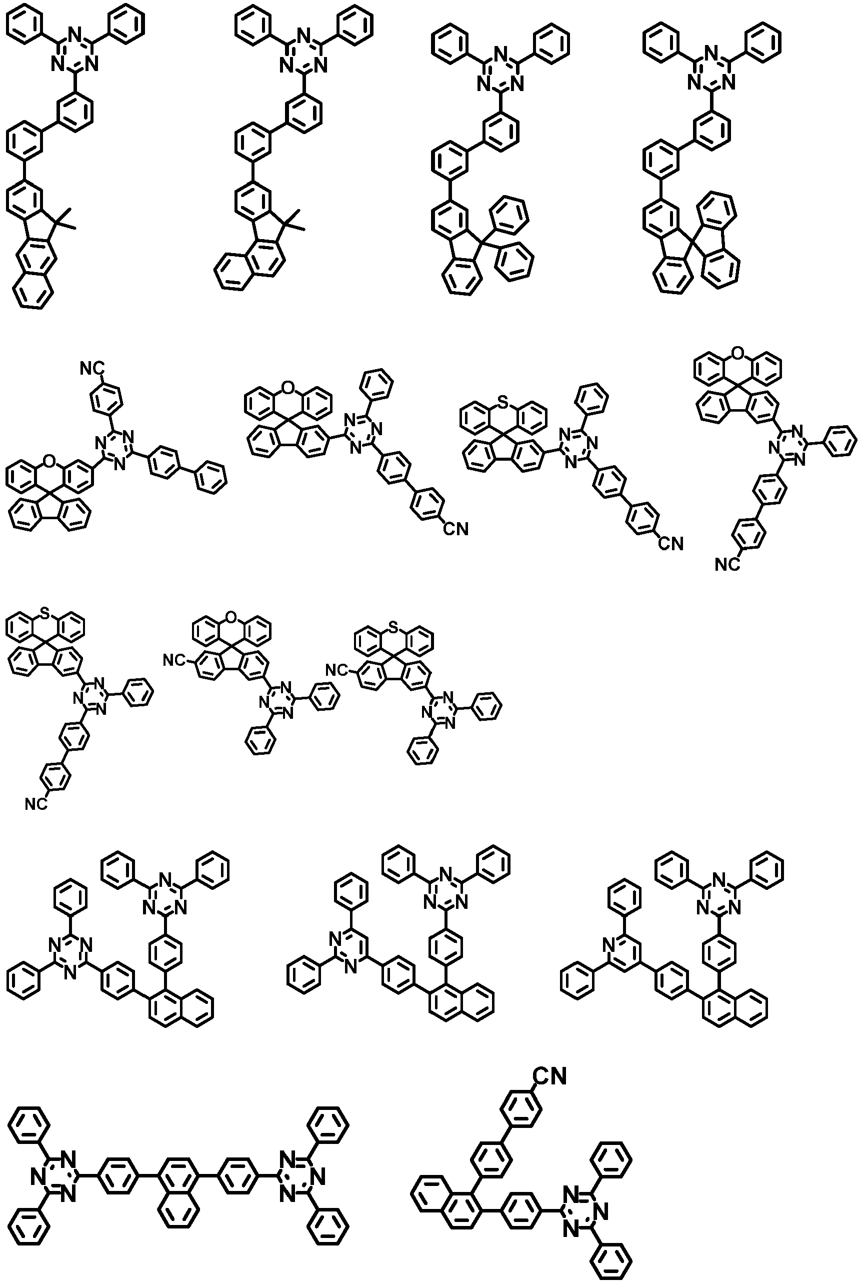

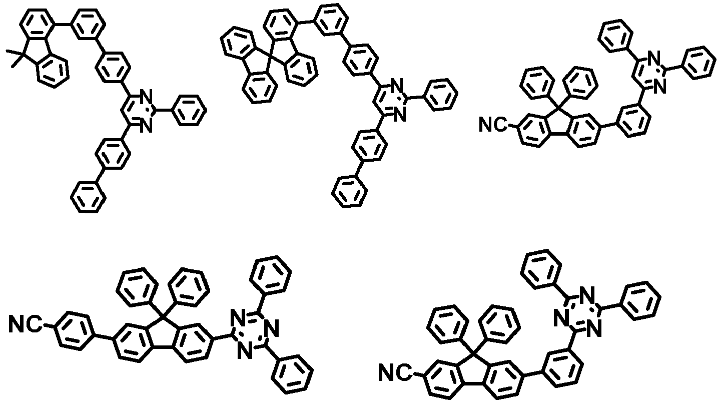

An embodiment of the present invention may provide an organic light emitting diode including the light emitting layer as the organic layer.

Another embodiment of the present invention may provide an organic light emitting diode including a hole transport region and the light emitting layer as the organic layer.

Another embodiment of the present invention may provide an organic light emitting diode including an electron transport region and the light emitting layer as the organic layer.

An embodiment of the present invention may provide an organic light emitting diode including a hole transport region 140 and an electron transport region 150 in addition to the light emitting layer 130 as the organic layer 105, as shown in FIG. 1.

In another embodiment of the present invention, an organic light emitting diode may further include an electron injection layer (not shown), a hole injection layer (not shown), etc. in addition to the light emitting layer as the organic layer.

The organic light emitting diodes may be manufactured by forming an anode or a cathode on a substrate, and then forming an organic layer by a dry film method such as vacuum deposition, sputtering, plasma plating and ion plating, and forming a cathode or an anode thereon.

The organic light emitting diode may be applied to an organic light emitting display device.

Hereinafter, the embodiments are illustrated in more detail with reference to examples. However, these examples are exemplary, and the present scope is not limited thereto.

Hereinafter, starting materials and reactants used in Examples and Synthesis Examples were purchased from Sigma-Aldrich Co. Ltd., TCI Inc., Tokyo chemical industry or P&H tech as far as there in no particular comment or were synthesized by known methods.

### (Synthesis of First Compound)

The compounds presented as a more specific example of the compound of the present invention were synthesized through the following steps.

### Synthesis Example 1: Synthesis of Compound 1-38

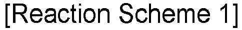

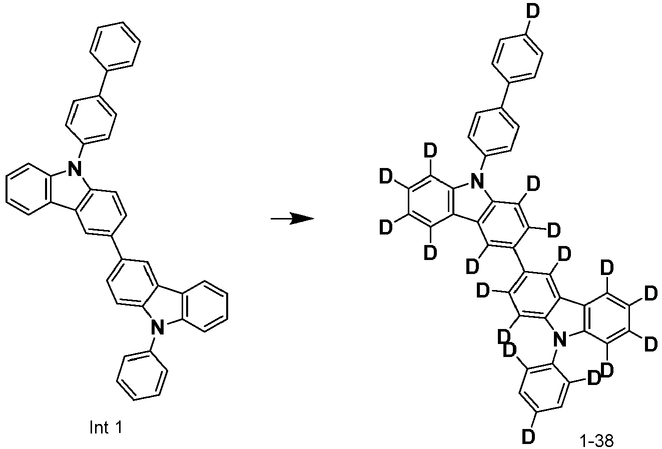

### 1st step: Synthesis of Compound Int 1

Compound Int 1 was synthesized by referring to the method disclosed in Korean Publication No. 2016-0049842.

### 2nd step: Synthesis of Compound 1-38

30 g (0.0535 mol) of Compound Int 1, 40 g (0.267 mol) of trifluoromethanesulfonic acid, and 282 g (3.35 mol) of D₆-benzene were put and then, stirred at 10 °C for 24 hours. Subsequently, purified water was added thereto and then, neutralized with a saturated K₃PO₄ solution. An organic layer therefrom was concentrated and column-purified to obtain 18 g of Compound 1-38 (a white solid, LC-Mass Mz 578.79, C₄₂H₁₀D₁₈N₂).

### Synthesis Example 2: Synthesis of Compound 1-110

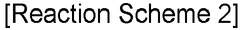

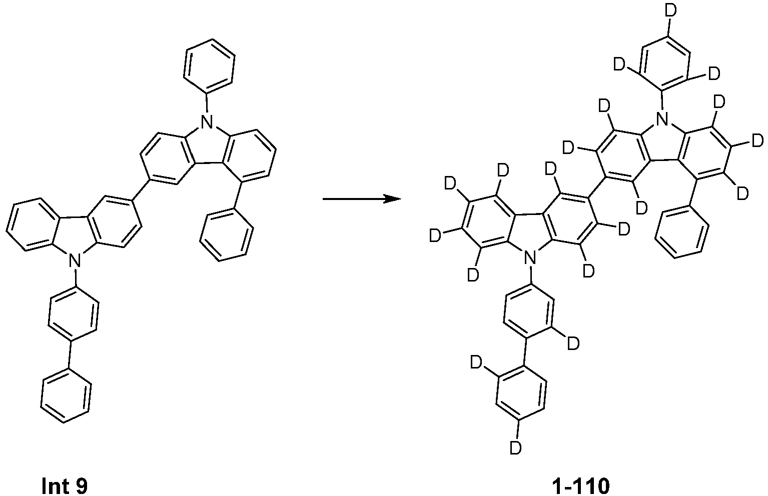

Compound Int 9 was synthesized by referring to the method disclosed in Korean Publication KR10-2018-0035076. Compound 1-110 was synthesized in the same manner as in Synthesis Example 1 except that compound Int 9 was used instead of Int 1 in the 2^{nd} step.

### (Synthesis of Second Compound)

### Synthesis Example 3: Synthesis of Compound 2-B-30

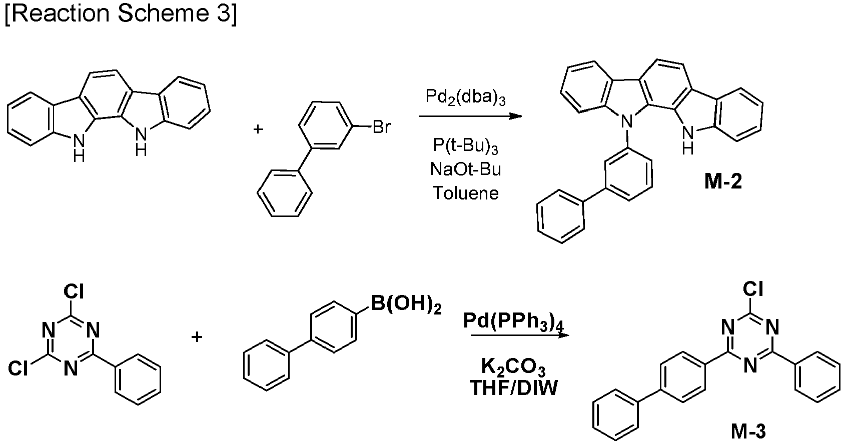

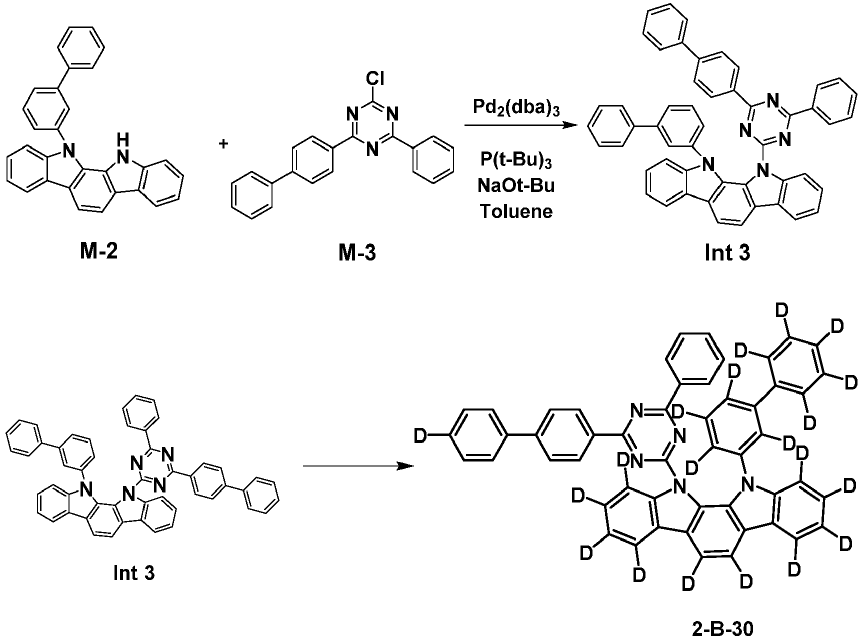

### 1st step: Synthesis of Intermediate M-2

11,12-dihydroindolo[2,3-a]carbazole (78.35 g, 305.69 mmol, CAS No. 60511-85-5), 3-bromobiphenyl (59.38 g, 254.74 mmol), NaOt-Bu (26.93 g, 280.22 mmol, and Pd₂(dba)₃ (7 g, 7.64 mmol) were suspended in 1,400 ml of toluene, and P(t-Bu)₃ (3.64 ml, 15.28 mmol) was added thereto and then, stirred under reflux for 12 hours. After adding distilled water to the reaction solution, the mixture was separated. The obtained product was purified through silica gel column to obtain Intermediate M-2 (68.7 g, 57%).

### 2nd step: Synthesis of Intermediate M-3

2,4-dichloro-6-phenyl-1,3,5-triazine (74.50 g, 329.56 mmol) and 4-biphenylboronic acid (55.47 g, 280.12 mmol) were dissolved in 0.7 L of a mixed solution of tetrahydrofuran (THF) and distilled water in a ratio of 3:1, and sodium tert-butoxide (68.32 g, 494.34 mmol) was added thereto and then, stirred under reflux for 12 hours. After cooling the reaction solution and separating layers, an organic layer therefrom was collected and concentrated. The concentrated residue was purified through silica gel column to obtain Intermediate M-3 (75.9 g, 67%).

### 3rd step: Synthesis of Compound Int 3

Compound Int 3 was obtained in the same manner as in the synthesis method of Intermediate M-2 by using Intermediate M-2 and Intermediate M-3.

### 4th step: Synthesis of Compound 2-B-30

20 g (0.0279 mol) of Compound Int 3, 12.33 ml (0.1397 mol) of trifluoromethanesulfonic acid, and 335.49 ml (3.4924 mol) of D₆-benzene were put and then, stirred at 10 °C for 24 hours. After adding purified water thereto and conducting neutralization with a saturated K₃PO₄ solution, an organic layer therefrom was concentrated and column-purified to obtain Compound 2-B-30 (14 g, 70%).

### Synthesis Example 4: Synthesis of Compound 2-B-22

Compound Int 4 and Compound 2-B-22 were respectively synthesized in the same manner as in the 1^{st} to 4^{th} steps of Synthesis Example 3 except that 3-biphenylboronic acid was used instead of the 4-biphenylboronic acid in the 2^{nd} step of Synthesis Example 3.

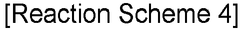

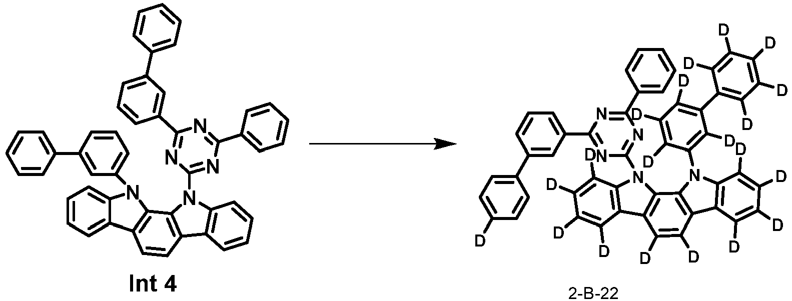

### Synthesis Example 5: Synthesis of Compound 2-B-80

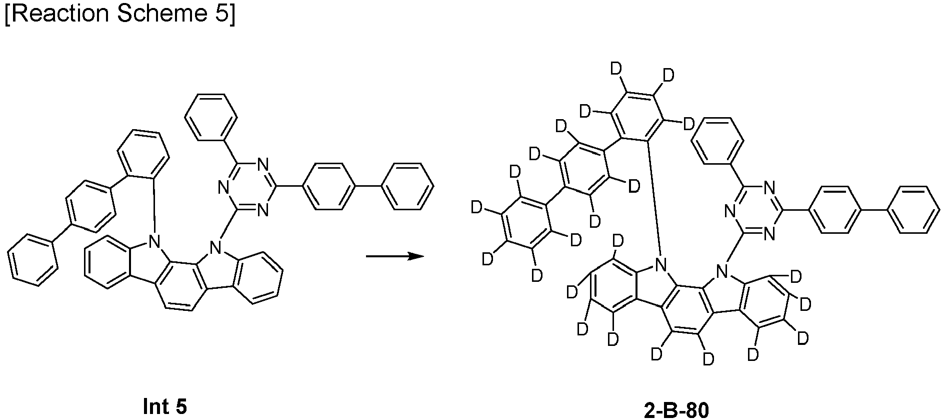

Compound Int 5 and Compound 2-B-80 were respectively synthesized in the same manner as in the 1^{st} to 4^{th} steps of Example 3 except that 2-bromo-1,1':4',1"-terphenyl (CAS No. 3282-24-4) was used instead of the 3-bromobiphenyl in the 1^{st} step of Synthesis Example 3.

### Synthesis Example 6: Synthesis of Compound 2-B-82

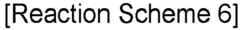

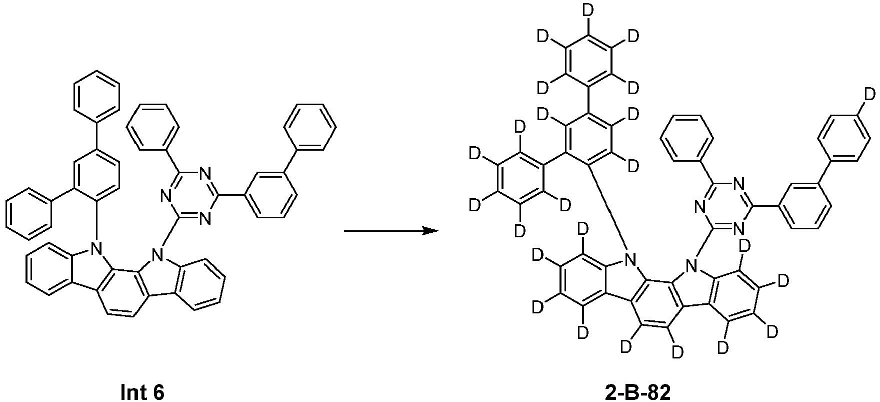

Compound Int 6 and Compound 2-B-82 were respectively synthesized in the same manner as in the 1^{st} to 4^{th} steps of Example 3 except that 4'-bromo-1,1':3',1"-terphenyl (CAS No. 60631-83-6) was used instead of the 3-bromobiphenyl in the 1^{st} step of Synthesis Example 3, and 3-biphenylboronic acid was used instead of the 4-biphenylboronic acid in the 2^{nd} step of Synthesis Example 3.

### Synthesis Example 7: Synthesis of Compound 2-B-36

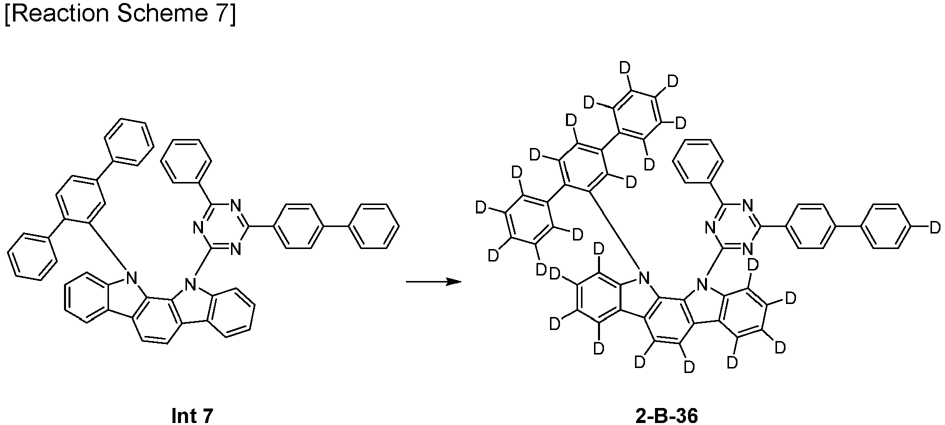

Compound Int 7 and Compound 2-B-36 were respectively synthesized in the same manner as in the 1^{st} to 4^{th} steps of Example 3 except that 2'-bromo-1,1':4',1"-terphenyl (CAS No. 3282-25-5) was used instead of the 3-bromobiphenyl in the 1^{st} step of Synthesis Example 3.

### Synthesis Example 8: Synthesis of Compound 2-B-2

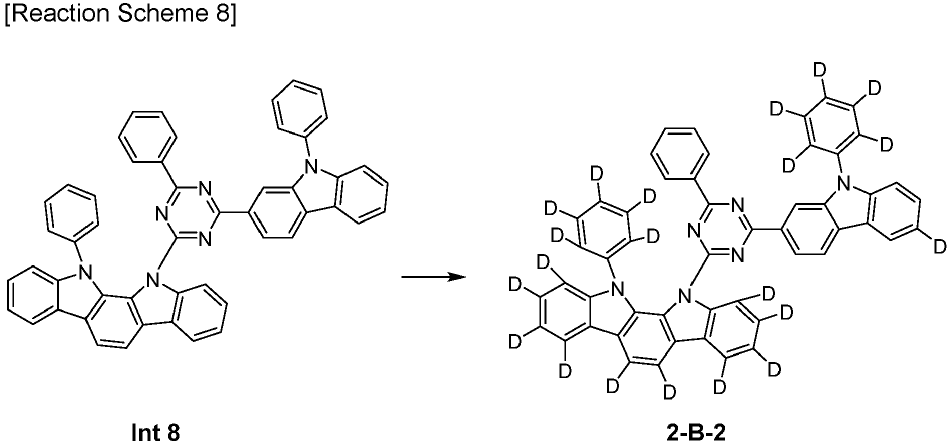

Compound Int 8 and Compound 2-B-2 were respectively synthesized in the same manner as in the 1^{st} to 4^{th} steps of Example 3 except that 11,12-dihydro-11-phenylindolo[2,3-a]carbazole (CAS No. 1024598-06-8) was used instead of Intermediate M-2 in the 3^{rd} step of Synthesis Example 3, and 9-phenylcarbazole-2-boronic acid (CAS No. 1001911-63-2) was used instead of the 4-biphenylboronic acid in the 2^{nd} step of Synthesis Example 3.

### (Manufacture of Organic Light Emitting Diode)

### Example 1

A glass substrate coated with ITO (indium tin oxide) was washed with distilled water ultrasonic wave. After washing with the distilled water, the glass substrate was washed with a solvent such as isopropyl alcohol, acetone, methanol, and the like ultrasonically and dried and then, moved to a plasma cleaner, cleaned by using oxygen plasma for 10 minutes, and moved to a vacuum depositor. This prepared ITO transparent electrode was used as an anode, Compound A doped with 3% NDP-9 (Novaled GmbH) was vacuum-deposited on the ITO substrate to form a 100 Å-thick hole injection layer, and Compound A is deposited on the hole injection layer to a thickness of 1350 Å to form a hole transport layer. Compound 8 was deposited on the hole transport layer to a thickness of 350 Å to form a hole transport auxiliary layer. On the hole transport auxiliary layer, a 330 Å-thick light emitting layer was formed by vacuum deposition simultaneously using Compound 1-38 obtained in Synthesis Example 1 and Compound 2-B-30 obtained in Synthesis Example 3 as a host and doping 7 wt% of PhGD. Subsequently, Compound C was deposited to form a 50 Å-thick electron transport auxiliary layer on the light emitting layer, and Compound D and Liq were simultaneously vacuum-deposited in a weight ratio of 1:1 to form a 300 Å-thick electron transport layer. On the electron transport layer, Liq and Al were sequentially vacuum-deposited to be 15 Å-thick and 1200 Å-thick, manufacturing an organic light emitting diode.

ITO/ Compound A (3% NDP-9 doping, 100 Å)/Compound A (1350 Å)/Compound B (350 Å)/EML[93 wt% of host (Compound 1-38: Compound 2-B-30 = 4:6 w/w): 7 wt% of PhGD] (330 Å)/ Compound C (50 Å)/ Compound D:LiQ (300 Å)/Liq (15 Å) / Al (1200 Å).
Compound A: N-(biphenyl-4-yl)-9,9-dimethyl-N-(4-(9-phenyl-9H-carbazol-3-yl)phenyl)-9H-fluoren-2-amine
Compound B: N,N-bis(9,9-dimethyl-9H-fluoren-4-yl)-9,9-spirobi(fluorene)-2-amine
Compound C: 2-[3'-(9,9-Dimethyl-9H-fluoren-2-yl)[1,1'-biphenyl]-3-yl]-4,6-diphenyl-1,3,5-triazine
Compound D: 2-[4-[4-(4'-Cyano-1,1'-biphenyl-4-yl)-1-naphthyl]phenyl]-4,6-diphenyl-1,3,5-triazine

### [PhGD]

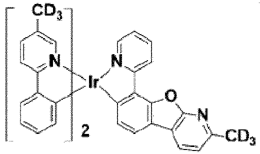

### Examples 2 to 6 and Comparative Examples 1 to 18

Organic light emitting diodes according to Examples 2 to 6 and Comparative Examples 1 to 18 were manufactured in the same manner as in Example 1, except for changing the host as described in Tables 1 to 6.

### Evaluation

### (1) Measurement of Current Density Change Depending on Voltage Change

The obtained organic light emitting diodes were measured regarding a current value flowing in the unit device, while increasing the voltage from 0 V to 10 V using a current-voltage meter (Keithley 2400), and the measured current value was divided by area to provide the results.

### (2) Measurement of Luminance Change Depending on Voltage Change

Luminance was measured by using a luminance meter (Minolta Cs-1000A), while the voltage of the organic light emitting diodes was increased from 0 V to 10 V.

### (3) Measurement of Luminous Efficiency

Luminous efficiency (cd/A) at the same current density (10 mA/cm²) were calculated by using the luminance and current density from (1) and (2) above.

### (4) Measurement of Life-span

The results were obtained by measuring a time when luminous efficiency (cd/A) was decreased down to 90%, while luminance (cd/m²) was maintained to be 6,000 cd/m².

The values shown in Tables 1 to 6 are relative values based on the values of Comparative Example 1, Comparative Example 4, Comparative Example 7, Comparative Example 10, Comparative Example 13, Comparative Example 16, Comparative Example 19, and Comparative Example 22, respectively.

**(Table 1)**

| | First compound | Second compound | Life-span ratio |
|---|---|---|---|
| Comparative Example 1 | Int 1 | Int 3 | 100% |
| Comparative Example 2 | Int 1 | 2-B-30 | 113% |
| Comparative Example 3 | 1-38 | Int 3 | 129% |
| Example 1 | 1-38 | 2-B-30 | 150% |

**(Table 2)**

| | First compound | Second compound | Life-span ratio |
|---|---|---|---|
| Comparative Example 4 | Int 1 | Int 2 | 100% |
| Comparative Example 5 | Int 1 | 2-B-22 | 128% |
| Comparative Example 6 | 1-38 | Int 4 | 124% |
| Example 2 | 1-38 | 2-B-22 | 150% |

**(Table 3)**

| | First compound | Second compound | Life-span ratio |
|---|---|---|---|
| Comparative Example 7 | Int 1 | Int 5 | 100% |
| Comparative Example 8 | Int 1 | 2-B-80 | 123% |
| Comparative Example 9 | 1-38 | Int 5 | 122% |
| Example 3 | 1-38 | 2-B-80 | 141% |

**(Table 4)**

| | First compound | Second compound | Life-span ratio |
|---|---|---|---|
| Comparative Example 10 | Int 1 | Int 6 | 100% |
| Comparative Example 11 | Int 1 | 2-B-82 | 136% |
| Comparative Example 12 | 1-38 | Int 6 | 122% |
| Example 4 | 1-38 | 2-B-82 | 168% |

**(Table 5)**

| | First compound | Second compound | Life-span ratio |
|---|---|---|---|
| Comparative Example 13 | Int 1 | Int 7 | 100% |
| Comparative Example 14 | Int 1 | 2-B-36 | 111 % |
| Comparative Example 15 | 1-38 | Int 7 | 122% |
| Example 5 | 1-38 | 2-B-36 | 141% |

**(Table 6)**

| | First compound | Second compound | Life-span ratio |
|---|---|---|---|
| Comparative Example 16 | 3-8 | Int 8 | 100% |
| Comparative Example 17 | 3-8 | 2-B-2 | 110% |
| Comparative Example 18 | 1-110 | Int 8 | 124% |
| Example 6 | 1-110 | 2-B-2 | 138% |

Referring to Tables 1 to 6, the life-span characteristics of the organic light emitting diodes according to the examples of the present invention are significantly improved compared to the organic light emitting diodes according to the comparative examples.

While this invention has been described in connection with what is presently considered to be practical example embodiments, it is to be understood that the invention is not limited to the disclosed embodiments, but, on the contrary, is intended to cover various modifications and equivalent arrangements included within the spirit and scope of the appended claims.

### <Description of symbols>

100: organic light emitting diode
105: organic layer
110: cathode
120: anode
130: light emitting layer
140: hole transport region
150: electron transport region

## Claims

1. A composition for an organic optoelectronic device, comprising
a first compound represented by Chemical Formula 1, and
a second compound represented by a combination of Chemical Formula 2 and Chemical Formula 3: 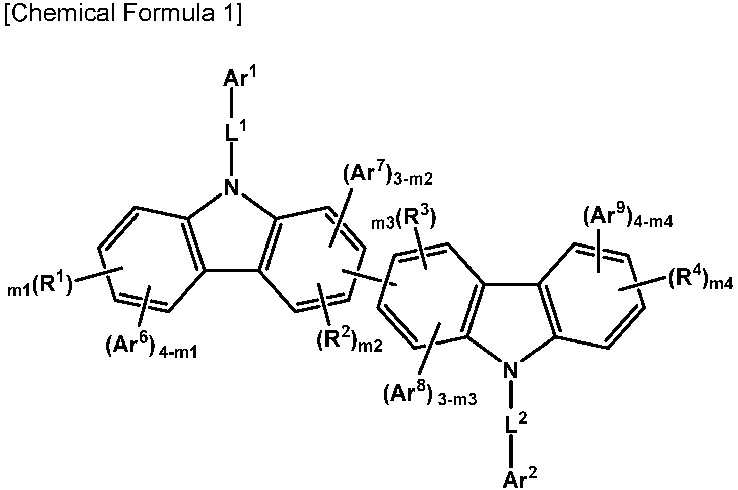
wherein, in Chemical Formula 1,
L¹ and L² are each independently a single bond or a substituted or unsubstituted C6 to C30 arylene group,
Ar¹ and Ar² are each independently a substituted or unsubstituted C6 to C30 aryl group or a substituted or unsubstituted C2 to C30 heterocyclic group,
R¹ to R⁴ are each independently hydrogen, deuterium or a substituted or unsubstituted C6 to C30 aryl group,
Ar⁶ to Ar⁹ are each independently hydrogen or a substituted or unsubstituted C6 to C30 aryl group, and
m1 and m4 are each independently an integer from 1 to 4,
m2 and m3 are each independently an integer from 1 to 3, and
Chemical Formula 1 simultaneously satisfies the following conditions (i) and (ii):
(i) at least one of Ar¹ and Ar² is a C6 to C30 aryl group substituted with at least one deuterium or a C2 to C30 heterocyclic group substituted with at least one deuterium, and
(ii) at least one of R¹ to R⁴ is deuterium; 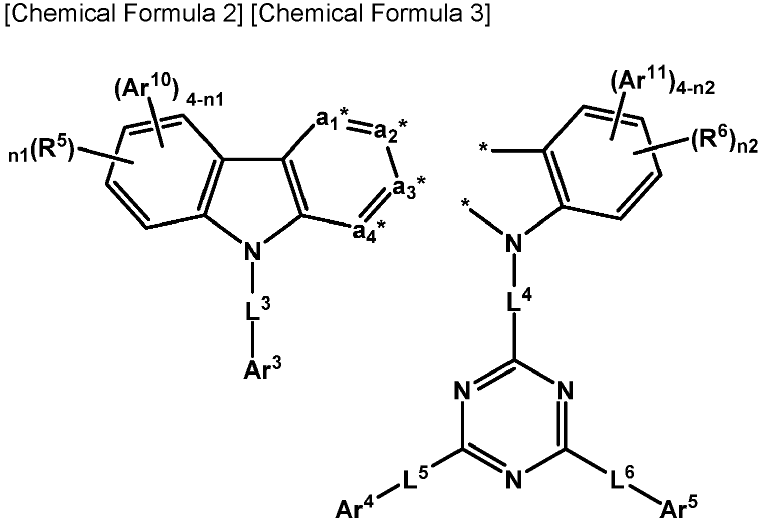
wherein, in Chemical Formula 2 and Chemical Formula 3,
Ar³ to Ar⁵ are each independently a substituted or unsubstituted C6 to C20 aryl group or a substituted or unsubstituted C2 to C30 heterocyclic group,
the two adjacent ones among a₁* to a₄* in Chemical Formula 2 are linking carbons each linked to * in Chemical Formula 3,
the remaining two of a₁* to a₄* in Chemical Formula 2 that are not linked to * in Chemical Formula 3 are CR^{a},
L³ to L⁶ are each independently a single bond, a substituted or unsubstituted C6 to C20 arylene group or a substituted or unsubstituted C2 to C20 heteroarylene group,
R^{a}, R⁵, and R⁶ are each independently hydrogen, deuterium, a cyano group, a halogen, a substituted or unsubstituted amino group, a substituted or unsubstituted C1 to C30 alkyl group, a substituted or unsubstituted C6 to C30 aryl group or a substituted or unsubstituted C2 to C30 heterocyclic group,
Ar¹⁰ and Ar¹¹ are each independently hydrogen or a substituted or unsubstituted C6 to C30 aryl group,
n1 and n2 are each independently an integer from 1 to 4, and
Chemical Formula 2 and Chemical Formula 3 satisfy at least one of the following conditions (iii) and (iv):
(iii) at least one of Ar3 to Ar5 is a C6 to C20 aryl group substituted with at least one deuterium or a C2 to C30 heterocyclic group substituted with at least one deuterium, and
(iv) at least one of R^{a}, R⁵, and R⁶ is deuterium.

2. The composition for the organic optoelectronic device of claim 1, wherein
the first compound is represented by any one of Chemical Formula 1-1 to Chemical Formula 1-10: 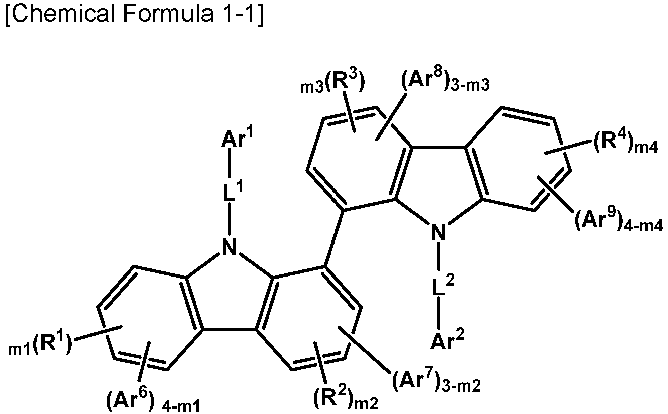 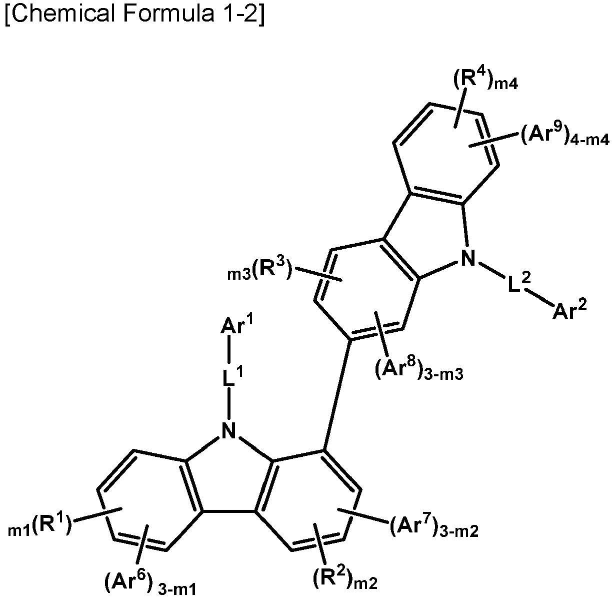 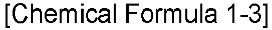 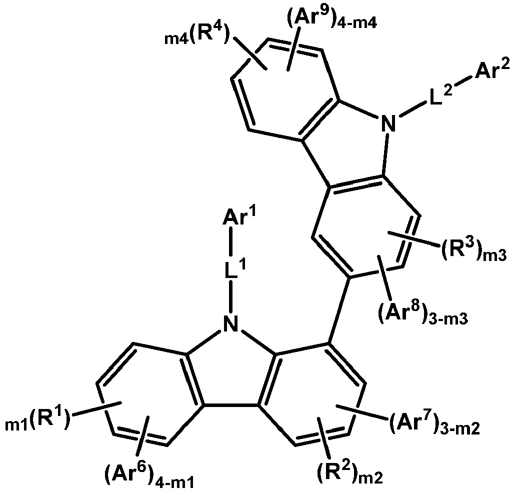 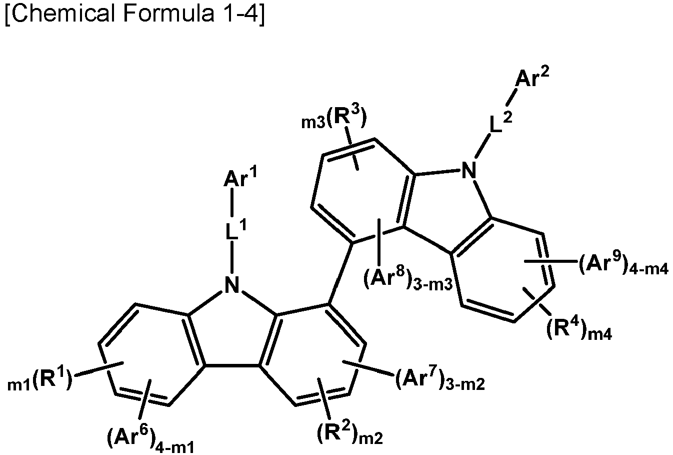 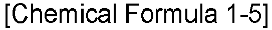 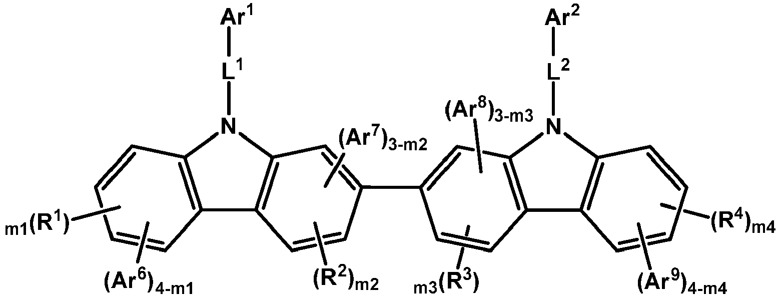 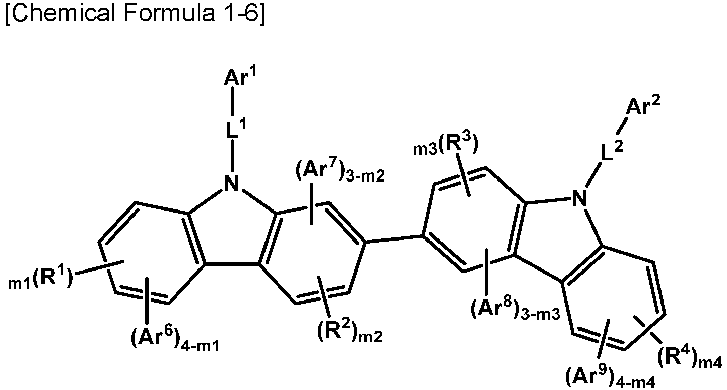 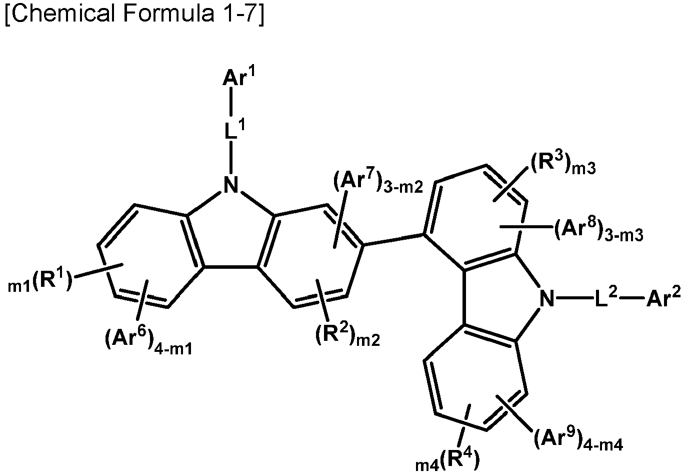 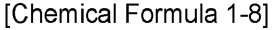 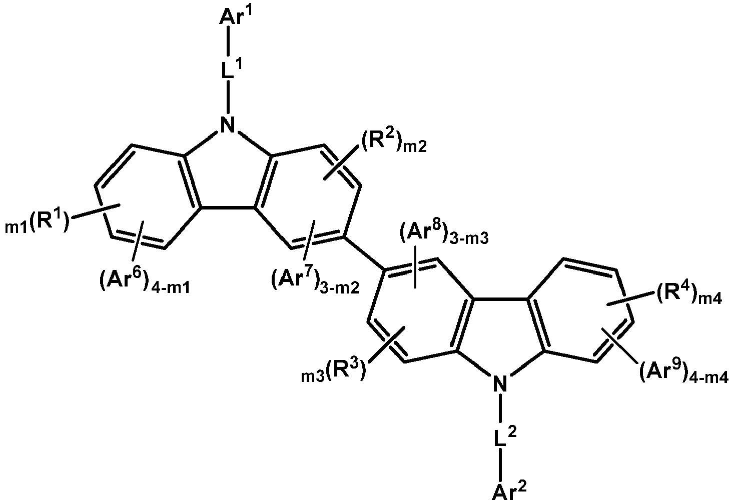 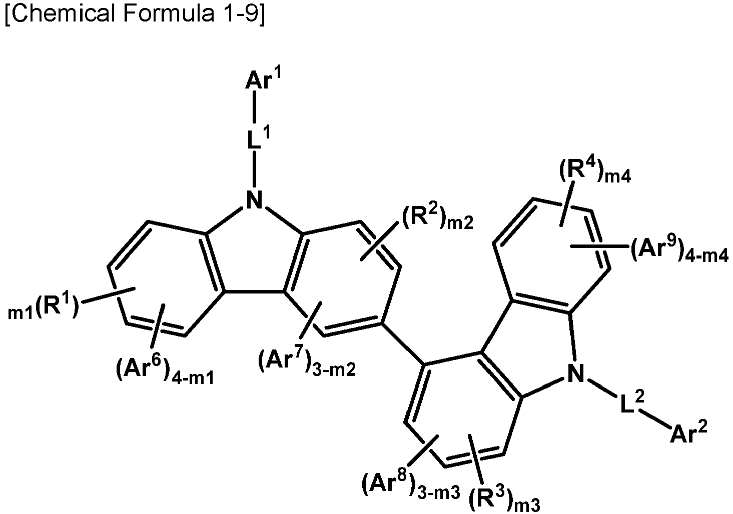 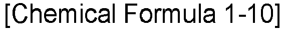 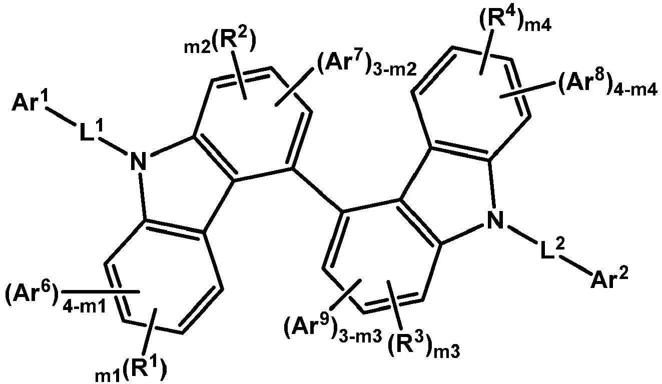
wherein, in Chemical Formula 1-1 to Chemical Formula 1-10,
L¹, L², Ar¹, Ar², Ar⁶ to Ar⁹, R¹ to R⁴, and m¹ to m⁴ are the same as described in claim 1.

3. The composition for the organic optoelectronic device of claim 1, wherein
in Chemical Formula 1, at least one of Ar¹ and Ar² is a phenyl group substituted with at least one deuterium, a biphenyl group substituted with at least one deuterium, a terphenyl group substituted with at least one deuterium, a naphthyl group substituted with at least one deuterium, an anthracenyl group substituted with at least one deuterium, a phenanthrenyl group substituted with at least one deuterium, a triphenylene group substituted with at least one deuterium, a fluorenyl group substituted with at least one deuterium, a dibenzofuranyl group substituted with at least one deuterium, or a dibenzothiophenyl group substituted with at least one deuterium.

4. The composition for the organic optoelectronic device of claim 1, wherein
L¹-Ar¹ and L²-Ar² of Chemical Formula 1 are each independently selected from the substituents listed in Group I -1, and
Group I -2, and at least one of L¹-Ar¹ and L²-Ar² is one selected from substituents listed in Group I -2: 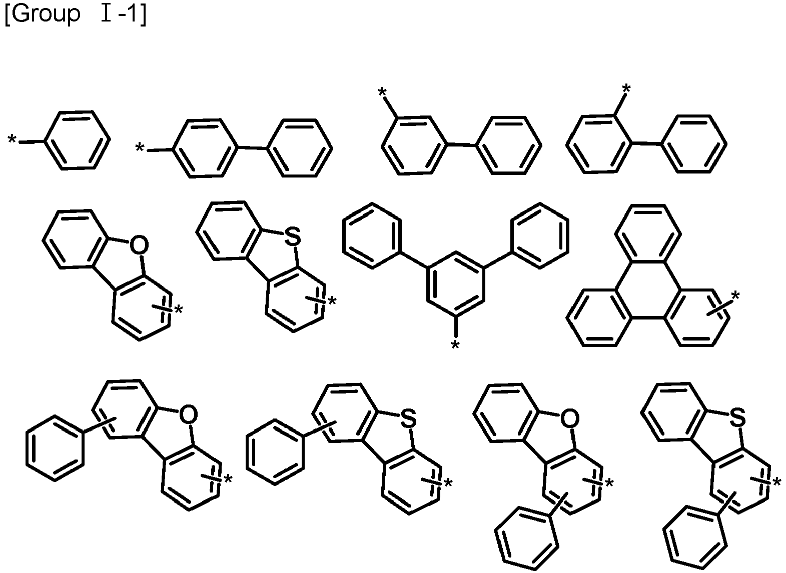 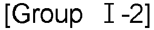 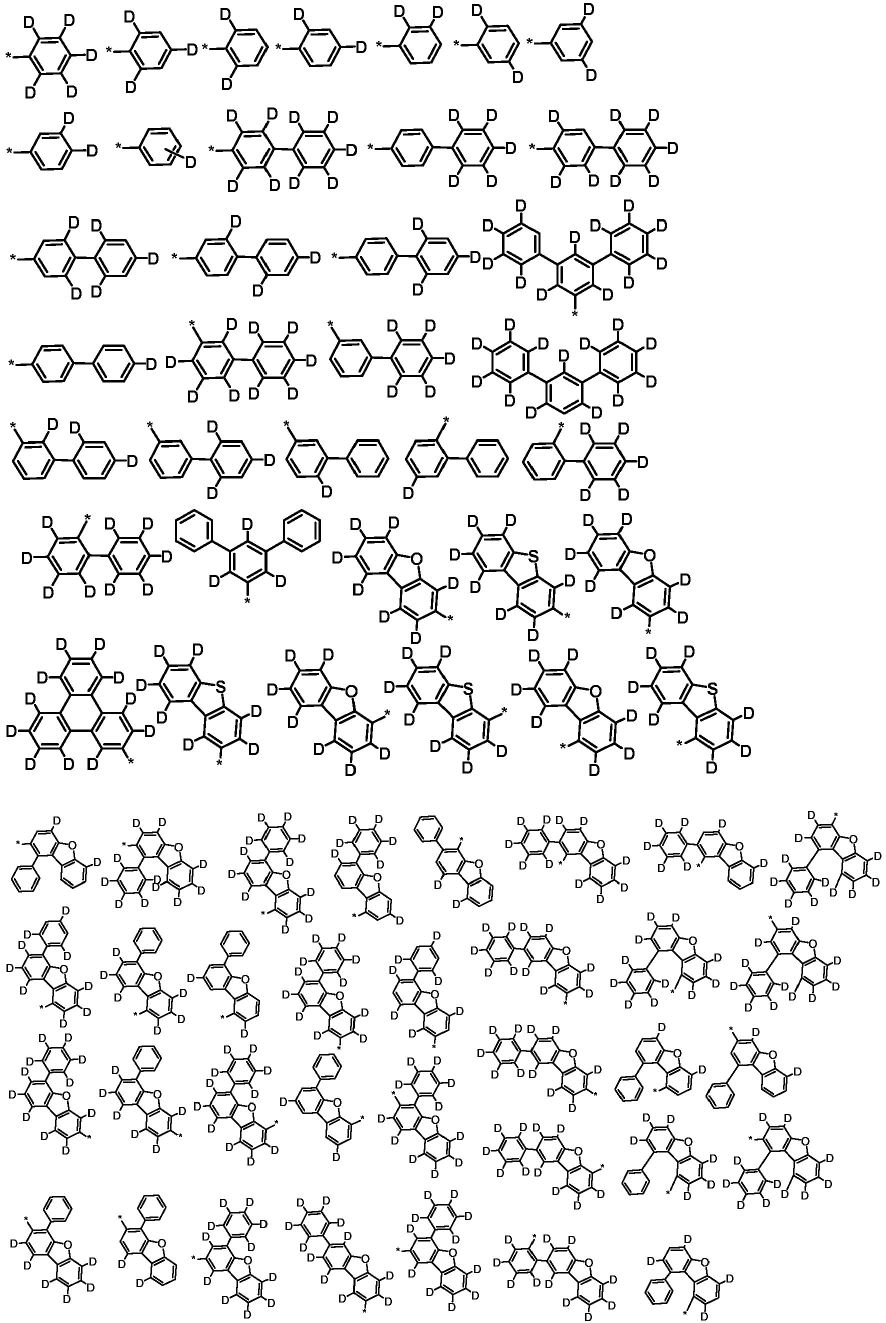 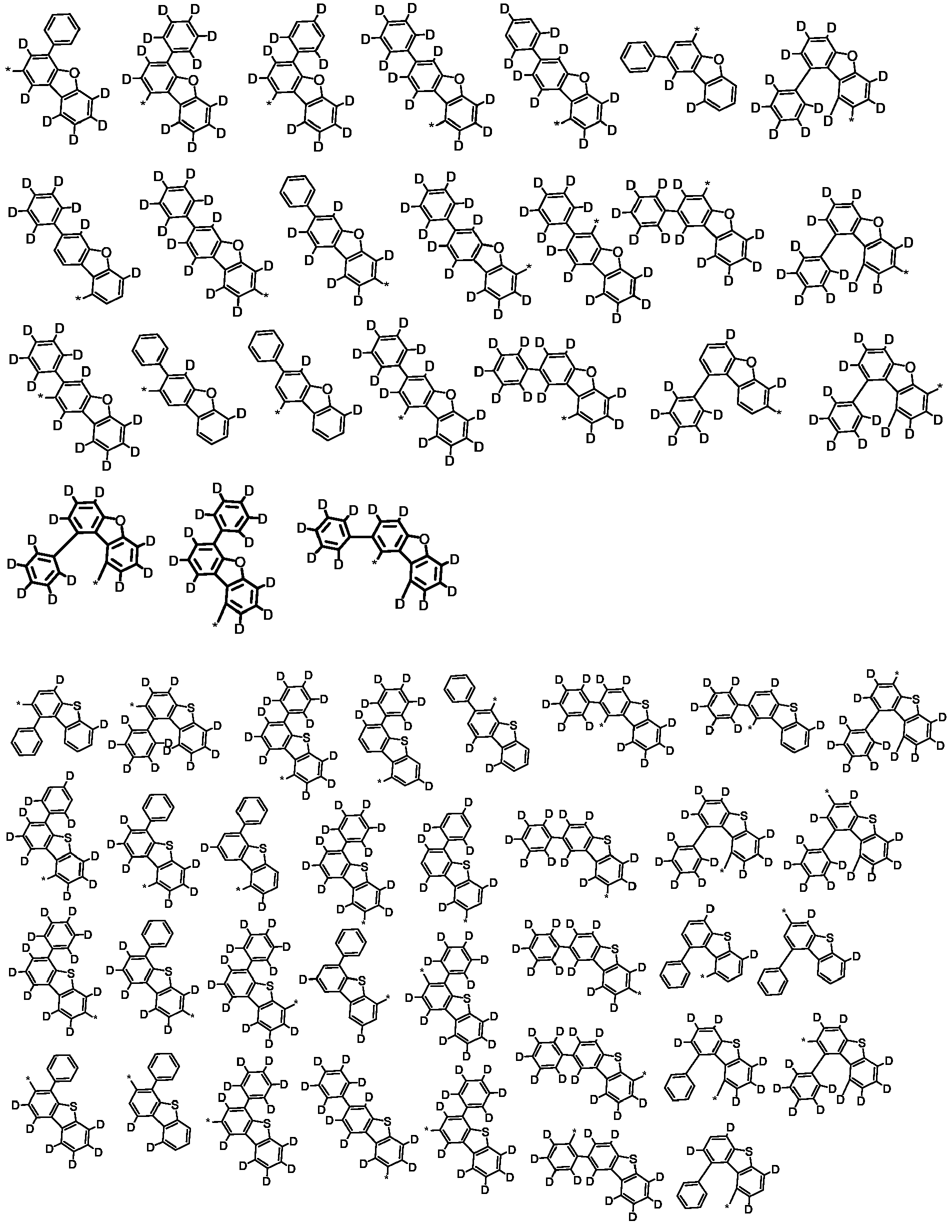 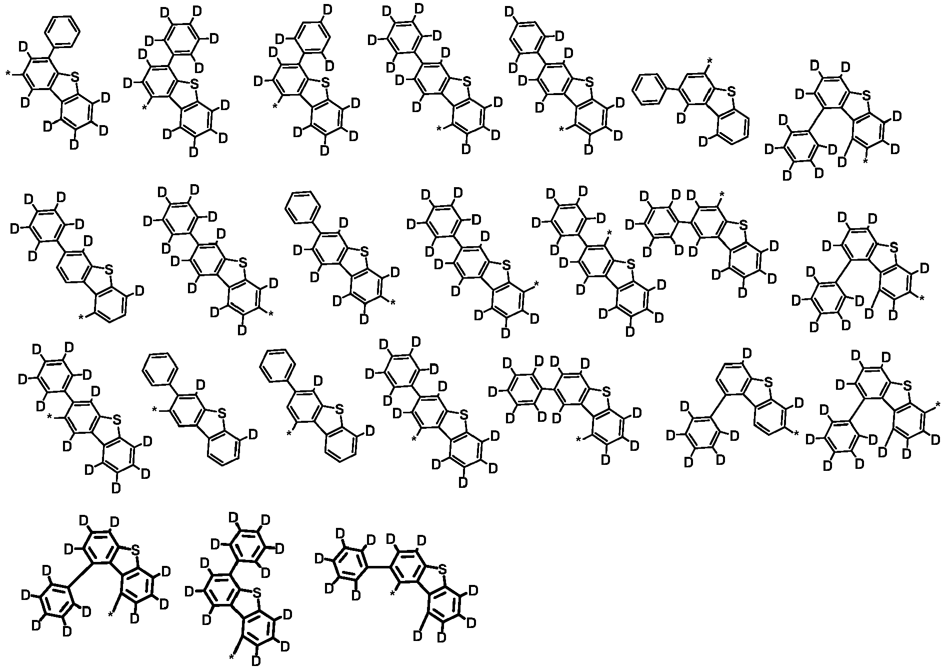
wherein, in Group I -1 and Group I -2, * is a linking point.

5. The composition for the organic optoelectronic device of claim 1, wherein
Chemical Formula 1 is represented by Chemical Formula 1-8a or 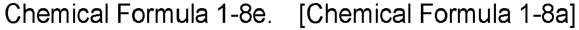 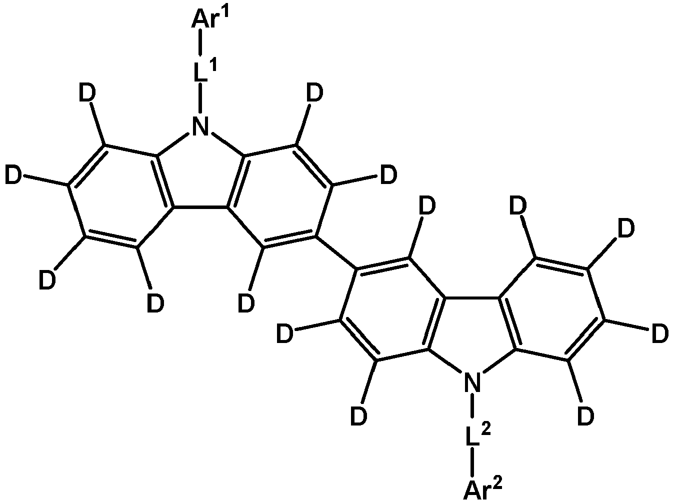 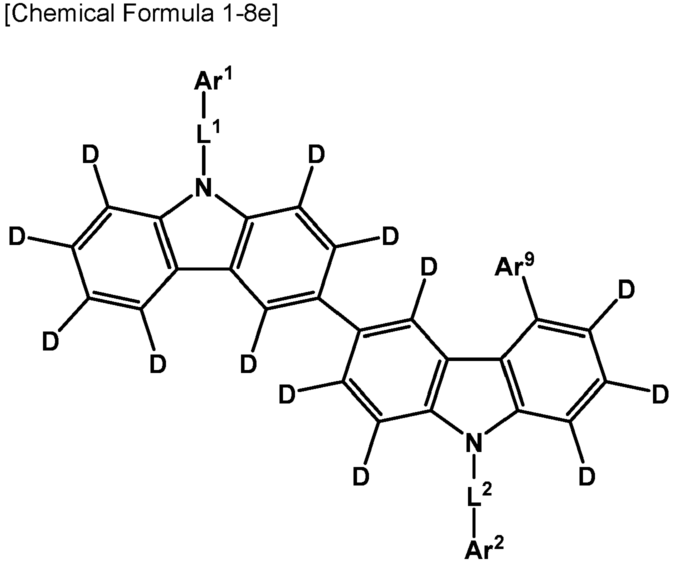
wherein, in Chemical Formula 1-8a and Chemical Formula 1-8e,
L¹, L², Ar¹, Ar², and Ar⁹ are the same as described in claim 1.

6. The composition for the organic optoelectronic device of claim 1, wherein
the first compound is one selected from compounds listed in Group 1:

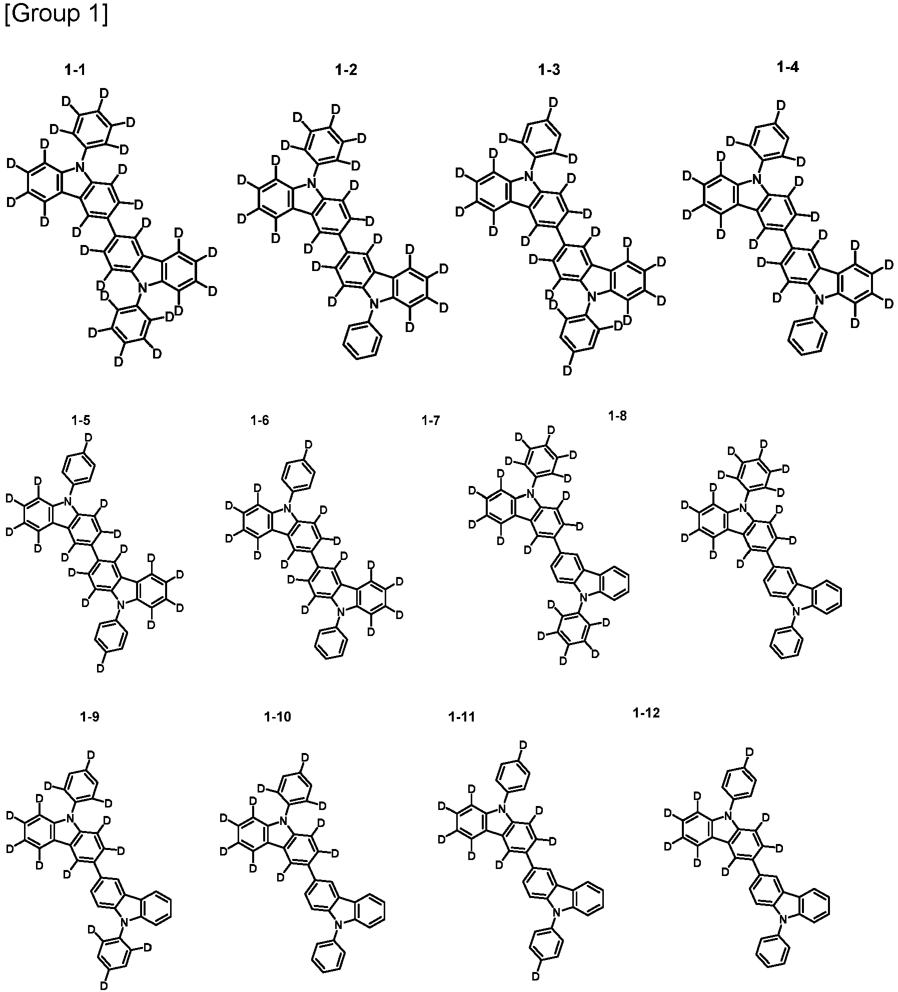

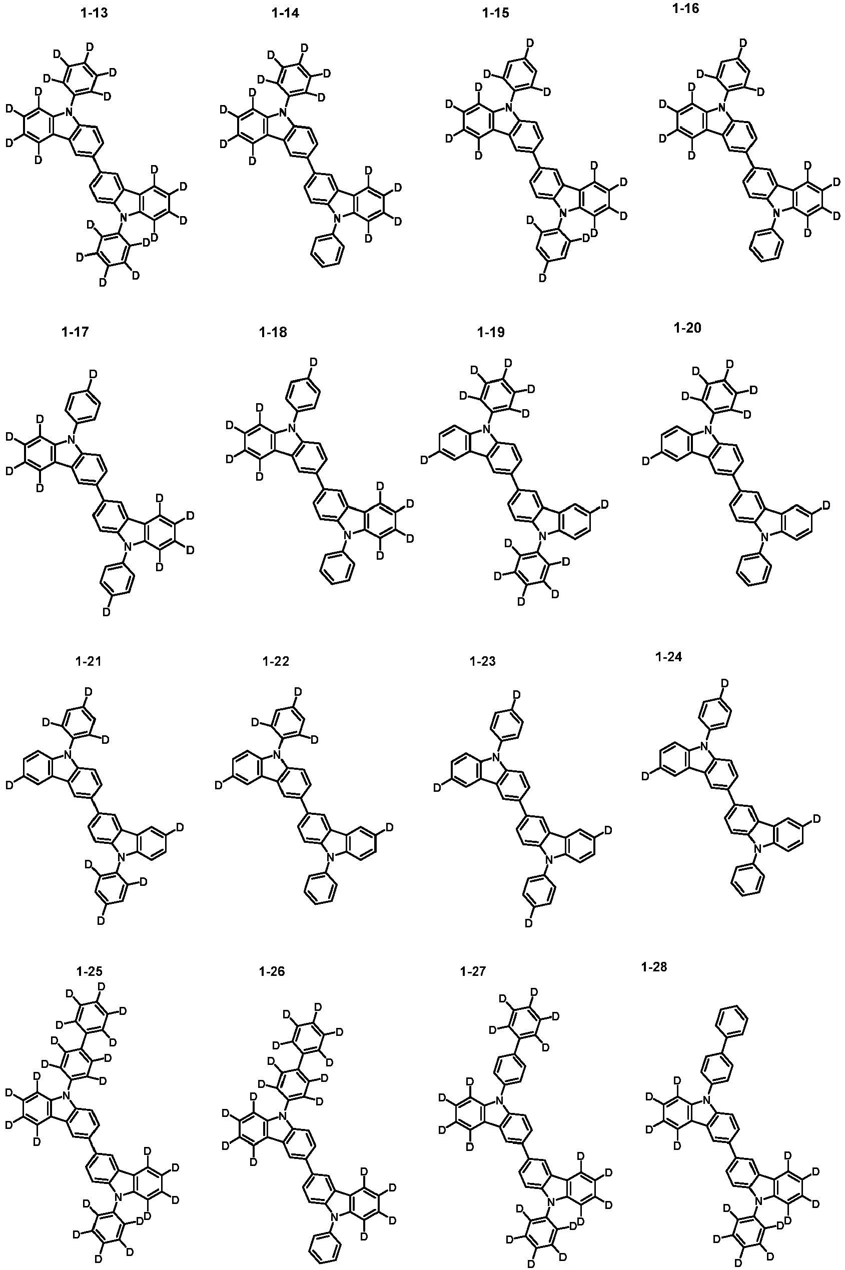

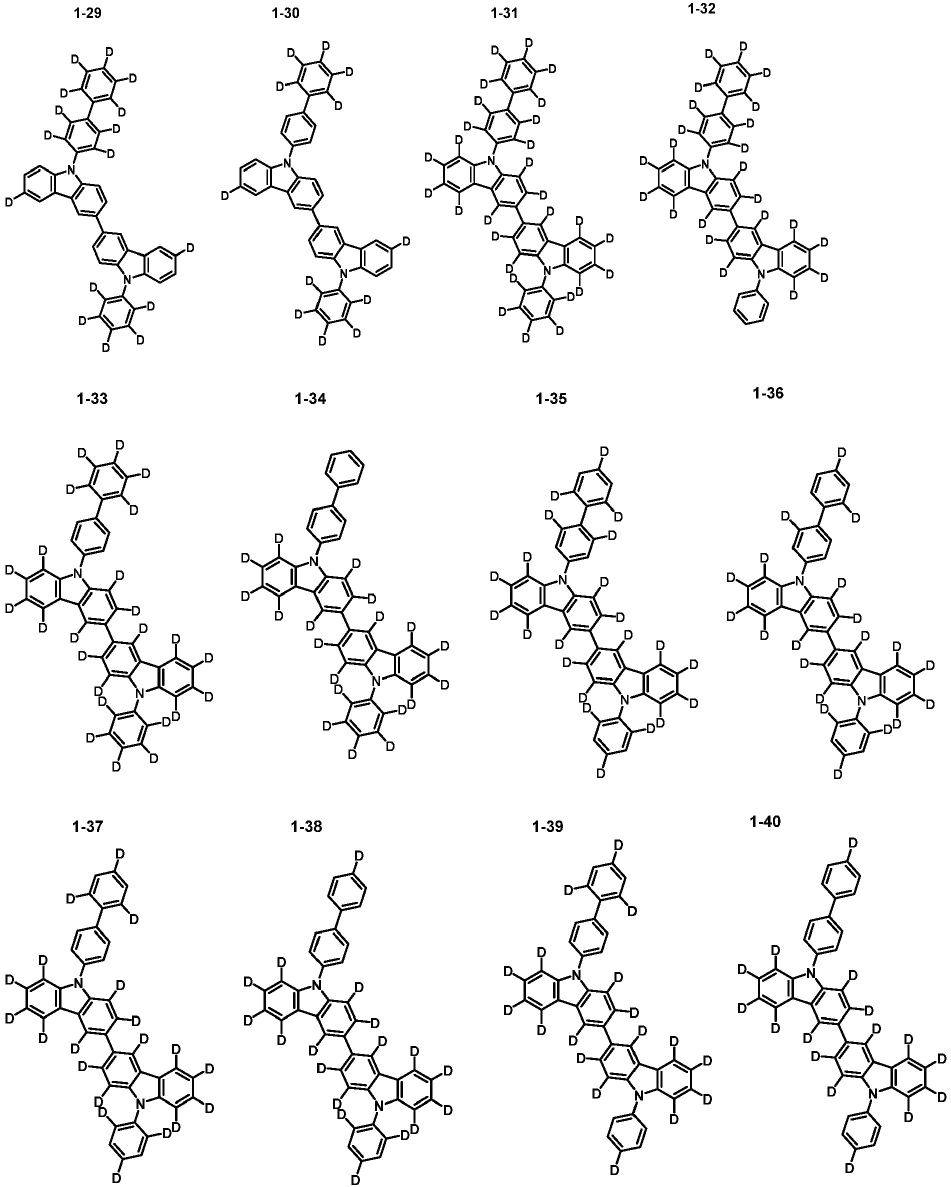

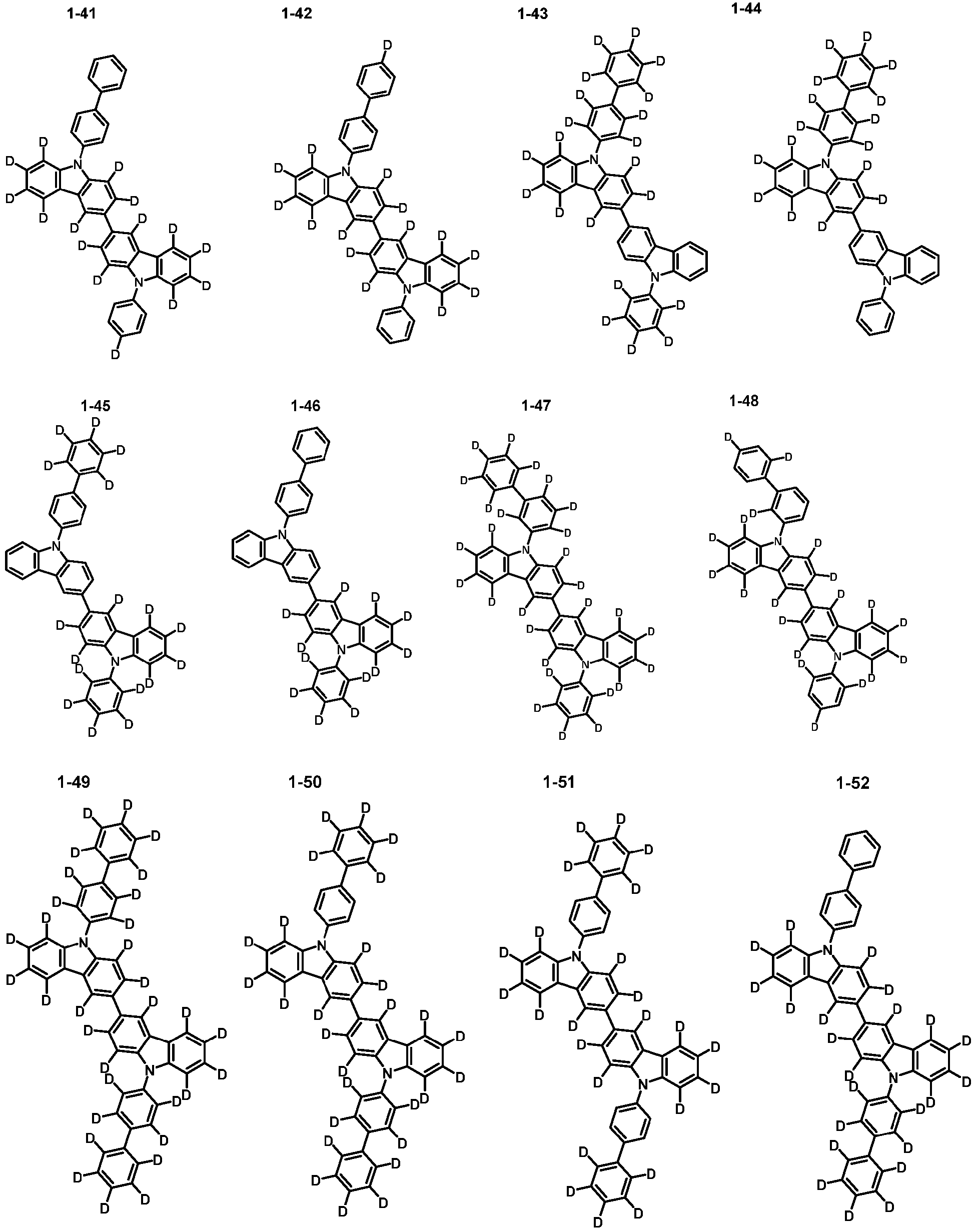

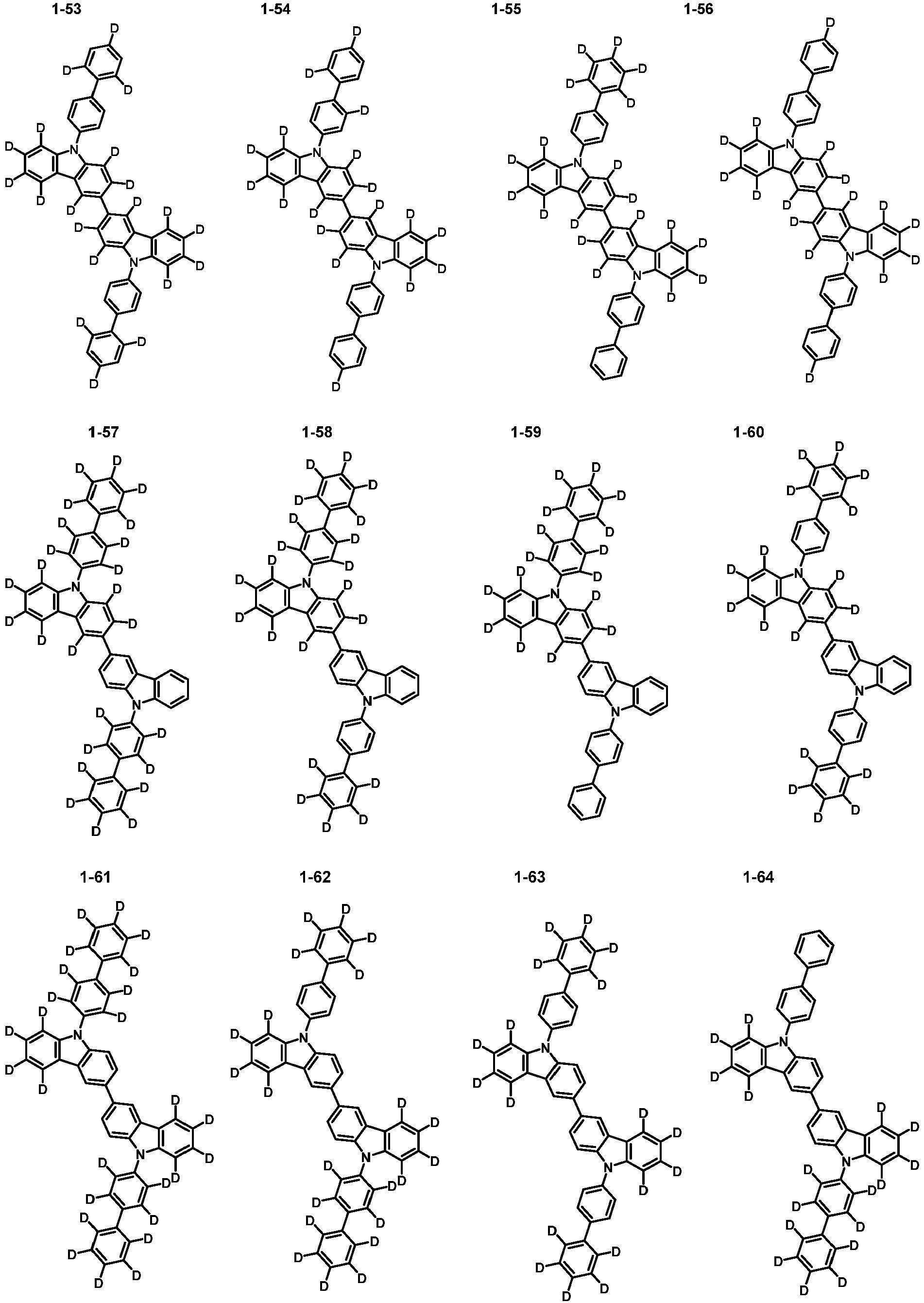

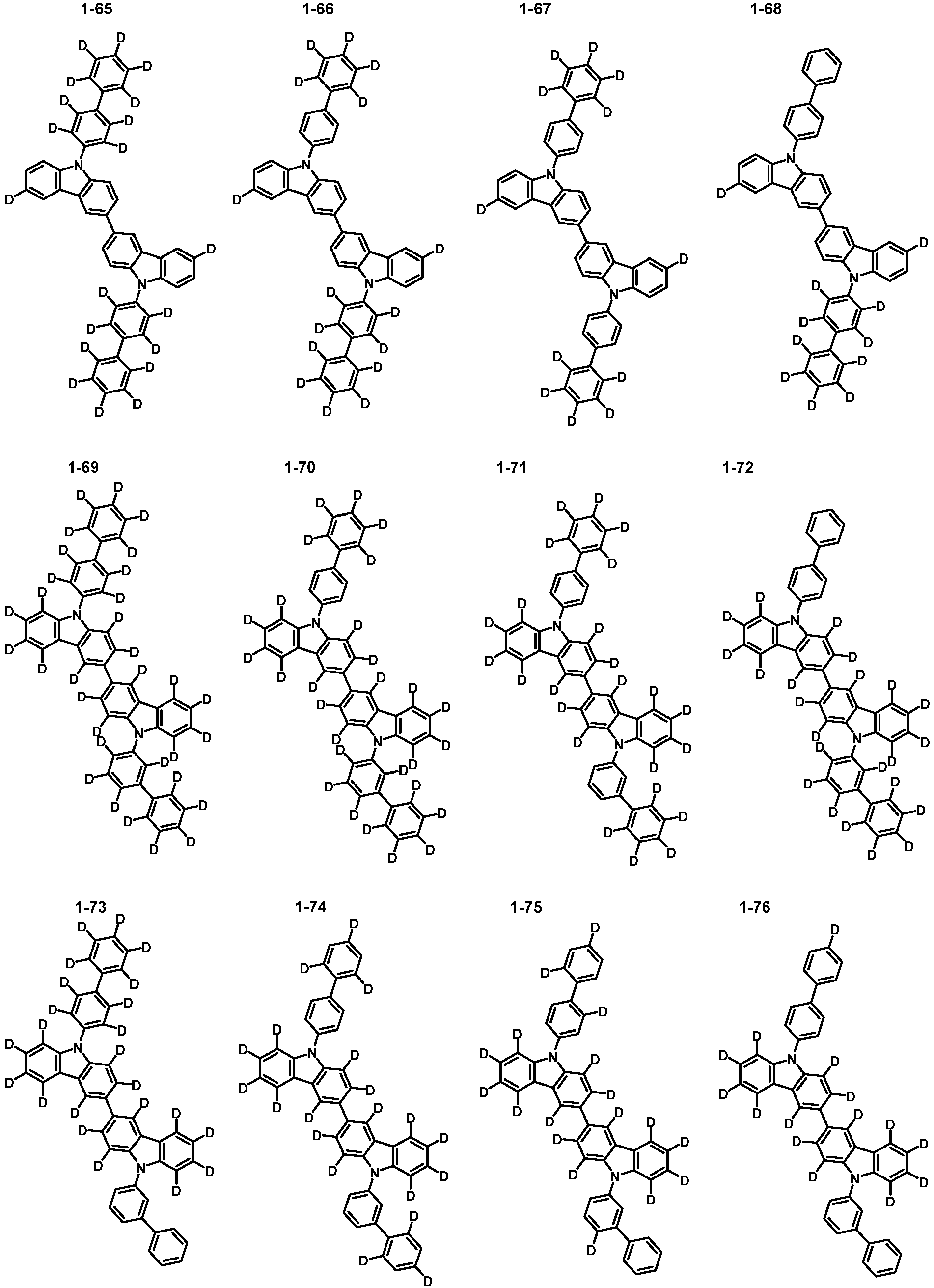

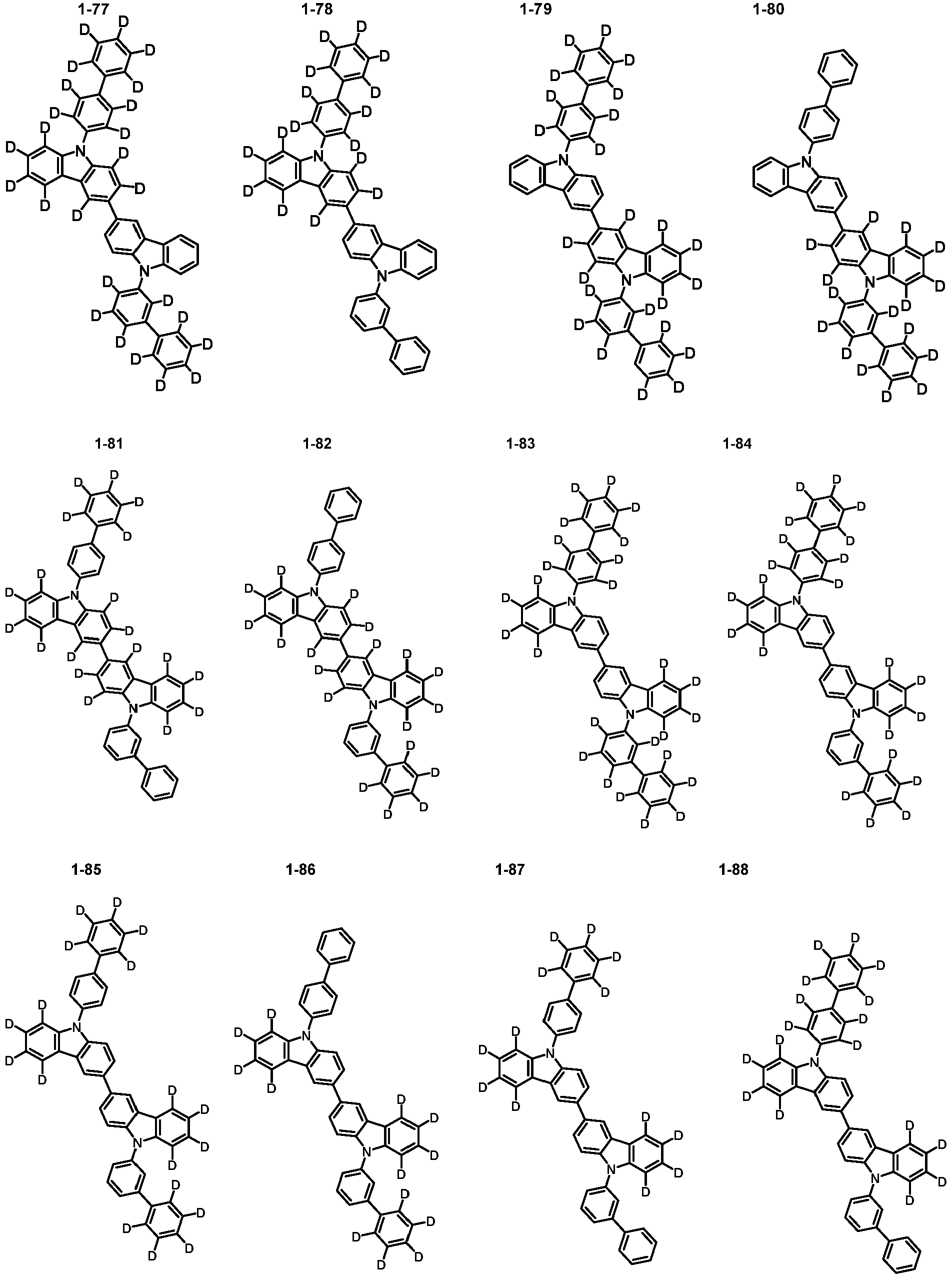

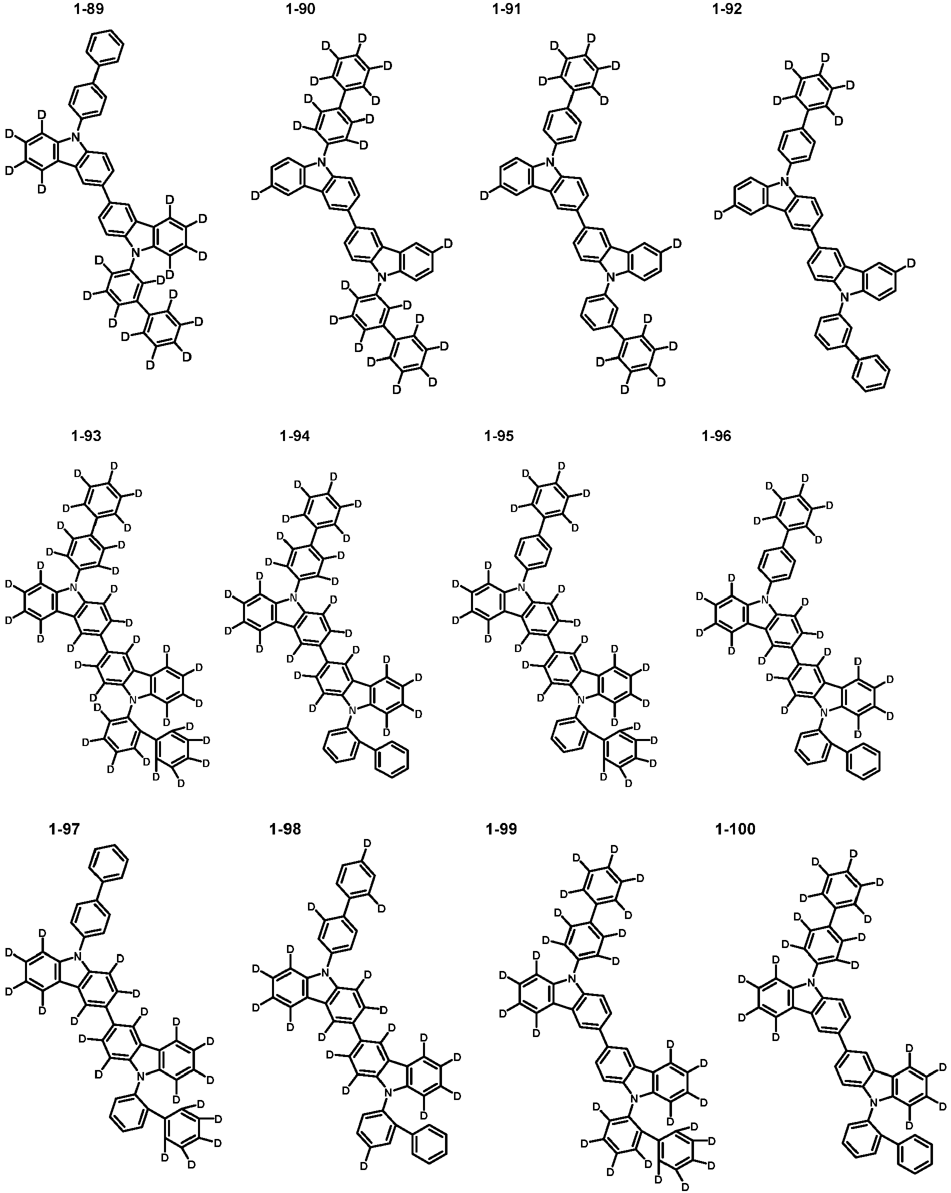

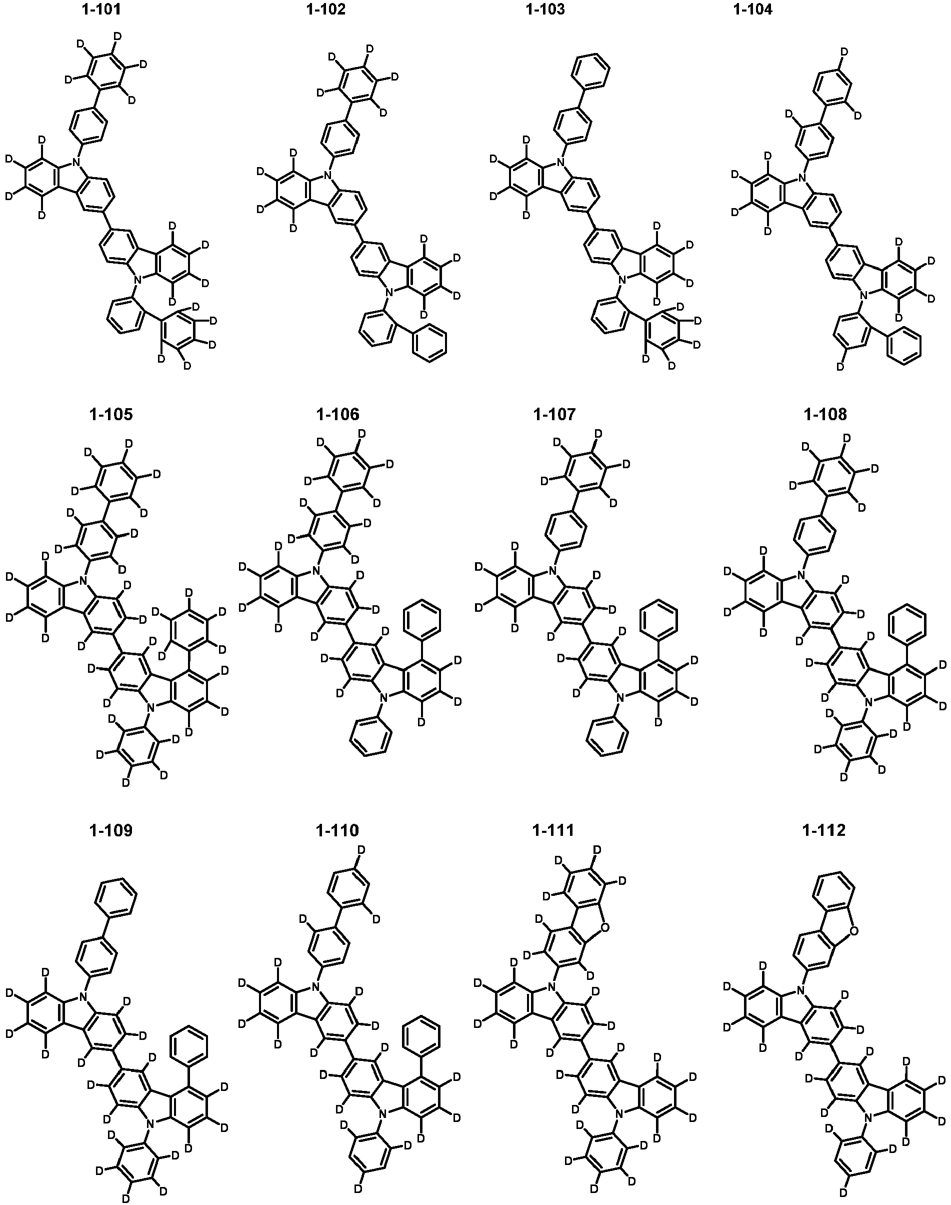

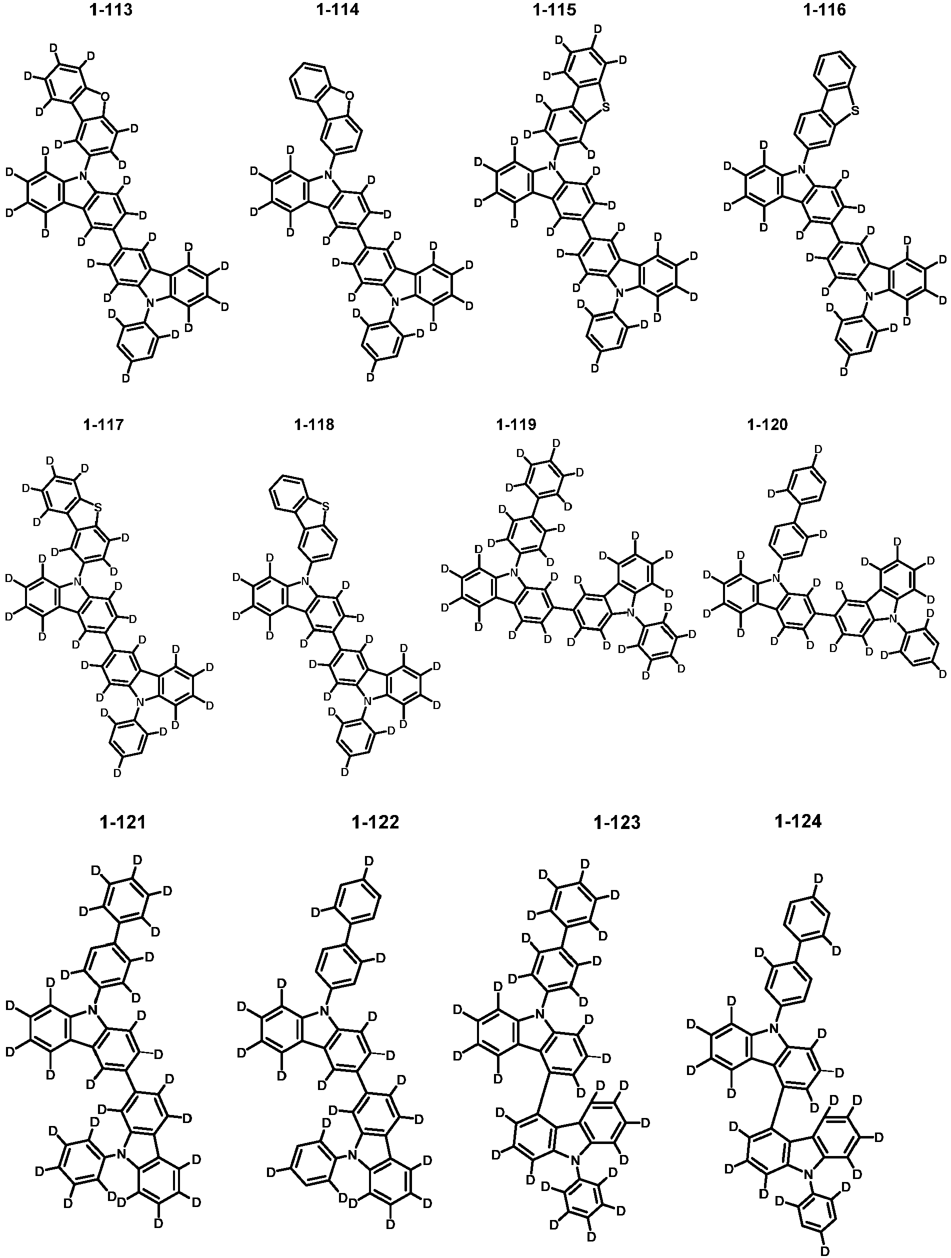

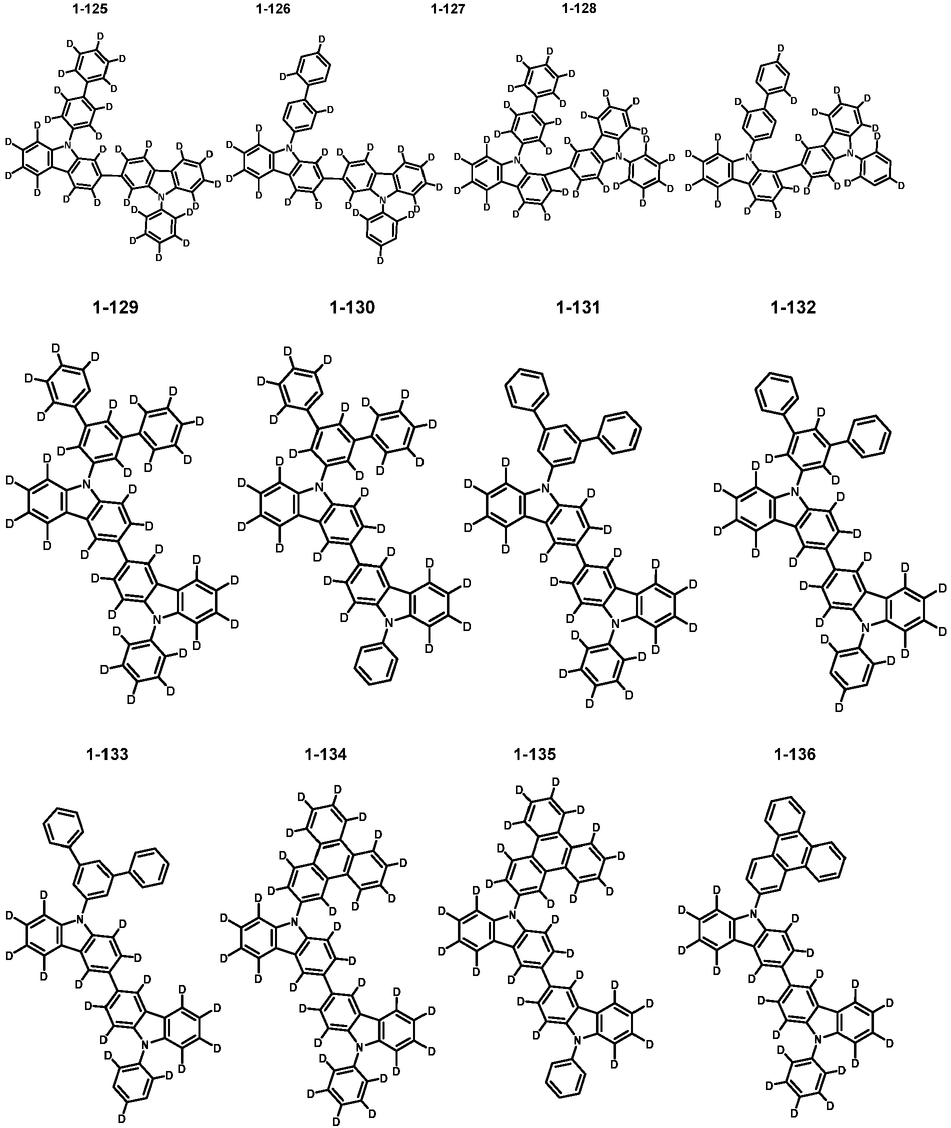

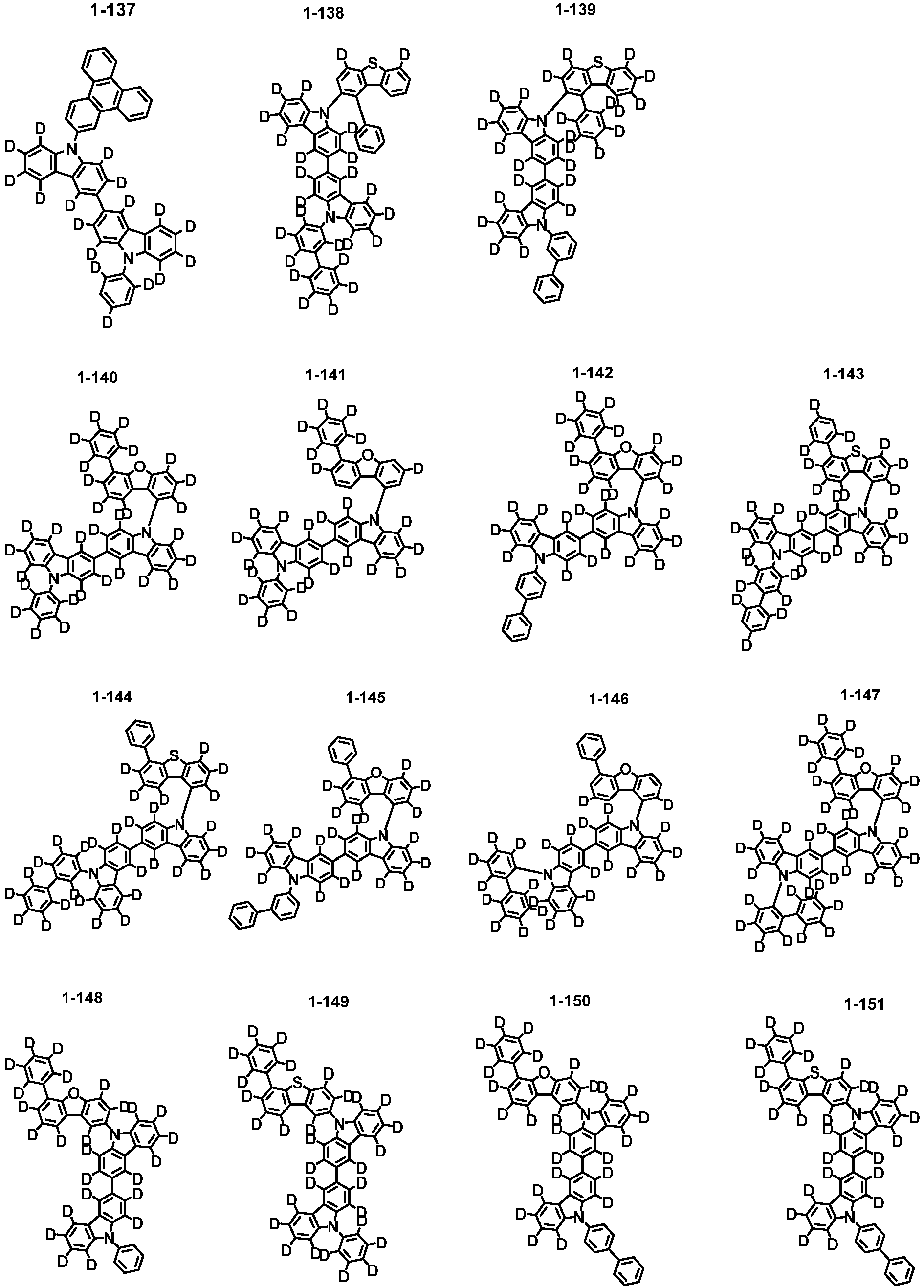

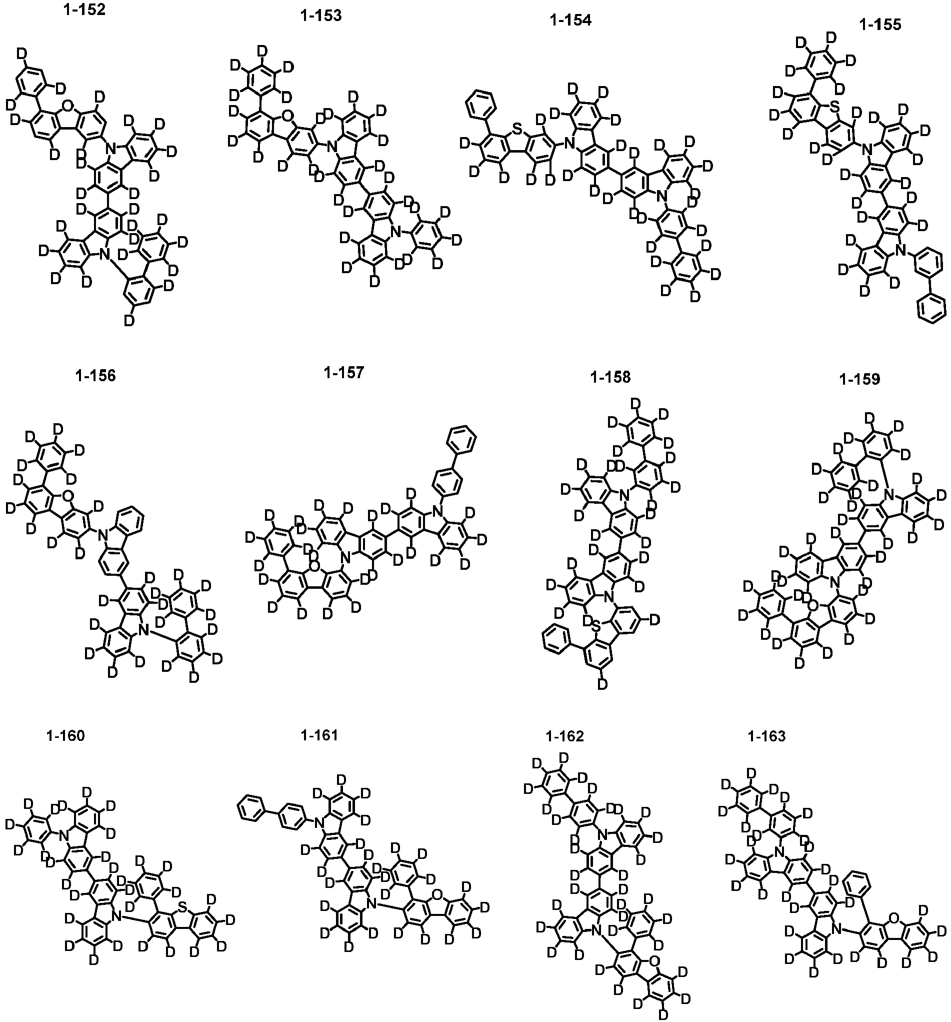

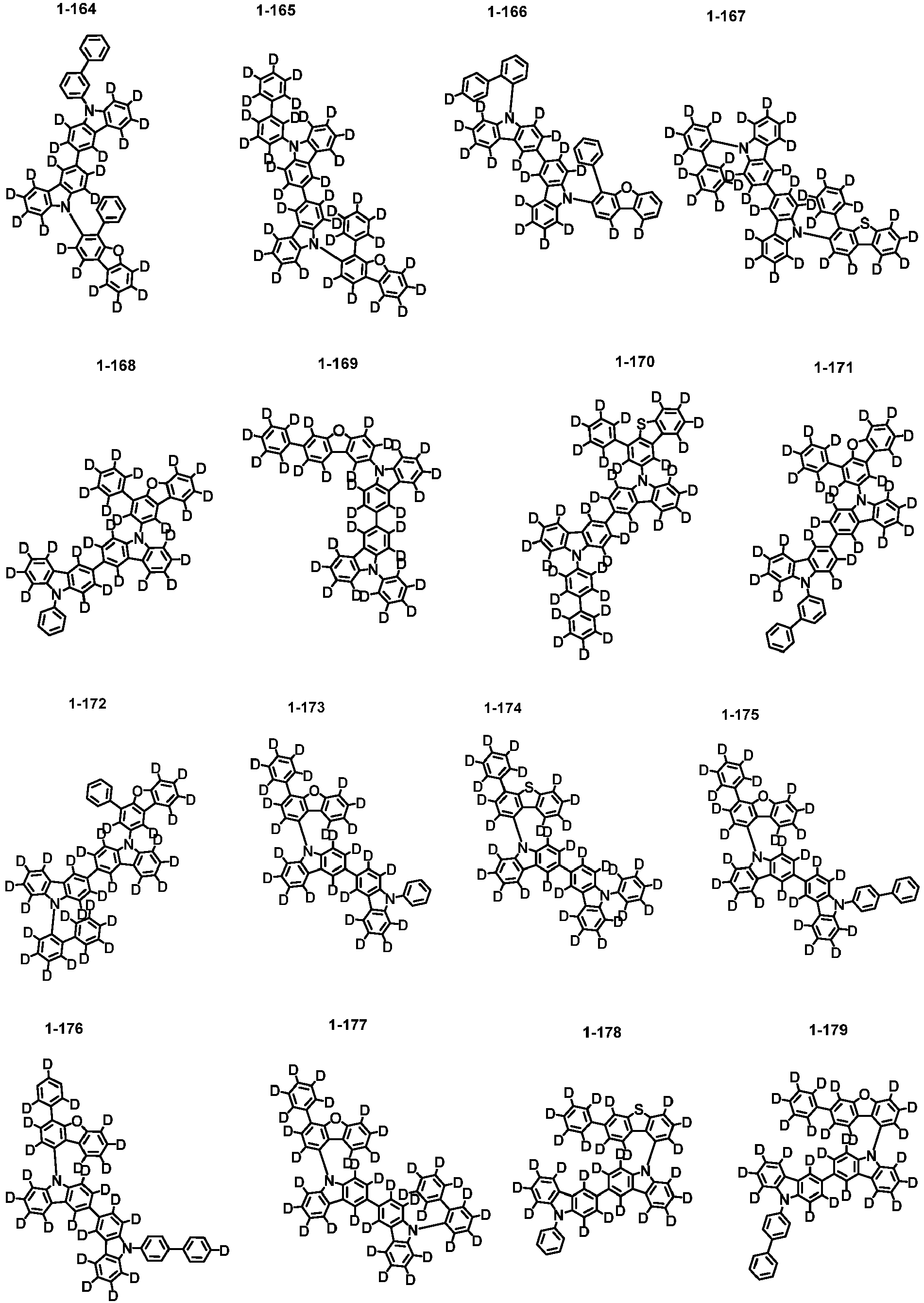

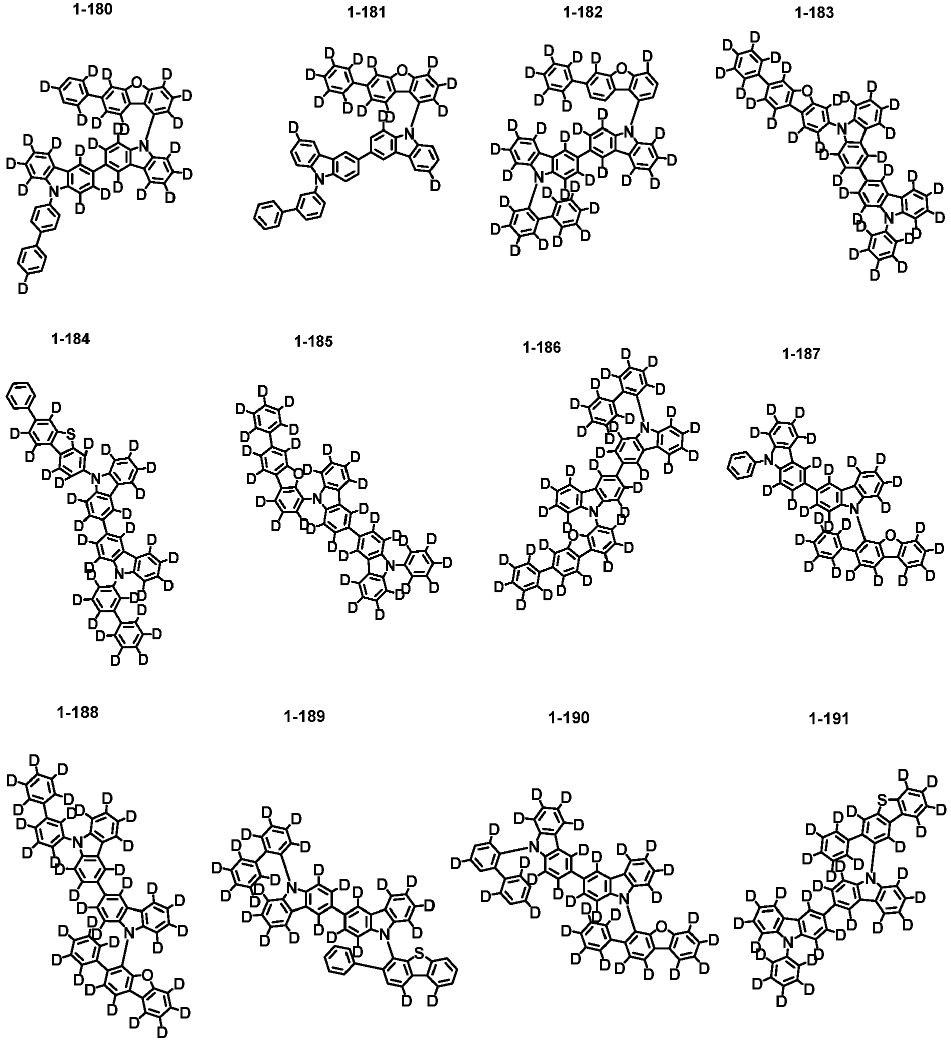

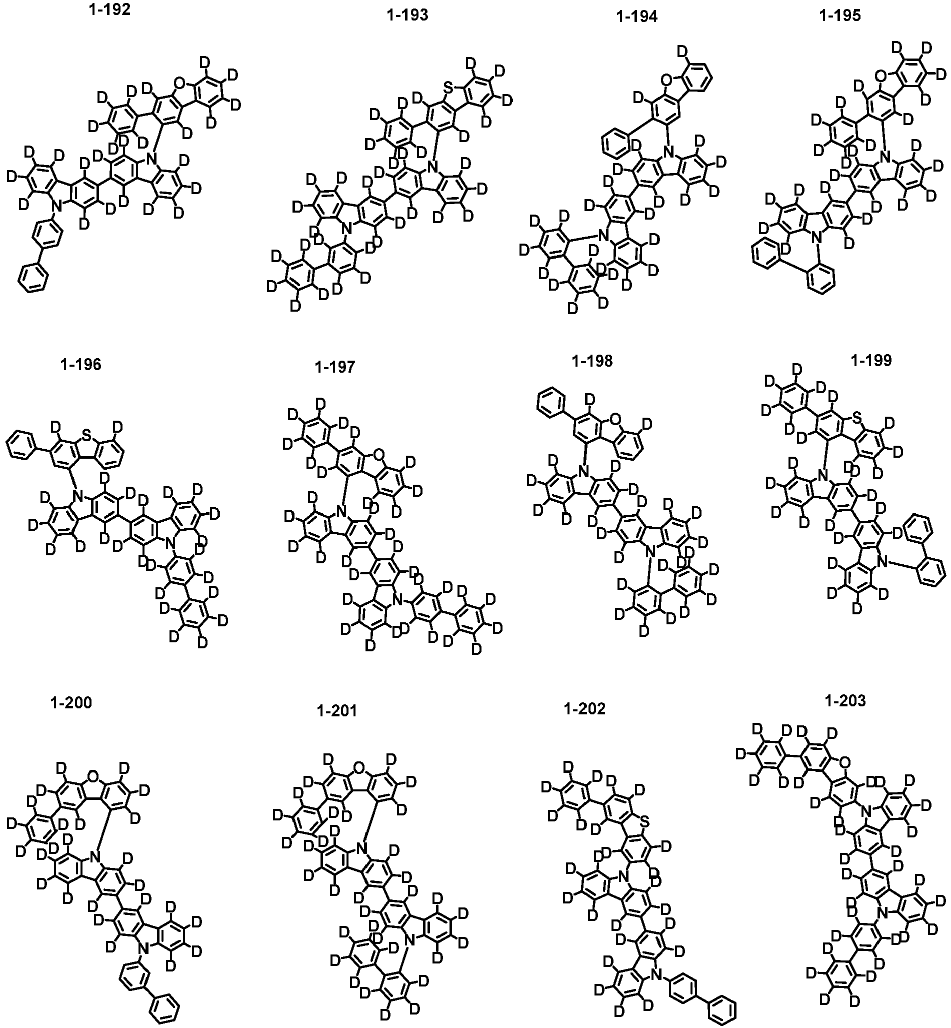

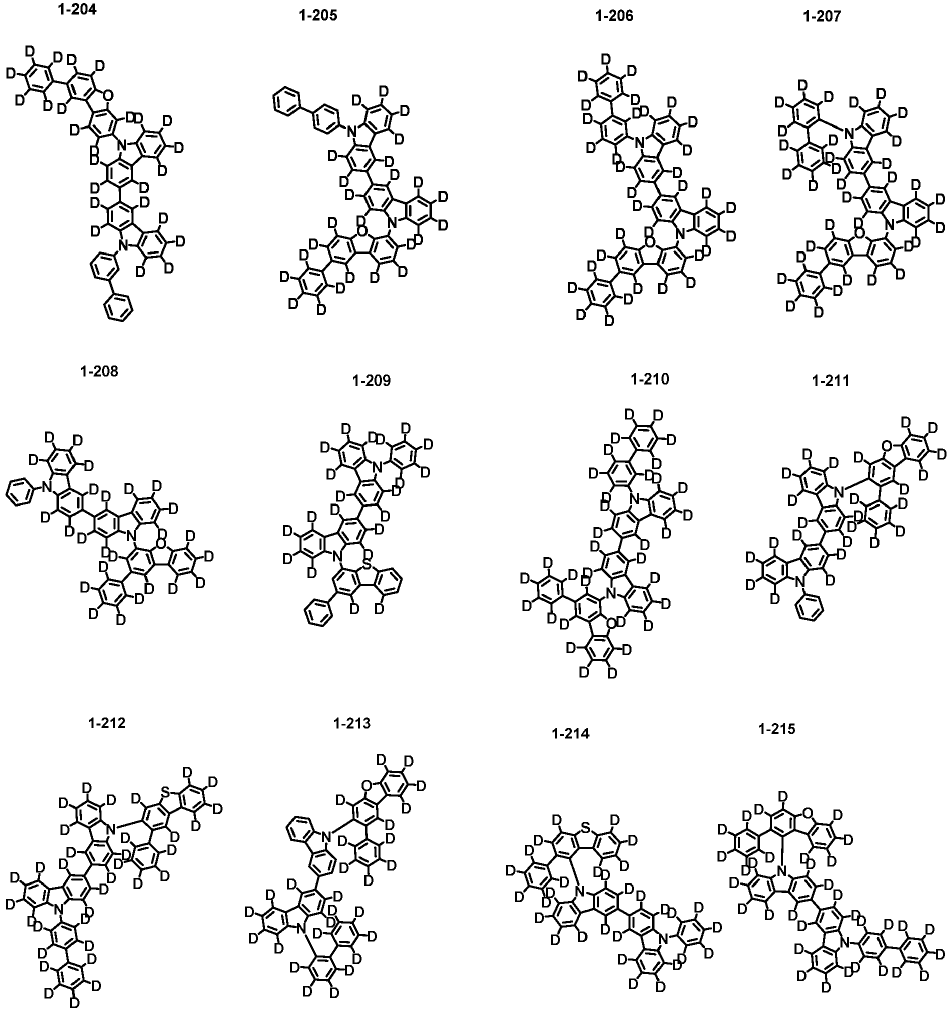

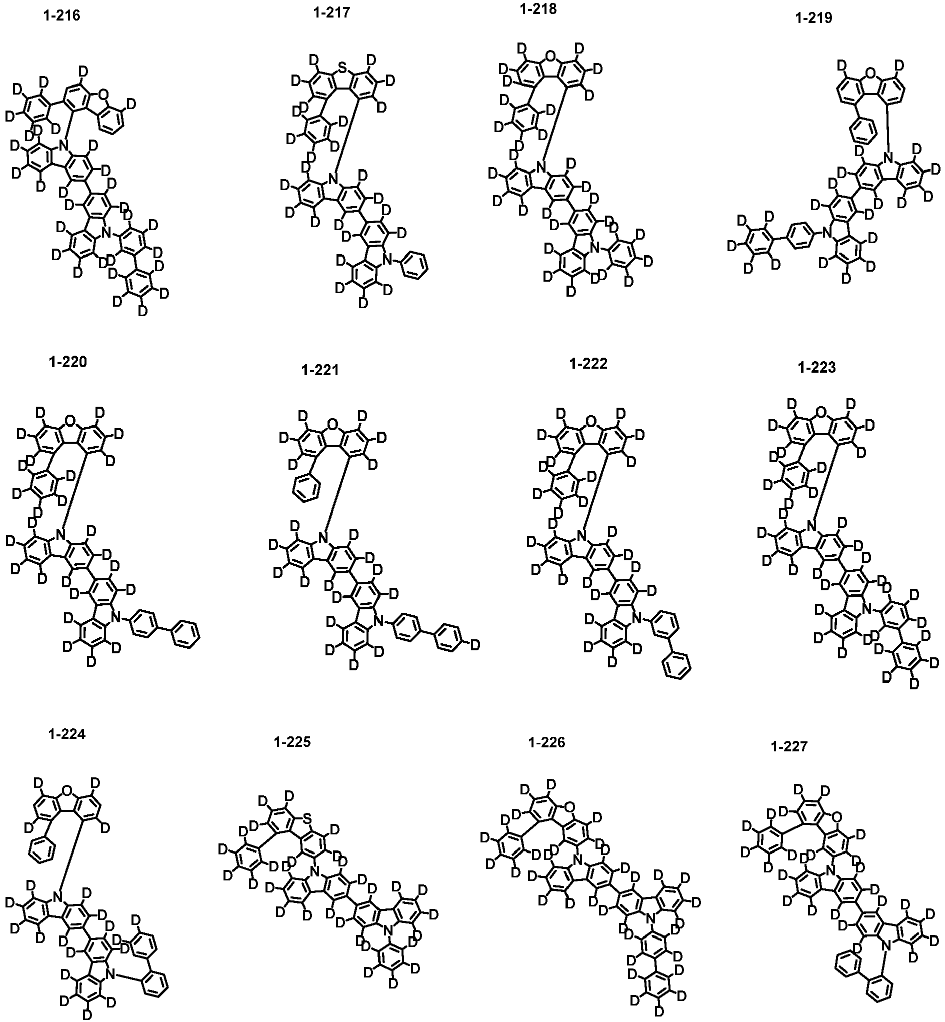

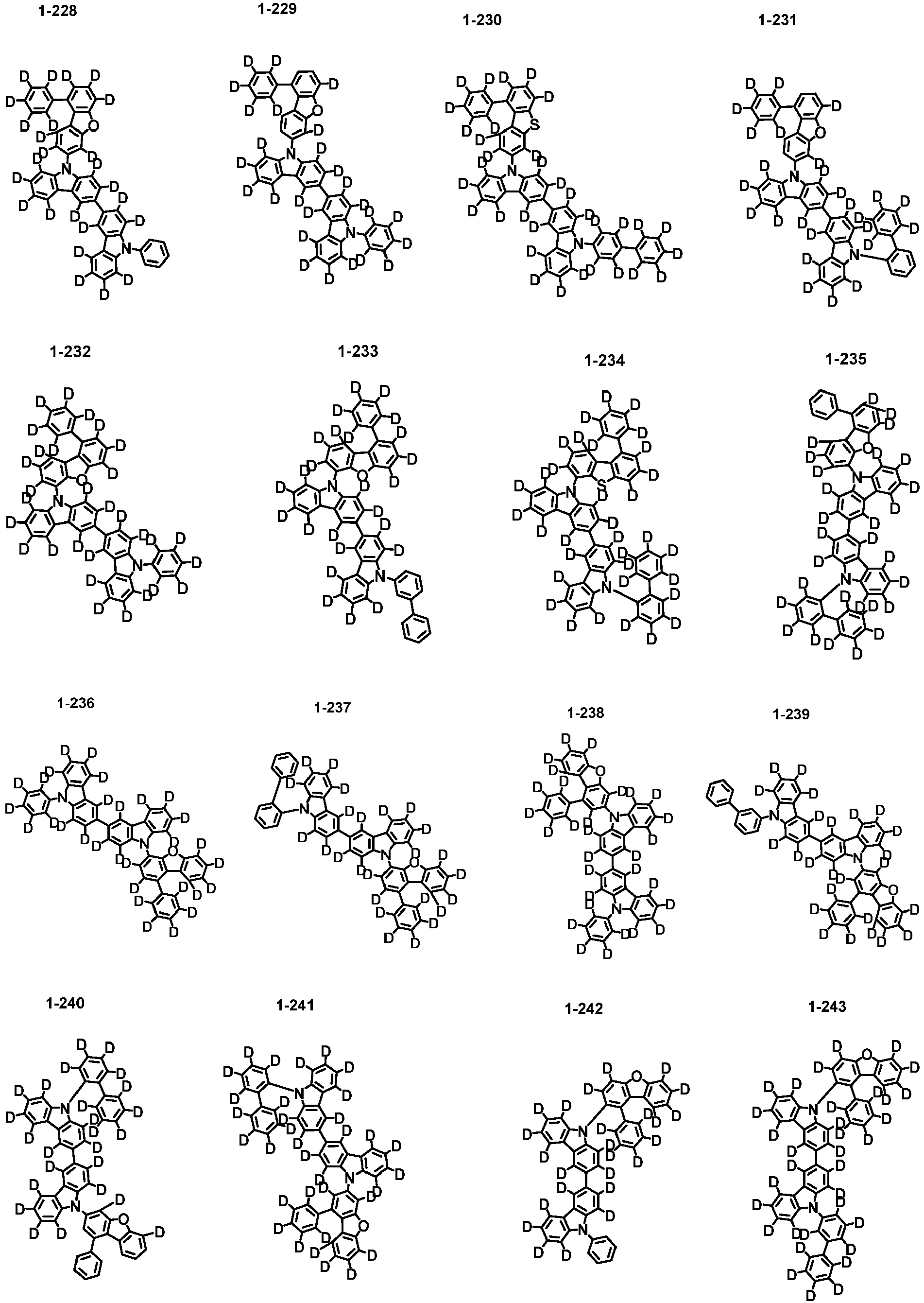

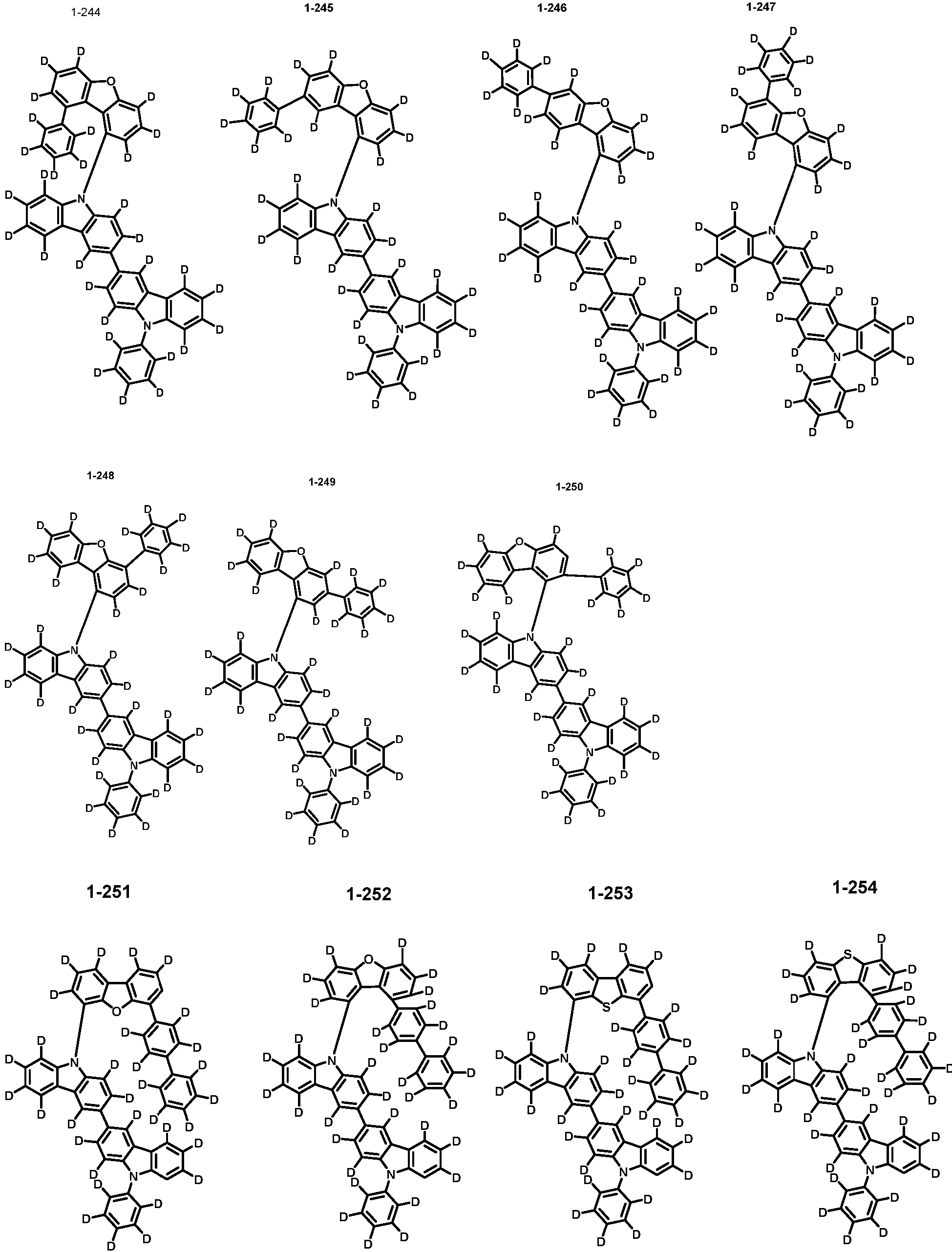

7. The composition for the organic optoelectronic device of claim 1, wherein
the second compound is represented by any one of Chemical Formula 2A to Chemical Formula 2F: 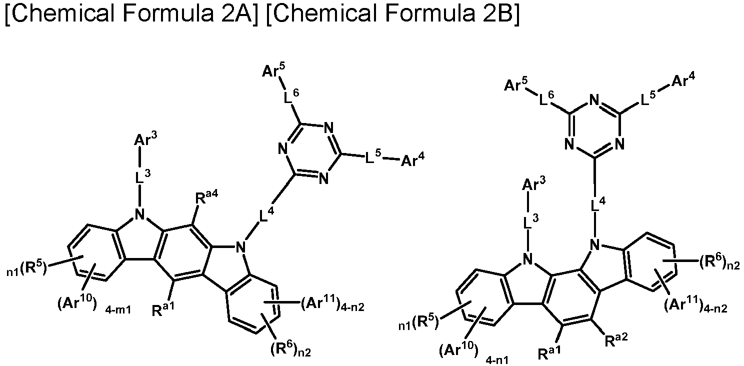 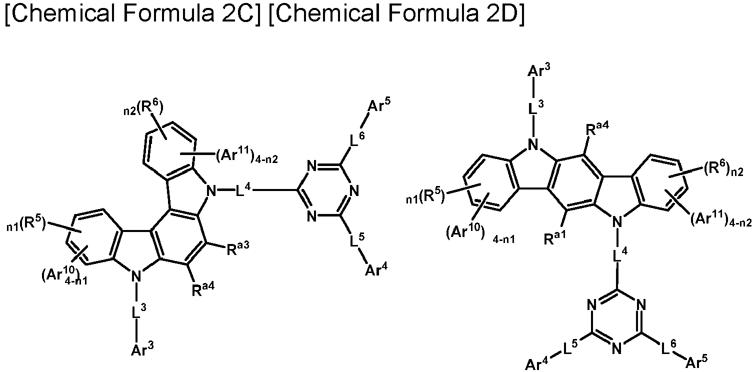 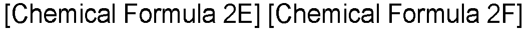 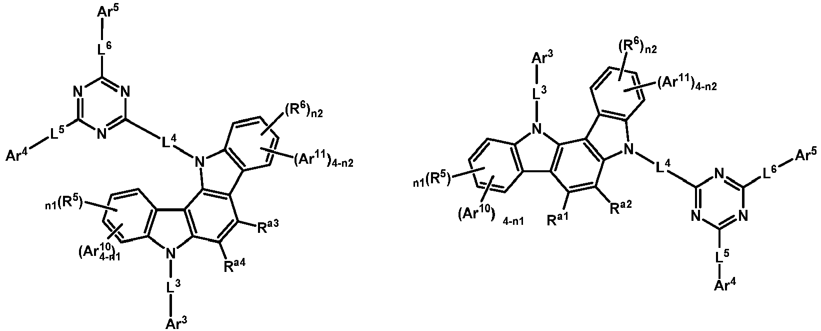
wherein, in Chemical Formula 2A to Chemical Formula 2F,
Ar³ to Ar⁵, Ar¹⁰, Ar¹¹, L³ to L⁶, R¹ to R⁴, n1, and n2 are the same as described in claim 1, and
R^{a1} to R^{a4} are each the same as R^{a} defined in claim 1.

8. The composition for the organic optoelectronic device of claim 1, wherein
the second compound is represented by Chemical Formula 2B-a: 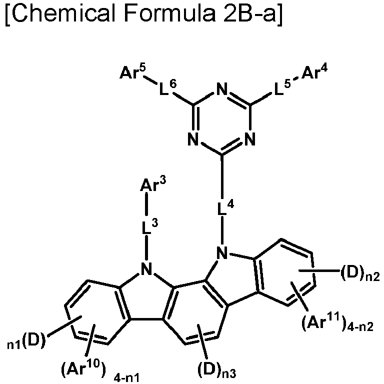
wherein, in Chemical Formula 2B-a,
Ar³ to Ar⁵, Ar¹⁰, Ar¹¹, and L³ to L⁶ are the same as described in claim 1,
n1 and n2 are each independently an integer from 1 to 4, and
n3 is an integer of 1 or 2.

9. The composition for the organic optoelectronic device of claim 1, wherein
at least one of Ar³ to Ar⁵ is a phenyl group substituted with at least one deuterium, a biphenyl group substituted with at least one deuterium, a terphenyl group substituted with at least one deuterium, a naphthyl group substituted with at least one deuterium, an anthracenyl group substituted with at least one deuterium, a phenanthrenyl group substituted with at least one deuterium, a triphenylene group substituted with at least one deuterium, a fluorenyl group substituted with at least one deuterium, a carbazolyl group substituted with at least one deuterium, a dibenzofuranyl group substituted with at least one deuterium, or a dibenzothiophenyl group substituted with at least one deuterium.

10. The composition for the organic optoelectronic device of claim 1, wherein
the L³-Ar³, L⁵-Ar⁴, and L⁶-Ar⁵ are each independently selected from the substituents listed in Group II-1 and Group II-2, and
at least one of L³-Ar³, L⁵-Ar⁴, and L⁶-Ar⁵ is one selected from substituents listed in Group II-2: 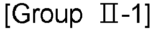 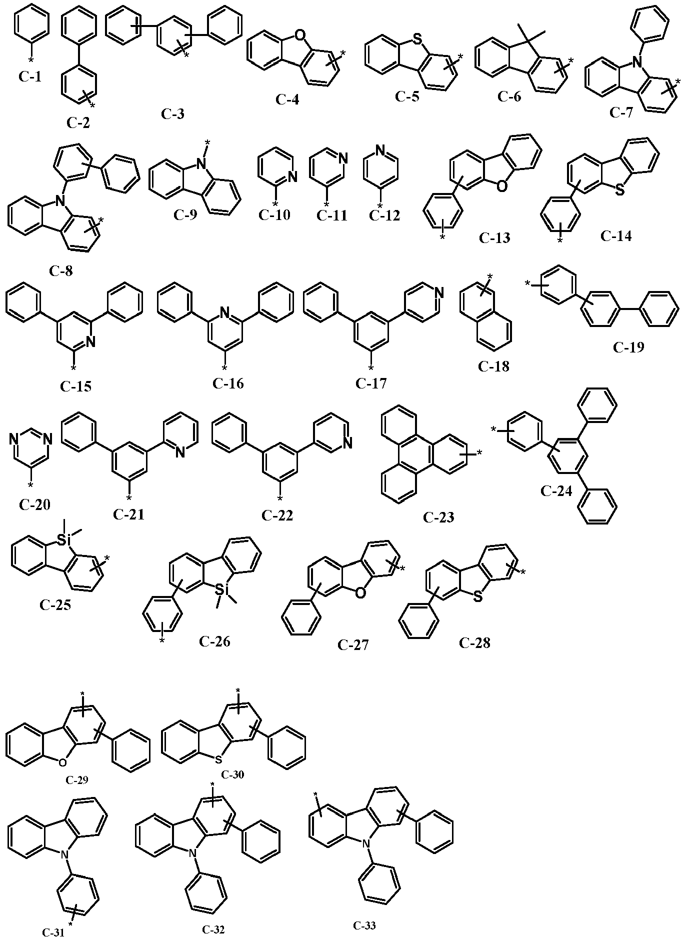 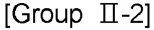 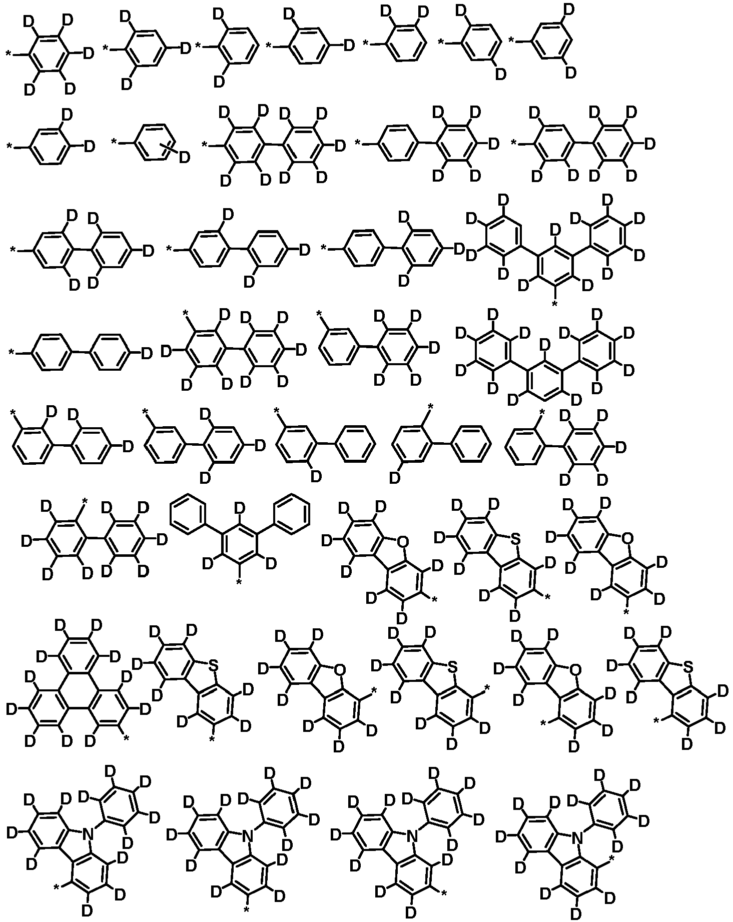
wherein, in Group II-1 and Group II-2, * is a linking point.

11. The composition for the organic optoelectronic device of claim 1, wherein
the second compound is one selected from the compounds of Group 2:

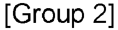

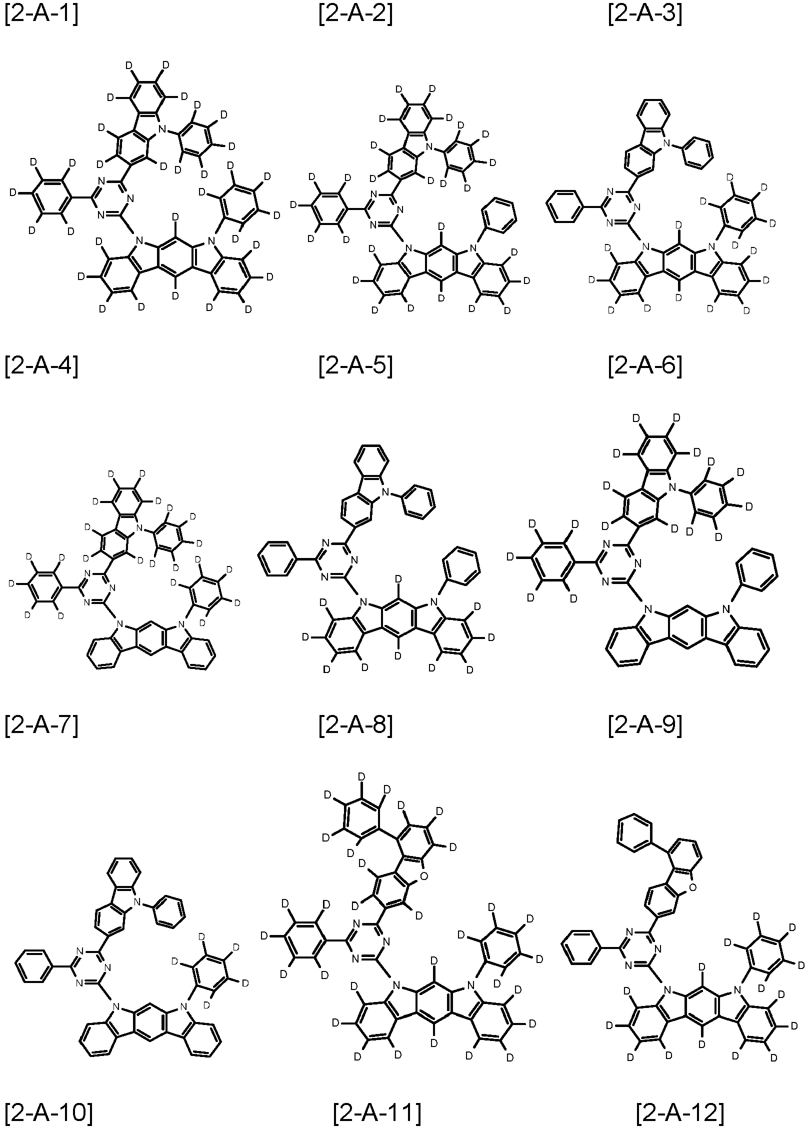

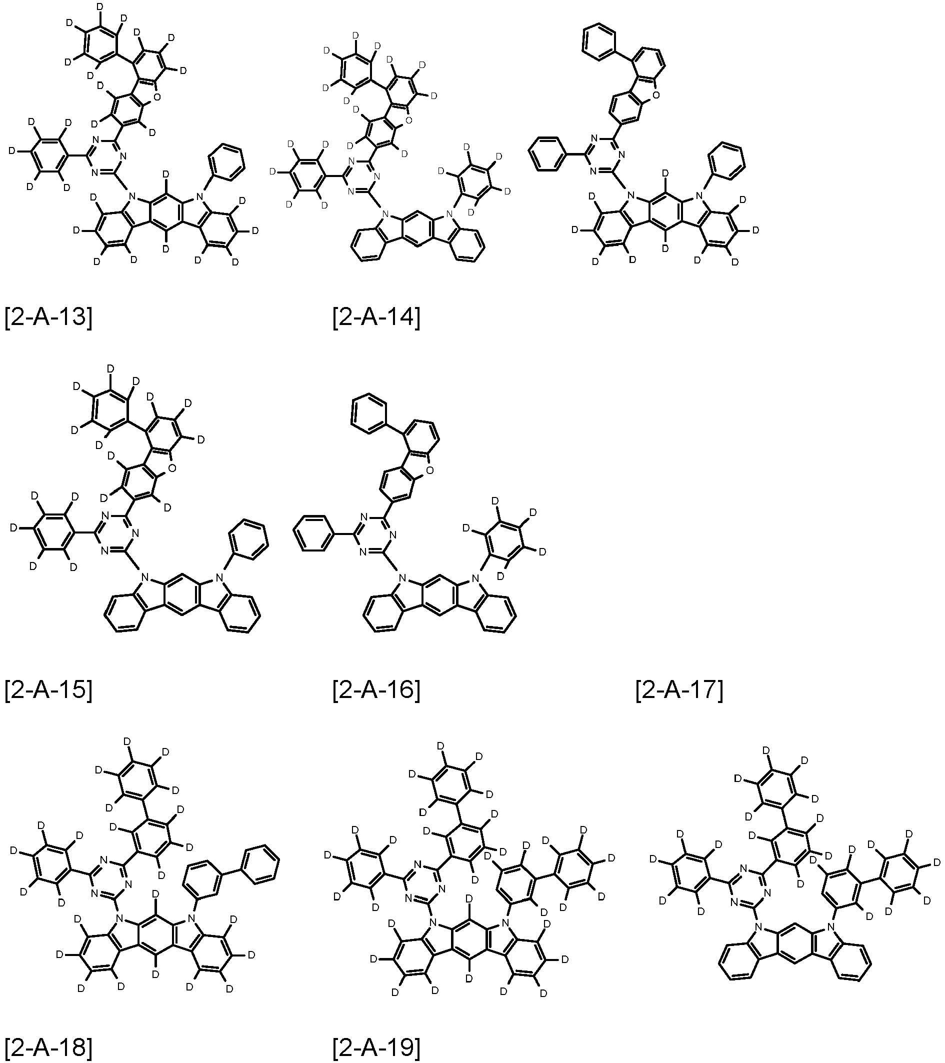

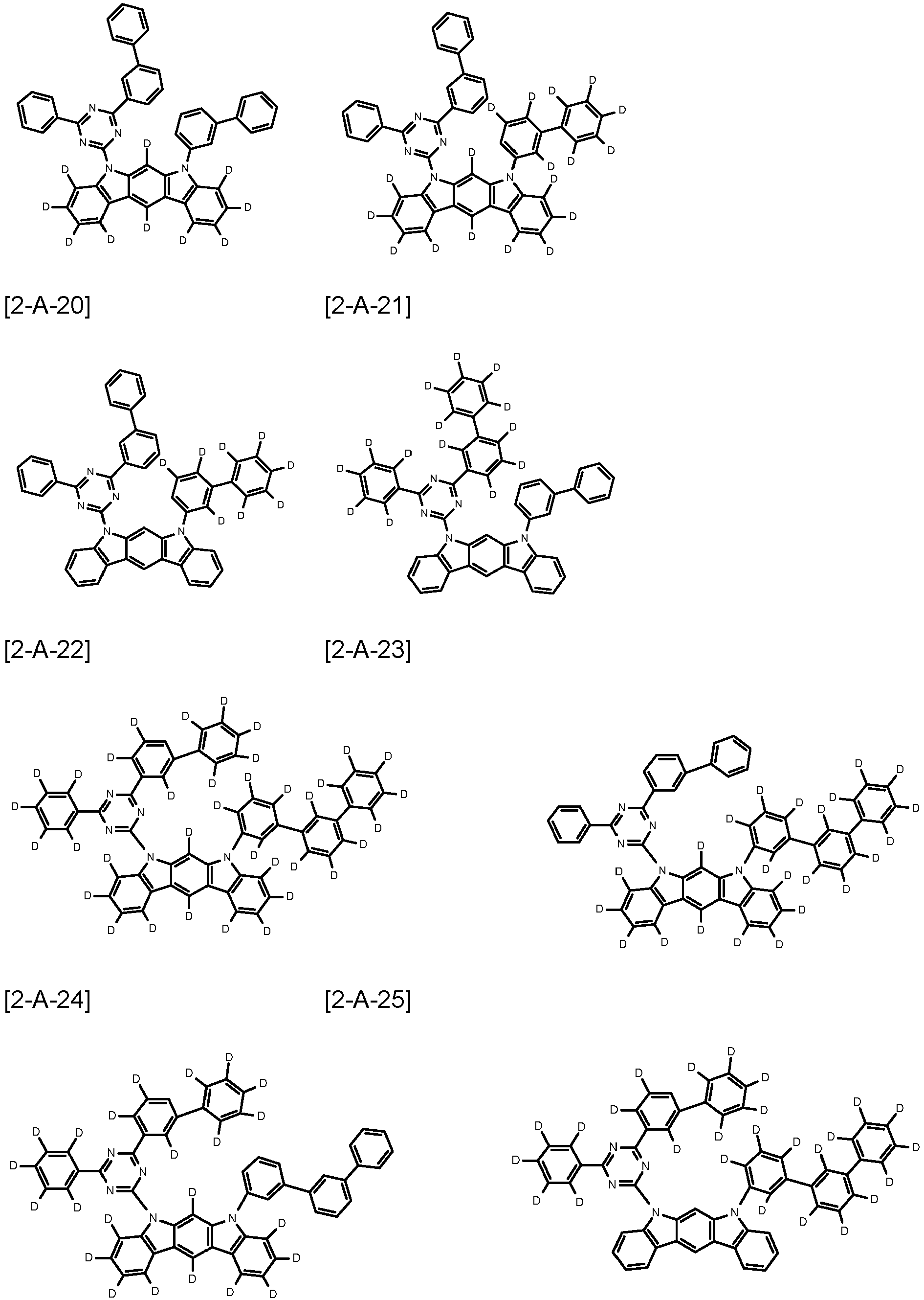

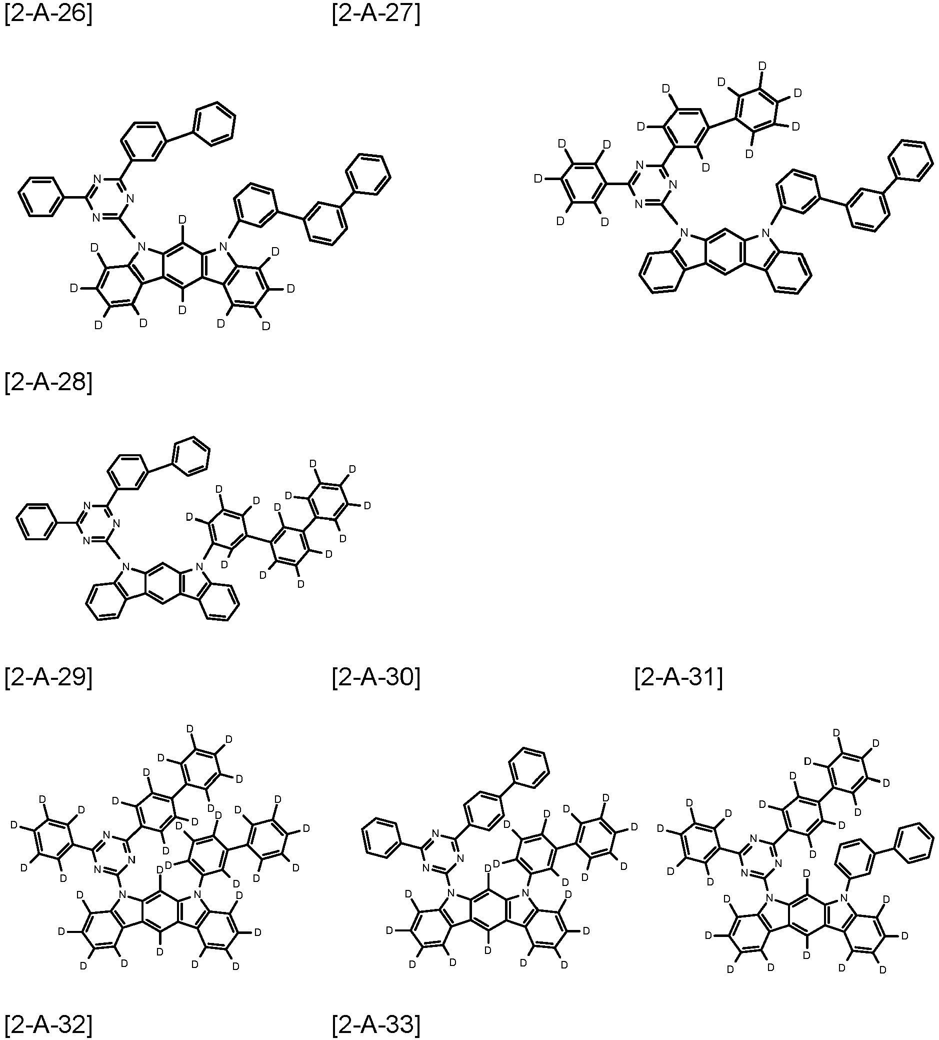

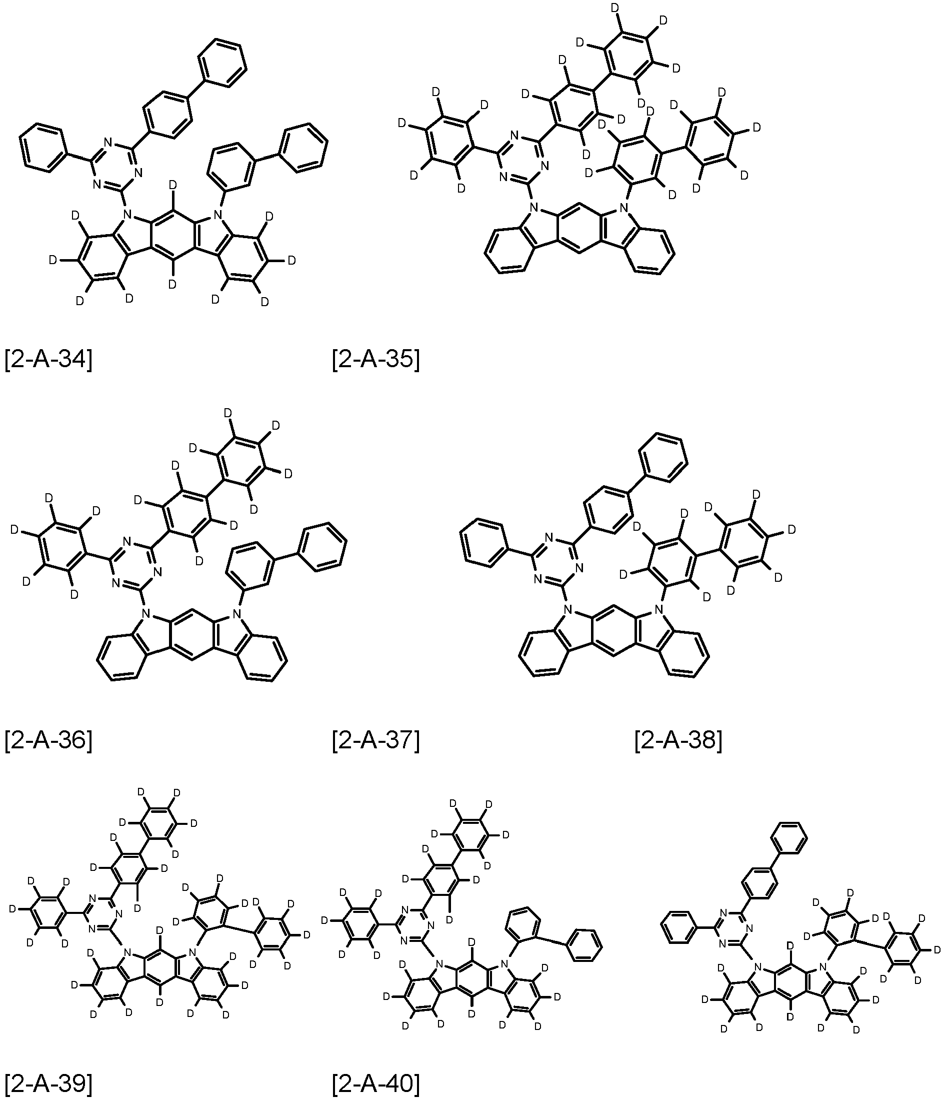

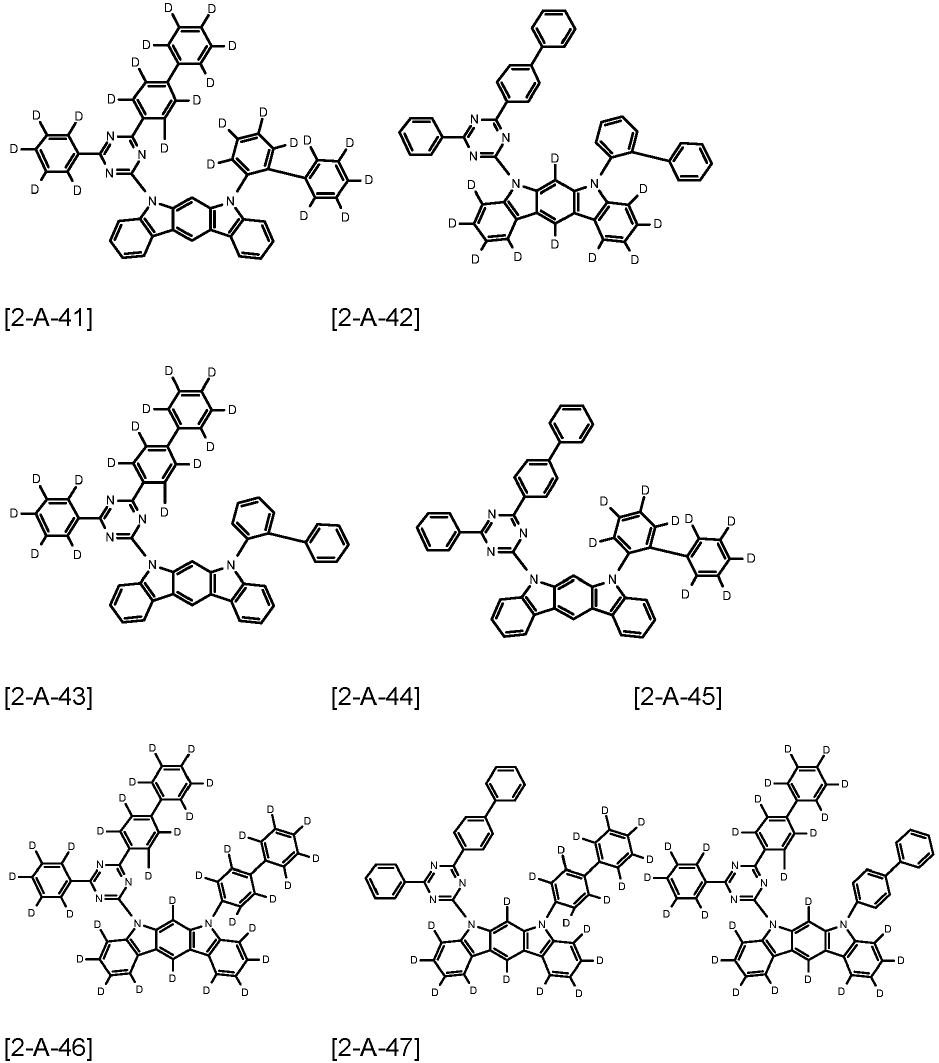

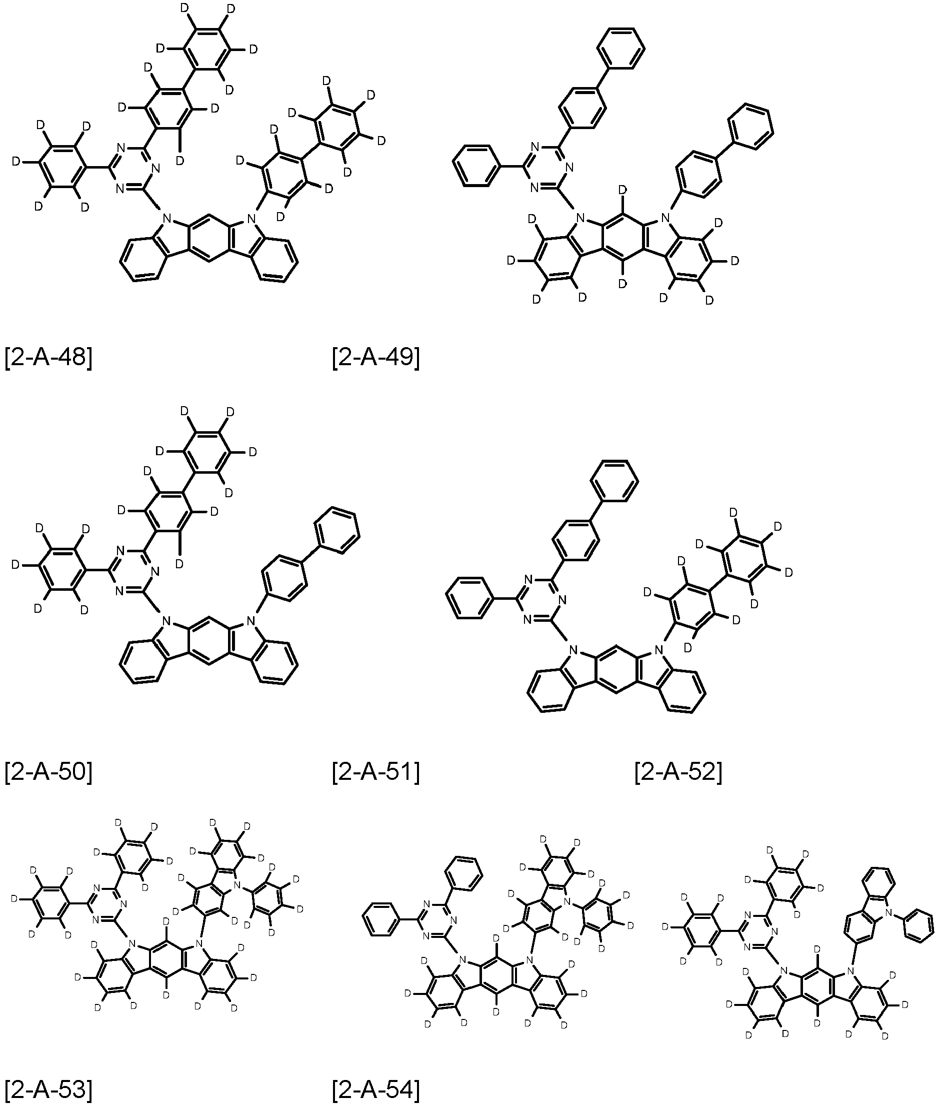

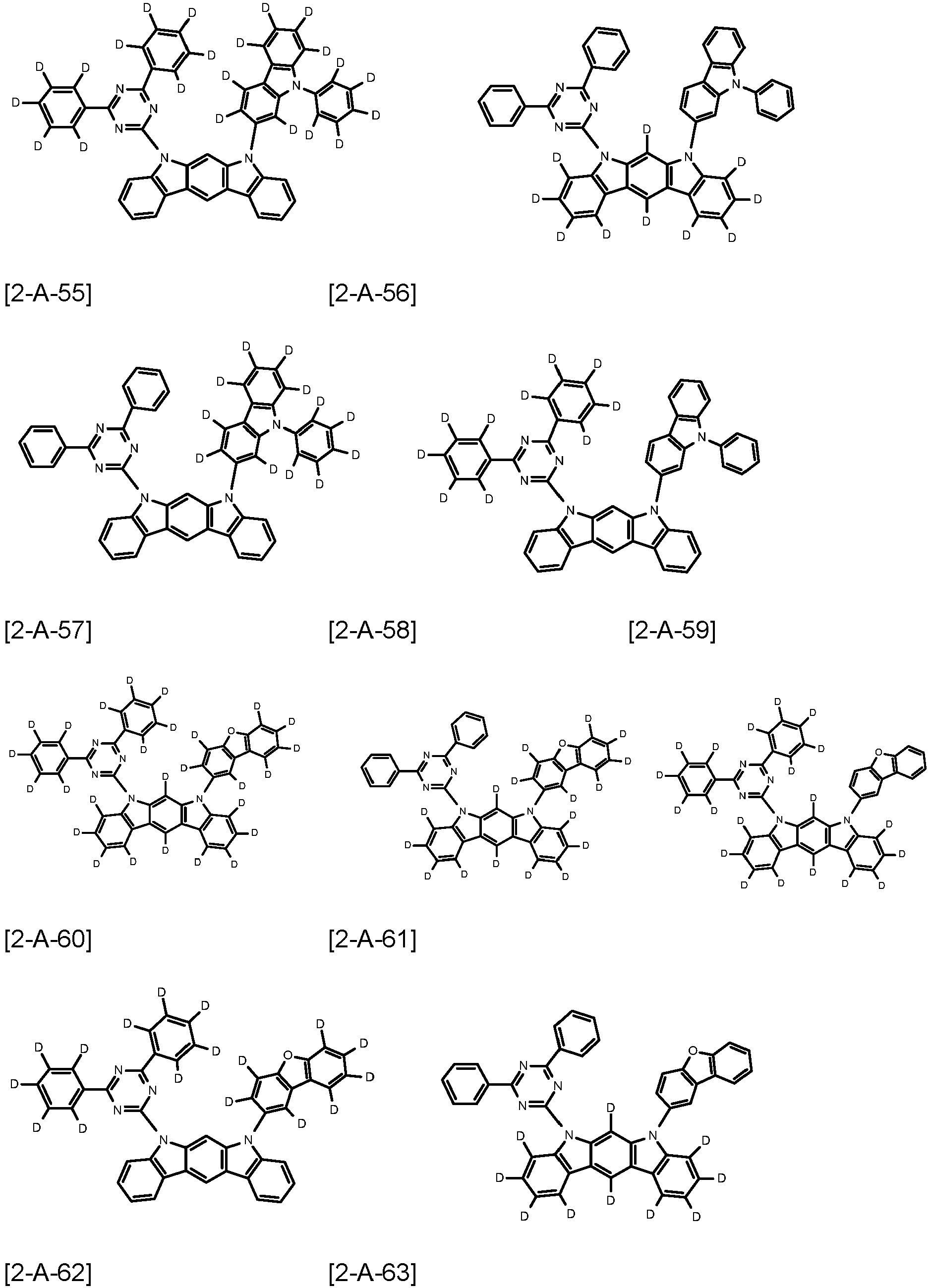

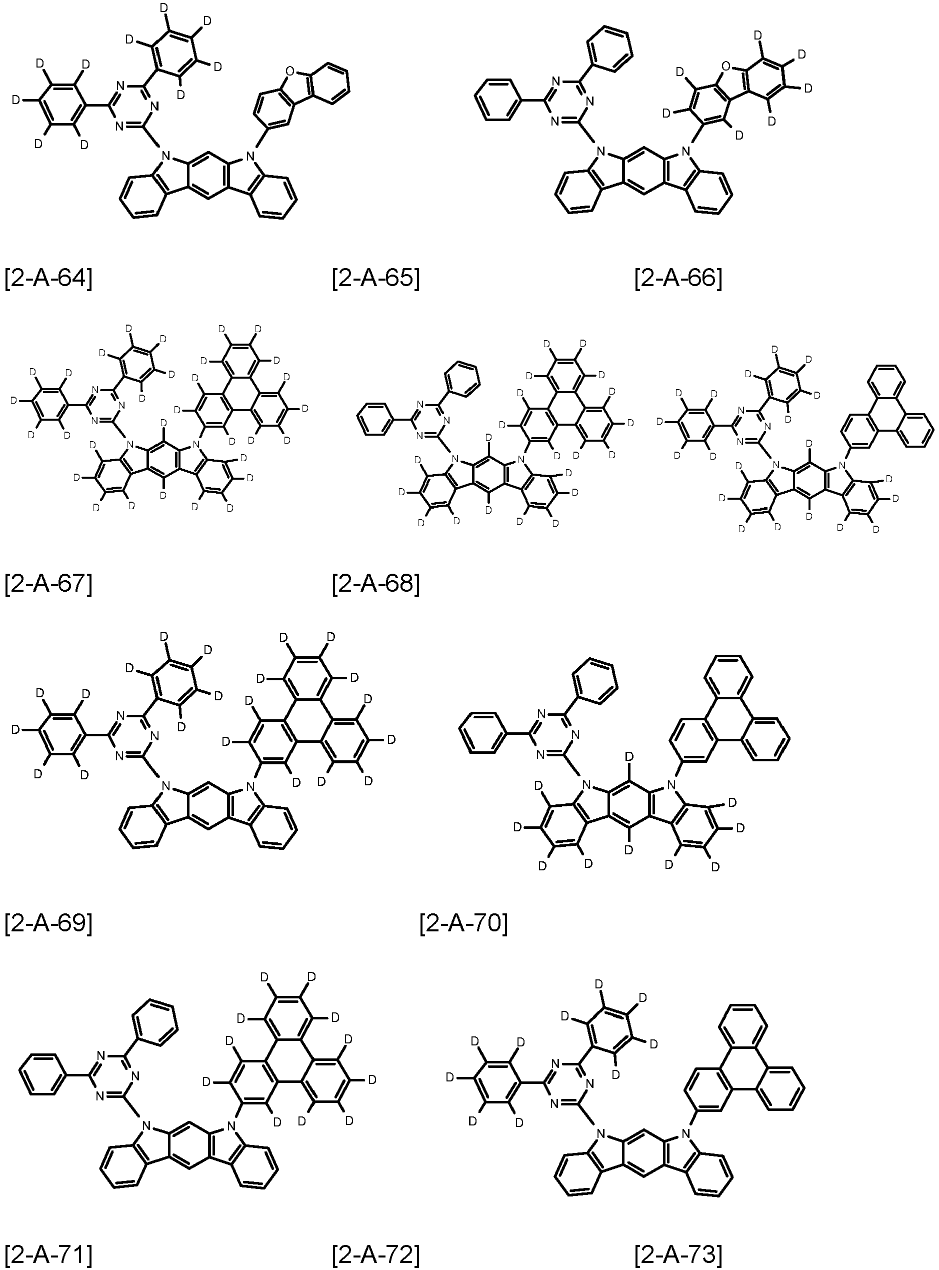

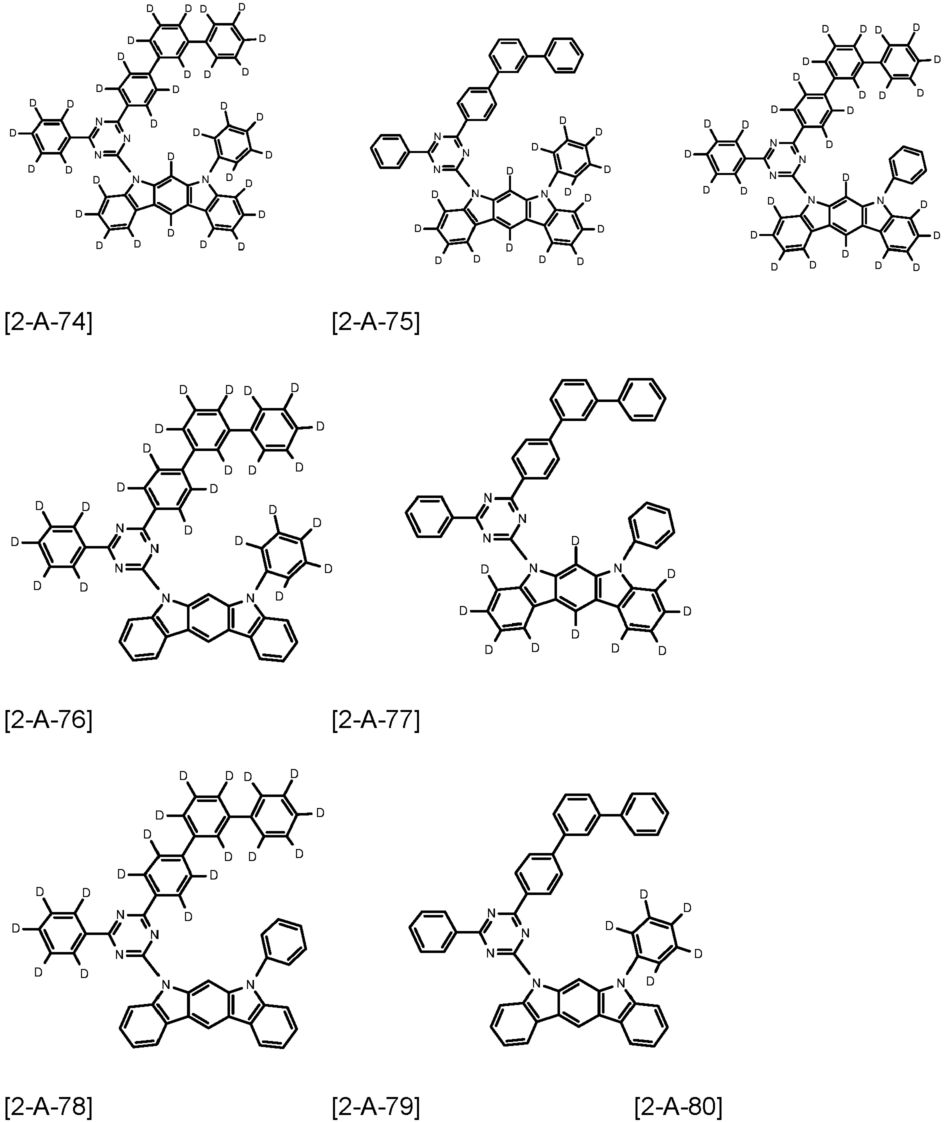

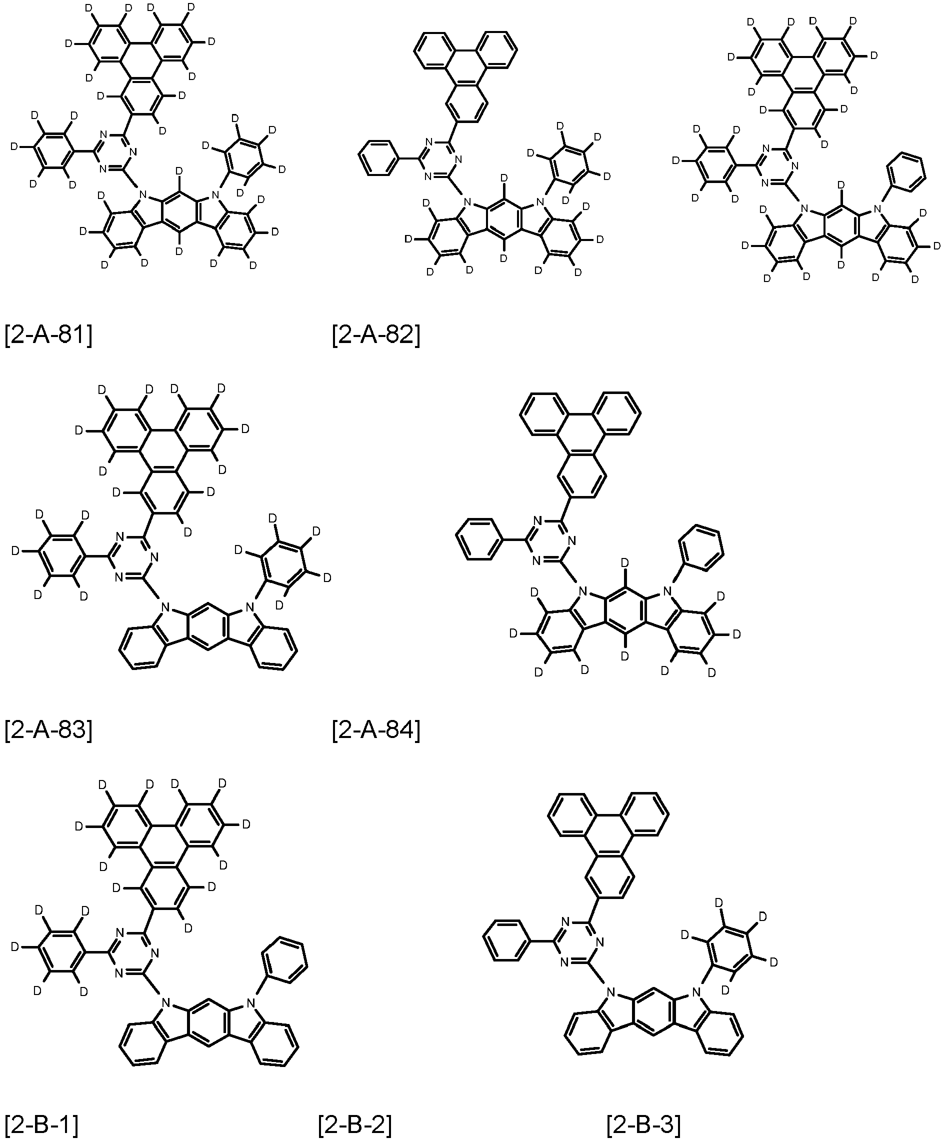

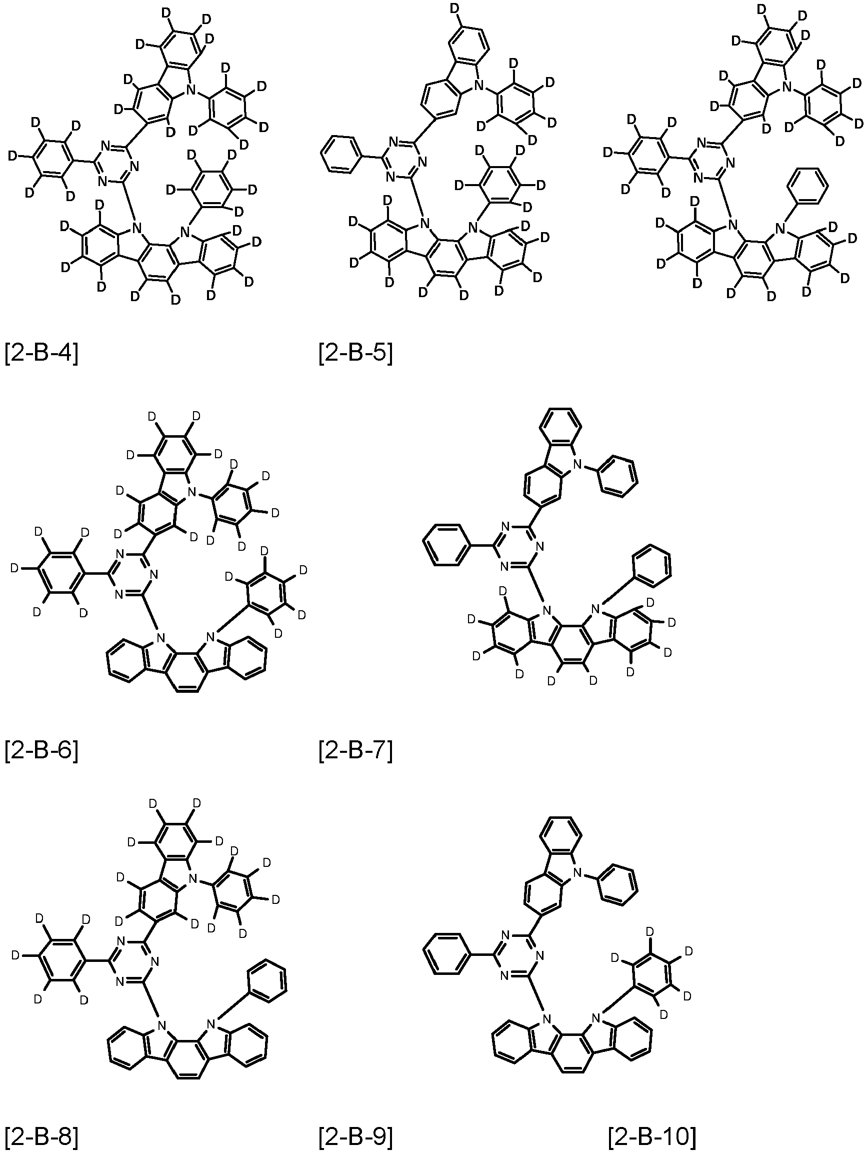

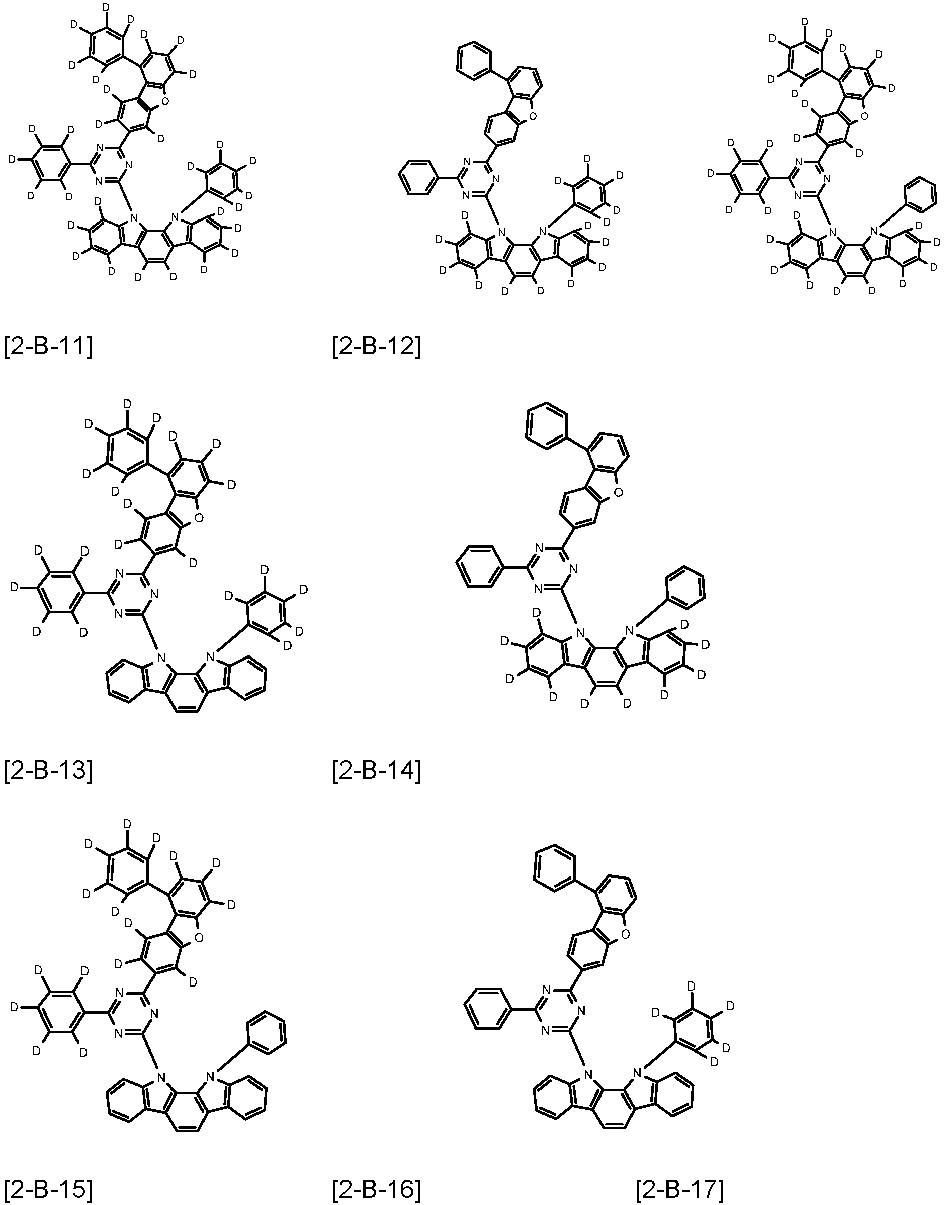

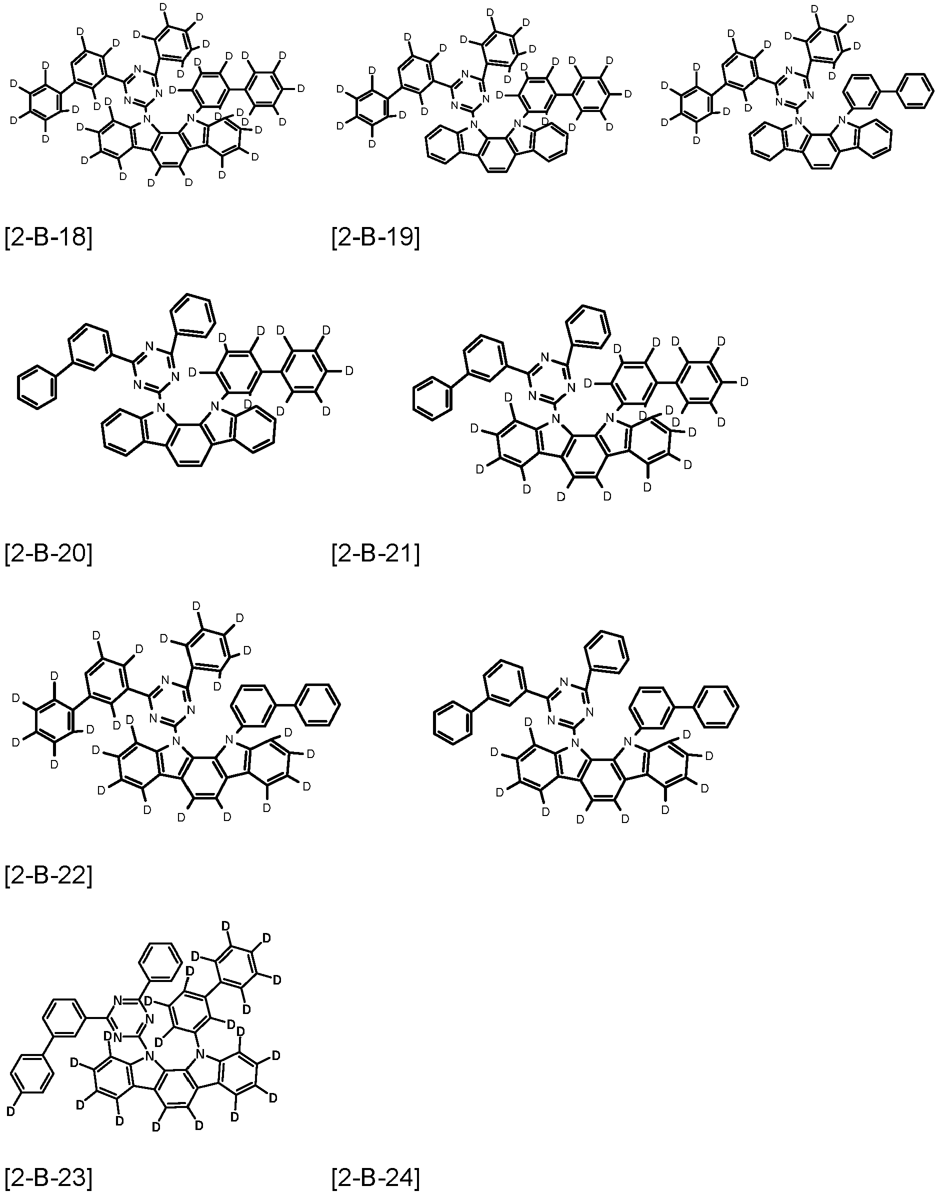

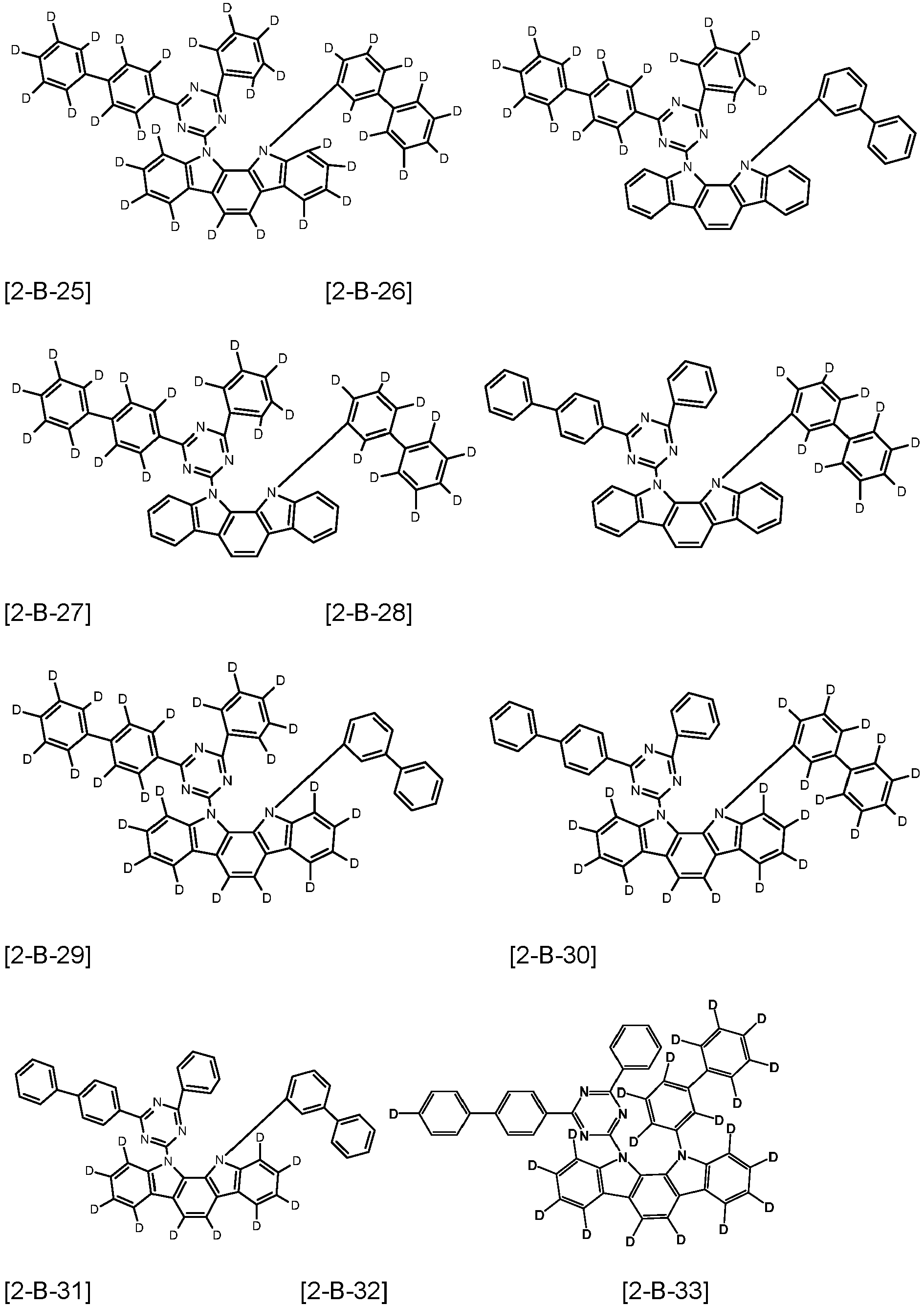

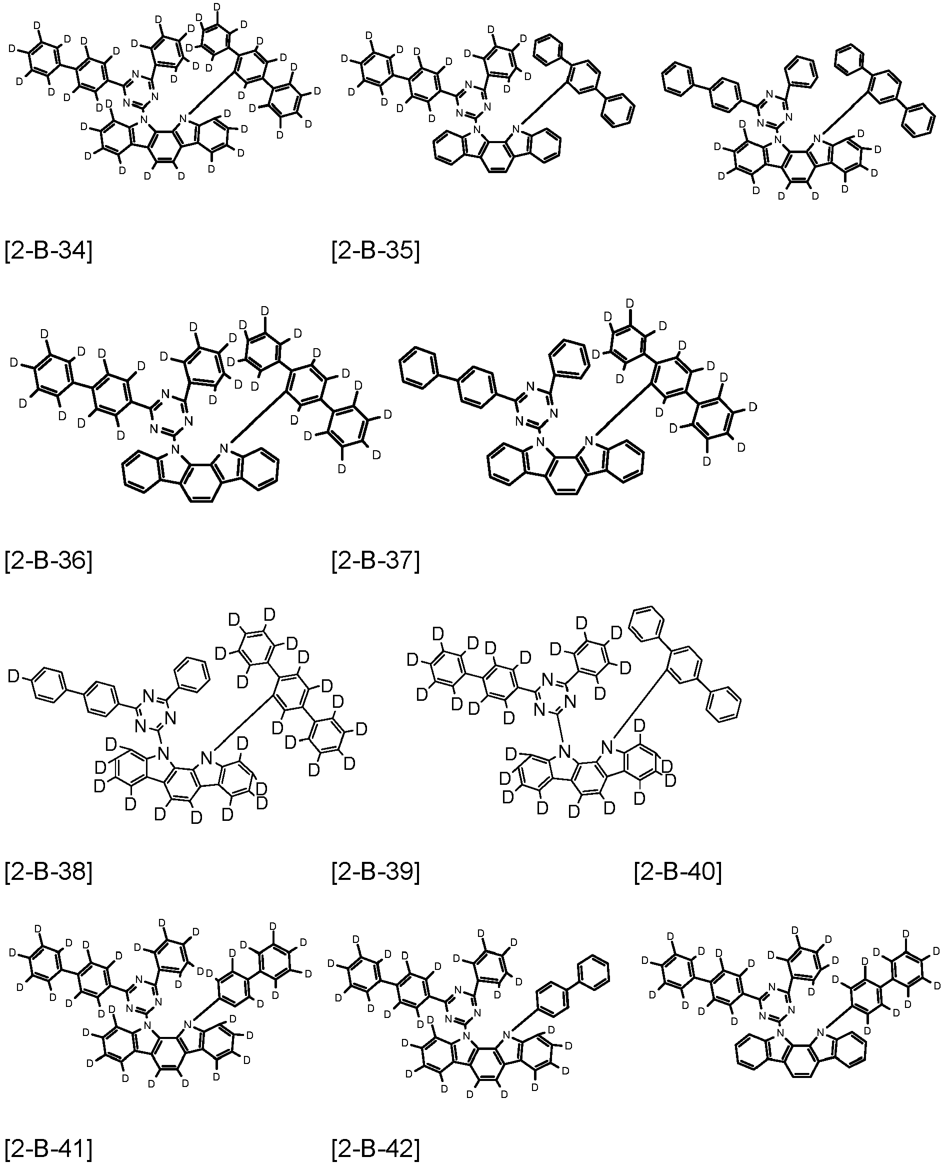

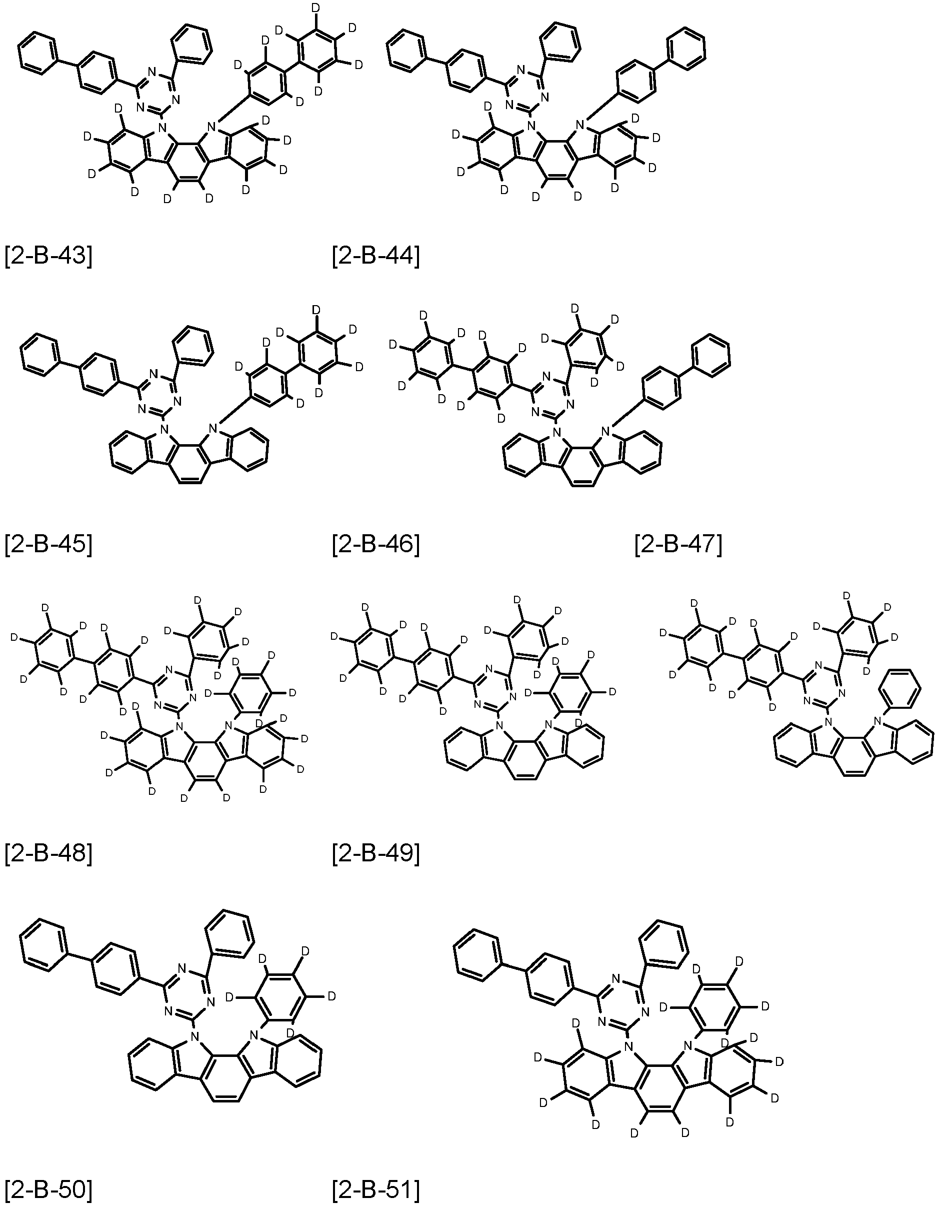

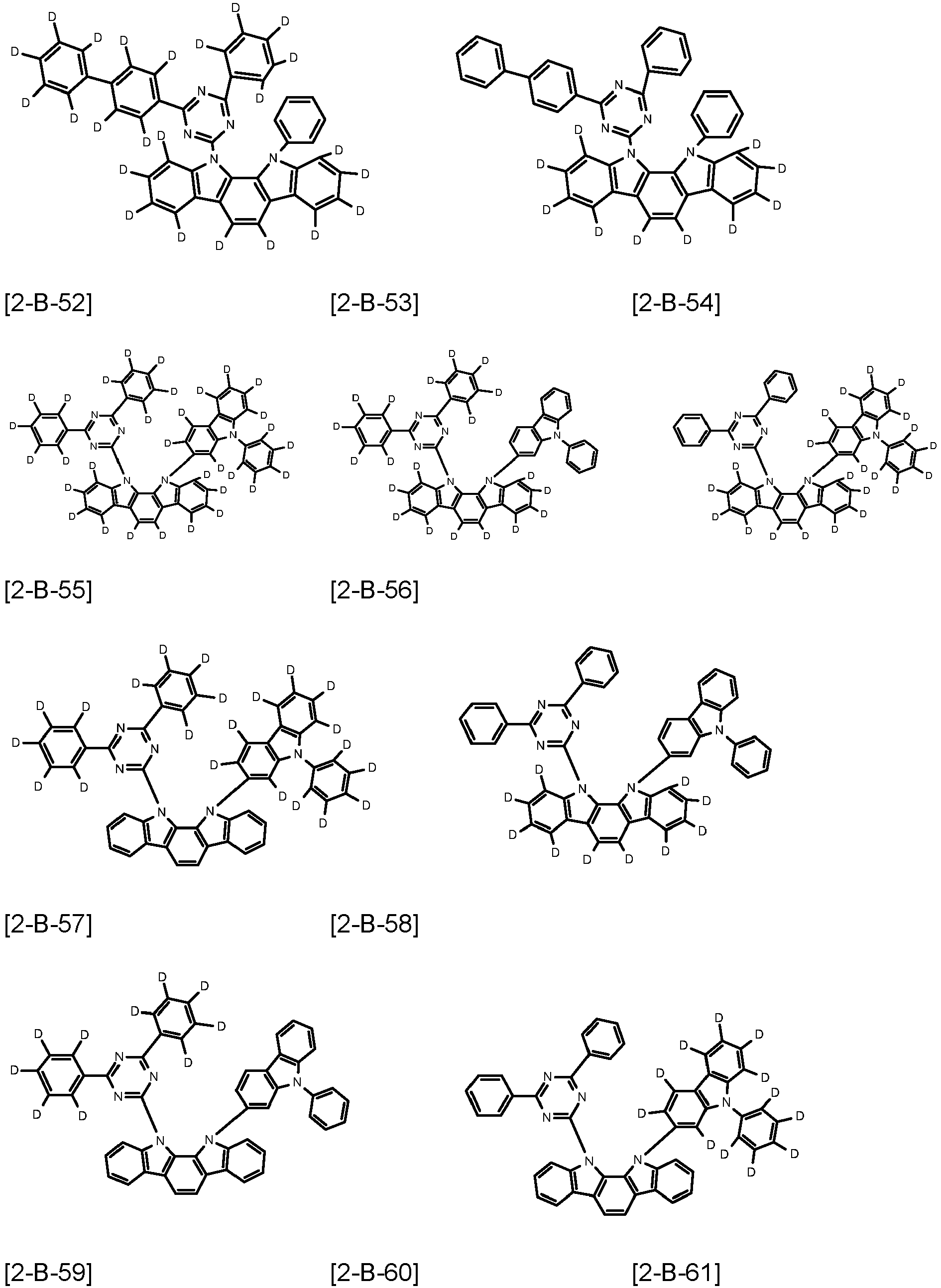

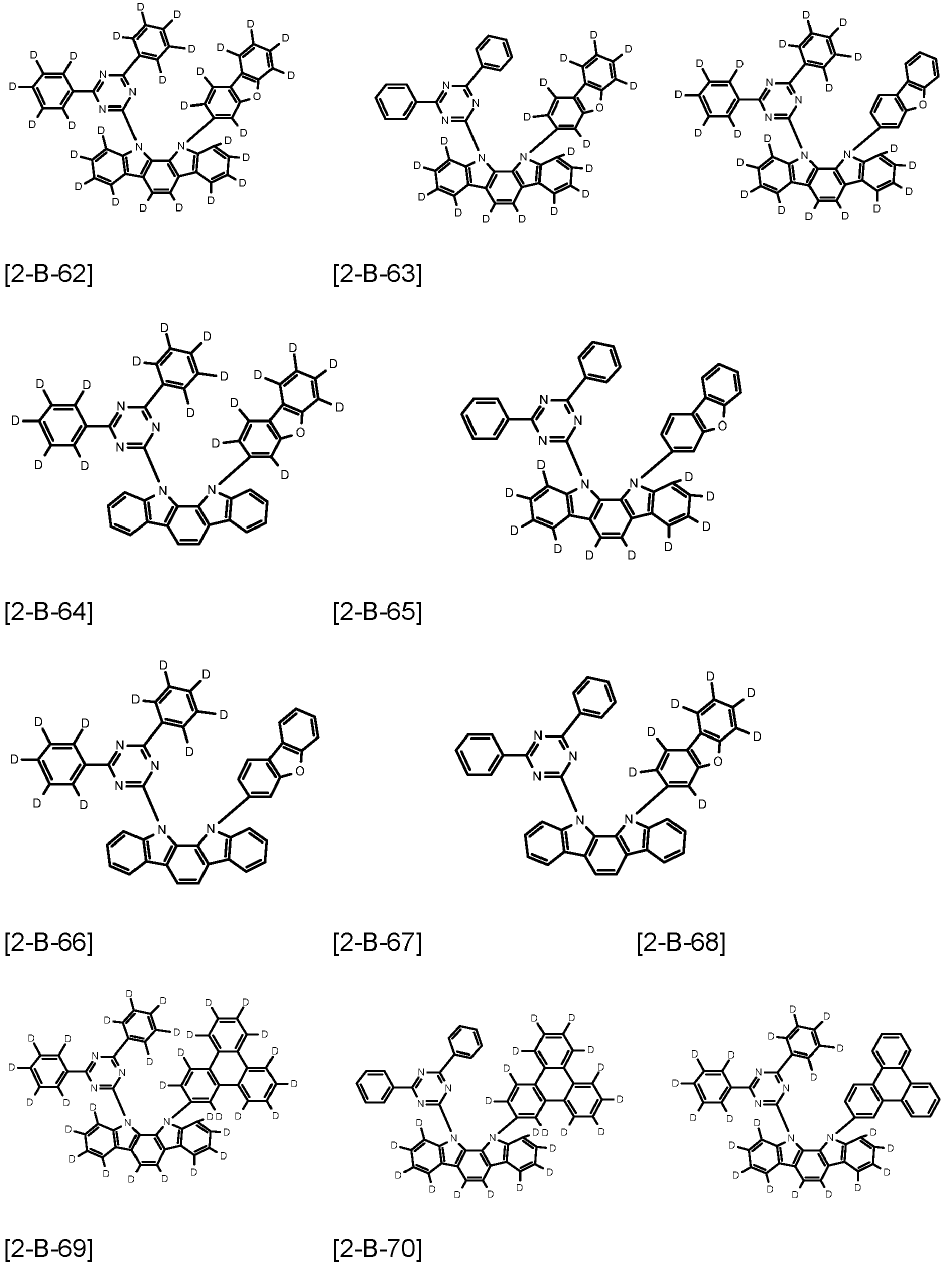

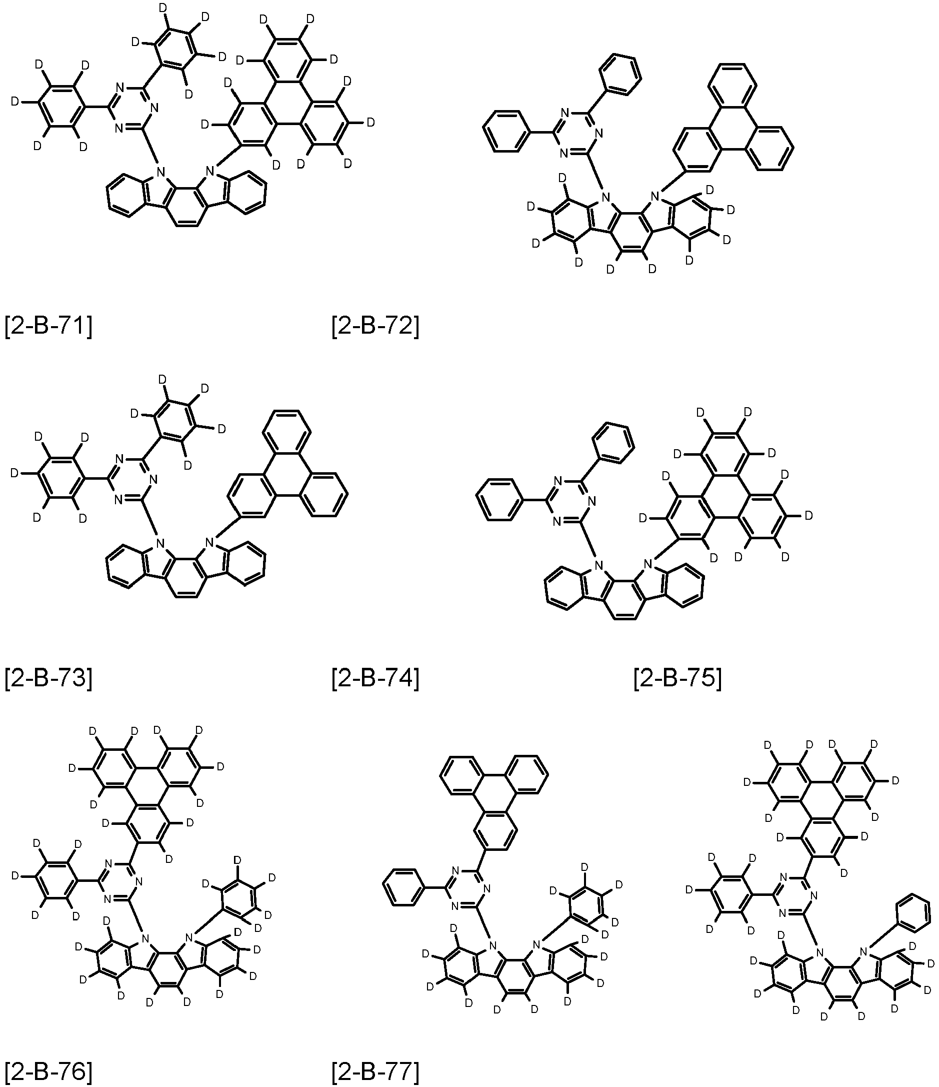

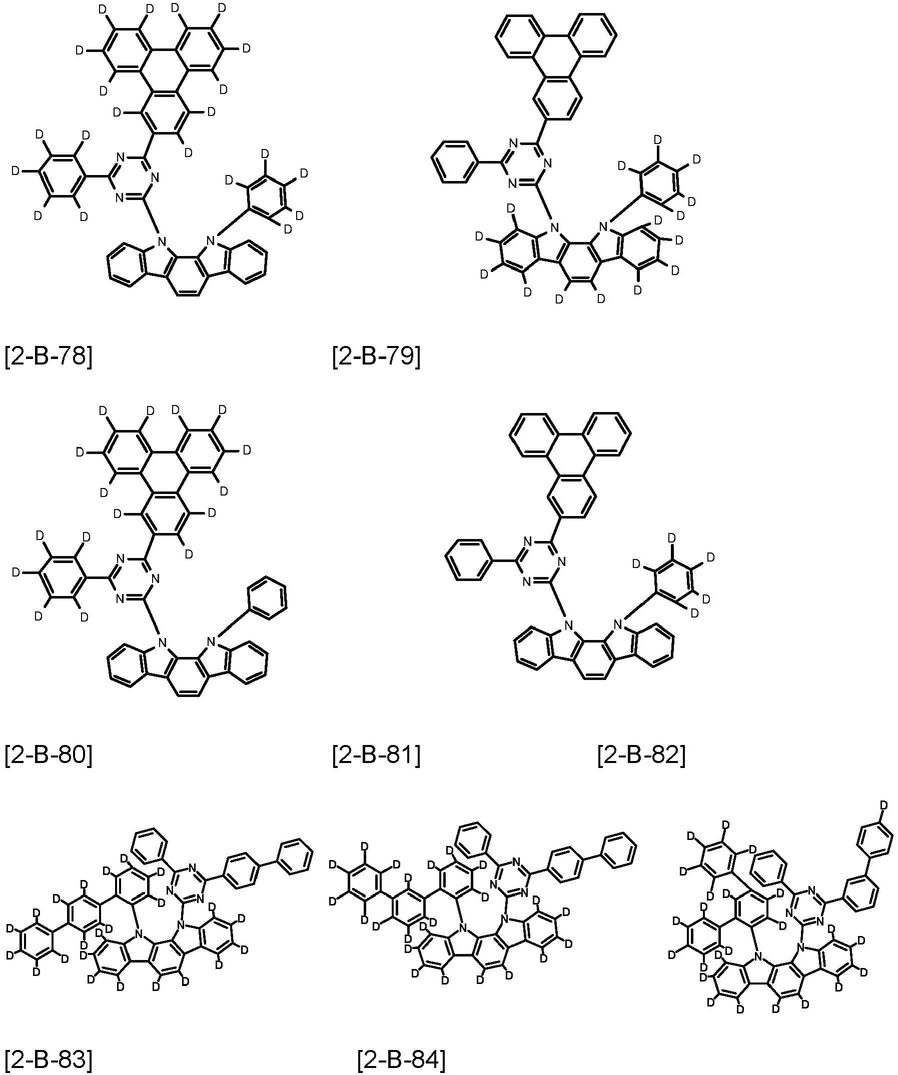

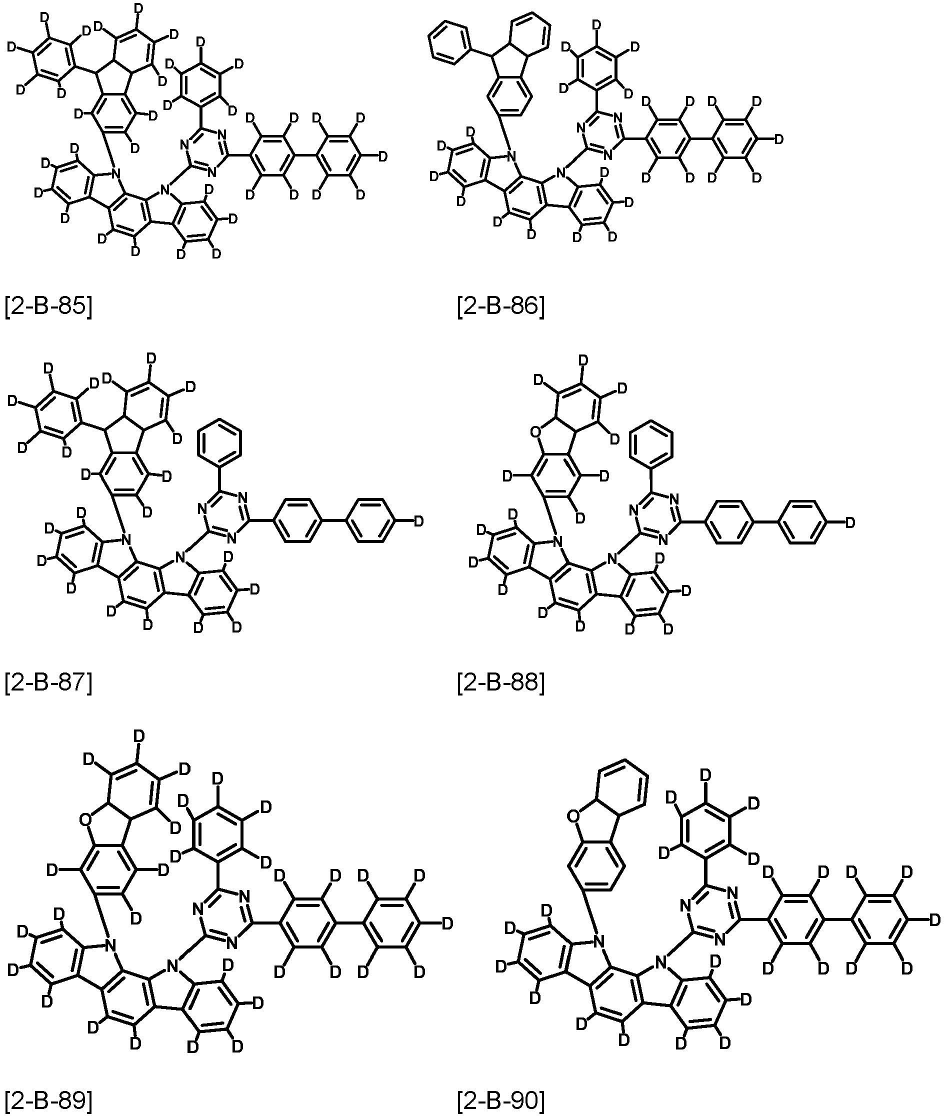

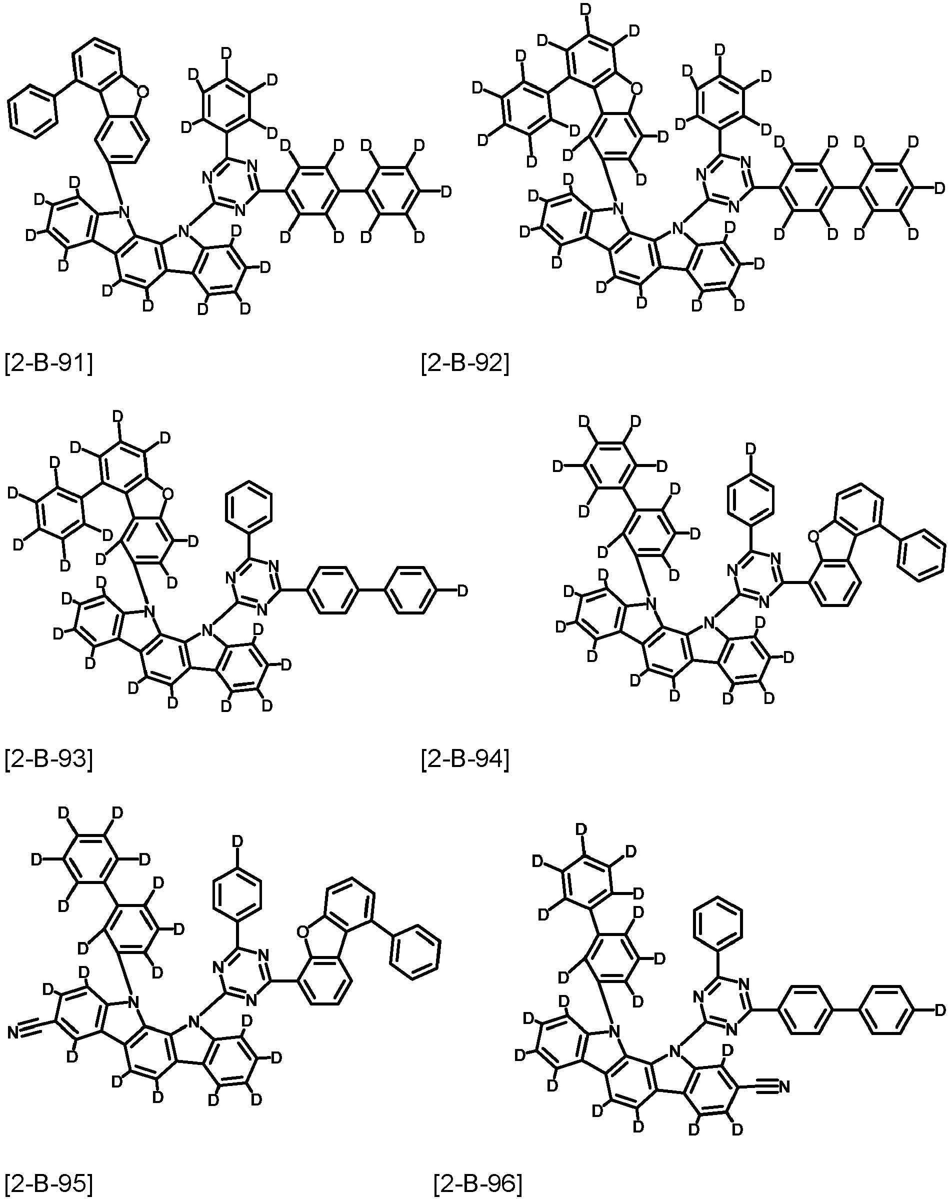

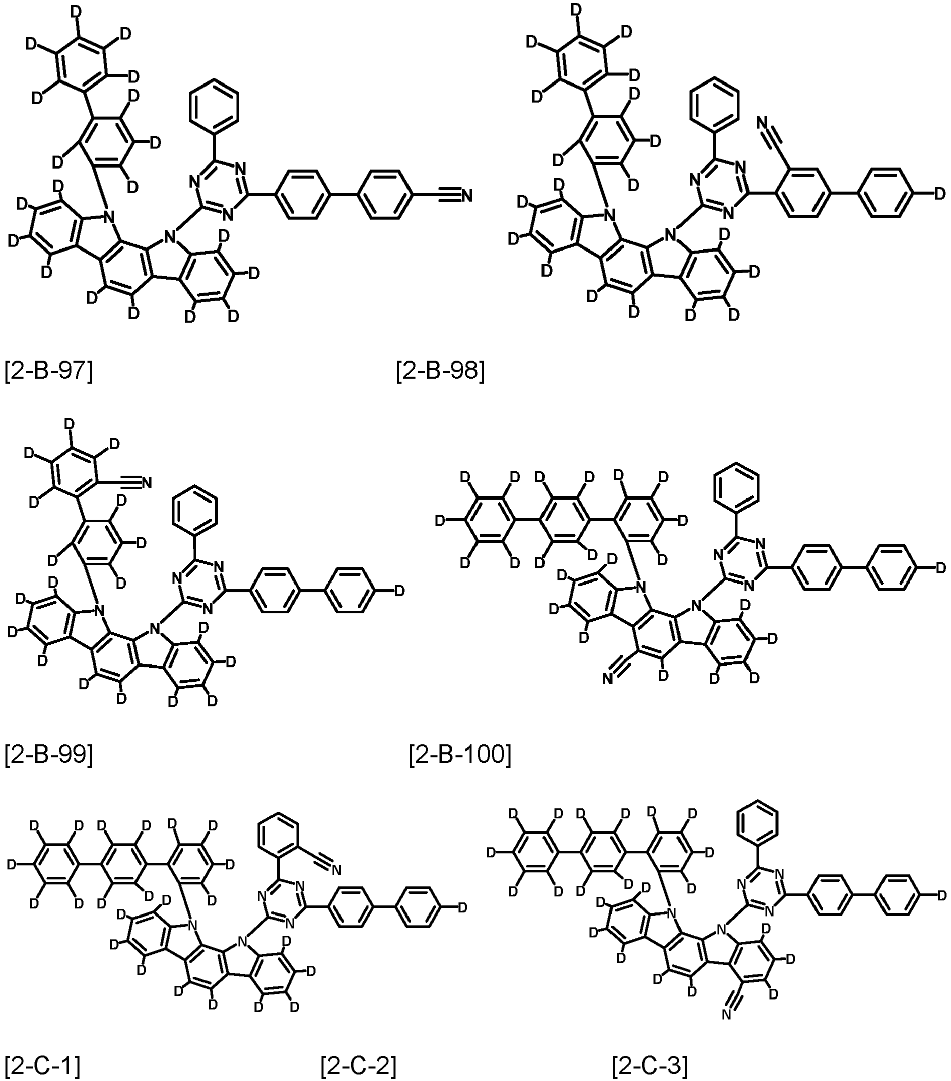

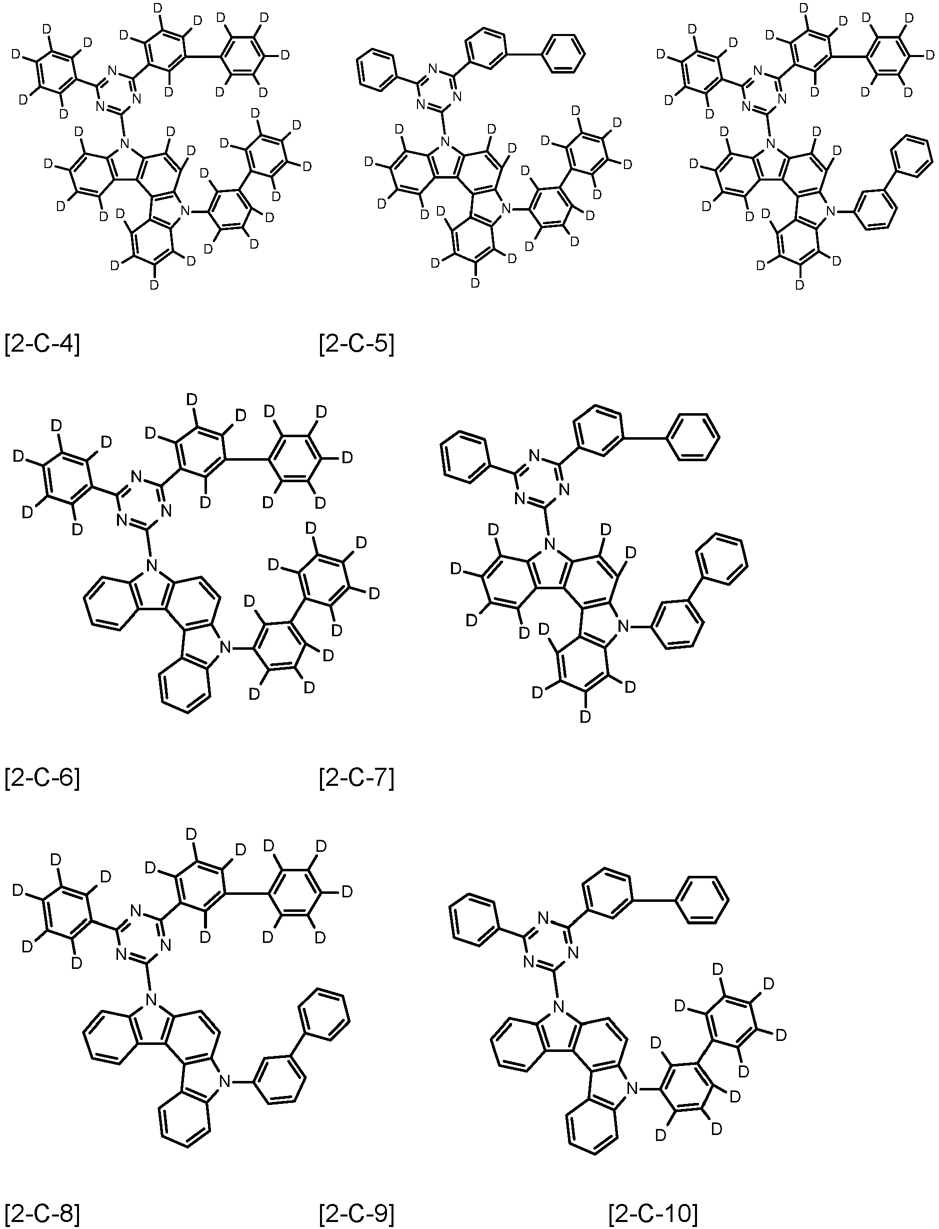

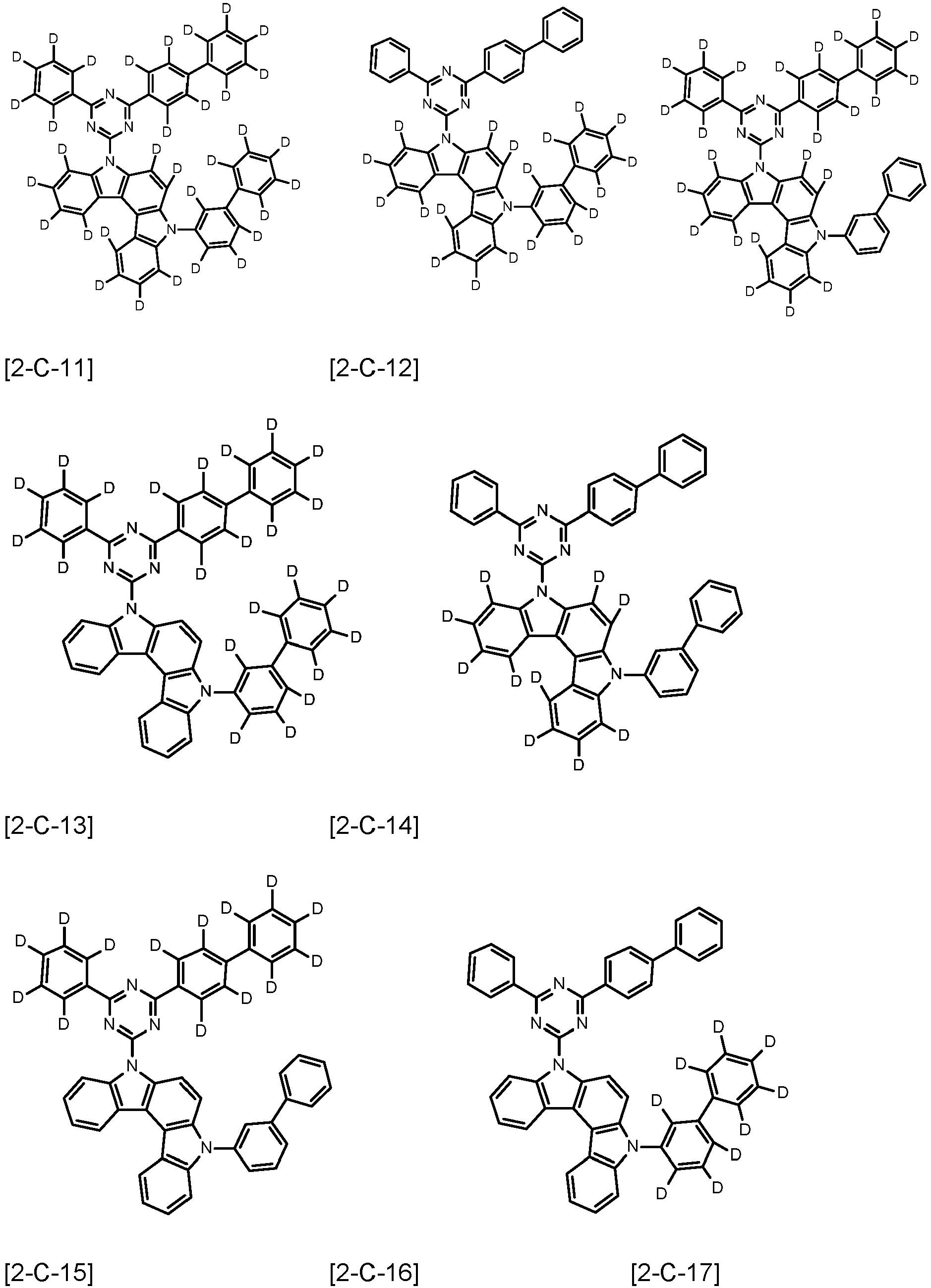

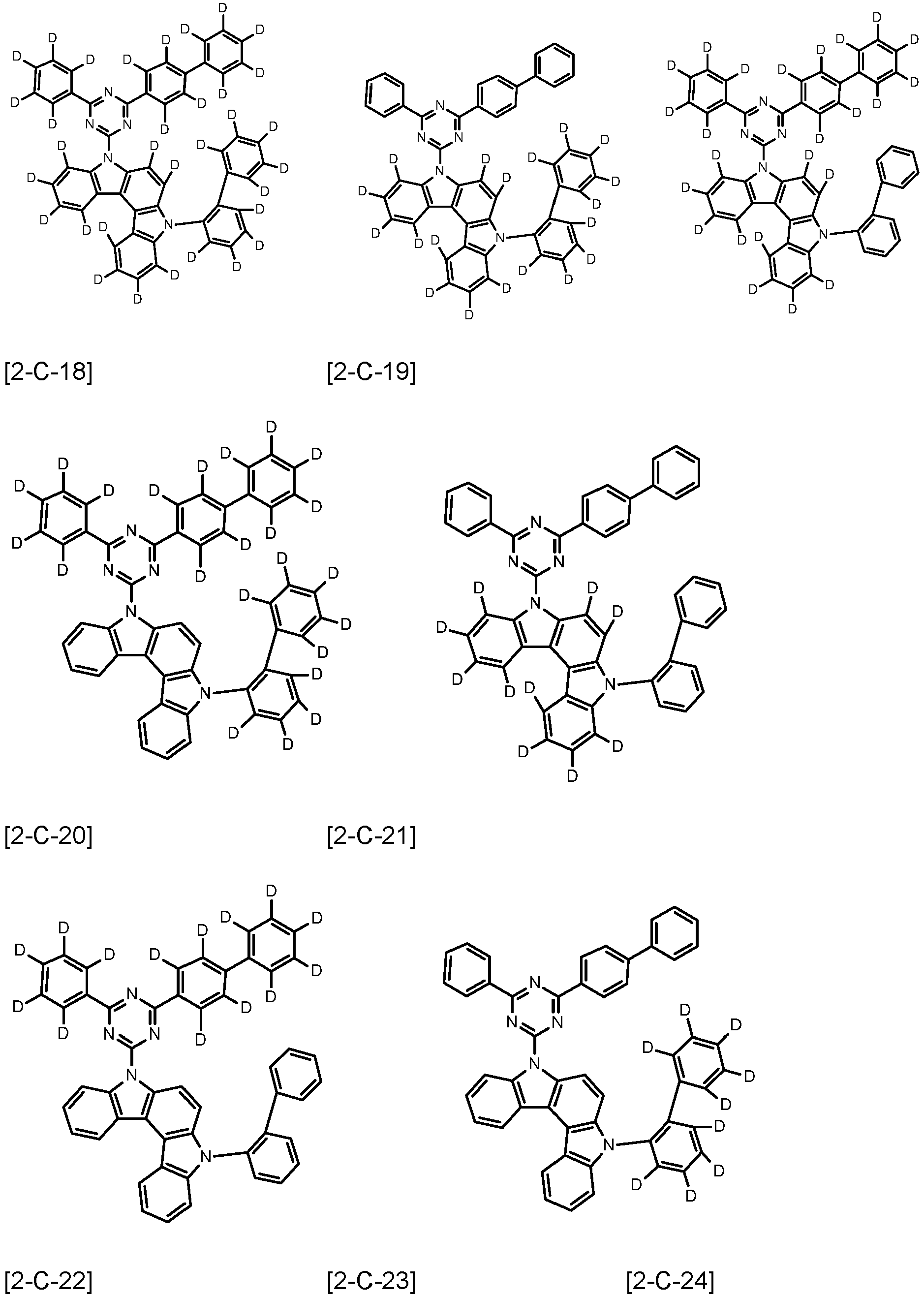

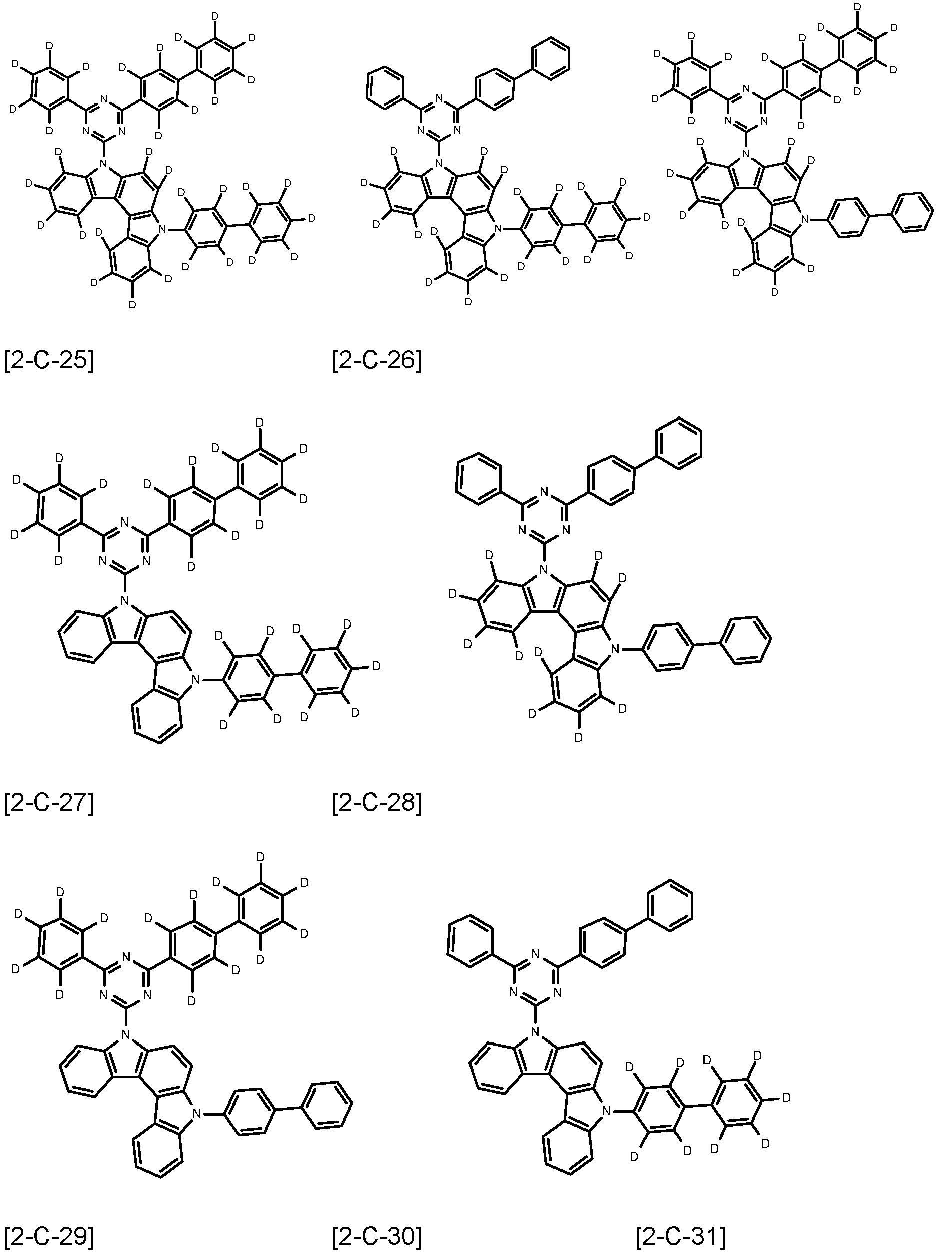

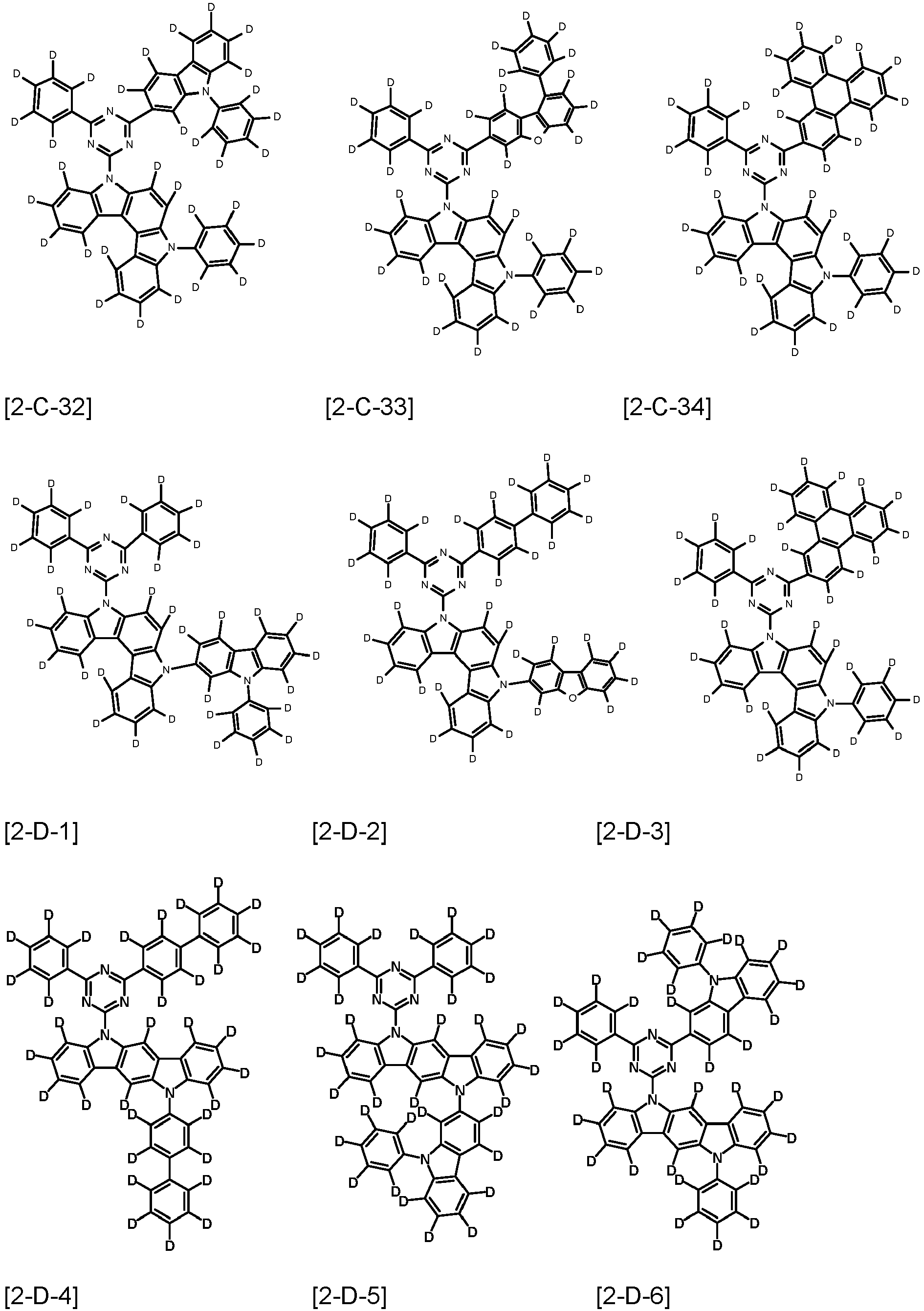

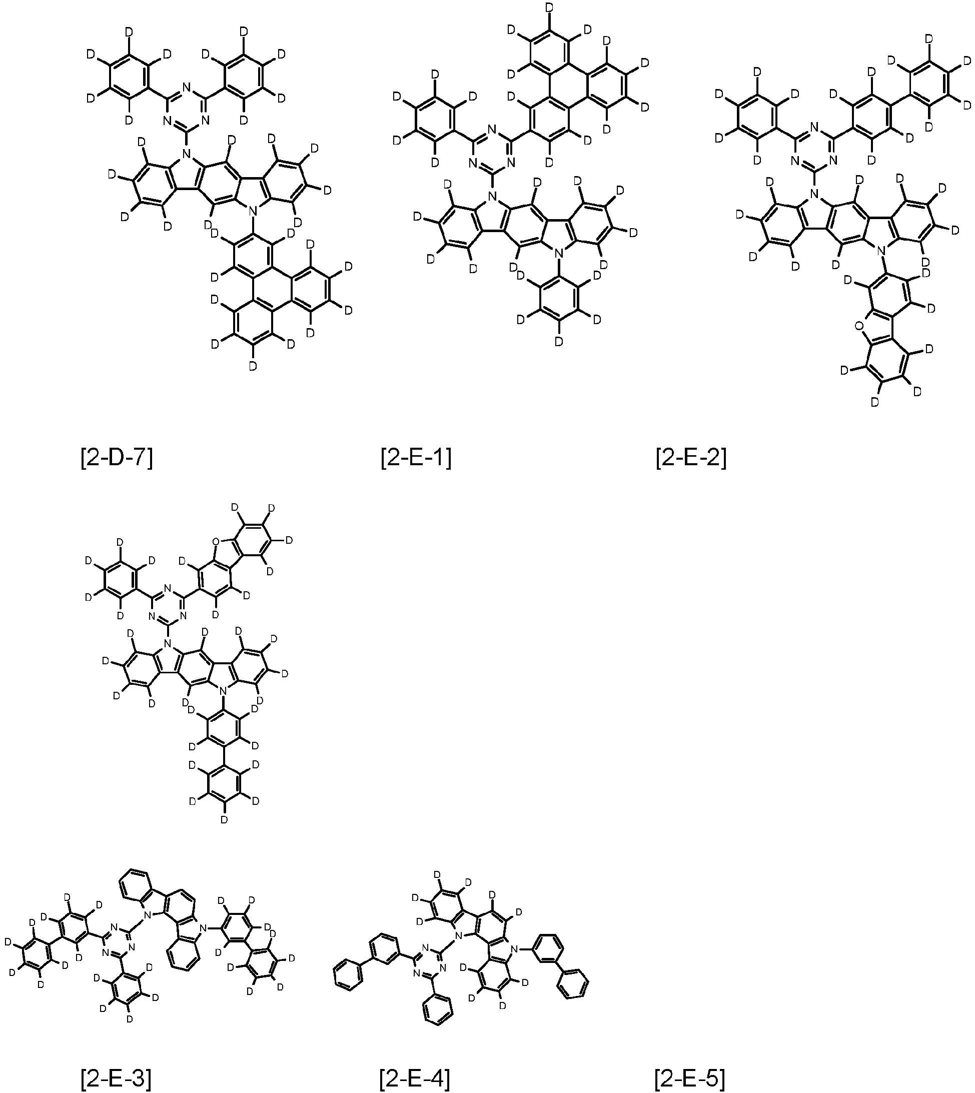

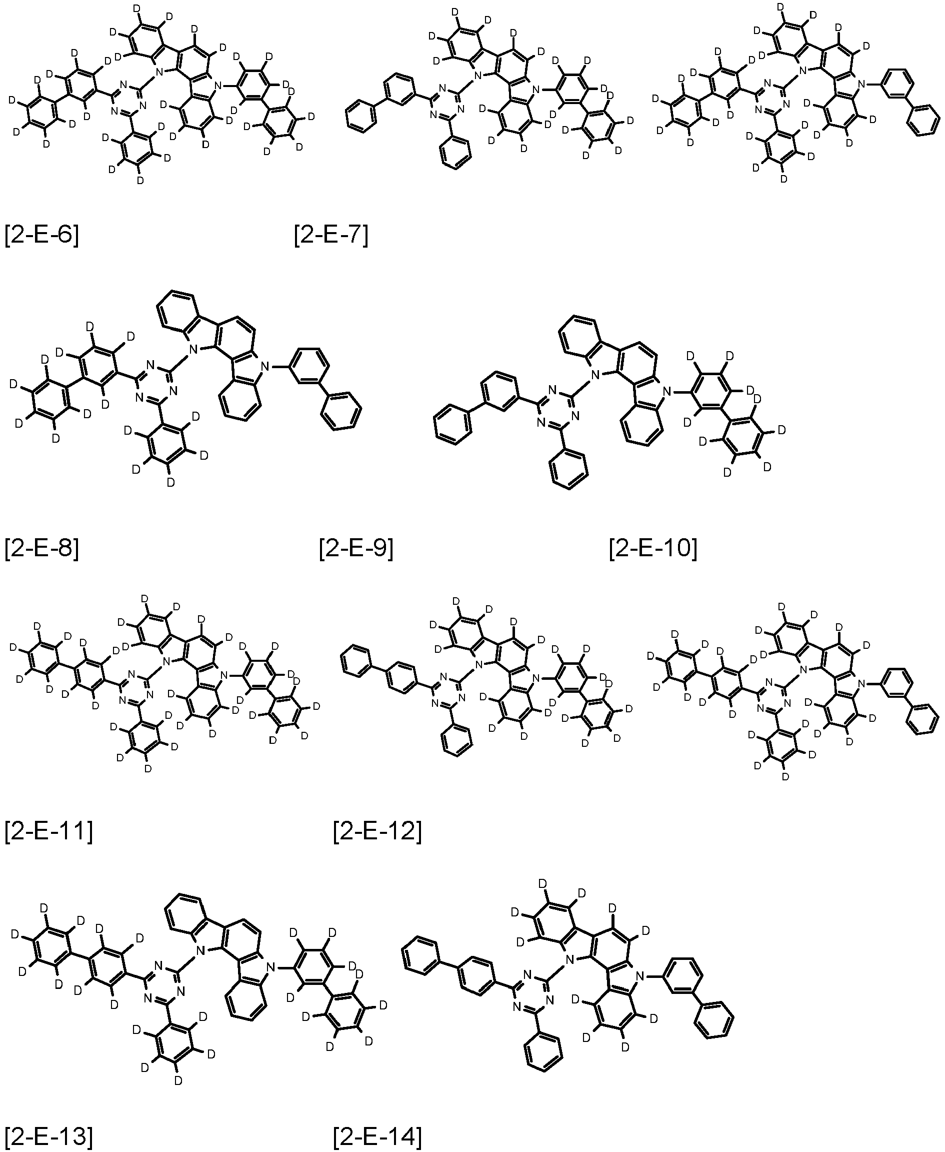

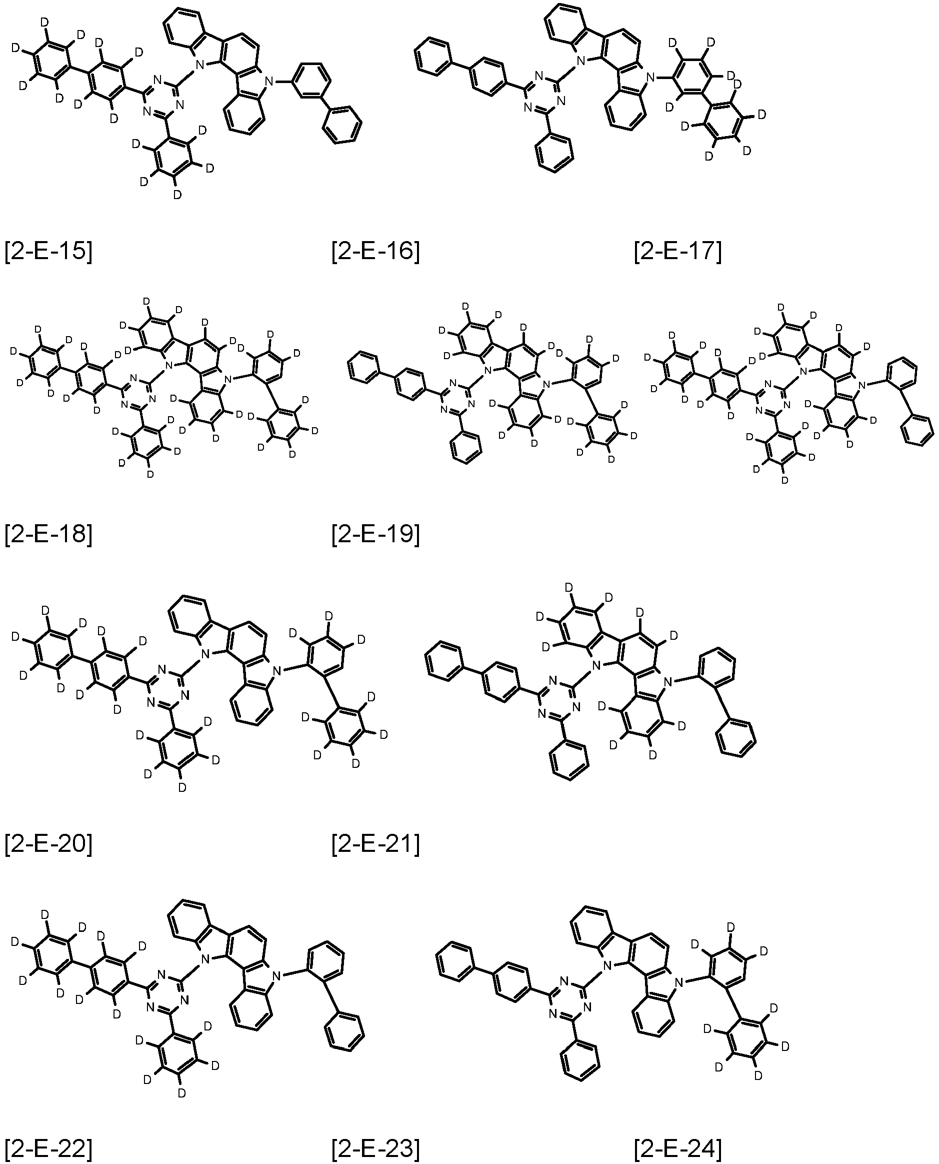

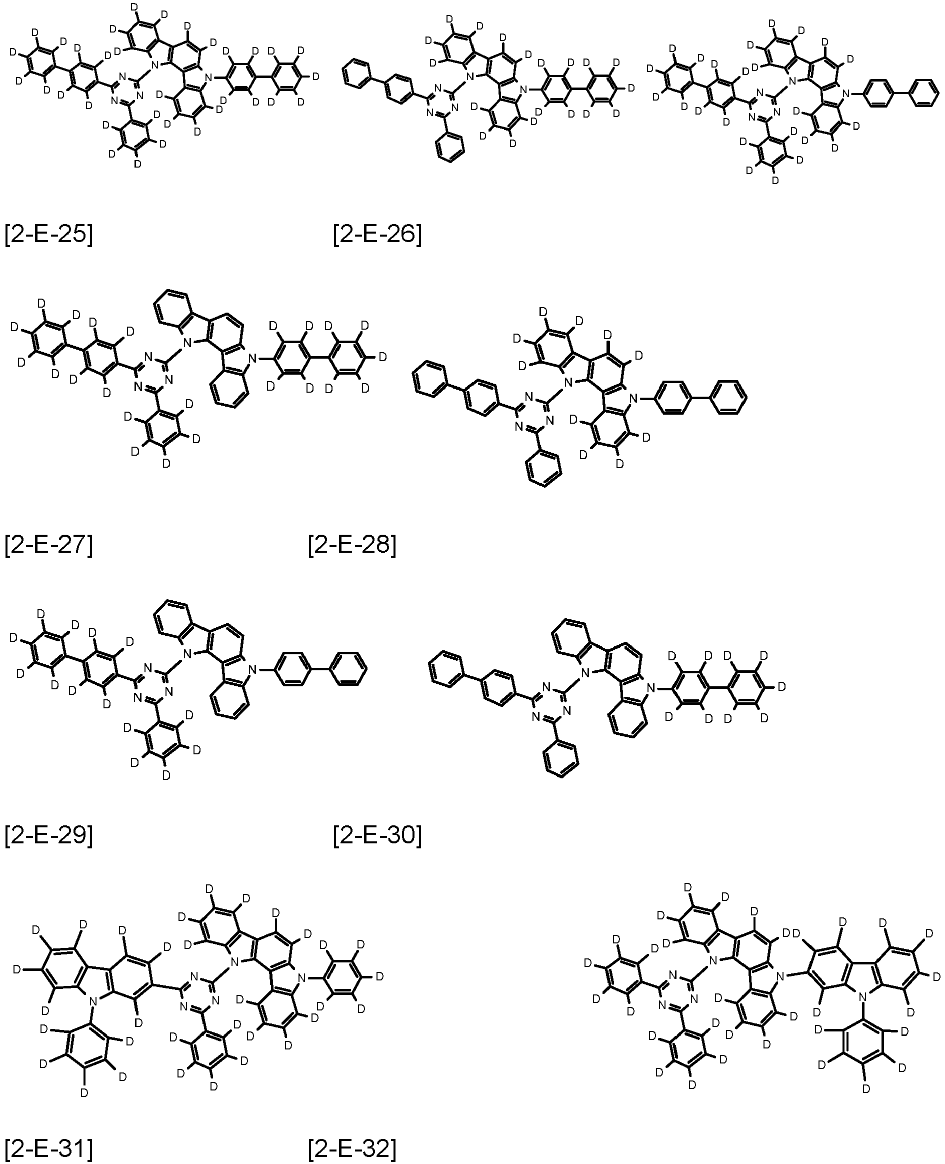

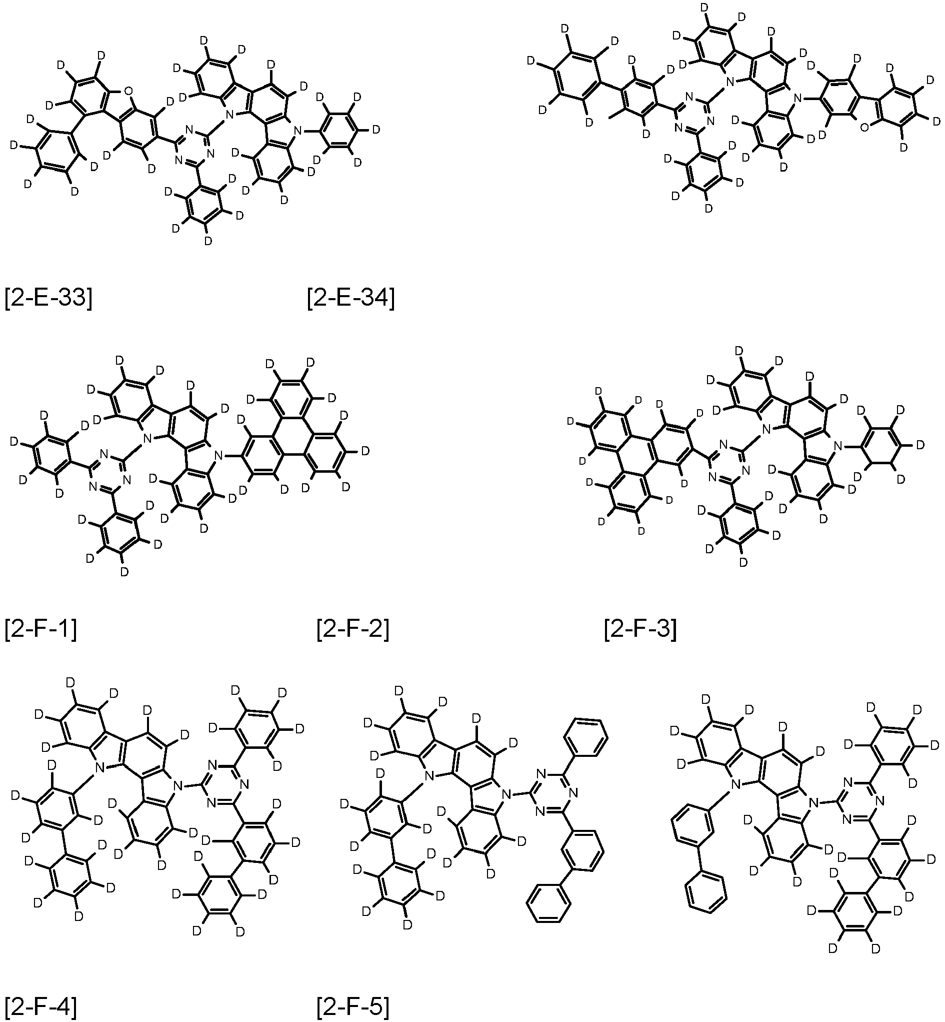

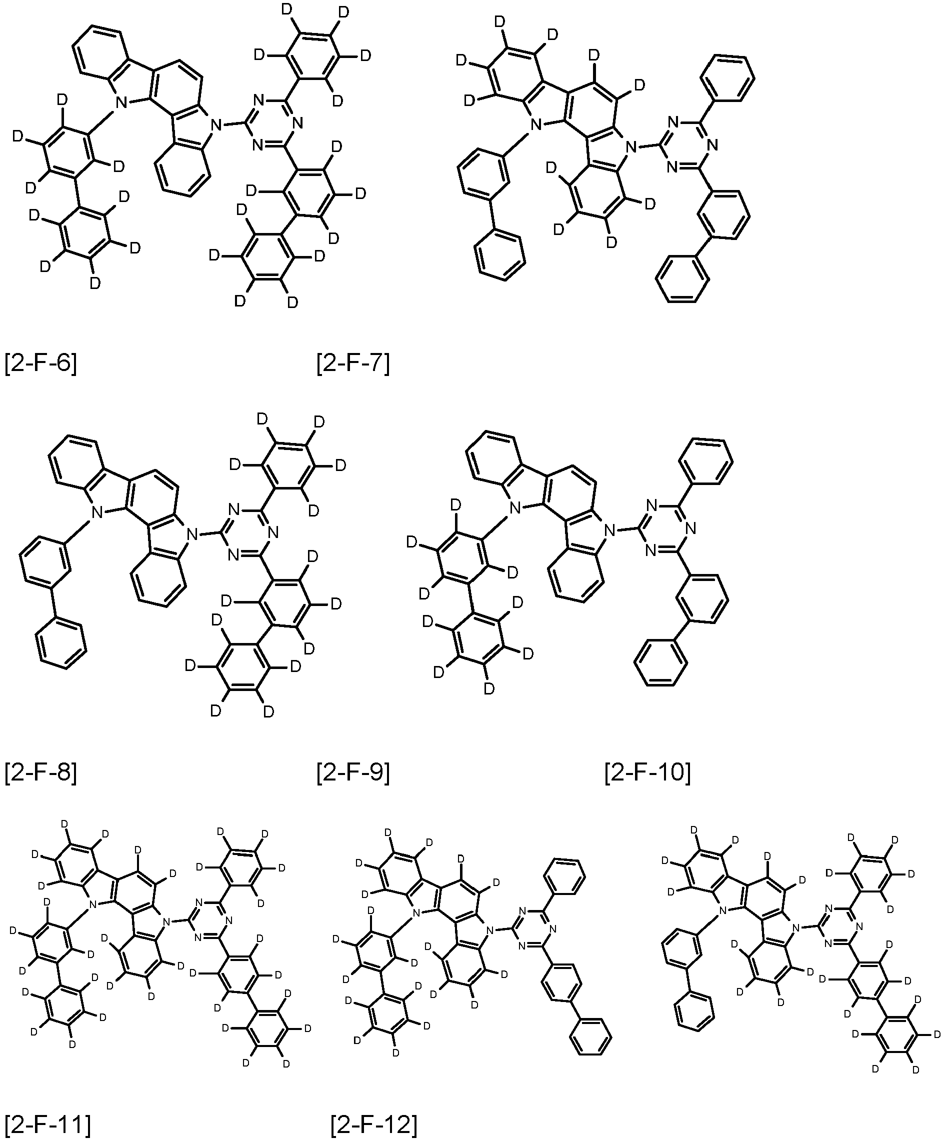

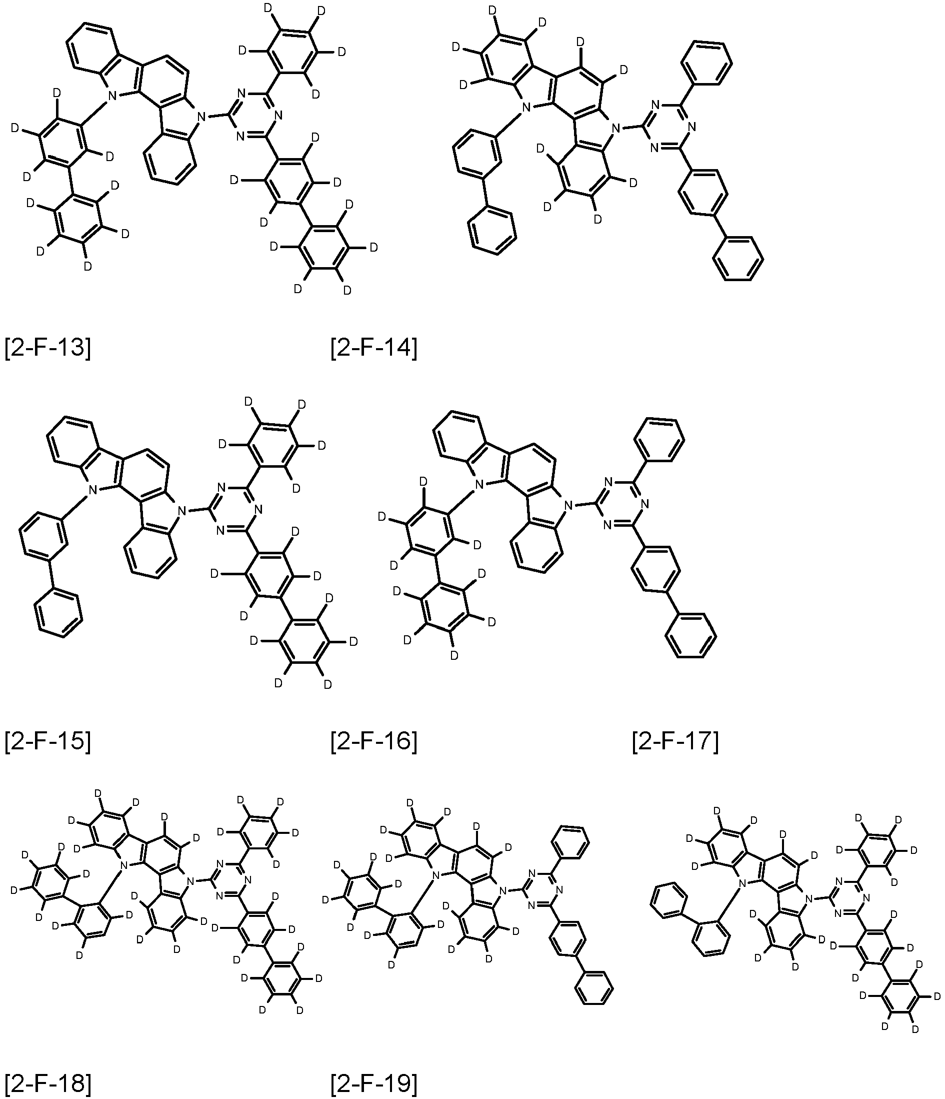

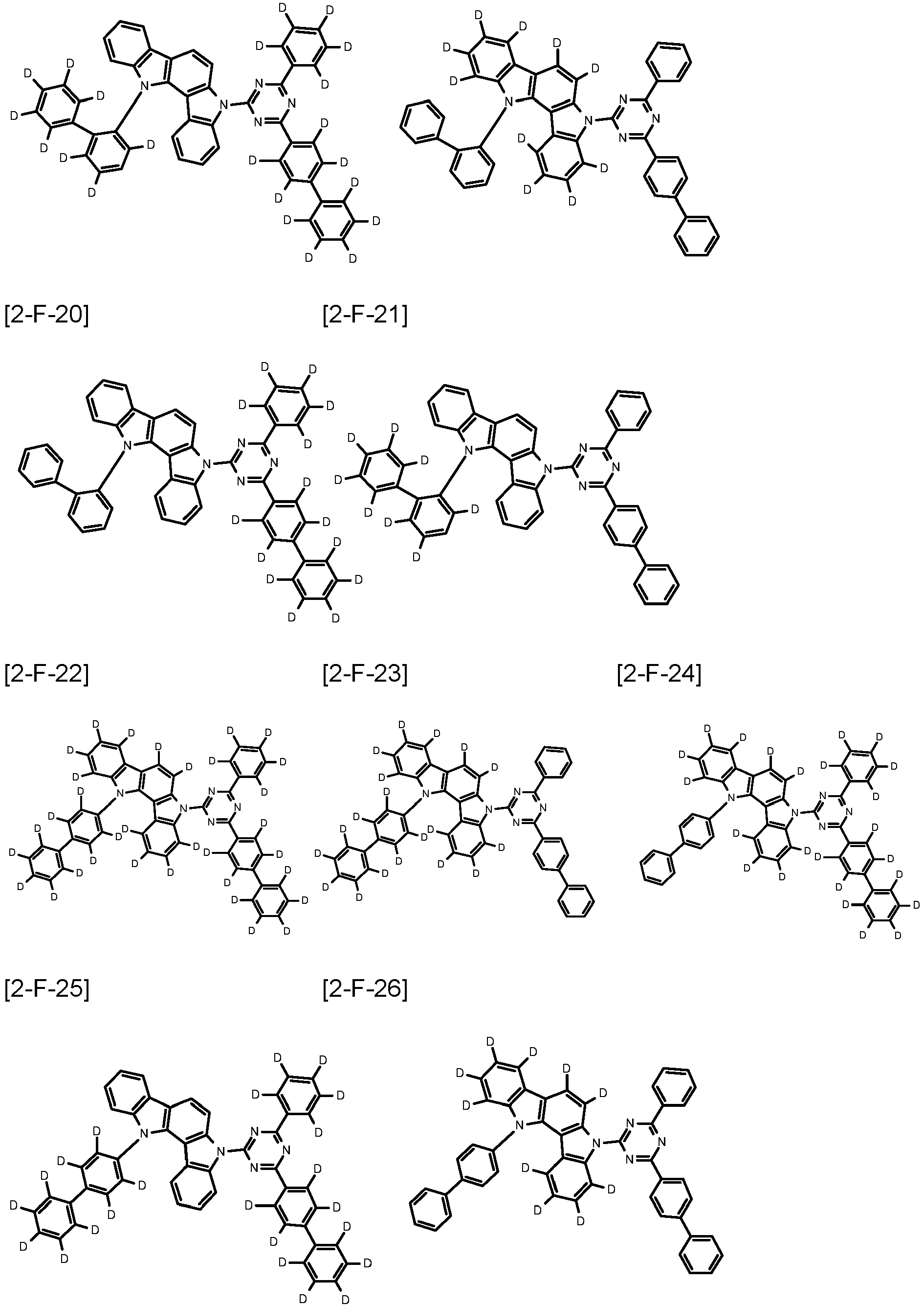

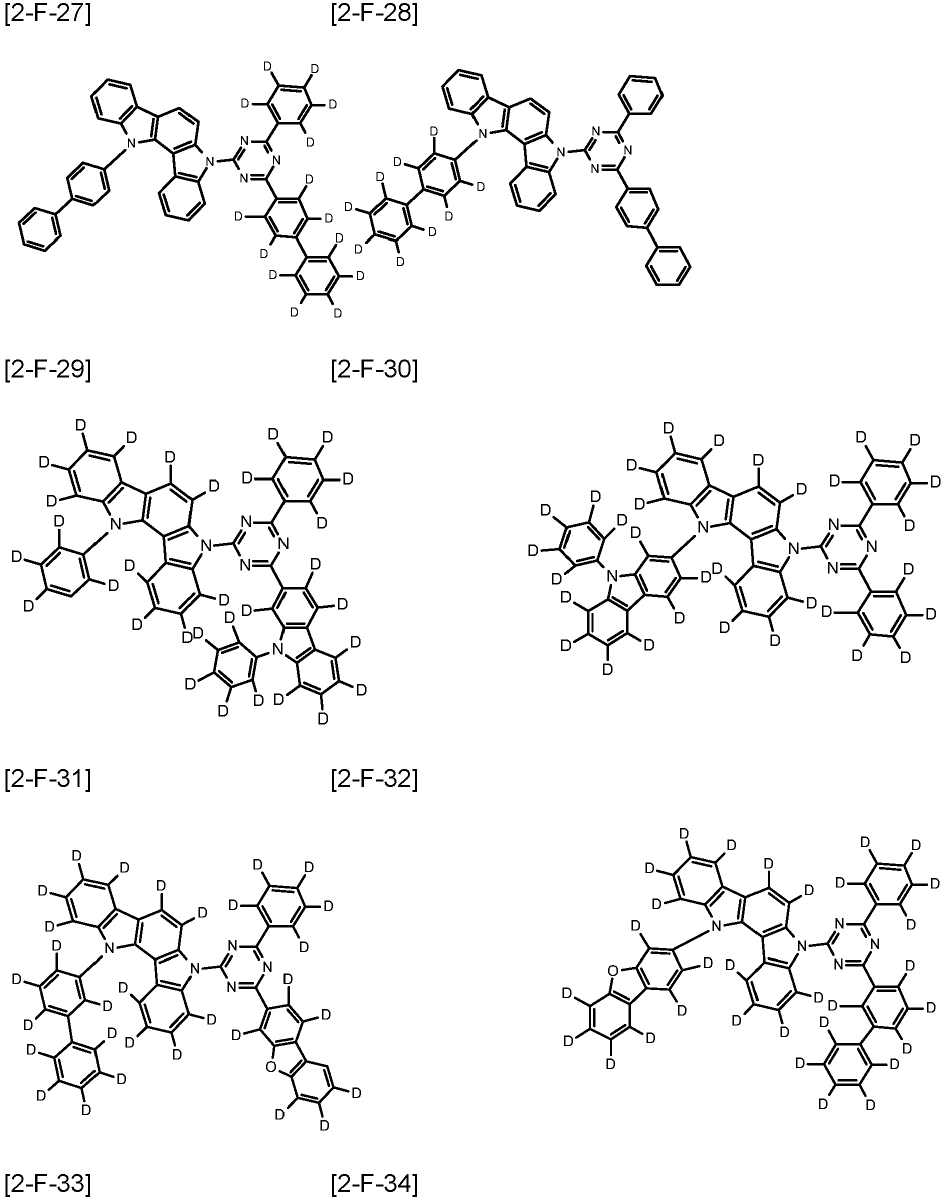

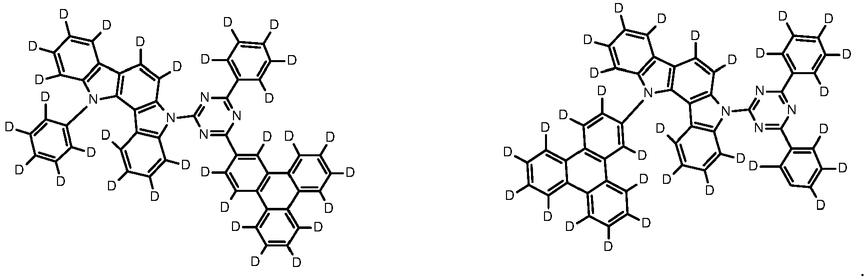

12. The composition for the organic optoelectronic device of claim 1, wherein
the first compound is represented by Chemical Formula 1-8a, and
the second compound is represented by Chemical Formula 2B-a-1: 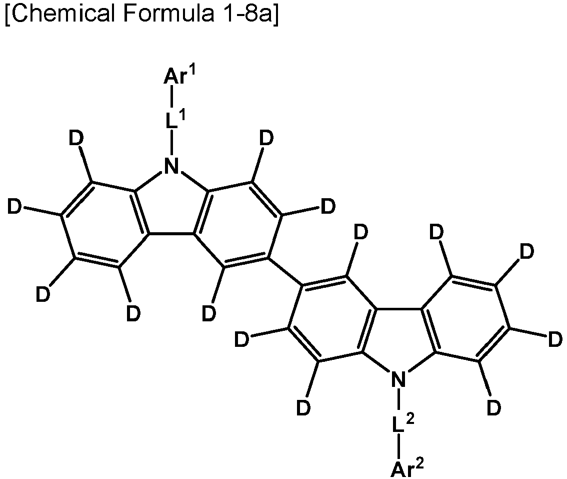
wherein, in Chemical Formula 1-8a,
L¹ and L² are a single bond or a substituted or unsubstituted phenylene group, and
Ar¹ and Ar² are each a phenyl group substituted with deuterium, a biphenyl group substituted with deuterium, a terphenyl group substituted with deuterium, or a triphenylene group substituted with deuterium; 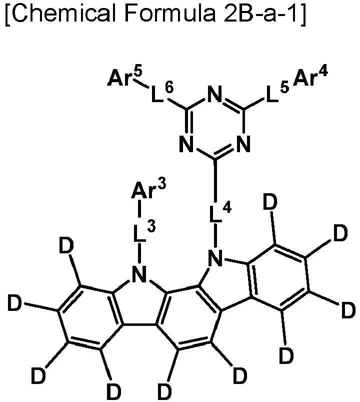
wherein, in Chemical Formula 2B-a-1,
Ar³ to Ar⁵ are each independently a substituted or unsubstituted phenyl group or a substituted or unsubstituted biphenyl group, and
L³ to L⁶ are each independently a single bond or a substituted or unsubstituted phenylene group.

13. An organic photoelectronic device, comprising
an anode and a cathode facing each other, and
at least one organic layer between the anode and the cathode,
wherein the organic layer includes the composition for the organic optoelectronic device of any one of claim 1 to claim 12.

14. The organic photoelectronic device of claim 13, wherein
the organic layer includes a light emitting layer, and
the light emitting layer includes the composition for an organic optoelectronic device.

15. A display device comprising the organic photoelectronic device of claim 13.
